(19) Europäisches Patentamt / European Patent Office / Office européen des brevets

(11) **EP 4 644 382 A1**

(12) **EUROPEAN PATENT APPLICATION**
published in accordance with Art. 153(4) EPC

(43) Date of publication:
**05.11.2025 Bulletin 2025/45**

(21) Application number: **23910563.8**

(22) Date of filing: **26.12.2023**

(51) International Patent Classification (IPC):
***C07D 401/14*** *(2006.01)* ***C07D 409/14*** *(2006.01)*
***C07D 417/14*** *(2006.01)* ***C07D 413/14*** *(2006.01)*
***A61K 31/40*** *(2006.01)* ***A61K 31/4545*** *(2006.01)*
***A61K 31/5377*** *(2006.01)* ***A61K 31/454*** *(2006.01)*
***A61P 29/00*** *(2006.01)* ***A61P 35/00*** *(2006.01)*
***A61P 25/00*** *(2006.01)*

(52) Cooperative Patent Classification (CPC):
**A61K 31/40; A61K 31/454; A61K 31/4545; A61K 31/5377; A61P 25/00; A61P 29/00; A61P 35/00; C07D 401/14; C07D 409/14; C07D 413/14; C07D 417/14**

(86) International application number:
**PCT/CN2023/141787**

(87) International publication number:
**WO 2024/140638 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2022 CN 202211684679**

(71) Applicant: **Gluetacs Therapeutics (Shanghai) Co., Ltd.**
**Shanghai 201306 (CN)**

(72) Inventors:
- **YANG, Xiaobao**
  **Shanghai 201306 (CN)**
- **SUN, Renhong**
  **Shanghai 201306 (CN)**
- **ZHOU, Yuedong**
  **Shanghai 201306 (CN)**
- **ZHAO, Baoyin**
  **Shanghai 201306 (CN)**
- **REN, Chaowei**
  **Shanghai 201306 (CN)**

(74) Representative: **Dragsted Partners A/S**
**Rådhuspladsen 16**
**1550 Copenhagen V (DK)**

# (54) COMPOUND BASED ON SULFUR/OXYGEN SUBSTITUTED GLUTARIMIDE-BASED ISOINDOLINONE SKELETON AND USE THEREOF

(57) The present disclosure relates to the technical field of molecular glue degraders, and particularly to a sulfur/oxygen substituted glutarimide-isoindolinone skeleton-based compound of Formula I, and uses thereof. The present disclosure further pertains to a compound of Formula A and its uses.

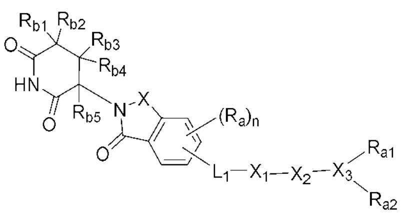

Formula I

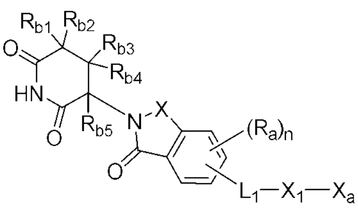

Formula A

EP 4 644 382 A1

## Description

## Technical Field

The present disclosure relates to the technical field of molecular glue degraders, and particularly to a sulfur/-oxygen-substituted glutarimidyl-isoindolinone scaffold-based compound and its applications.

## Background

Although Thalidomide, lenalidomide and other phthalimide immunomodulatory drugs (IMiDs) have shown significant efficacy in the treatment of multiple myeloma and autoimmune diseases, it was not until 2010 that the E3 ubiquitin ligase cereblon (CRBN) was identified as a direct target of IMiDs. Subsequent research confirmed that these drugs function as molecular glue, inducing the interaction between transcription factors IKZF1/3 and CRBN protein, ultimately leading to their ubiquitination and degradation. Compared with traditional small molecule inhibitors, molecular glue protein degraders have natural mechanism advantages: the former works by occupying the functional domain of the target protein for a long time to inhibit its function, while the protein degraders directly degrade and eliminate the entire target protein, often having a much greater efficacy than traditional small molecule inhibitors. The protein degraders may target "non-drugable" targets and have low requirements for binding affinity, catalytic capacity and low concentration, which may overcome the clinical drug resistance problem of traditional small molecules. Moreover, molecular glue degraders usually have small molecular weights and desirable druggability, so the research and development of such drugs have received great attention. Based on the CRBN E3 ubiquitin ligase and molecular glue degradation mechanism, a series of compounds have been developed, such as pomalidomide, which has been launched and CC-122 of Bristol-Myers Squibb (BMS), which is undergoing clinical trials, CC-220, CC-90009, CC-99282 and CC-92480, DKY709 from Novartis and CFT7455 from C4 Therapeutics. The degradation substrates of these molecular glues have also expanded from the initially discovered transcription factors IKZF1/3 to include casein kinase 1$\alpha$ (CK1$\alpha$), zinc finger protein 91 (ZFP91) and translation factor GSPT1. The degradation of these protein substrates enables the molecular glues to exert therapeutic efficacy against diseases or disorders associated with said substrates, including but not limited to immunomodulation, anti-inflammatory, and anti-tumor effects. Currently, the number of identified molecular glue candidate compounds is quite limited. Moreover, there is a wide variety of pathogenic proteins that may theoretically serve as degradation substrates for the development of molecular glues. Therefore, it is necessary to design and develop more molecular glues through rationalization and diversification approaches to degrade and eliminate more pathogenic proteins, and to apply them to the treatment of diseases associated with these pathogenic proteins.

The present discolsure develops more novel, highly efficient, low-toxic, stable, and cost-effective molecular glue degraders based on the fused phenyl-glutarimide skeleton through rational design to meet the demands of clinical applications.

## Summary

The objects of the present disclosure are to provide a sulfur/oxygen-substituted glutarimidyl-isoindolinone scaffold-based compound and its applications, aiming to address the limitations in the prior art.

To achieve the above objectives and other related goals, in one aspect, the present disclosure provides a compound of Formula I:

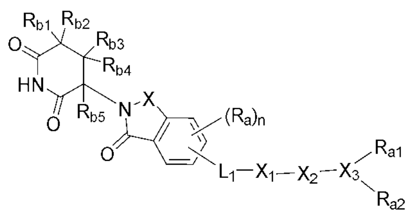

Formula I

or salts, enantiomers, stereoisomers, polymorphs or solvates thereof, wherein

X is selected from the group consisting of CO or $CH_2$;
$L_1$ is selected from the group consisting of S or O;
$X_1$ is selected from the group consisting of unsubstituted or substituted linear or branched alkylene, or $X_1$ is absent;
$X_2$ represents the structure of Formula II:

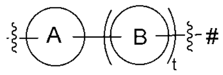

II

wherein ring A and ring B are each independently selected from the group consisting of unsubstituted or substituted arylene, unsubstituted or substituted heteroarylene, unsubstituted or substituted heterocyclylene, or unsubstituted or substituted cycloalkylene;
t represents an integer of 0, 1 or 2; and
symbol # indicates the point of attachment to $X_3$;

$X_3$ is selected from the group consisting of $CR_1$, N, or CO, wherein $R_1$ is selected from the group consisting of hydrogen, deuterium, halogen, nitro, cyano, hydroxy, mercapto, trifluoromethoxy, unsubstituted or substituted amino, unsubstituted or substituted silyl, unsubstituted or substituted boryl, unsubstituted or substituted linear or branched alkyl, or unsubstituted or substituted cycloalkyl;
$R_{a1}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl;
$R_{a2}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl, or $R_{a2}$ is absent; and when $X_3$ represents CO, $R_{a2}$ is absent and $R_{a1}$ is neither hydrogen nor deuterium, and when $R_{a2}$ represents hydrogen or deuterium, $X_3$ represents $CR_1$ or N, and $R_{a1}$ is neither hydrogen nor deuterium;
$R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl;
$R_{b1}$, $R_{b2}$, $R_{b3}$, $R_{b4}$, and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl; and
n is an integer of 0, 1, 2 or 3, and when n is an integer of 2 or 3, each $R_a$ is independently identical to or different from each other.

In another aspect, the present disclosure provides a method for preparing the compound or salts, enantiomers, stereoisomers, polymorphs or solvates thereof of the present disclosure, comprising:

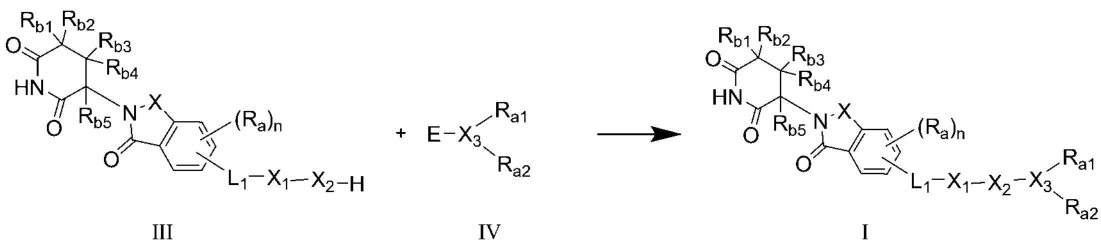

III IV I

wherein (1) E-$X_3$ represents CO, $R_{a2}$ represents hydrogen; or (2) E-$X_3$ represents halogen-CH, MsO-CH, TsO-CH, or NsO-CH; or (3) E-$X_3$ represents CO, $R_{a2}$ represents hydroxy.

In a further aspect, the present disclosure provides the compound or salts, enantiomers, stereoisomers, polymorphs or solvates thereof of the present disclosure, for use as a medicament.

In a further aspect, the present disclosure provides a pharmaceutical composition comprising the compound or salts, enantiomers, stereoisomers, polymorphs or solvates thereof of the present disclosure, and at least one pharmaceutically acceptable salt, and at least one pharmaceutically acceptable carrier or excipient.

In a further aspect, the present disclosure provides use of the compound or salts, enantiomers, stereoisomers, polymorphs or solvates thereof of the present disclosure, or the pharmaceutical composition of the present disclosure for the manufacture of a medicament for the treatment of a disease or disorder associated with cereblon protein.

In a further aspect, the present disclosure provides a method for treating or preventing a disease or disorder associated with cereblon protein, comprising administering to a subject a therapeutically effective amount of the compounds or salts, enantiomers, stereoisomers, polymorphs or solvates thereof of the present disclosure, or the pharmaceutical composition of the present disclosure.

In another aspect, the present disclosure provides a compound of Formula A:

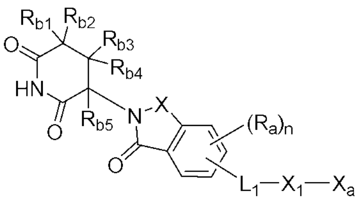

Formula A

or salts, enantiomers, stereoisomers, polymorphs or solvates thereof, wherein

X is selected from the group consisting of CO or $CH_2$;
$L_1$ is selected from the group consisting of S or O;
$X_1$ is selected from the group consisting of unsubstituted or substituted linear or branched alkylene, or $X_1$ is absent;
$X_a$ represents the structure of Formula A1:

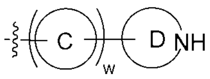

Formula A1

wherein ring C is selected from the group consisting of unsubstituted or substituted arylene, unsubstituted or substituted heteroarylene, unsubstituted or substituted heterocyclylene, or unsubstituted or substituted cycloalkylene, and w represents an integer of 0 or 1; and
ring D represents unsubstituted or substituted nitrogen-containing heterocyclyl;

$R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl;
$R_{b1}$, $R_{b2}$, $R_{b3}$, $R_{b4}$ and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl; and
n is an integer of 0, 1, 2 or 3, and when n is an integer of 2 or 3, each $R_a$ is independently identical to or different from each other.

In a further aspect, the present disclosure provides the compound of Formula A or salts, enantiomers, stereoisomers, polymorphs or solvates thereof of the present disclosure, for use as a medicament.

In a further aspect, the present disclosure provides a pharmaceutical composition comprising the compound of Formula A or salts, enantiomers, stereoisomers, polymorphs or solvates thereof of the present disclosure, and at least one pharmaceutically acceptable salt, and at least one pharmaceutically acceptable carrier or excipient.

In a further aspect, the present disclosure provides use of the compound of Formula A or salts, enantiomers, stereoisomers, polymorphs or solvates thereof of the present disclosure, or the pharmaceutical composition of the present disclosure for the manufacture of a medicament for the treatment of a disease or disorder associated with cereblon protein.

In a further aspect, the present disclosure provides a method for treating or preventing a disease or disorder associated with cereblon protein, comprising administering to a subject a therapeutically effective amount of the compound of Formula A or salts, enantiomers, stereoisomers, polymorphs or solvates thereof of the present disclosure, or the pharmaceutical composition of the present disclosure.

Compared with the prior art, the advantageous effects of the present invention are as follows:
The compounds provided by the present invention feature a sulfur/oxygen-substituted glutarimidyl-isoindolinone scaffold. Based on this scaffold, rationally designed novel compounds have been developed as molecular glues, exhibiting high efficacy, low toxicity, excellent stability, and cost-effectiveness. These compounds are suitable for preparing immunomodulatory drugs, anti-inflammatory agents, and antitumor medications to meet clinical needs.

## Description of Drawings

Figure 1 shows the Western blot analysis results of the compounds of the present disclosure. The compounds of the present disclosure effectively degrade substrate proteins (such as Wee1, IKZF1, IKZF2, IKZF3, GSPT1, and CK1α) in human primary peripheral blood mononuclear cells (hPBMCs).

## Detailed Description of the Invention

Through extensive research, the present inventors have developed novel compounds based on a sulfur/oxygen-substituted glutarimidyl-isoindolinone scaffold. These compounds serve as molecular glues with the following advantages: structural novelty, high efficacy and low toxicity, excellent stability and cost-effectiveness. They are suitable for manufacturing medicament for treating CRBN (cereblon protein)-associated diseases or disorders, leading to the completion of the present invention.

## Definitions

Unless otherwise specified, the following words, phrases and symbols used herein generally have the meanings as described below.

In general, the nomenclature used herein (e.g., the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" may mean at least a second or more.

It should be understood that whenever the term "comprise" or "include" is used herein to describe various aspects, other similar aspects described by "consisting of" and/or "consisting essentially of" are also provided.

Herein, a bond interrupted by a wavy line shows the point of attachment of the depicted group to the rest of the molecule. For example, the group $L_3$ depicted below

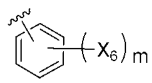

shows the point of attachment of the group $L_3$ to the group $L_2$ of the compound of Formula I.

Salts or pharmaceutically acceptable salts, enantiomers, stereoisomers, solvates, polymorphs of the compounds of Formula I of the present disclosure are also encompassed within the scope of the present disclosure.

As used herein, the term "enantiomer" shall be construed to mean, when a specific compound (or generic structure) is designated as the (R)- or (S)-enantiomer/having an absolute (R)- or (S)-configuration, the corresponding compound (or generic structure) in an enriched, particularly substantially pure enantiomeric form.

As used herein, the term "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "polymorph" refers to solid crystalline forms of the compounds of the present disclosure or complexes thereof. Different polymorphs of the same compound exhibit distinct physical, chemical, and/or spectroscopic properties. Physical property variations include but are not limited to stability (e.g., thermal or photostability), compressibility and density (critical for formulation and manufacturing), and dissolution rate (may affect bioavailability). Stability differences may cause variations in chemical reactivity (e.g., differential oxidation, as evidenced by faster discoloration observed in one polymorph compared to another), mechanical properties (e.g., conversion of tablets containing a kinetically preferred polymorph into a more thermodynamically stable crystalline form during storage), or both (e.g., tablets containing one polymorph being more susceptible to degradation under high humidity conditions). The additional physical properties of polymorphs may affect their processing behavior. For example, one polymorph may be more prone to solvate formation due to e.g., its distinct crystal habit or particle size distribution, or may present greater filtration or washing challenges compared to another polymorph.

As used herein, the term "salts or pharmaceutically acceptable salts" of the present disclosure refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-ketoglutarates, hippurates, D-glucuronates, D-gluconates, α-D-glucoheptates, glycolates, mucates, L-ascorbates,

orotates, picrates, glycinates, alaninates, argininates, cinnamates, laurates, pamoates, sebacates, benzenesulfonates, methanesulfonates, ethanesulfonates, ethanedisulfonates, formates, acetates, 2,2-dichloroacetates, trimethylacetates, propionates, valerates, palmitates, triphenylacetates, 2-ethyl-butanedioates, iodates, nicotiniates, L-pyroglutamates, L-prolinates, ferulates, 2-hydroxyethanesulfonates, nitrates, gentisates, cholates, salicylate, terephthalates, glutarates, adipates, stearates, oleoates, undecylenates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, p-chlorobenzenesulfonates, 1,5-naphthalenedisulfonates, 3-hydroxy-2-naphthoates, 1-hydroxy-2-naphthoates, 2-naphthalenesulfonates, glycolates, or *p*-toluenesulfonates, etc.

As used herein, the term "solvate" refers to a compound of the present disclosure or its pharmaceutically acceptable salt that incorporates either stoichiometric or non-stoichiometric amounts of solvent molecules bound through non-covalent intermolecular forces. Preferably, the solvent is volatile, non-toxic, and suitable for administration in minimal doses to humans. Examples of solvents include, but are not limited to water, isopropanol, ethanol, methanol, DMSO, ethyl acetate, acetic acid, and ethanolamine. The term "hydrate" refers to a complex where the solvent molecule is water.

As used herein, the wording "optionally substituted with..." and "unsubstituted or substituted" may be used interchangeably. The term "substituted" generally indicates that one or more hydrogens in the referenced structure are replaced by the same or different specific substituents. By way of example, substituents may optionally be selected from the group consisting of: deuterium, halogen, cyano, nitro, hydroxy, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkylaryl, alkylamino, aralkylamino, heteroarylamino, arylamino, arylphosphino, heteroaryl, heterocyclyl, acenaphthenyl, and oxo; or the substituents may be formed by linking two or more of the aforementioned exemplary substituents. For example, "substituents formed by linking two or more of the aforementioned exemplary substituents" may include biaryl groups (e.g., biphenyl) and biheteroaryl groups (e.g., bipyridyl).

As used herein, the term "unsubstituted or substituted amino" may be represented by the formula $-NY_1Y_2$, wherein $Y_1$ and $Y_2$ may each independently be hydrogen, deuterium, alkenyl, alkyl (e.g., $C_1$-$C_3$ alkyl), cycloalkyl (e.g., $C_{3-20}$ cycloalkyl), heterocyclyl (e.g., 4- to 20-membered heterocyclyl), aryl (e.g., $C_{6-20}$ aryl), or heteroaryl (e.g., 5- to 20-membered heteroaryl). Non-limiting examples of the unsubstituted or substituted amino groups include amino, N-methylamino, N-ethylamino, N-phenylamino, N,N-dimethylamino, N,N-diethylamino, etc.

As used herein, the term "unsubstituted or substituted silyl" may be represented by the formula $-SiY_aY_bY_c$, wherein $Y_a$, $Y_b$ and $Y_c$ may each independently be hydrogen, deuterium, halogen, alkoxy, halogenated alkoxy, alkenyl, alkyl (e.g., $C_1$-$C_3$ alkyl), cycloalkyl (e.g., $C_{3-20}$ cycloalkyl), heterocyclyl (e.g., 4- to 20-membered heterocyclyl), aryl (e.g., $C_{6-20}$ aryl), or heteroaryl (e.g., 5- to 20-membered heteroaryl). Non-limiting examples of the unsubstituted or substituted silyl include trimethylsilyl, triethylsilyl, tert-butyldimethylsilyl, vinyldimethylsilyl, propyldimethylsilyl, triphenylsilyl, diphenylsilyl, phenylsilyl, etc.

As used herein, the term "unsubstituted or substituted boryl" may be represented by the formula $-BY_dY_e$, wherein $Y_d$ and $Y_e$ may each independently be hydrogen, deuterium, alkoxy, halogenated alkoxy, alkenyl, alkyl (e.g., $C_1$-$C_3$ alkyl), cycloalkyl (e.g., $C_{3-20}$ cycloalkyl), heterocyclyl (e.g., 4- to 20-membered heterocyclyl), aryl (e.g., $C_{6-20}$ aryl), or heteroaryl (e.g., 5- to 20-membered heteroaryl). Non-limiting examples of the unsubstituted or substituted boryl include trimethylboryl, triethylboryl, tert-butyldimethylboryl, triphenylboryl, phenylboryl, etc.

As used herein, the term "...is absent", used alone or in combination, means that the referenced group serves as a bond linker, with the two adjacent groups being directly connected. For example, the expression "$X_1$ is absent" means that $X_1$ is a bond linker. In other words, when $X_1$ is absent, group $L_1$ of the compound of Formula I is directly connected to group $X_2$. Alternatively, for example, the expression "when $X_3$ represents CO, $R_{a2}$ is absent, and $R_{a1}$ is neither hydrogen nor deuterium" means that the group

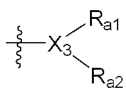

of the compound of Formula I represents

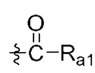.

As used herein, the term "aryl", used alone or in combination, refers to a monovalent aromatic hydrocarbon group containing from 5 to 30 carbon atoms and optionally one or more fused rings, such as $C_5$-$C_{30}$ aryl, $C_5$-$C_{25}$ aryl, $C_5$-$C_{20}$ aryl, $C_5$-$C_{15}$ aryl etc. Aryl may be monocyclic or polycyclic aryl. In some embodiments, monocyclic aryl includes but is not limited

to phenyl, biphenyl, terphenyl, quaterphenyl, and quinquephenyl. Polycyclic aryl includes but is not limited to naphthyl, anthryl, phenanthryl, pyrenyl, perylenyl, and fluorenyl etc. Fluorenyl may be substituted (e.g., 9,9'-dimethylfluorenyl and 9,9'-diphenylfluorenyl etc.); furthermore, two of the substituents may combine with each other to form a spirocyclic structure such as 9,9'-spirobifluorenyl. As used herein, "aryl" refers to an optionally substituted aryl group. A substituted aryl refers to an aryl group substituted one or more times (e.g., 1-4, 1-3, or 1-2 times) with a substituent(s). For example, aryl is mono-, di-, or tri-substituted with a substituent(s) optionally selected from the group consisting of e.g., deuterated $C_1$-$C_6$ alkyl, deuterated $C_3$-$C_6$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, cycloalkyl, alkenyl, aryl, aralkyl, arylalkenyl, alkylaryl, aralkylamino, heteroarylamino, arylamino, arylphosphino, heteroaryl, heterocyclyl, acenaphthenyl, and any combination thereof.

As used herein, the term "arylene", used alone or in combination, refers to a divalent aromatic hydrocarbon group containing from 5 to 30 carbon atoms and optionally one or more fused rings, such as $C_5$-$C_{30}$ arylene, $C_5$-$C_{25}$ arylene, $C_5$-$C_{20}$ arylene, $C_5$-$C_{15}$ arylene etc. $C_5$-$C_{30}$ arylene may be monocyclic arylene or polycyclic arylene. Monocyclic arylene includes but is not limited to phenylene, biphenylene, terphenylene, quaterphenylene, and quinquephenylene. Polycyclic arylene includes but is not limited to naphthylene, anthrylene, phenanthrylene, pyrenylene, perylenylene, and fluorenylene etc. Fluorenylene may be substituted (e.g., 9,9'-dimethylfluorenylene and 9,9'-diphenylfluorenylene); furthermore, two of the substituents may combine with each other to form a spirocyclic structure such as 9,9'-spirobifluorenylene. As used herein, "arylene" refers to an optionally substituted arylene group. A substituted arylene refers to an arylene group substituted one or more times (e.g., 1-4, 1-3, or 1-2 times) with a substituent(s). For example, arylene is mono-, di-, or tri-substituted with a substituent(s) optionally selected from the group consisting of e.g., deuterated $C_1$-$C_6$ alkyl, deuterated $C_3$-$C_6$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, cycloalkyl, alkenyl, aryl, aralkyl, arylalkenyl, alkylaryl, aralkylamino, heteroarylamino, arylamino, arylphosphino, heteroaryl, heterocyclyl, acenaphthenyl, and any combination thereof.

As used herein, the term "heteroaryl", used alone or in combination, refers to a 5- to 30-membered (optionally 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monovalent monocyclic, bicyclic, or polycyclic heteroaryl containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and may be carbocyclic ring or contain one or more heteroatoms independently selected from O, S and N. Heteroaryl includes, but is not limited to, furanyl, oxazolyl, isoxazolyl, oxadiazolyl, thienyl, thiazolyl, isothiazolyl, thiadiazolyl, pyrrolyl, imidazolyl, pyrazolyl, pyridyl, pyrimidinyl, pyridazinyl, pyrazinyl, triazolyl, tetrazolyl, pyranyl, thiopyranyl, thiazinyl, triazinyl, tetrazinyl, indolyl, indolinyl, isoindolyl, isoindolinyl, benzofuranyl, isobenzofuranyl, benzothienyl, indazolyl, quinolinyl, isoquinolinyl, quinazolinyl, quinoxalinyl, cinnolinyl, naphthyridinyl, acridinyl, xanthenyl, phenanthridinyl, diazanaphthyl, triazaindenyl, indolizinyl, phthalazinyl, pyrazolopyridinyl, pyrazolopyrimidinyl, pyridopyrimidinyl, pyridopyrazinyl, pyrazinopyrazinyl, benzothiazolyl, benzoxazolyl, benzimidazolyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, pyrrolopyridinyl, pyrrolothiazolyl, imidazothiazolyl, pyrenyl, dinaphthofuranyl, naphthobenzofuranyl, dinaphthothienyl, or naphthobenzothienyl etc. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, aryl, aralkyl, arylalkenyl, alkylaryl, aralkylamino, heteroarylamino, arylamino, arylphosphino, heteroaryl, heterocyclyl, acenaphthenyl, or any combination thereof.

As used herein, the term "heteroarylene", used alone or in combination, refers to a 5- to 30-membered (optionally 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) divalent monocyclic, bicyclic, or polycyclic heteroaryl containing one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining rings which may be a saturated, partially unsaturated or aromatic ring and may be carbocyclic ring or contain one or more heteroatoms independently

selected from O, S and N. Heteroarylene includes, but is not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, pyranylene, thiopyranylene, thiazinylene, triazinylene, tetrazinylene, indolylene, indolinylene, isoindolylene, isoindolinylene, benzofuranylene, isobenzofuranylene, benzothienylene, indazolylene, quinolinylene, isoquinolinylene, quinazolinylene, quinoxalinylene, cinnolinylene, naphthyridinylene, acridinylene, xanthenylene, phenanthridinylene, diazanaphthylene, triazaindenylene, indolizinylene, phthalazinylene, pyrazolopyridylene, pyrazolopyrimidinylene, pyridopyrimidinylene, pyridopyrazinylene, pyrazinopyrazinylene, benzothiazolylene, benzoxazolylene, benzimidazolylene, dibenzothienylene, dibenzofuranylene, carbazolylene, benzocarbazolylene, dibenzocarbazolylene, indolocarbazolylene, indenocarbazolylene, phenazinylene, imidazopyridinylene, phenanthridinylene, phenanthrolinylene, phenothiazinylene, imidazopyridinylene, imidazophenanthridinylene, pyrrolopyridinylene, pyrrolothiazolylene, imidazothiazolylene, benzimidazoquinazolinylene, benzimidazophenanthridinylene, pyrenylene, dinaphthofuranylene, naphthobenzofuranylene, dinaphthothienylene, or naphthobenzothienylene etc. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, aryl, aralkyl, arylalkenyl, alkylaryl, aralkylamino, heteroarylamino, arylamino, arylphosphino, heteroaryl, heterocyclyl, acenaphthenyl, or any combination thereof.

As used herein, the term "heterocyclyl", used alone or in combination, refers to a monocyclic, bicyclic, tricyclic, or polycyclic saturated or partially unsaturated (i.e., containing one or more double bonds without full conjugation) cycloalkyl group containing one or more heteroatoms (e.g., 1 to 5, 1 to 4, 1 to 3, 1 to 2, or 1 heteroatom) independently selected from sulfur, oxygen, and nitrogen, with no specific limitation on the number of carbon atoms. Representative examples include, but are not limited to, 4- to 30-membered heterocyclyl, 4- to 25-membered heterocyclyl, 4- to 20-membered heterocyclyl, 4- to 15-membered heterocyclyl, 4- to 10-membered heterocyclyl, 10- to 15-membered heterocyclyl, 7- to 15-membered heterocyclyl, and 5- to 15-membered heterocyclyl. Bicyclic, tricyclic, and polycyclic heterocyclyl include bridged heterocyclyl (e.g., 5- to 20-membered, 5- to 15-membered, 5- to 11-membered, 5- to 10-membered, 7- to 20-membered, 7- to 15-membered, 7- to 9-membered bridged heterocyclyl), fused heterocyclyl (e.g., 5- to 20-membered, 5- to 15-membered, 5- to 11-membered, 5- to 10-membered, 7- to 20-membered, 7- to 15-membered, 7- to 9-membered fused heterocyclyl) and spiro-heterocyclyl (e.g., 5- to 20-membered, 5- to 15-membered, 5- to 11-membered, 5- to 10-membered, 7- to 20-membered, 7- to 15-membered, 7- to 9-membered spiro-heterocyclyl). By way of example, the heterocyclyl group may contain 1-3 heteroatoms, 1-2 heteroatoms, or 1 heteroatom, with each heteroatom independently selected from the group consisting of O, N, and S. In additional embodiments, the heterocyclyl includes, but is not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, azacyclooctyl, diazacycloheptanyl (e.g., 1,4-diazacycloheptanyl, 4,5-diazacycloheptanyl, or 1,3-diazacycloheptanyl etc.), diazacyclooctyl, azabicyclohexyl (e.g., 3-azabicyclo[3.1.0]hexyl etc.), azabicycloheptanyl (e.g., 3-azabicyclo[3.1.1]heptanyl, 3-azabicyclo[3.2.0]heptanyl, 6-azabicyclo[3.1.1]heptan-3-yl etc.), diazabicycloheptanyl (e.g., 2,5-diazabicyclo[2.2.1]heptan-2-yl, 3,6-diazabicyclo[3.1.1]heptan-3-yl), azabicyclooctyl (e.g., cis-7-azabicyclo[3.3.0]octanyl, 3-azabicyclo[3.2.1]octan-8-yl etc.), diazabicyclooctyl (e.g., 3,8-diazabicyclo[3.2.1]octan-8-yl, 3,8-diazabicyclo [3.2.1]octan-3-yl, 2,5-diazabicyclo[2.2.2]octan-2-yl), spiro-heterocyclyl (e.g., 5- to 15-membered or 7- to 15-membered spiro-heterocyclyl, 7-azaspiro[3.5]nonanyl, 3-azaspiro[5,5]undecanyl etc.). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, alkenyl, aryl, aralkyl, arylalkenyl, alkylaryl, aralkylamino, heteroarylamino, arylamino, arylphosphino, heteroaryl, heterocyclyl, acenaphthenyl, and any combination thereof.

As used herein, the term "nitrogen-containing heterocyclyl", used alone or in combination, refers to 4- to 30-membered (e.g., 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 4- to 9-membered, 5- to 20-membered, 5- to 15-membered, 7- to 20-membered, or 7- to 15-membered) saturated or partially unsaturated (i.e., containing one or more double bonds without full conjugation) monocyclic, bicyclic, tricyclic, or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of "nitrogen-containing heterocyclyl" include, but are not limited to, nitrogen-containing monocyclic heterocyclyl (e.g., 4- to 30-membered or 4- to 20-membered nitrogen-containing monocyclic heterocyclyl), nitrogen-containing bridged heterocyclyl (e.g., 5- to 20-membered nitrogen-containing bridged heterocyclyl, or 7- to 20-membered nitrogen-containing

bridged heterocyclyl), nitrogen-containing fused heterocyclyl (e.g., 5- to 20-membered or 7- to 20-membered nitrogen-containing fused heterocyclyl) and nitrogen-containing spiro-heterocyclyl (e.g., 5- to 20-membered or 7- to 20-membered nitrogen-containing spiro-heterocyclyl). Representative examples of nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptanyl, azacyclooctyl, diazacycloheptanyl (e.g., 1,4-diazacycloheptan-1-yl) and diazacyclooctyl. Examples of nitrogen-containing bridged heterocyclyl, nitrogen-containing fused heterocyclyl and nitrogen-containing spiro-heterocyclyl include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo [3.2.1]octan-8-yl, 3,8-diazabicyclo[3.2.1]octan-3-yl, 2,5-diazabicyclo[2.2.2]octan-2-yl, and azaspiro-heterocyclyl (e.g., 5- to 20-membered azaspiro-heterocyclyl, such as 3-azaspiro[5.5]undecan-3-yl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, alkenyl, aryl, aralkyl, arylalkenyl, alkylaryl, aralkylamino, heteroarylamino, arylamino, arylphosphino, alkyl, cycloalkyl, heteroaryl, heterocyclyl, acenaphthenyl, and any combination thereof.

As used herein, the term "heterocyclylene", used alone or in combination, refers to a monocyclic, bicyclic, tricyclic, or polycyclic saturated or partially unsaturated (i.e., containing one or more double bonds without full conjugation) divalent cycloalkyl group containing one or more heteroatoms (e.g., 1 to 5, 1 to 4, 1 to 3, 1 to 2, or 1 heteroatom) independently selected from sulfur, oxygen, and nitrogen, with no specific limitation on the number of carbon atoms. Representative examples include, but are not limited to, 4- to 30-membered heterocyclylene, 4- to 25-membered heterocyclylene, 4- to 20-membered heterocyclylene, 4- to 15-membered heterocyclylene, 4- to 10-membered heterocyclylene, 10- to 15-membered heterocyclylene, 7- to 15-membered heterocyclylene, and 5- to 15-membered heterocyclylene etc. Bicyclic, tricyclic, and polycyclic heterocyclylene include bridged heterocyclylene (e.g., 5- to 20-membered, 5- to 15-membered, 5- to 11-membered, 5- to 10-membered, 7- to 20-membered, 7- to 15-membered, 7- to 9-membered bridged heterocyclylene), fused heterocyclylene (e.g., 5- to 20-membered, 5- to 15-membered, 5- to 11-membered, 5- to 10-membered, 7- to 20-membered, 7- to 15-membered, 7-to 9-membered fused heterocyclylene) and spiro-heterocyclylene (e.g., 5- to 20-membered, 5- to 15-membered, 5- to 11-membered, 5- to 10-membered, 7- to 20-membered, 7- to 15-membered, 7- to 9-membered spiro-heterocyclylene). By way of example, the heterocyclylene group may contain 1-3 heteroatoms, 1-2 heteroatoms, or 1 heteroatom, with each heteroatom independently selected from the group consisting of O, N, and S. In additional embodiments, the heterocyclylene includes, but is not limited to, azetidinylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, azacyclooctylene, or diazacycloheptanylene (e.g., 1,4-diazacycloheptanylene , 4,5-diazacycloheptanylene, or 1,3-diazacycloheptanylene etc.), diazacyclooctylene, azabicyclohexylene (e.g., 3-azabicyclo[3.1.0]hexylene etc.), azabicycloheptanylene (e.g., 3-azabicyclo[3.1.1]heptanylene, 3-azabicyclo[3.2.0]heptanylene, 6-azabicyclo[3.1.1]heptanylene etc.), azabicyclooctylene (e.g., cis-7-azabicyclo[3.3.0]octanylene, 3-azabicyclo[3.2.1]octanylene etc.), diazabicyclooctylene (e.g., 3,8-diazabicyclo[3.2.1]octanylene, 2,5-diazabicyclo[2.2.2]octanylene), or spiro-heterocyclylene (e.g., 5- to 15-membered or 7- to 15-membered spiro-heterocyclylene, 7-azaspiro[3.5]nonanylene, 3-azaspiro[5,5]undecanylene etc.). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, alkenyl, aryl, aralkyl, arylalkenyl, alkylaryl, aralkylamino, heteroarylamino, arylamino, arylphosphino, alkyl, cycloalkyl, heteroaryl, heterocyclyl, acenaphthenyl, and any combination thereof.

As used herein, the term "cycloalkyl", used alone or in combination, refers to a saturated or partially unsaturated (i.e., containing one or more double bonds without full conjugation) monocyclic or bicyclic or polycyclic cyclic hydrocarbon radical, which in some embodiments contains from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl), or from 4 to 20 carbon atoms (i.e., $C_{4-20}$ cycloalkyl), or from 4 to 15 carbon atoms (i.e., $C_{4-15}$ cycloalkyl), or from 5 to 20 carbon atoms (i.e., $C_{5-20}$ cycloalkyl), or from 5 to 15 carbon atoms (i.e., $C_{5-15}$ cycloalkyl). The term " $C_3$-$C_{20}$ cycloalkyl" refers to a saturated or partially unsaturated (i.e., containing one or more double bonds without full conjugation) monocyclic or bicyclic or polycyclic cyclic hydrocarbon radical contains from 3 to 20 carbon atoms. The term "cycloalkyl" includes monocyclic, bicyclic, tricyclic and polycyclic

cycloalkyl. The bicyclic, tricyclic and polycyclic cycloalkyl include bridged cycloalkyl (e.g., $C_{5-20}$ bridged cycloalkyl, $C_{5-15}$ bridged cycloalkyl, $C_{7-20}$ bridged cycloalkyl, $C_{7-15}$ bridged cycloalkyl, $C_{7-11}$ bridged cycloalkyl), fused cycloalkyl (e.g., $C_{5-20}$ fused cycloalkyl, $C_{5-15}$ fused cycloalkyl, $C_{7-20}$ fused cycloalkyl, $C_{7-15}$ fused cycloalkyl, $C_{7-11}$ fused cycloalkyl) and spiro-cycloalkyl (e.g., $C_{5-20}$ spiro-cycloalkyl, $C_{5-15}$ spiro-cycloalkyl, $C_{7-20}$ spiro-cycloalkyl, $C_{7-15}$ spiro-cycloalkyl, $C_{7-11}$ spiro-cycloalkyl). Representative examples of cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_{5-20}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), adamantanyl, noradamantanyl, bornyl, norbornyl (also named as bicyclo[2.2.1] heptyl by the IUPAC system) and cubanyl. As used herein, the "cycloalkyl" is optionally substituted. The substituents of the substituted "cycloalkyl" may preferably be one or more (e.g., 1-5, 1-4, 1-3, 1-2, or 1) substituents selected from the group consisting of trifluoromethyl, mercapto, hydroxy, amino, halogen, cyano, $C_1$-$C_3$ alkoxy, trifluoromethyl, heterocyclyl, deuterium, nitro, carbonyl, ester group, imide group, amino, phosphine oxide group, deuterated alkoxy, trifluoromethoxy, aryloxy, alkylthio, arylthio, alkylsulfonyl, arylsulfonyl, silyl, boryl, alkyl (e.g., $C_1$-$C_3$ alkyl), deuterated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo, or any combination thereof.

As used herein, the term "cycloalkylene", used alone or in combination, refers to a saturated or partially unsaturated (i.e., containing one or more double bonds without full conjugation) divalent monocyclic or bicyclic or polycyclic cyclic hydrocarbon radical, which in some embodiments contains from 3 to 20 carbon atoms (i.e., $C_{3-20}$ cycloalkylene), or from 3 to 15 carbon atoms (i.e., $C_{3-15}$ cycloalkylene), or from 3 to 12 carbon atoms (i.e., $C_{3-12}$ cycloalkylene), or from 3 to 11 carbon atoms (i.e., $C_{3-11}$ cycloalkylene), or from 3 to 10 carbon atoms (i.e., $C_{3-10}$ cycloalkylene), or from 3 to 8 carbon atoms ( i.e., $C_{3-8}$ cycloalkylene), or from 3 to 7 carbon atoms (i.e., $C_{3-7}$ cycloalkylene), or from 3 to 6 carbon atoms (i.e., $C_{3-6}$ cycloalkylene), or from 4 to 20 carbon atoms (i.e., $C_{4-20}$ cycloalkylene), or from 4 to 15 carbon atoms (i.e., $C_{4-15}$ cycloalkylene), or from 5 to 20 carbon atoms (i.e., $C_{5-20}$ cycloalkylene), or from 5 to 15 carbon atoms (i.e., $C_{5-15}$ cycloalkylene). The term "$C_3$-$C_{20}$ cycloalkylene" refers to a saturated or partially unsaturated (i.e., containing one or more double bonds without full conjugation) divalent monocyclic or bicyclic or polycyclic cyclic hydrocarbon radical contains from 3 to 20 carbon atoms. The term "cycloalkylene" includes monocyclic, bicyclic, tricyclic and polycyclic cycloalkylene. The bicyclic, tricyclic and polycyclic cycloalkylene include bridged cycloalkylene (e.g., $C_{5-20}$ bridged cycloalkylene, $C_{5-15}$ bridged cycloalkylene, $C_{7-20}$ bridged cycloalkylene, $C_{7-15}$ bridged cycloalkylene, $C_{7-11}$ bridged cycloalkylene), fused cycloalkylene (e.g., $C_{5-20}$ fused cycloalkylene, $C_{5-15}$ fused cycloalkylene, $C_{7-20}$ fused cycloalkylene, $C_{7-15}$ fused cycloalkylene, $C_{7-11}$ fused cycloalkylene) and spiro-cycloalkylene (e.g., $C_{5-20}$ spiro-cycloalkylene, $C_{5-15}$ spiro-cycloalkylene, $C_{7-20}$ spiro-cycloalkylene, $C_{7-15}$ spiro-cycloalkylene, $C_{7-11}$ spiro-cycloalkylene). Representative examples of cycloalkylene include, but are not limited to, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_{5-20}$ spiro-cycloalkylene, such as spiro[3.3]heptanylene, spiro [2.5]octanylene, spiro[3.5]nonylene, spiro[4.4]nonylene, spiro[4.5]decylene, or spiro[5.5]undecylene), adamantanylene, noradamantanylene, bornylene, norbornylene (also named as bicyclo[2.2.1]heptanylene by the IUPAC system) and cubanylene. The cycloalkylene may be unsubstituted or substituted as explicitly defined. The substituents of the substituted "cycloalkylene" may preferably be one or more (e.g., 1-5, 1-4, 1-3, 1-2, or 1) substituents selected from the group consisting of trifluoromethyl, mercapto, hydroxy, amino, halogen, cyano, $C_1$-$C_3$ alkoxy, trifluoromethyl, heterocyclyl, deuterium, nitro, carbonyl, ester group, imide group, phosphine oxide group, deuterated alkoxy, trifluoromethoxy, aryloxy, alkylthio, arylthio, alkylsulfonyl, arylsulfonyl, silyl, boryl, alkyl (e.g., $C_1$-$C_3$ alkyl), deuterated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo, or any combination thereof.

As used herein, the term "alkyl", used alone or in combination, refers to a linear or branched alkyl group, with no specific limitation on the number of carbon atoms. For example, it may be $C_1$-$C_{30}$, $C_1$-$C_{25}$, $C_1$-$C_{20}$, $C_1$-$C_{15}$, $C_1$-$C_{10}$, $C_1$-$C_5$ alkyl, etc. By way of example, alkyl includes but is not limited to methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, etc. As used herein, the "alkyl" is optionally substituted by one or more substituents optionally selected from the group consisting of D (i.e., deuterium), halogen, hydroxy, cyano, $C_1$-$C_3$ alkoxy, trifluoromethyl, heterocyclyl (e.g., 4- to 8-membered heterocyclyl), nitro, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl (e.g., $C_1$-$C_3$ alkyl), deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkylaryl, alkylamino, aralkylamino, heteroarylamino, arylamino, arylphosphino, heteroaryl,

acenaphthenyl, oxo, or any combination thereof.

As used herein, the term "alkylene", used alone or in combination, refers to a linear or branched divalent saturated hydrocarbon group composed of carbon and hydrogen atoms, with no specific limitation on the number of carbon atoms. For example, it may be $C_1$-$C_{30}$, $C_1$-$C_{25}$, $C_1$-$C_{20}$, $C_1$-$C_{15}$, $C_1$-$C_{10}$, $C_1$-$C_5$ alkylebe, etc. By way of example, alkylene includes but is not limited to methylene, ethylene, propylene, isopropylene, butylene, n-butylene, isobutylene, tert-butylene, sec-butylene, 1-methyl-butylene, 1-ethyl-butylene, pentylene, n-pentylene, isopentylene, neopentylene, tert-pentylene, hexylene, n-hexylene, 1-methylpentylene, 2-methylpentylene, 4-methyl-2-pentylene, 3,3-dimethylbutylene, 2-ethylbutylene, heptylene, n-heptylene, 1-methylhexylene, cyclopentylmethylene, cyclohexylmethylene, octylene, n-octylene, tert-octylene, 1-methylheptylene, 2-ethylhexylene, 2-propylpentylene, nonylene, 2,2-dimethylheptylene, 1-ethyl-propylene, 1,1-dimethyl-propylene, isohexylene, 4-methylhexylene, 5-methylhexylene, etc. As used herein, the "alkylene" is optionally substituted by one or more substituents optionally selected from the group consisting of D (i.e., deuterium), halogen, hydroxy, cyano, $C_1$-$C_3$ alkoxy, trifluoromethyl, heterocyclyl (e.g., 4- to 8-membered heterocyclyl), nitro, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo, or any combination thereof.

As used herein, the term "alkoxy", used alone or in combination, refers to -O-alkyl. Optionally, the alkyl portion of the alkoxy group may contain 1-10 (or 1-6, 1-5, 1-4, or 1-3) carbon atoms. Alkoxy includes, but is not limited to, e.g., methoxy, ethoxy, propoxy, isopropoxy, n-butoxy, isobutoxy, tert-butoxy, sec-butoxy, n-pentyloxy, neopentyloxy, isopentyloxy, n-hexyloxy, 3,3-dimethylbutoxy, 2-ethylbutoxy, n-octyloxy, n-nonyloxy, n-decyloxy, etc.

As used herein, the term "halogen atom" or "halogen", used alone or in combination, refers to fluorine, chlorine, bromine, or iodine.

As used herein, the term "halogenated alkyl", used alone or in combination, refers to a linear or branched alkyl in which one or more hydrogen atoms are each replaced with a halogen atom. Examples of the halogenated alkyl include, but are not limited to, $-CF_3$, $-CHF_2$, $-CH_2F$, $-CBr_3$, $-CHBr_2$, $-CH_2Br$, $-CCl_3$, $-CHCl_2$, $-CH_2Cl$, $-CI_3$, $-CHI_2$, $-CH_2I$, $-CH_2-CF_3$, $-CH_2-CHF_2$, $-CH_2-CH_2F$, $-CH_2-CBr_3$, $-CH_2-CHBr_2$, $-CH_2-CH_2Br$, $-CH_2-CCl_3$, $-CH_2-CHCl_2$, $-CH_2-C H_2Cl$, $-CH_2-CI_3$, $-CH_2-CHI_2$, $-CH_2-CH_2I$ etc. Herein, the alkyl and halogen are as defined above.

As used herein, the term "halogenated alkoxy", used alone or in combination, refers to a alkoxy group in which one or more hydrogen atoms are each replaced with a halogen atom. Examples of the halogenated alkoxy include, but are not limited to, trifluoromethoxy etc. Herein, the alkoxy and halogen are as defined above.

As used herein, the term "pharmaceutically acceptable carrier" generally refers to carriers used for the administration of therapeutic agents, which do not themselves induce the production of antibodies harmful to the individual receiving the composition and exhibit no excessive toxicity upon administration. These carriers are well-known to those skilled in the art, e.g., relevant information on pharmaceutically acceptable carriers disclosed in Remington's Pharmaceutical Sciences (Mack Pub. Co., N.J., 1991). Specifically, the carrier may comprise, but is not limited to, one or more selected from the group consisting of saline, buffers, glucose, water, glycerol, ethanol, adjuvants, and combinations thereof.

As used herein, the term "pharmaceutically acceptable excipient" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they may perform their intended function. Generally, such constructs are carried or transported from one organ or part of the body to another organ or part of the body. The excipient is compatible with the other ingredients of the formulation, including the compounds useful in the present disclosure, and is not harmful to the patient, and the excipient must be "acceptable". Some examples of materials that may be used as pharmaceutically acceptable excipients include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

## Compounds

In one aspect, the present disclosure provides an embodiment 1): the compound of Formula I:

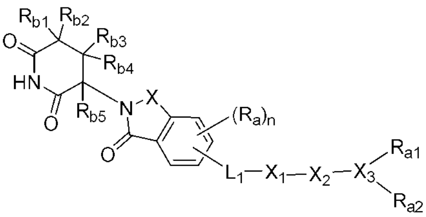

I

or salts, enantiomers, stereoisomers, polymorphs or solvates thereof,

wherein X is selected from the group consisting of CO or $CH_2$;
$L_1$ is selected from the group consisting of S or O;
$X_1$ is selected from the group consisting of unsubstituted or substituted linear or branched alkylene, or $X_1$ is absent;
$X_2$ represents the structure of Formula II:

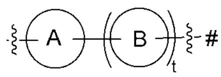

II

wherein ring A and ring B are each independently selected from the group consisting of unsubstituted or substituted arylene, unsubstituted or substituted heteroarylene, unsubstituted or substituted heterocyclylene, or unsubstituted or substituted cycloalkylene;
t represents an integer of 0, 1 or 2; and
symbol # indicates the point of attachment to $X_3$;

$X_3$ is selected from the group consisting of $CR_1$, N, or CO, wherein $R_1$ is selected from the group consisting of hydrogen, deuterium, halogen, nitro, cyano, hydroxy, mercapto, trifluoromethoxy, unsubstituted or substituted amino, unsubstituted or substituted silyl, unsubstituted or substituted boryl, unsubstituted or substituted linear or branched alkyl, or unsubstituted or substituted cycloalkyl;
$R_{a1}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl;
$R_{a2}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl, or $R_{a2}$ is absent; and when $X_3$ represents CO, $R_{a2}$ is absent and $R_{a1}$ is neither hydrogen nor deuterium, and when $R_{a2}$ represents hydrogen or deuterium, $X_3$ represents $CR_1$ or N and $R_{a1}$ is neither hydrogen nor deuterium;
$R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl;
$R_{b1}$, $R_{b2}$, $R_{b3}$, $R_{b4}$ and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl;
n is an integer of 0, 1, 2 or 3, and when n is an integer of 2 or 3, each $R_a$ is independently identical to or different from each other.

Embodiment 2): Pertaining to the compound of Formula I or salts, enantiomers, stereoisomers, polymorphs or solvates thereof as described in embodiment 1), wherein X in Formula I is CO.
Embodiment 3): Pertaining to the compound of Formula I or salts, enantiomers, stereoisomers, polymorphs or solvates thereof as described in embodiment 1), wherein X in Formula I is $CH_2$.
Embodiment 4): Pertaining to the compound of Formula I or salts, enantiomers, stereoisomers, polymorphs or solvates thereof as described in embodiment 1), wherein $L_1$ in Formula I is S.
Embodiment 5): Pertaining to the compound of Formula I or salts, enantiomers, stereoisomers, polymorphs or solvates thereof as described in embodiment 1), wherein $L_1$ in Formula I is O.
Embodiment 6): Pertaining to the compound of Formula I or salts, enantiomers, stereoisomers, polymorphs or solvates thereof as described in any one of embodiments 1)-5), wherein $X_1$ is unsubstituted or substituted linear or

branched alkylene. When $X_1$ is unsubstituted or substituted linear or branched alkylene, the $X_1$ represents unsubstituted or substituted linear or branched $C_1$-$C_5$ alkylene, or unsubstituted or substituted linear or branched $C_1$-$C_3$ alkylene.

Embodiment 7): Pertaining to the compound of Formula I or salts, enantiomers, stereoisomers, polymorphs or solvates thereof as described in any one of embodiments 1)-6), wherein when $X_1$ is unsubstituted or substituted linear or branched $C_1$-$C_5$ alkylene, the linear or branched $C_1$-$C_5$ alkylene may be methylene, ethylene, propylene, n-propylene, isopropylene, butylene, n-butylene, isobutylene, tert-butylene, sec-butylene, 1-methyl-butylene, 1-ethyl-butylene, pentylene, n-pentylene, isopentylene, neopentylene, or tert-pentylene.

Embodiment 8): Pertaining to the compound of Formula I or salts, enantiomers, stereoisomers, polymorphs or solvates thereof as described in any one of embodiments 1)-7), wherein when $X_1$ is unsubstituted or substituted linear or branched $C_1$-$C_3$ alkylene, the linear or branched alkylene may be e.g., methylene, ethylene, propylene, or isopropylene.

Embodiment 9): Pertaining to the compound of Formula I or salts, enantiomers, stereoisomers, polymorphs or solvates thereof as described in any one of embodiments 1)-8), wherein the $X_1$ is selected from the group consisting of methylene.

Embodiment 10): Pertaining to the compound of Formula I or salts, enantiomers, stereoisomers, polymorphs or solvates thereof as described in any one of embodiments 1)-8), wherein the substituents of the substituted $X_1$ is optionally selected from the group consisting of deuterium, halogen, cyano, nitro, hydroxy, mercapto, carbonyl, ester group, imide group, amino, phosphine oxide group, alkoxy, halogenated alkoxy, aryloxy, alkylthio, arylthio, alkylsulfonyl, arylsulfonyl, silyl, boryl, alkyl, cycloalkyl, alkenyl, aryl, aralkyl, arylalkenyl, alkylaryl, alkylamino, aralkylamino, heteroarylamino, arylamino, arylphosphino, heteroaryl, and acenaphthenyl etc., or any combination thereof. Optionally, the substituents of the substituted $X_1$ may be e.g., one or more substituents selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl), nitro, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo or any combination thereof. Further optionally, the substituents of the substituted $X_1$ may be e.g., selected from the group consisting of deuterium, halogen, nitro, mercapto, hydroxy, cyano, $C_1$-$C_3$ alkyl, $C_1$-$C_3$ alkoxy, halogenated $C_1$-$C_3$ alkoxy, halogenated $C_1$-$C_3$ alkyl, amino, silyl, boryl, or any combination thereof. Further optionally, the substituents of the substituted $X_1$ may be e.g., selected from the group consisting of deuterium, F, Cl, methoxy, methyl, hydroxy, or any combination thereof.

Embodiment 11): Pertaining to the compound of Formula I or salts, enantiomers, stereoisomers, polymorphs or solvates thereof as described in any one of embodiments 1)-5), wherein $X_1$ is absent.

Embodiment 12): Pertaining to the compound of Formula I or salts, enantiomers, stereoisomers, polymorphs or solvates thereof as described in any one of embodiments 1)-11), wherein $X_2$ represents the structure of Formula II:

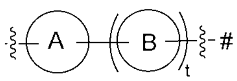

II

wherein ring A is selected from the group consisting of unsubstituted or substituted $C_5$-$C_{30}$ arylene, unsubstituted or substituted 5- to 30-membered heteroarylene, unsubstituted or substituted 4- to 30-membered heterocyclylene, or unsubstituted or substituted $C_3$-$C_{30}$ cycloalkylene; preferably, ring A is selected from the group consisting of unsubstituted or substituted $C_5$-$C_{20}$ arylene, unsubstituted or substituted 5- to 20-membered heteroarylene, unsubstituted or substituted 4- to 20-membered heterocyclylene, or unsubstituted or substituted $C_3$-$C_{20}$ cycloalkylene;
ring B is selected from the group consisting of unsubstituted or substituted $C_5$-$C_{30}$ arylene, unsubstituted or substituted 5- to 30-membered heteroarylene, unsubstituted or substituted 4- to 30-membered heterocyclylene, or unsubstituted or substituted $C_3$-$C_{30}$ cycloalkylene; preferably, ring B is selected from the group consisting of unsubstituted or substituted $C_5$-$C_{20}$ arylene, unsubstituted or substituted 5- to 20-membered heteroarylene, unsubstituted or substituted 4- to 20-membered heterocyclylene, or unsubstituted or substituted $C_3$-$C_{20}$ cycloalkylene;
t represents an integer of 0, 1 or 2; preferably, t represents an integer of 0 or 1; and
symbol # indicates the point of attachment to $X_3$.

Embodiment 13): Pertaining to the compound of Formula I or salts, enantiomers, stereoisomers, polymorphs or solvates thereof as described in any one of embodiments 1)-12), wherein $X_2$ represents the structure of Formula II:

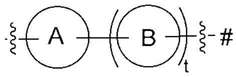

II

wherein ring A is selected from the group consisting of unsubstituted or substituted $C_5$-$C_{15}$ arylene, unsubstituted or substituted 5- to 15-membered heteroarylene, unsubstituted or substituted 4- to 15-membered heterocyclylene, or unsubstituted or substituted $C_3$-$C_{15}$ cycloalkylene, wherein the substituents of the substituted ring A are optionally one or more substituents selected from the group consisting of halogen, mercapto, hydroxy, amino, cyano, $C_1$-$C_6$ alkyl, $C_1$-$C_6$ alkoxy, halogenated $C_1$-$C_6$ alkyl or any combination thereof;
ring B is selected from the group consisting of unsubstituted or substituted $C_5$-$C_{15}$ arylene, unsubstituted or substituted 5- to 15-membered heteroarylene, unsubstituted or substituted 4- to 15-membered heterocyclylene, or unsubstituted or substituted $C_3$-$C_{15}$ cycloalkylene; wherein the substituents of the substituted ring B are optionally one or more substituents selected from the group consisting of halogen, mercapto, hydroxy, amino, cyano, $C_1$-$C_6$ alkyl, $C_1$-$C_6$ alkoxy, halogenated $C_1$-$C_6$ alkyl, or any combination thereof;
t represents an integer of 0, 1 or 2; preferably, t represents an integer of 0 or 1; and
symbol # indicates the point of attachment to $X_3$.

Embodiment 14): Pertaining to the compound of Formula I or salts, enantiomers, stereoisomers, polymorphs or solvates thereof as described in any one of embodiments 1)-13), wherein ring A and ring B are each independently selected from the group consisting of:

azetidinylene, azacyclopentylene, azacyclohexylene, azacycloheptanylene, azacyclooctylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, azacyclooctylene, diazacycloheptanylene, diazacyclooctylene, bridged heterocyclylene (e.g., 4- to 15-membered bridged heterocyclylene, 5- to 15-membered bridged heterocyclylene, and 7- to 15-membered bridged heterocyclylene, such as azabicyclohexylene, azabicycloheptanylene or azabicyclooctylene), or spiro-heterocyclylene (e.g., 4- to 15-membered spiro-heterocyclylene, 5- to 15-membered spiro-heterocyclylene, and 7- to 15-membered spiro-heterocyclylene, such as 3-azaspiro[5.5]undecanylene or 7-azaspiro[3.5]nonanylene); or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo [2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene; or
cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_3$-$C_{15}$ spiro-cycloalkylene or $C_5$-$C_{15}$ spiro-cycloalkylene or $C_7$-$C_{15}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), or bridged cycloalkylene (e.g., $C_3$-$C_{15}$ bridged cycloalkylene or $C_5$-$C_{15}$ bridged cycloalkylene or $C_7$-$C_{15}$ bridged cycloalkylene, adamantanylene, noradamantanylene, bornylene, bicyclo[2.2.1]heptylene, 2-oxobicyclo[2.2.1]heptylene, or bicyclo [2.2.1]heptentylene); or
phenylene or naphthylene;
wherein ring A and ring B are each independently optionally substituted by one or more substituents selected from the group consisting of deuterium, halogen, nitro, mercapto, hydroxy, cyano, $C_1$-$C_6$ alkyl, $C_1$-$C_6$ alkoxy, halogenated $C_1$-$C_6$ alkoxy, halogenated $C_1$-$C_6$ alkyl, unsubstituted or substituted amino, unsubstituted or substituted silyl, unsubstituted or substituted boryl or any combination thereof.

Embodiment 15): Pertaining to the compound of Formula I or salts, enantiomers, stereoisomers, polymorphs or solvates thereof as described in any one of embodiments 12)-14), wherein $X_2$ represents optionally substituted nitrogen-containing heterocyclyl, optionally substituted aryl-$NH_2$, optionally substituted heteroaryl-$NH_2$, or optionally substituted cycloalkyl-$NH_2$. Further, $X_2$ is selected from the group consisting of

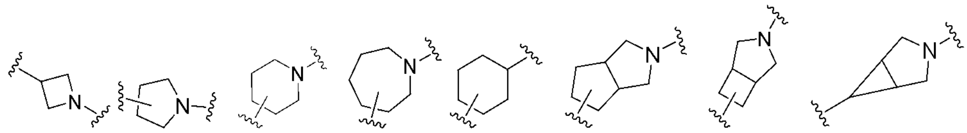

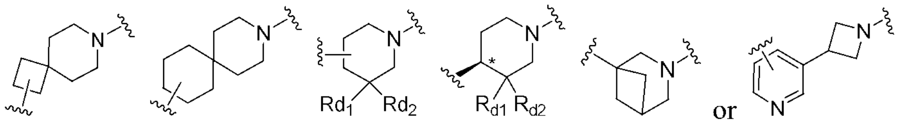
or

etc.

Embodiment 16): Pertaining to the compound of Formula I or salts, enantiomers, stereoisomers, polymorphs or solvates thereof as described in embodiment 15), wherein $R_{d1}$ and $R_{d2}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, and unsubstituted or substituted linear or branched $C_1$-$C_5$ alkyl; and $R_{d1}$ and $R_{d2}$ are not hydrogen at the same time. In specific embodiments, optionally, the halogen may be e.g., F, Cl, Br or I. Optionally, the $C_1$-$C_6$ alkoxy includes but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, and 2-ethylbutoxy etc. Optionally, the halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino etc. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_5$ alkyl includes but is not limited to methyl, ethyl, propyl, isopropyl, butyl, n-butyl, isobutyl, tert-butyl, sec-butyl, 1-methyl-butyl, 1-ethyl-butyl, pentyl, n-pentyl, isopentyl, neopentyl, and tert-pentyl etc., wherein said linear or branched $C_1$-$C_5$ alkyl is optionally substituted with one or more substituents optioanlly selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl) or any combination thereof.

Embodiment 17): Pertaining to the compound of Formula I or salts, enantiomers, stereoisomers, polymorphs or solvates thereof as described in any one of embodiments 1)-16), wherein $X_3$ is $CR_1$; and $R_1$ is selected from the group consisting of hydrogen, deuterium, halogen, nitro, cyano, hydroxy, mercapto, trifluoromethoxy, unsubstituted or substituted amino, unsubstituted or substituted silyl, unsubstituted or substituted boryl, unsubstituted or substituted linear or branched alkyl, or unsubstituted or substituted cycloalkyl.

Embodiment 18): Pertaining to the compound of Formula I or salts, enantiomers, stereoisomers, polymorphs or solvates thereof as described in embodiment 17), wherein $R_1$ is selected from the group consisting of hydrogen, deuterium, F, Cl, Br, I, nitro, cyano, hydroxy, mercapto, trifluoromethoxy, unsubstituted or substituted amino, unsubstituted or substituted silyl, unsubstituted or substituted boryl, unsubstituted or substituted linear or branched $C_1$-$C_{10}$ alkyl, or unsubstituted or substituted $C_3$-$C_{15}$ cycloalkyl.

Embodiment 19): Pertaining to the compound of Formula I or salts, enantiomers, stereoisomers, polymorphs or solvates thereof as described in embodiment 17), wherein when $X_3$ is selected from the group consisting of $CR_1$, $R_1$ is hydrogen.

Embodiment 20): Pertaining to the compound of Formula I or salts, enantiomers, stereoisomers, polymorphs or solvates thereof as described in embodiment 17), wherein when $X_3$ is selected from the group consisting of $CR_1$, $R_1$ is deuterium.

Embodiment 21): Pertaining to the compound of Formula I or salts, enantiomers, stereoisomers, polymorphs or solvates thereof as described in embodiment 17), wherein when $X_3$ is selected from the group consisting of $CR_1$, $R_1$ is halogen. Optionally, when $R_1$ is halogen, the halogen is F, Cl, Br, or I.

Embodiment 22): Pertaining to the compound of Formula I or salts, enantiomers, stereoisomers, polymorphs or solvates thereof as described in embodiment 17), wherein when $X_3$ is selected from the group consisting of $CR_1$, $R_1$ is nitro.

Embodiment 23): Pertaining to the compound of Formula I or salts, enantiomers, stereoisomers, polymorphs or solvates thereof as described in embodiment 17), wherein when $X_3$ is selected from the group consisting of $CR_1$, $R_1$ is cyano.

Embodiment 24): Pertaining to the compound of Formula I or salts, enantiomers, stereoisomers, polymorphs or solvates thereof as described in embodiment 17), wherein when $X_3$ is selected from the group consisting of $CR_1$, $R_1$ is amino.

Embodiment 25): Pertaining to the compound of Formula I or salts, enantiomers, stereoisomers, polymorphs or solvates thereof as described in embodiment 17), wherein when $X_3$ is selected from the group consisting of $CR_1$, $R_1$ is

hydroxy.

Embodiment 26): Pertaining to the compound of Formula I or salts, enantiomers, stereoisomers, polymorphs or solvates thereof as described in embodiment 17), wherein when $X_3$ is selected from the group consisting of $CR_1$, $R_1$ is mercapto.

Embodiment 27): Pertaining to the compound of Formula I or salts, enantiomers, stereoisomers, polymorphs or solvates thereof as described in embodiment 17), wherein when $X_3$ is selected from the group consisting of $CR_1$, $R_1$ is trifluoromethoxy.

Embodiment 28): Pertaining to the compound of Formula I or salts, enantiomers, stereoisomers, polymorphs or solvates thereof as described in embodiment 17), wherein when $X_3$ is selected from the group consisting of $CR_1$, $R_1$ is unsubstituted or substituted amino. Optionally, the unsubstituted or substituted amino includes amino, N-methylamino, N-ethylamino, N-phenylamino, N,N-dimethylamino, and N,N-diethylamino etc.

Embodiment 29): Pertaining to the compound of Formula I or salts, enantiomers, stereoisomers, polymorphs or solvates thereof as described in embodiment 17), wherein when $X_3$ is selected from the group consisting of $CR_1$, $R_1$ is unsubstituted or substituted silyl. Optionally, the unsubstituted or substituted silyl is specifically trimethylsilyl, triethylsilyl, tert-butyldimethylsilyl, vinyldimethylsilyl, propyldimethylsilyl, triphenylsilyl, diphenylsilyl, and phenylsilyl etc.

Embodiment 30): Pertaining to the compound of Formula I or salts, enantiomers, stereoisomers, polymorphs or solvates thereof as described in embodiment 17), wherein when $X_3$ is selected from the group consisting of $CR_1$, $R_1$ is unsubstituted or substituted boryl. Optionally, the unsubstituted or substituted boryl is specifically trimethylboryl, triethylboryl, tert-butyldimethylboryl, triphenylboryl, and phenylboryl etc.

Embodiment 31): Pertaining to the compound of Formula I or salts, enantiomers, stereoisomers, polymorphs or solvates thereof as described in embodiment 17), wherein when $X_3$ is selected from the group consisting of $CR_1$, $R_1$ is unsubstituted or substituted alkyl. Optionally, when the $R_1$ is unsubstituted or substituted linear or branched alkyl, the number of carbon atoms may be for example $C_1$-$C_{30}$, $C_1$-$C_{25}$, $C_1$-$C_{20}$, $C_1$-$C_{15}$, $C_1$-$C_{10}$, $C_1$-$C_5$, and $C_1$-$C_3$ etc, preferably linear or branched $C_1$-$C_{10}$ alkyl. Further optionally, the linear or branched $C_1$-$C_{10}$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, nonyl, 2,2-dimethylheptyl, 1-ethylpropyl, 1,1-dimethylpropyl, isohexyl, 4-methylhexyl, or 5-methylhexyl etc. Optionally, the linear or branched $C_1$-$C_{10}$ alkyl includes but is not limited to methyl, ethyl, and propyl etc. Further, the alkyl is optionally substituted by a substituent optionally selected from the group consisting of e.g., deuterium, halogen, cyano, nitro, hydroxy, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo, or any combination thereof. The substituent is preferably one or more substituents selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl) or any combination thereof.

Embodiment 32): Pertaining to the compound of Formula I or salts, enantiomers, stereoisomers, polymorphs or solvates thereof as described in embodiment 17), wherein when $R_1$ is unsubstituted or substituted cycloalkyl, it has 3 to 20 carbon atoms (i.e., $C_{3-20}$ cycloalkyl), 3 to 15 carbon atoms (i.e., $C_{3-15}$ cycloalkyl), 3 to 12 carbon atoms (i.e., $C_{3-12}$ cycloalkyl), 3 to 11 carbon atoms (i.e., $C_{3-11}$ cycloalkyl), 3 to 10 carbon atoms (i.e., $C_{3-10}$ cycloalkyl), 3 to 8 carbon atoms (i.e., $C_{3-8}$ cycloalkyl), 3 to 7 carbon atoms (i.e., $C_{3-7}$ cycloalkyl), or 3 to 6 carbon atoms (i.e., $C_{3-6}$ cycloalkyl). Preferably, $R_1$ is unsubstituted or substituted $C_3$-$C_{15}$ cycloalkyl. Optionally, $C_3$-$C_{15}$ cycloalkyl includes but is not limited to cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_{5-20}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), adamantanyl, noradamantanyl, bornyl, norbornyl (Also named as bicyclo[2.2.1]heptyl by the IUPAC system) and cubanyl etc. Further optionally, $C_3$-$C_{15}$ cycloalkyl includes but is not limited to cyclopropyl, cyclobutyl, and cyclopentyl etc. Wherein, said cycloalkyl is optionally substituted with one or more (e.g., 1-5, 1-4, 1-3, 1-2, or 1) substituents optioanlly selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, amino, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, acenaphthenyl, oxo or any combination thereof. The substituents are preferably selected from the group consisting of trifluoromethyl, mercapto, hydroxy, amino, halogen, cyano, $C_1$-$C_3$ alkyl, $C_1$-$C_3$ alkoxy, trifluoromethoxy, heterocyclyl or any combination thereof.

Embodiment 33): Pertaining to the compound of Formula I or salts, enantiomers, stereoisomers, polymorphs or solvates thereof as described in any one of embodiments 1)-17), wherein $X_3$ is N.

Embodiment 34): Pertaining to the compound of Formula I or salts, enantiomers, stereoisomers, polymorphs or solvates thereof as described in any one of embodiments 1)-17), wherein $X_3$ is CO.

Embodiment 35): Pertaining to the compound of Formula I or salts, enantiomers, stereoisomers, polymorphs or solvates thereof as described in any one of embodiments 1)-17), wherein when $X_3$ represents CO, $R_{a2}$ is absent and $R_{a1}$ is neither hydrogen nor deuterium.

Embodiment 36): Pertaining to the compound of Formula I or salts, enantiomers, stereoisomers, polymorphs or solvates thereof as described in any one of embodiments 1)-17), wherein when $R_{a2}$ represents hydrogen or deuterium, $X_3$ represents $CR_1$ or N, and $R_{a1}$ is neither hydrogen nor deuterium.

Embodiment 37): Pertaining to the compound of Formula I or salts, enantiomers, stereoisomers, polymorphs or solvates thereof as described in any one of embodiments 1)-36), wherein $R_{a1}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl.

Embodiment 38): Pertaining to the compound of Formula I or salts, enantiomers, stereoisomers, polymorphs or solvates thereof as described in embodiment 37), wherein $R_{a1}$ is selected from the group consisting of hydrogen, deuterium, unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl; preferably, $R_{a1}$ is selected from the group consisting of hydrogen, deuterium, unsubstituted or substituted $C_5$-$C_{20}$ aryl, unsubstituted or substituted 5- to 20-membered heteroaryl, unsubstituted or substituted $C_3$-$C_{20}$ cycloalkyl, or unsubstituted or substituted 4- to 20-membered heterocyclyl.

Embodiment 39): Pertaining to the compound of Formula I or salts, enantiomers, stereoisomers, polymorphs or solvates thereof as described in embodiment 37), wherein $R_{a1}$ is hydrogen.

Embodiment 40): Pertaining to the compound of Formula I or salts, enantiomers, stereoisomers, polymorphs or solvates thereof as described in embodiment 37), wherein $R_{a1}$ is deuterium.

Embodiment 41): Pertaining to the compound of Formula I or salts, enantiomers, stereoisomers, polymorphs or solvates thereof as described in embodiment 37), wherein $R_{a1}$ is unsubstituted or substituted aryl. Optionally, the unsubstituted or substituted aryl is unsubstituted or substituted $C_5$-$C_{30}$ aryl. Optionally, the unsubstituted or substituted $C_5$-$C_{30}$ aryl is unsubstituted or substituted $C_5$-$C_{25}$, $C_5$-$C_{20}$, $C_5$-$C_{15}$ aryl. Optionally, the $C_5$-$C_{30}$ aryl may be monocyclic or polycyclic aryl. In some embodiments, monocyclic aryl includes but is not limited to phenyl, biphenyl, terphenyl, quaterphenyl, and quinquephenyl etc. Polycyclic aryl includes but is not limited to naphthyl, anthryl, phenanthryl, pyrenyl, perylenyl, and fluorenyl etc. Fluorenyl is optionally substituted, with examples including 9,9'-dimethylfluorenyl and 9,9'-diphenylfluorenyl etc. Furthermore, two of the substituents may combine with each other to form a spirocyclic structure such as 9,9'-spirobifluorenyl etc. The aryl is optionally substituted aryl. The substituted aryl refers to an aryl group substituted one or more times (e.g., 1-4, 1-3, or 1-2 times) with a substituent. For example, aryl is mono-, di-, or tri-substituted with a substituent optionally selected from the group consisting of e.g., deuterated $C_1$-$C_6$ alkyl, deuterated $C_3$-$C_6$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, unsubstituted or substituted cycloalkyl, alkenyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof (preferably, the substituent is selected from the group consisting of deuterated $C_1$-$C_6$ alkyl, deuterated $C_3$-$C_6$ cycloalkyl, hydroxy, amino, mercapto, halogen, cyano, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl, and any combination thereof). Further, when $R_{a1}$ is substituted aryl and the substituent of aryl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.) (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, or $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, or $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof). Further, when $R_{a1}$ is substituted aryl and the substituent of aryl is unsubstituted or substituted aryl, the

unsubstituted or substituted aryl is selected from the group consisting of unsubstituted or substituted $C_5$-$C_{20}$ aryl (e.g., unsubstituted or substituted $C_5$-$C_{15}$ aryl and unsubstituted or substituted $C_5$-$C_{10}$ aryl etc.) (optionally, the unsubstituted or substituted aryl is selected from the group consisting of biphenyl, terphenyl, naphthyl, anthryl, phenanthryl, fluorenyl, or 9,9'-dimethylfluorenyl etc.; and the unsubstituted or substituted aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof). Further, when $R_{a1}$ is substituted aryl and the substituent of aryl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl, or unsubstituted or substituted 5- to 10-membered heteroaryl) (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl; the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof). Further, when $R_{a1}$ is substituted aryl and the substituent of aryl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.) (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl, or 5- to 15-membered bridged heterocyclyl, or 7- to 15-membered bridged heterocyclyl, such as azabicyclohexyl, azabicycloheptanyl, diazabicycloheptanyl, azabicyclooctyl, and diazabicyclooctyl), or spiro-heterocyclyl (e.g., 4- to 15-membered spiro-heterocyclyl, or 5- to 15-membered spiro-heterocyclyl, or 7- to 15-membered spiro-heterocyclyl, such as 3-azaspiro[5.5]undecanyl or 7-azaspiro[3.5]nonanyl); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof).

Embodiment 42): Pertaining to the compound of Formula I or salts, enantiomers, stereoisomers, polymorphs or solvates thereof as described in embodiment 37), wherein $R_{a1}$ is unsubstituted or substituted heteroaryl. When the $R_{a1}$ is unsubstituted or substituted heteroaryl, the unsubstituted or substituted heteroaryl is unsubstituted or substituted 5- to 30-membered heteroaryl (optionally, the unsubstituted or substituted 5- to 30-membered heteroaryl is unsubstituted or substituted 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered heteroaryl). Optionally, the heteroaryl includes but is not limited to pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, tetrazolyl, pyranyl, thiopyranyl, pyrazinyl, thiazinyl, triazinyl, tetrazinyl, quinolinyl, isoquinolinyl, quinazolinyl, quinoxalinyl, cinnolinyl, naphthyridinyl, acridinyl, xanthenyl, phenanthridinyl, phthalazinyl, triazaindenyl, indolyl, indolinyl, indolizinyl, phthalazinyl, pyrazolopyridyl, pyrazolopyrimidinyl, pyridopyrimidinyl, pyridopyrazinyl, pyrazinopyrazinyl, benzothiazolyl, benzoxazolyl, benzimidazolyl, benzothienyl, benzofuranyl, isobenzofuranyl, dibenzothienyl, dibenzofuranyl, indazolyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, pyrrolopyridinyl, pyrrolothiazolyl, imidazothiazolyl, pyrenyl, dinaphthofuranyl, naphthobenzofuranyl, dinaphthothienyl, or naphthobenzothienyl etc. Wherein the heteroaryl is unsubstituted or substituted. The substituted heteroaryl refers to a

heteroaryl substituted one or more times (e.g., 1-4, 1-3, or 1-2 times) with a substituent optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, or any combination thereof (preferably, the substituent is optionally selected from the group consisting of $C_1$-$C_3$ alkyl, $C_3$-$C_6$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, unsubstituted or substituted cycloalkyl, unsubstituted or substituted aryl, unsubstituted or substituted heteroaryl, unsubstituted or substituted heterocyclyl, or any combination thereof). Further, when $R_{a1}$ is substituted heteroaryl and the substituent of heteroaryl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.) (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, or $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged heterocyclyl (e.g., $C_3$-$C_{15}$ bridged heterocyclyl, $C_5$-$C_{15}$ bridged heterocyclyl, or $C_7$-$C_{15}$ bridged heterocyclyl, such as adamantanyl, noradamantanyl, bornyl, bicyclo[2.2.1]heptyl, 2-oxobicyclo[2.2.1]heptyl, or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a1}$ is substituted heteroaryl and the substituent of heteroaryl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.) (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof. Further, when $R_{a1}$ is substituted heteroaryl and the substituent of heteroaryl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl, or unsubstituted or substituted 5- to 10-membered heteroaryl) (optionally, the unsubstituted or substituted heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl; and the unsubstituted or substituted heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $R_{a1}$ is substituted heteroaryl and the substituent of heteroaryl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptanyl, diazacycloheptanyl, diazacyclooctyl, bridged heterocyclyl (e.g., 4- to 15-membered bridged heterocyclyl or 5- to 15-membered bridged

heterocyclyl or 7- to 15-membered bridged heterocyclyl, such as azabicyclohexyl, azabicycloheptanyl, diazabicycloheptanyl, or azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_5$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 43): Pertaining to the compound of Formula I or salts, enantiomers, stereoisomers, polymorphs or solvates thereof as described in embodiment 37), wherein $R_{a1}$ is unsubstituted or substituted cycloalkyl. When the $R_{a1}$ is unsubstituted or substituted cycloalkyl, the unsubstituted or substituted cycloalkyl is unsubstituted or substituted $C_3$-$C_{20}$, $C_3$-$C_{15}$, $C_3$-$C_{12}$, $C_3$-$C_{11}$, $C_3$-$C_{10}$, $C_3$-$C_8$, $C_3$-$C_7$, $C_3$-$C_6$ cycloalkyl etc. Optionally, the cycloalkyl includes but is not limited to cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl) etc. Wherein, the "cycloalkyl" is optionally substituted, and the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, amino, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, unsubstituted or substituted cycloalkyl, deuterated cycloalkyl, alkenyl, unsubstituted or substituted aryl, unsubstituted or substituted heteroaryl, unsubstituted or substituted heterocyclyl, aralkyl, arylalkenyl, alkyl aryl, alkylamino, aralkylamino, heteroarylamino, arylamino, arylphosphino, acenaphthenyl, oxo, or any combination thereof. Further, the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, unsubstituted or substituted cycloalkyl, unsubstituted or substituted aryl, unsubstituted or substituted heteroaryl, unsubstituted or substituted heterocyclyl, or any combination thereof. Further, when $R_{a1}$ is substituted cycloalkyl and the substituent of cycloalkyl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.) (optionally, the unsubstituted or substituted cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged heterocyclyl (e.g., $C_3$-$C_{15}$ bridged heterocyclyl or $C_5$-$C_{15}$ bridged heterocyclyl or $C_7$-$C_{15}$ bridged heterocyclyl, such as adamantanyl, noradamantanyl, bornyl, bicyclo[2.2.1]heptyl, 2-oxobicyclo[2.2.1]heptyl or bicyclo[2.2.1]heptentyl); wherein when the unsubstituted or substituted cycloalkyl is substituted cycloalkyl, the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof). Further, when $R_{a1}$ is substituted cycloalkyl and the substituent of cycloalkyl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.) (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a1}$ is substituted cycloalkyl and the substituent of cycloalkyl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl, or unsubstituted or substituted 5- to 10-membered heteroaryl) (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a1}$ is substituted cycloalkyl and the substituent of cycloalkyl is unsubstituted or substituted heterocyclyl, the heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl, or 5- to 15-membered bridged heterocyclyl, or 7- to 15-membered bridged heterocyclyl, such as azabicyclohexyl, azabicycloheptanyl, diazabicycloheptanyl, and azabicyclooctyl), or spiro-heterocyclyl (e.g., 4- to 15-membered spiro-heterocyclyl, or 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 44): Pertaining to the compound of Formula I or salts, enantiomers, stereoisomers, polymorphs or solvates thereof as described in embodiment 37), wherein $R_{a1}$ is unsubstituted or substituted heterocyclyl. When the $R_{a1}$ is unsubstituted or substituted heterocyclyl, the unsubstituted or substituted heterocyclyl is unsubstituted or substituted 4- to 30-membered heterocyclyl, 4- to 25-membered heterocyclyl, 4- to 20-membered heterocyclyl, 4- to 15-membered heterocyclyl, 4- to 10-membered heterocyclyl, 10- to 15-membered heterocyclyl, 5- to 15-membered heterocyclyl, 7- to 15-membered heterocyclyl etc. Optionally, the heterocyclyl contains 1-3 heteroatoms, or 1-2 heteroatoms, or 1 heteroatom etc, with each heteroatom independently selected from the group consisting of O, N, and S etc. Further optionally, the heterocyclyl includes but is not limited to azetidinyl, oxetanyl, pyrrolidinyl, imidazolyl, pyrazolidyl, triazolyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl (e.g., 1, 4-diazacycloheptanyl, 4, 5-diazacycloheptanyl, or 1, 3-diazacycloheptanyl etc.), azabicyclohexyl (e.g., 3-azabicyclo[3.1.0]hexyl etc.), azabicycloheptanyl (e.g., 3-azabicyclo[3.1.1]heptanyl, 3-azabicyclo[3.2.0]heptanyl etc.), azabicyclooctyl (e.g., cis-7-azabicyclo[3.3.0]octyl etc.), and spiro-heterocyclyl (7-azaspiro[3.5]nonanyl, 3-azaspiro[5,5]undecanyl etc.). The heterocyclyl may be unsubstituted or substituted as explicitly defined (e.g., mono-, di-, tri-, or poly-substituted) by a substituent optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, oxo and any combination thereof (preferably, the substituent is selected from the group consisting of deuterium, hydroxy, amino, mercapto, nitro, halogen, cyano, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl, and any combination thereof). Further, when $R_{a1}$ is substituted heterocyclyl and the substituent of heterocyclyl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl or $C_5$-$C_{15}$ bridged cycloalkyl or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is

optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a1}$ is substituted heterocyclyl and the substituent of heterocyclyl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a1}$ is substituted heterocyclyl and the substituent of heterocyclyl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a1}$ is substituted heterocyclyl and the substituent of heterocyclyl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.) (optionally, the unsubstituted or substituted heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl); and the unsubstituted or substituted heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof).

Embodiment 45): Pertaining to the compound of Formula I or salts, enantiomers, stereoisomers, polymorphs or solvates thereof as described in any one of embodiments 1)-44), wherein $R_{a2}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl, or $R_{a2}$ is absent; and when $X_3$ represents CO, $R_{a2}$ is absent and $R_{a1}$ is neither hydrogen nor deuterium, and when $R_{a2}$ represents hydrogen or deuterium, $X_3$ represents $CR_1$ or N, and $R_{a1}$ is neither hydrogen nor deuterium.

Embodiment 46): Pertaining to the compound of Formula I or salts, enantiomers, stereoisomers, polymorphs or solvates thereof as described in embodiment 45), wherein $R_{a2}$ is selected from the group consisting of hydrogen, deuterium, unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl, or $R_{a2}$ is absent.

Embodiment 47): Pertaining to the compound of Formula I or salts, enantiomers, stereoisomers, polymorphs or

solvates thereof as described in embodiment 45) or 46), wherein $R_{a2}$ is selected from the group consisting of hydrogen, deuterium, unsubstituted or substituted $C_5$-$C_{20}$ aryl, unsubstituted or substituted 5- to 20-membered heteroaryl, unsubstituted or substituted $C_3$-$C_{20}$ cycloalkyl, or unsubstituted or substituted 4- to 20-membered heterocyclyl, or $R_{a2}$ is absent.

Embodiment 48): Pertaining to the compound of Formula I or salts, enantiomers, stereoisomers, polymorphs or solvates thereof as described in any one of embodiments 45)-47), wherein $R_{a2}$ is selected from the group consisting of hydrogen.

Embodiment 49): Pertaining to the compound of Formula I or salts, enantiomers, stereoisomers, polymorphs or solvates thereof as described in any one of embodiments 45)-47), wherein $R_{a2}$ is selected from the group consisting of deuterium.

Embodiment 50): Pertaining to the compound of Formula I or salts, enantiomers, stereoisomers, polymorphs or solvates thereof as described in embodiment 45), wherein $R_{a2}$ is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.). The aryl may be monocyclic or polycyclic aryl. In some embodiments, the monocyclic aryl includes but is not limited to phenyl, biphenyl, terphenyl, quaterphenyl, and quinquephenyl etc. The polycyclic aryl includes but is not limited to naphthyl, anthryl, phenanthryl, pyrenyl, perylenyl, and fluorenyl etc. The fluorenyl may be substituted (e.g., 9,9'-dimethylfluorenyl and 9,9'-diphenylfluorenyl etc.). Preferably, $R_{a2}$ is selected from the group consisting of biphenyl, terphenyl, naphthyl, anthryl, phenanthryl, fluorenyl, and 9,9'-dimethylfluorenyl. Furthermore, two of the substituents may combine with each other to form a spirocyclic structure such as 9,9'-spirobifluorenyl etc. The aryl is optionally substituted aryl. The substituted aryl refers to an aryl group substituted one or more times (e.g., 1-4, 1-3, or 1-2 times) with a substituent. For example, aryl is mono-, di-, or tri-substituted with a substituent optionally selected from the group consisting of e.g., deuterated $C_1$-$C_6$ alkyl, deuterated $C_3$-$C_6$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, unsubstituted or substituted cycloalkyl, alkenyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof (preferably, the substituent is selected from the group consisting of deuterated $C_1$-$C_6$ alkyl, deuterated $C_3$-$C_6$ cycloalkyl, hydroxy, amino, mercapto, halogen, cyano, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl, and any combination thereof). Further, when $R_{a2}$ is substituted aryl and the substituent of aryl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.) (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl or $C_5$-$C_{15}$ bridged cycloalkyl or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted aryl and the substituent of aryl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.) (optionally, the unsubstituted or substituted aryl is selected from the group consisting of biphenyl, terphenyl, naphthyl, anthryl, phenanthryl, fluorenyl, or 9,9'-dimethylfluorenyl etc.); and the unsubstituted or substituted aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $R_{a2}$ is substituted aryl and the substituent of aryl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl) (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl,

tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted aryl and the substituent of aryl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.) (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 51): Pertaining to the compound of Formula I or salts, enantiomers, stereoisomers, polymorphs or solvates thereof as described in embodiment 45), wherein $R_{a2}$ is selected from the group consisting of unsubstituted or substituted 5- to 25-membered heteroaryl (preferably, unsubstituted or substituted 5- to 20-membered heteroaryl, or unsubstituted or substituted 5- to 15-membered heteroaryl); more preferably, $R_{a2}$ is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl. Wherein the heteroaryl may be unsubstituted or substituted. The substituted heteroaryl refers to a heteroaryl substituted one or more times (e.g., 1-4, 1-3, or 1-2 times) with a substituent optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, or any combination thereof (preferably, the substituent is optionally selected from the group consisting of $C_1$-$C_3$ alkyl, $C_3$-$C_6$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, unsubstituted or substituted cycloalkyl, unsubstituted or substituted aryl, unsubstituted or substituted heteroaryl, unsubstituted or substituted heterocyclyl, or any combination thereof). Further, when $R_{a1}$ is substituted heteroaryl and the substituent of heteroaryl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.) (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl or $C_5$-$C_{15}$ bridged cycloalkyl or $C_7$-$C_{15}$

bridged cycloalkyl, such as adamantanyl, noradamantanyl, bornyl, bicyclo[2.2.1]heptyl, 2-oxobicyclo[2.2.1]heptyl or bicyclo[2.2.1]heptentyl); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted heteroaryl and the substituent of heteroaryl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.) (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted heteroaryl and the substituent of heteroaryl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl) (optionally, the unsubstituted or substituted heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl; and the unsubstituted or substituted heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $R_{a2}$ is substituted heteroaryl and the substituent of heteroaryl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.) (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 52): Pertaining to the compound of Formula I or salts, enantiomers, stereoisomers, polymorphs or solvates thereof as described in embodiment 45), wherein $R_{a2}$ is selected from the group consisting of unsubstituted or substituted 4- to 20-membered heterocyclyl (e.g., unsubstituted or substituted 4- to 15-membered heterocyclyl); preferably, $R_{a2}$ is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl). The heterocyclyl may be unsubstituted or substituted pyrrolidinyl,citly defined (e.g., mono-, di-, tri-, or poly-substituted) by a substituent optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof (preferably, the substituent is selected from the group consisting of deuterium, hydroxy, amino, mercapto, nitro, halogen, cyano, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_1$-$C_6$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocycly,1 and any combination thereof). Further, when $R_{a2}$ is substituted heterocyclyl and the substituent of heterocyclyl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl or $C_5$-$C_{15}$ bridged cycloalkyl or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted heterocyclyl and the substituent of heterocyclyl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted heterocyclyl and the substituent of heterocyclyl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted heterocyclyl and the substituent of heterocyclyl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.) (optionally, the unsubstituted or substituted heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl,

dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl); and the unsubstituted or substituted heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof).

Embodiment 53): Pertaining to the compound of Formula I or salts, enantiomers, stereoisomers, polymorphs or solvates thereof as described in embodiment 45), wherein $R_{a2}$ is selected from the group consisting of unsubstituted or substituted $C_3$-$C_{20}$ cycloalkyl (e.g., unsubstituted or substituted $C_3$-$C_{15}$ cycloalkyl); preferably, $R_{a2}$ is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl or $C_5$-$C_{15}$ bridged cycloalkyl or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl). Wherein the "cycloalkyl" is optionally substituted, and the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, amino, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, unsubstituted or substituted cycloalkyl, deuterated cycloalkyl, alkenyl, unsubstituted or substituted aryl, unsubstituted or substituted heteroaryl, unsubstituted or substituted heterocyclyl, aralkyl, arylalkenyl, alkyl aryl, alkylamino, aralkylamino, heteroarylamino, arylamino, arylphosphino, acenaphthenyl, oxo or any combination thereof. Further, the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, unsubstituted or substituted cycloalkyl, unsubstituted or substituted aryl, unsubstituted or substituted heteroaryl, unsubstituted or substituted heterocyclyl, or any combination thereof. Further, when $R_{a2}$ is substituted cycloalkyl and the substituent of cycloalkyl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.) (optionally, the unsubstituted or substituted cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl or $C_5$-$C_{15}$ bridged cycloalkyl or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl); and the unsubstituted or substituted cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof). Further, when $R_{a2}$ is substituted cycloalkyl and the substituent of cycloalkyl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof. Further, when $R_{a2}$ is substituted cycloalkyl and the substituent of cycloalkyl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl,

triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $R_{a2}$ is substituted cycloalkyl and the substituent of cycloalkyl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 54): Pertaining to the compound of Formula I or salts, enantiomers, stereoisomers, polymorphs or solvates thereof as described in any one of embodiments 45)-53), wherein the substituents of the substituted $R_{a2}$ are optionally one or more selected from the group consisting of deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, linear or branched $C_1$-$C_5$ alkyl, halogenated $C_1$-$C_5$ alkyl, optionally substituted alkyl, optionally substituted aryl, optionally substituted heteroaryl, and optionally substituted heterocyclyl. Further, the optionally substituted alkyl is e.g., optionally substituted $C_1$-$C_{30}$ alkyl, optionally substituted $C_1$-$C_{20}$ alkyl, optionally substituted $C_1$-$C_{15}$ alkyl, and optionally substituted $C_1$-$C_{10}$ alkyl etc., wherein the substituents of the optionally substituted alkyl are e.g., selected from the group consisting of $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. The optionally substituted aryl is e.g., optionally substituted $C_5$-$C_{30}$ aryl, optionally substituted $C_5$-$C_{25}$ aryl, optionally substituted $C_5$-$C_{15}$ aryl, and optionally substituted $C_5$-$C_{10}$ aryl etc., wherein the substituent of the optionally substituted aryl is e.g., selected from the group consisting of $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. The optionally substituted heteroaryl is e.g., optionally substituted 5- to 30-membered heteroaryl, unsubstituted or substituted 5- to 15-membered, 5- to 12-membered, 5- to 11-membered, 5- to 10-membered, 5- to 9-membered, 5- to 8-membered, 5- to 7-membered, 5- to 6-membered, 6- to 15-membered or 6- to 9-membered heteroaryl, wherein the substituent of the optionally substituted heteroaryl is e.g., selected from the group consisting of $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. The optionally substituted heterocyclyl is optionally substituted 4- to 30-membered heterocyclyl, optionally substituted 4- to 25-membered heterocyclyl, optionally substituted 4- to 20-membered heterocyclyl, optionally substituted 4- to 15-membered heterocyclyl, optionally substituted 4- to 10-membered heterocyclyl, or optionally substituted 10- to 15-membered heterocyclyl, wherein the substituent of the optionally substituted heterocyclyl is e.g., selected from the group consisting of $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 55): Pertaining to the compound of Formula I or salts, enantiomers, stereoisomers, polymorphs or solvates thereof as described in embodiment 45), wherein $R_{a2}$ is absent.

Embodiment 56): Pertaining to the compound of Formula I or salts, enantiomers, stereoisomers, polymorphs or solvates thereof as described in embodiment 45), wherein $R_{a1}$ and $R_{a2}$ cannot simultaneously be hydrogen or deuterium.

Embodiment 57): Pertaining to the compound of Formula I or salts, enantiomers, stereoisomers, polymorphs or solvates thereof as described in embodiment 45), wherein when $R_{a2}$ is absent, $X_3$ represents CO.

Embodiment 58): Pertaining to the compound of Formula I or salts, enantiomers, stereoisomers, polymorphs or solvates thereof as described in any one of embodiments 1)-53) and 55), wherein $R_{a2}$ is selected from the group consisting of

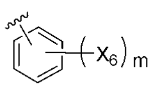 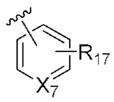 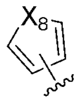 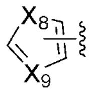 or 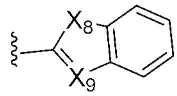 ;

wherein $X_6$ is selected from the group consisting of deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, halogenated $C_1$-$C_6$ alkyl, or linear or branched $C_1$-$C_6$ alkyl. Optionally, halogen may be e.g., F, Cl, Br, I. Optionally, $C_1$-$C_6$ alkoxy may include, but is not limited to, e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like. Optionally, tne halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino, and the like. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_6$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, and tert-penty letc, and the linear or branched $C_1$-$C_6$ alkyl is optionally substituted with one or more substituents optioanlly selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl) or any combination thereof;

m is an integer of 0, 1, 2, 3, 4 or 5, and when m is an integer of 2, 3, 4 or 5, each $X_6$ is independently identical to or different from each other;

$X_7$ and $X_9$ are each independently selected from the group consisting of CH or N;

each $X_8$ is independently selected from the group consisting of O, S or NH;

$R_{17}$ is selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, halogenated $C_1$-$C_5$ alkyl, or linear or branched $C_1$-$C_5$ alkyl, wherein the halogen may optionally be e.g., F, Cl, Br, or I. Optionally, the $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like. Optionally, the halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino, and the like. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_5$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, and tert-pentyl etc., and the linear or branched $C_1$-$C_5$ alkyl is optionally substituted with one or more substituents optioanlly selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl), or any combination thereof.

Embodiment 59): Pertaining to the compound of Formula I or salts, enantiomers, stereoisomers, polymorphs or solvates thereof as described in any one of embodiments 1)-58), wherein $R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl.

Embodiment 60): Pertaining to the compound of Formula I or salts, enantiomers, stereoisomers, polymorphs or solvates thereof as described in embodiment 59), wherein $R_a$ is selected from the group consisting of deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl.

Embodiment 61): Pertaining to the compound of Formula I or salts, enantiomers, stereoisomers, polymorphs or solvates thereof as described in embodiment 59) or 60), wherein $R_a$ is selected from the group consisting of deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl.

Embodiment 62): Pertaining to the compound of Formula I or salts, enantiomers, stereoisomers, polymorphs or solvates thereof as described in embodiment 59), wherein $R_a$ is deuterium.

Embodiment 63): Pertaining to the compound of Formula I or salts, enantiomers, stereoisomers, polymorphs or solvates thereof as described in embodiment 59), wherein $R_a$ is halogen, which is optionally F, Cl, Br, or I.

Embodiment 64): Pertaining to the compound of Formula I or salts, enantiomers, stereoisomers, polymorphs or solvates thereof as described in embodiment 59), wherein $R_a$ is nitro.

Embodiment 65): Pertaining to the compound of Formula I or salts, enantiomers, stereoisomers, polymorphs or solvates thereof as described in embodiment 59), wherein $R_a$ is cyano.

Embodiment 66): Pertaining to the compound of Formula I or salts, enantiomers, stereoisomers, polymorphs or solvates thereof as described in embodiment 59), wherein $R_a$ is amino.

Embodiment 67): Pertaining to the compound of Formula I or salts, enantiomers, stereoisomers, polymorphs or solvates thereof as described in embodiment 59), wherein $R_a$ is hydroxy.

Embodiment 68): Pertaining to the compound of Formula I or salts, enantiomers, stereoisomers, polymorphs or solvates thereof as described in embodiment 59), wherein $R_a$ is mercapto.

Embodiment 69): Pertaining to the compound of Formula I or salts, enantiomers, stereoisomers, polymorphs or solvates thereof as described in embodiment 59), wherein $R_a$ is alkoxy. Optionally, when the $R_a$ is alkoxy, the alkoxy is $C_1$-$C_6$ alkoxy. $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like.

Embodiment 70): Pertaining to the compound of Formula I or salts, enantiomers, stereoisomers, polymorphs or solvates thereof as described in embodiment 59), wherein $R_a$ is halogenated alkoxy. Optionally, when the $R_a$ is halogenated alkoxy, the halogenated alkoxy is halogenated $C_1$-$C_6$ alkoxy; further optionally the halogenated $C_1$-$C_6$ alkoxy is trifluoromethoxy etc.

Embodiment 71): Pertaining to the compound of Formula I or salts, enantiomers, stereoisomers, polymorphs or solvates thereof as described in embodiment 59), wherein $R_a$ is unsubstituted or substituted amino. Optionally, the unsubstituted or substituted amino includes amino, N-methylamino, N-ethylamino, N-phenylamino, N,N-dimethylamino, and N,N-diethylamino, and the like.

Embodiment 72): Pertaining to the compound of Formula I or salts, enantiomers, stereoisomers, polymorphs or solvates thereof as described in embodiment 59), wherein $R_a$ is unsubstituted or substituted silyl. Optionally, the unsubstituted or substituted silyl is specifically trimethylsilyl, triethylsilyl, tert-butyldimethylsilyl, vinyldimethylsilyl, propyldimethylsilyl, triphenylsilyl, diphenylsilyl, and phenylsilyl etc.

Embodiment 73): Pertaining to the compound of Formula I or salts, enantiomers, stereoisomers, polymorphs or solvates thereof as described in embodiment 59), wherein $R_a$ is unsubstituted or substituted boryl. Optionally, the unsubstituted or substituted boryl is specifically trimethylboryl, triethylboryl, tert-butyldimethylboryl, triphenylboryl, and phenylboryl etc.

Embodiment 74): Pertaining to the compound of Formula I or salts, enantiomers, stereoisomers, polymorphs or solvates thereof as described in embodiment 59), wherein $R_a$ is unsubstituted or substituted linear or branched alkyl. Optionally, the number of carbon atoms may be for example $C_1$-$C_{30}$, $C_1$-$C_{25}$, $C_1$-$C_{20}$, $C_1$-$C_{15}$, $C_1$-$C_{10}$, $C_1$-$C_5$, $C_1$-$C_3$ etc, with linear or branched $C_1$-$C_{10}$ alkyl being preferred. Further optionally, the linear or branched $C_1$-$C_{10}$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, nonyl, 2,2-dimethylheptyl, 1-ethylpropyl, 1,1-dimethylpropyl, isohexyl, 4-methylhexyl, or 5-methylhexyl etc. Optionally, the linear or branched $C_1$-$C_{10}$ alkyl includes but is not limited to methyl, ethyl, and propyl etc. Wherein the alkyl is optionally substituted, and the substituent may be e.g., one or more optionally selected from the group consisting of deuterium, halogen, cyano, nitro, hydroxy, mercapto, carbonyl, ester group, imide group,

amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo, or any combination thereof. The substituent is preferably one or more selected from the group consisting of D (i.e., deuterium), halogen, hydroxy, cyano, $C_{1-3}$ alkyl, $C_{1-3}$ alkoxy, trifluoromethyl, heterocyclyl (e.g., 4- to 8-membered heterocyclyl) or any combination thereof.

Embodiment 75): Pertaining to the compound of Formula I or salts, enantiomers, stereoisomers, polymorphs or solvates thereof as described in embodiment 59), wherein $R_a$ is unsubstituted or substituted cycloalkyl. Optionally, when the $R_a$ is unsubstituted or substituted cycloalkyl, it has 3 to 20 carbon atoms (i.e., $C_{3-20}$ cycloalkyl), 3 to 15 carbon atoms (i.e., $C_{3-15}$ cycloalkyl), 3 to 12 carbon atoms (i.e., $C_{3-12}$ cycloalkyl), 3 to 11 carbon atoms (i.e., $C_{3-11}$ cycloalkyl), 3 to 10 carbon atoms (i.e., $C_{3-10}$ cycloalkyl), 3 to 8 carbon atoms (i.e., $C_{3-8}$ cycloalkyl), 3 to 7 carbon atoms (i.e., $C_{3-7}$ cycloalkyl), or 3 to 6 carbon atoms (i.e., $C_{3-6}$ cycloalkyl). $R_1$ is preferably unsubstituted or substituted $C_3$-$C_{15}$ cycloalkyl. Optionally, $C_3$-$C_{15}$ cycloalkyl includes but is not limited to cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl) etc. Further optionally, $C_3$-$C_{15}$ cycloalkyl includes but is not limited to cyclopropyl, cyclobutyl, cyclopentyl etc. Wherein the cycloalkyl is optionally substituted with one or more (e.g., 1-5, 1-4, 1-3, 1-2, or 1) substituents optioanlly selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, amino, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, acenaphthenyl, oxo, or any combination thereof. The substituents are preferably one or more selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl or any combination thereof.

Embodiment 76): Pertaining to the compound of Formula I or salts, enantiomers, stereoisomers, polymorphs or solvates thereof as described in embodiment 59), wherein $R_a$ is unsubstituted or substituted heterocyclyl. Optionally, when the $R_a$ is unsubstituted or substituted heterocyclyl, the unsubstituted or substituted heterocyclyl is unsubstituted or substituted 4- to 30-membered heterocyclyl, 4- to 25-membered heterocyclyl, 4- to 20-membered heterocyclyl, 4- to 15-membered heterocyclyl, 4- to 10-membered heterocyclyl, 10- to 15-membered heterocyclyl, 5- to 15-membered heterocyclyl, or 7- to 15-membered heterocyclyl etc. Optionally, the heterocyclyl contains 1-3 heteroatoms, or 1-2 heteroatoms, or 1 heteroatom etc, with each heteroatom independently selected from the group consisting of O, N, and S etc. Further optionally, the heterocyclyl includes but is not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl (e.g., 1, 4-diazacycloheptanyl, 4, 5-diazacycloheptanyl, or 1, 3-diazacycloheptanyl etc), azabicyclohexyl (e.g., 3-azabicyclo[3.1.0]hexyl etc), azabicycloheptanyl (e.g., 3-azabicyclo[3.1.1]heptanyl, 3-azabicyclo[3.2.0]heptanyl etc), azabicyclooctyl (e.g., cis-7-azabicyclo[3.3.0]octyl etc), and spiro-heterocyclyl (e.g., 4- to 15-membered spiro-heterocyclyl or 5- to 15-membered spiro-heterocyclyl or 7-to 15-membered spiro-heterocyclyl, such as 7-azaspiro[3.5]nonanyl, 3-azaspiro[5,5]undecanyl etc). The heterocyclyl may be unsubstituted or substituted as explicitly defined (e.g., mono-, di-, tri-, or poly-substituted) by a substituent optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_{3}$-$_{6}$ cycloalkyl, optionally deuterated $C_1$-$C_6$ alkoxy, $C_{1}$-$_{4}$ alkyl-NH-, $NH_2$-$C_1$-$_6$ alkylene, $C_1$-$_4$ alkyl-NHC(O)-, $C_1$-$_4$ alkyl-C(O)NH-, alkenyl, aryl, aralkyl, arylalkenyl, alkylaryl, aralkylamino, heteroarylamino, arylamino, arylphosphino, heteroaryl, acenaphthenyl, and any combination thereof. Preferably, the substituent is selected from the group consisting of deuterium, hydroxy, amino, mercapto, nitro, halogen, cyano, oxo, optionally deuterated $C_1$-$C_6$ alkyl, halogenated $C_1$-$C_6$ alkyl, optionally deuterated $C_3$-$_6$ cycloalkyl, optionally deuterated $C_1$-$C_6$ alkoxy, $C_1$-$_4$ alkyl-NH-, $NH_2$-$C_1$-$_6$ alkylene, $C_1$-$_4$ alkyl-NHC(O)-, $C_1$-$_4$ alkyl-C(O)NH-, and any combination thereof.

Embodiment 77): Pertaining to the compound of Formula I or salts, enantiomers, stereoisomers, polymorphs or solvates thereof as described in embodiment 59), wherein $R_a$ is unsubstituted or substituted aryl. Optionally, the unsubstituted or substituted aryl is unsubstituted or substituted $C_5$-$_{30}$ aryl. Optionally, the unsubstituted or substituted $C_5$-$_{30}$ aryl is unsubstituted or substituted $C_5$-$_{25}$, $C_5$-$_{20}$, or $C_5$-$_{15}$ aryl. Optionally, the $C_5$-$_{30}$ aryl may be monocyclic or polycyclic aryl. In some embodiments, monocyclic aryl includes but is not limited to phenyl, biphenyl, terphenyl, quaterphenyl, and quinquephenyl etc. Polycyclic aryl includes but is not limited to naphthyl, anthryl, phenanthryl, pyrenyl, perylenyl, and fluorenyl etc. Fluorenyl is optionally substituted, with examples including 9,9'-dimethylfluorenyl and 9,9'-diphenylfluorenyl etc. Furthermore, two of the substituents may combine with each other to form a spirocyclic structure such as 9,9'-spirobifluorenyl etc. The aryl is optionally substituted aryl. The substituted aryl refers to an aryl group substituted one or more times (e.g., 1-4, 1-3, or 1-2 times) with a substituent. For example, aryl is mono-, di-, or tri-substituted with a substituent optionally selected from the group consisting of e.g., deuterated $C_1$-$C_6$ alkyl, deuterated $C_{3-6}$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, cycloalkyl, alkenyl, aryl, aralkyl, arylalkenyl, alkylaryl, aralkylamino, heteroarylamino, arylamino, arylphosphino, heteroaryl, acenaphthenyl, and any combination thereof. Preferably, the substituent is selected from the group consisting of deuterated $C_1$-$C_6$ alkyl, deuterated $C_{3-6}$ cycloalkyl, hydroxy, amino, mercapto, halogen, cyano, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 78): Pertaining to the compound of Formula I or salts, enantiomers, stereoisomers, polymorphs or solvates thereof as described in embodiment 59), wherein $R_a$ is unsubstituted or substituted heteroaryl. When the $R_a$ is unsubstituted or substituted heteroaryl, the unsubstituted or substituted heteroaryl is unsubstituted or substituted 5- to 30-membered heteroaryl. Optionally, the unsubstituted or substituted 5- to 30-membered heteroaryl is unsubstituted or substituted 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered heteroaryl. Optionally, the heteroaryl includes but is not limited to pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, tetrazolyl, pyranyl, thiopyranyl, pyrazinyl, thiazinyl, triazinyl, tetrazinyl, quinolinyl, isoquinolinyl, quinazolinyl, quinoxalinyl, cinnolinyl, naphthyridinyl, acridinyl, xanthenyl, phenanthridinyl, phthalazinyl, triazaindenyl, indolyl, indolinyl, indolizinyl, phthalazinyl, pyrazolopyridyl, pyrazolopyrimidinyl, pyridopyrimidinyl, pyridopyrazinyl, pyrazinopyrazinyl, benzothiazolyl, benzoxazolyl, benzimidazolyl, benzothienyl, benzofuranyl, isobenzofuranyl, dibenzothienyl, dibenzofuranyl, indazolyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, pyrrolopyridinyl, pyrrolothiazolyl, imidazothiazolyl, pyrenyl, dinaphthofuranyl, naphthobenzofuranyl, dinaphthothienyl, and naphthobenzothienyl etc. Wherein the heteroaryl is unsubstituted or substituted. The substituted heteroaryl refers to a heteroaryl substituted one or more times (e.g., 1-4, 1-3, or 1-2 times) with a substituent optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, aryl, aralkyl, arylalkenyl, alkylaryl, aralkylamino, heteroarylamino, arylamino, arylphosphino, heteroaryl, acenaphthenyl, or any combination thereof. Preferably, the substituent is optionally selected from the group consisting of $C_1$-$C_3$ alkyl, $C_3$-$C_6$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, or any combination thereof.

Embodiment 79): Pertaining to the compound of Formula I or salts, enantiomers, stereoisomers, polymorphs or solvates thereof as described in any one of embodiments 1)-78), wherein $R_{b1}$, $R_{b2}$, $R_{b3}$, $R_{b4}$, and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, and unsubstituted or substituted linear or branched alkyl.

Embodiment 80): Pertaining to the compound of Formula I or salts, enantiomers, stereoisomers, polymorphs or solvates thereof as described in any one of embodiments 1)-78), wherein $R_{b1}$, $R_{b2}$, $R_{b3}$, $R_{b4}$, and $R_{b5}$ are each independently selected from the group consisting of hydrogen, deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, or unsubstituted or substituted linear or branched $C_1$-$C_5$ alkyl.

Embodiment 81): Pertaining to the compound of Formula I or salts, enantiomers, stereoisomers, polymorphs or solvates thereof as described in any one of embodiments 1)-78), wherein $R_{b1}$, $R_{b2}$, $R_{b3}$, $R_{b4}$, and $R_{b5}$ are each independently selected from the group consisting of hydrogen, deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, or unsubstituted or substituted linear or branched $C_1$-$C_5$ alkyl.

Embodiment 82): Pertaining to the compound of Formula I or salts, enantiomers, stereoisomers, polymorphs or solvates thereof as described in any one of embodiments 1)-78), wherein $R_{b1}$, $R_{b2}$, $R_{b3}$, $R_{b4}$, and $R_{b5}$ are each independently selected from the group consisting of hydrogen.

Embodiment 83): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of deuterium.

Embodiment 84): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of halogen. Optionally, when the $R_{b1}$, $R_{b2}$, $R_{b3}$, $R_{b4}$, and $R_{b5}$ are each independently selected from the group consisting of halogen, the halogen is F, Cl, Br, or I.

Embodiment 85): Pertaining to the compound of Formula I or salts, enantiomers, stereoisomers, polymorphs or

solvates thereof as described in embodiment 81), wherein $R_{b1}$, $R_{b2}$, $R_{b3}$, $R_{b4}$, and $R_{b5}$ are each independently selected from the group consisting of nitro.

Embodiment 86): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of cyano.

Embodiment 87): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of amino.

Embodiment 88): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of hydroxy.

Embodiment 89): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of mercapto.

Embodiment 90): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of alkoxy. Optionally, when the $R_{b1}$, $R_{b2}$, $R_{b3}$, $R_{b4}$, and $R_{b5}$ are each independently selected from the group consisting of alkoxy, the alkoxy is $C_1$-$C_6$ alkoxy. Further optionally, the alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like.

Embodiment 91): Pertaining to the compound of Formula I or salts, enantiomers, stereoisomers, polymorphs or solvates thereof as described in embodiment 81), wherein $R_{b1}$, $R_{b2}$, $R_{b3}$, $R_{b4}$, and $R_{b5}$ are each independently selected from the group consisting of halogenated alkoxy. Optionally, when the $R_{b1}$, $R_{b2}$, $R_{b3}$, $R_{b4}$, and $R_{b5}$ are each independently selected from the group consisting of halogenated alkoxy, the halogenated alkoxy is halogenated $C_1$-$C_6$ alkoxy. Further, the halogenated $C_1$-$C_6$ alkoxy is trifluoromethoxy etc.

Embodiment 92): Pertaining to the compound of Formula I or salts, enantiomers, stereoisomers, polymorphs or solvates thereof as described in embodiment 81), wherein the $R_{b1}$, $R_{b2}$, $R_{b3}$, $R_{b4}$, and $R_{b5}$ are each independently selected from the group consisting of unsubstituted or substituted amino. Optionally, the unsubstituted or substituted amino includes amino, N-methylamino, N-ethylamino, N-phenylamino, N,N-dimethylamino, and N,N-diethylamino, and the like.

Embodiment 93): Pertaining to the compound of Formula I or salts, enantiomers, stereoisomers, polymorphs or solvates thereof as described in embodiment 81), wherein the $R_{b1}$, $R_{b2}$, $R_{b3}$, $R_{b4}$, and $R_{b5}$ are each independently selected from the group consisting of unsubstituted or substituted silyl. Optionally, the unsubstituted or substituted silyl is specifically trimethylsilyl, triethylsilyl, tert-butyldimethylsilyl, vinyldimethylsilyl, propyldimethylsilyl, triphenylsilyl, diphenylsilyl, and phenylsilyl etc.

Embodiment 94): Pertaining to the compound of Formula I or salts, enantiomers, stereoisomers, polymorphs or solvates thereof as described in embodiment 81), wherein the $R_{b1}$, $R_{b2}$, $R_{b3}$, $R_{b4}$, and $R_{b5}$ are each independently selected from the group consisting of unsubstituted or substituted boryl. Optionally, the unsubstituted or substituted boryl is specifically trimethylboryl, triethylboryl, tert-butyldimethylboryl, triphenylboryl, and phenylboryl etc.

Embodiment 95): Pertaining to the compound of Formula I or salts, enantiomers, stereoisomers, polymorphs or solvates thereof as described in embodiment 81), wherein the $R_{b1}$, $R_{b2}$, $R_{b3}$, $R_{b4}$, and $R_{b5}$ are each independently selected from the group consisting of unsubstituted or substituted linear or branched alkyl. Optionally, the number of carbon atoms may be for example $C_1$-$C_{30}$, $C_1$-$C_{25}$, $C_1$-$C_{20}$, $C_1$-$C_{15}$, $C_1$-$C_{10}$, $C_1$-$C_5$ or $C_1$-$C_3$ etc, with linear or branched $C_1$-$C_{10}$ alkyl being preferred. Further optionally, the linear or branched $C_1$-$C_{10}$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, nonyl, 2,2-dimethylheptyl, 1-ethylpropyl, 1,1-dimethylpropyl, isohexyl, 4-methylhexyl, and 5-methylhexyl etc. Optionally, the linear or branched $C_1$-$C_{10}$ alkyl includes but is not limited to methyl, ethyl, and propyl etc. Wherein the alkyl is optionally substituted, and the substituent may be e.g., selected from the group consisting of deuterium, halogen, cyano, nitro, hydroxy, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo or any combination thereof. The substituent is preferably one or more selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl) or any combination thereof.

Embodiment 96): Pertaining to the compound of Formula I or salts, enantiomers, stereoisomers, polymorphs or solvates thereof as described in any one of embodiments 1)-95), wherein n is an integer of 0, 1, 2 or 3, and when n is an

integer of 2 or 3, each $R_a$ is independently identical to or different from each other. In some embodiments, n is an integer of 0, 1 or 2.

Embodiment 97): Pertaining to the compound of Formula I or salts, enantiomers, stereoisomers, polymorphs or solvates thereof as described in any one of embodiments 1)-96), wherein for better characterization of $R_{a1}$, it is further represented by $-L_2-L_3$ moiety. The chemical structure of said compound is shown in Formula I-1 or Formula I-2:

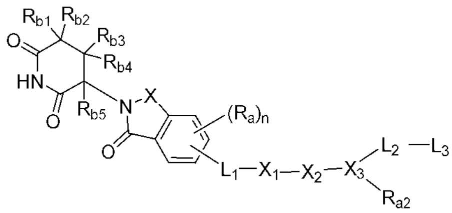

I-1

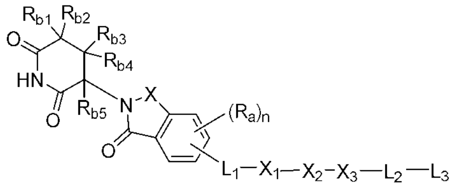

I-2

in Formula I-1 or Formula 1-2, X, $L_1$, $X_1$, $X_2$, $R_a$, $R_{b1}$, $R_{b2}$, $R_{b3}$, $R_{b4}$, $R_{b5}$, and n are as defined in the compounds of Formula I in any one of the preceding embodiments 1)-96);

$L_2$ is selected from the group consisting of unsubstituted or substituted $C_5$-$C_{30}$ arylene, unsubstituted or substituted 5- to 30-membered heteroarylene, unsubstituted or substituted $C_3$-$C_{30}$ cycloalkylene, or unsubstituted or substituted 4- to 30-membered heterocyclylene, or $L_2$ is absent;

$L_3$ is selected from the group consisting of unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl;

$R_{a2}$ is selected from the group consisting of unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl;

wherein in Formula 1-1, $X_3$ is selected from the group consisting of $CR_1$ or N, and in Formula 1-2, $X_3$ is selected from the group consisting of $CR_1$, N or CO, wherein $R_1$ is as defined in the compounds of Formula I in any one of the preceding embodiments 1)-96).

Embodiment 98): Pertaining to the compounds of Formula 1-1, Formula I-2 or salts, enantiomers, stereoisomers, polymorphs or solvates thereof as described in embodiment 97), wherein $L_2$ is selected from the group consisting of unsubstituted or substituted 5- to 20-membered heteroarylene (e.g., unsubstituted or substituted 5- to 15-membered heteroarylene); preferably, the heteroarylene is selected from the group consisting of pyridylene, pyrrolylene, pyrimidinylene, pyridazinylene, furanylene, thienylene, imidazolylene, pyrazolylene, oxazolylene, isoxazolylene, thiazolylene, isothiazolylene, triazolylene, oxadiazolylene, thiadiazolylene, tetrazolylene, pyranylene, thiopyranylene, pyrazinylene, pyridazinylene, thiazinylene, triazinylene, tetrazinylene, quinolinylene, isoquinolinylene, quinazolinylene, quinoxalinylene, cinnolinylene, naphthyridinylene, acridinylene, xanthenylene, phenanthridinylene, phthalazinylene, triazaindenylene, indolylene, indolinylene, indolizinylene, phthalazinylene, pyrazolopyridylene, pyrazolopyrimidinylene, pyridopyrimidinylene, pyridopyrazinylene, pyrazinopyrazinylene, benzothiazolylene, benzoxazolylene, benzimidazolylene, benzothienylene, benzofuranylene, isobenzofuranylene, dibenzothienylene, dibenzofuranylene, indazolylene, carbazolylene, benzocarbazolylene, dibenzocarbazolylene, indolocarbazolylene, indenocarbazolylene, phenazinylene, imidazopyridinylene, phenanthridinylene, phenanthrolinylene, phenothiazinylene, imidazopyridinylene, imidazophenanthridinylene, pyrrolopyridinylene, pyrrolothiazolylene, imidazothiazolylene, benzimidazoquinazolinylene, benzimidazophenanthridinylene, pyrenylene, dinaphthofuranylene, naphthobenzofuranylene, dinaphthothienylene, or naphthobenzothienylene. Wherein the heteroarylene may be unsubstituted or substituted as explicitly defined. The substituted heteroarylene refers to a heteroarylene substituted one or more times (e.g., 1-4, 1-3, or 1-2 times) with a substituent optionally selected

from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, or any combination thereof. Preferably, the substituent is optionally selected from the group consisting of $C_1$-$C_3$ alkyl, $C_3$-$C_6$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, unsubstituted or substituted cycloalkyl, unsubstituted or substituted aryl, unsubstituted or substituted heteroaryl, unsubstituted or substituted heterocyclyl, or any combination thereof. Further, when $L_2$ is substituted heteroarylene and the substituent of heteroarylene is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl and unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted heteroarylene and the substituent of heteroarylene is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted heteroarylene and the substituent of heteroarylene is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted heteroarylene and the substituent of heteroarylene is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4-to 15-membered bridged heterocyclyl, 5- to 15-membered bridged heterocyclyl, or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl, 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 99): Pertaining to the compounds of Formula I-1, Formula I-2 or salts, enantiomers, stereoisomers, polymorphs or solvates thereof as described in embodiment 97), wherein $L_2$ is selected from the group consisting of unsubstituted or substituted $C_3$-$C_{20}$ cycloalkylene (e.g., unsubstituted or substituted $C_3$-$C_{15}$ cycloalkylene); (preferably, $L_2$ is selected from the group consisting of cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_3$-$C_{15}$ spiro-cycloalkylene,$C_5$-$C_{15}$ spiro-cycloalkylene, or $C_7$-$C_{15}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), or bridged cycloalkylene (e.g., $C_3$-$C_{15}$ bridged cycloalkylene,$C_5$-$C_{15}$ bridged cycloalkylene, or $C_7$-$C_{15}$ bridged cycloalkylene, adamantanylene, noradamantanylene, bornylene, bicyclo[2.2.1]heptylene, 2-oxobicyclo[2.2.1]heptylene, or bicyclo[2.2.1]heptentylene)). Wherein the "cycloalkylene" is optionally substituted, and the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, unsubstituted or substituted cycloalkyl, deuterated cycloalkyl, alkenyl, unsubstituted or substituted aryl, unsubstituted or substituted heteroaryl, unsubstituted or substituted heterocyclyl, aralkyl, arylalkenyl, alkyl aryl, alkylamino, aralkylamino, heteroarylamino, arylamino, arylphosphino, acenaphthenyl, oxo or any combination thereof. Further, the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, unsubstituted or substituted cycloalkyl, unsubstituted or substituted aryl, unsubstituted or substituted heteroaryl, unsubstituted or substituted heterocyclyl, or any combination thereof. Further, when $L_2$ is substituted cycloalkylene and the substituent of cycloalkylene is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl,$C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl,$C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted cycloalkylene and the substituent of cycloalkylene is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted cycloalkylene and the substituent of cycloalkylene is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted cycloalkylene and the substituent of cycloalkylene is unsubstituted or substituted heterocyclyl, the heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl, 5- to 15-membered bridged heterocyclyl, or 7- to 15-membered bridged heterocyclyl, such as azabicyclohexyl, azabicycloheptanyl, diazabicycloheptanyl, azabicyclooctyl), or spiroheterocyclyl (e.g., 4- to 15-membered spiro-heterocyclyl, 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 100): Pertaining to the compounds of Formula I-1, Formula I-2 or salts, enantiomers, stereoisomers, polymorphs or solvates thereof as described in embodiment 97), wherein $L_2$ is selected from the group consisting of unsubstituted or substituted $C_5$-$C_{20}$ arylene (e.g., unsubstituted or substituted $C_5$-$C_{15}$ arylene); preferably, $L_2$ is selected from the group consisting of phenylene, biphenylene, terphenylene, naphthylene, anthrylene, phenanthrylene, fluorenylene, and 9,9'-dimethylfluorenylene. The arylene is optionally substituted arylene. The substituted arylene refers to an arylene group substituted one or more times (e.g., 1-4, 1-3, or 1-2 times) with a substituent. For example, arylene is mono-, di-, or tri-substituted with a substituent optionally selected from the group consisting of e.g., deuterated $C_1$-$C_6$ alkyl, deuterated $C_3$-$C_6$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, unsubstituted or substituted cycloalkyl, alkenyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof. Preferably, the substituent is selected from the group consisting of deuterated $C_1$-$C_6$ alkyl, deuterated $C_3$-$C_6$ cycloalkyl, hydroxy, amino, mercapto, halogen, cyano, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl, and any combination thereof. Further, when $L_2$ is substituted arylene and the substituent of arylene is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl and unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo [2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted arylene and the substituent of arylene is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, and unsubstituted or substituted $C_5$-$C_{10}$ aryl etc.) (optionally,

the aryl is selected from the group consisting of biphenyl, terphenyl, naphthyl, anthryl, phenanthryl, fluorenyl, or 9,9'-dimethylfluorenyl etc.; and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $L_2$ is substituted arylene and the substituent of arylene is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl and unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof. Further, when $L_2$ is substituted arylene and the substituent of arylene is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, and unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4-to 15-membered bridged heterocyclyl, 5- to 15-membered bridged heterocyclyl, or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl, 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 101): Pertaining to the compounds of Formula I-1, Formula I-2 or salts, enantiomers, stereoisomers, polymorphs or solvates thereof as described in embodiment 97), wherein $L_2$ is selected from the group consisting of unsubstituted or substituted 4- to 20-membered heterocyclylene (e.g., unsubstituted or substituted 4- to 15-membered heterocyclylene); preferably, $L_2$ is selected from the group consisting of azetidinylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, diazacycloheptanylene, diazacyclooctylene, bridged heterocyclylene (e.g., 4- to 15-membered bridged heterocyclylene, 5- to 15-membered bridged heterocyclylene, or 7- to 15-membered bridged heterocyclylene, such as azabicyclohexylene, azabicycloheptanylene, diazabicycloheptanylene, azabicyclooctylene), or spiro-heterocyclylene (e.g., 4- to 15-membered spiro-heterocyclylene, 5- to 15-membered spiro-heterocyclylene, or 7- to 15-membered spiro-heterocyclylene, such as 3-azaspiro[5.5]undecanylene or 7-azaspiro[3.5]nonanylene). The heterocyclylene may be unsubstituted or substituted as explicitly defined (e.g., mono-, di-, tri-, or poly-substituted) by a substituent optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_{1-6}$ alkylene, $C_1$-$C_4$ alkyl-NHC(O)-, $C_1$-$C_4$ alkyl-C(O)NH-, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof. Preferably, the substituent is selected from the group

consisting of deuterium, hydroxy, amino, mercapto, nitro, halogen, cyano, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_{1-6}$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl, and any combination thereof. Further, when $L_2$ is substituted heterocyclylene and the substituent of heterocyclylene is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, and unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted heterocyclylene and the substituent of heterocyclylene is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_2$ is substituted heterocyclylene and the substituent of heterocyclylene is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, ditriazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. When $L_2$ is substituted heterocyclylene and the substituent of heterocyclylene is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4-to 15-membered bridged heterocyclyl, 5- to 15-membered bridged heterocyclyl, or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl, 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy,

$C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 102): Pertaining to the compounds of Formula I-1, Formula I-2 or salts, enantiomers, stereoisomers, polymorphs or solvates thereof as described in embodiment 97), wherein $L_2$ is absent.

Embodiment 103): Pertaining to the compounds of Formula I-1, Formula I-2 or salts, enantiomers, stereoisomers, polymorphs or solvates thereof as described in any one of embodiments 97)-102), wherein $L_2$ is selected from the group consisting of

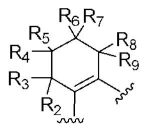, 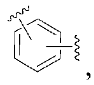, 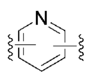, 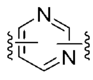, 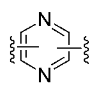, 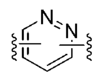,

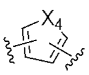, 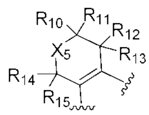, 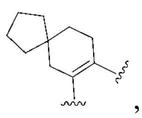, 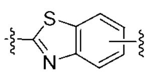, or 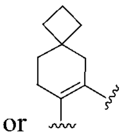;

wherein $X_4$ is selected from the group consisting of O, S, or NH;

$X_5$ is selected from the group consisting of O, S, or $NR_{16}$, where $R_{16}$ represents hydrogen or $C_1$-$C_3$ alkyl;

$R_2$, $R_3$, $R_4$, $R_5$, $R_6$, $R_7$, $R_8$, $R_9$, $R_{10}$, $R_{11}$, $R_{12}$, $R_{13}$, $R_{14}$, and $R_{15}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, halogenated $C_1$-$C_5$ alkyl, or linear or branched $C_1$-$C_5$ alkyl. Optionally, halogen may be e.g., F, Cl, Br, and I. Optionally, $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like. Optionally, halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino, and the like. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_5$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, and tert-pentyl etc., and the linear or branched $C_1$-$C_5$ alkyl is optionally substituted with one or more substituents optioanlly selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl) or any combination thereof.

Embodiment 104): Pertaining to the compounds of Formula I-1, Formula I-2 or salts, enantiomers, stereoisomers, polymorphs or solvates thereof as described in any one of embodiments 97)-103), wherein $L_3$ is selected from the group consisting of unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl.

Embodiment 105): Pertaining to the compounds of Formula I-1, Formula I-2 or salts, enantiomers, stereoisomers, polymorphs or solvates thereof as described in embodiment 104), wherein $L_3$ is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.). preferably, $L_3$ is selected from the group consisting of biphenyl, terphenyl, naphthyl, anthryl, phenanthryl, fluorenyl, and 9,9'-dimethylfluorenyl. The aryl may be monocyclic or polycyclic aryl. In some embodiments, the monocyclic aryl includes but is not limited to phenyl, biphenyl, terphenyl, quaterphenyl, and quinquephenyl etc. The polycyclic aryl includes but is not limited to naphthyl, anthryl, phenanthryl, pyrenyl, perylenyl, and fluorenyl etc. The fluorenyl may be substituted (e.g., 9,9'-dimethylfluorenyl and 9,9'-diphenylfluorenyl etc.). Preferably, $L_3$ is selected from the group consisting of biphenyl, terphenyl, naphthyl, anthryl, phenanthryl, fluorenyl, 9,9'-dimethylfluorenyl. Furthermore, two of the substituents may combine with each other to form a spirocyclic structure such as 9,9'-spirobifluorenyl etc. The aryl is optionally substituted aryl. The substituted aryl refers to an aryl group substituted one or more times (e.g., 1-4, 1-3, or 1-2 times) with a substituent. For example, aryl is mono-, di-, or tri-substituted with a substituent optionally selected from the group consisting of e.g., deuterated $C_1$-$C_6$ alkyl, deuterated $C_3$-$C_6$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-

C(O)NH-, alkyl, unsubstituted or substituted cycloalkyl, alkenyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof (preferably, the substituent is selected from the group consisting of deuterated $C_1$-$C_6$ alkyl, deuterated $C_3$-$C_6$ cycloalkyl, hydroxy, amino, mercapto, halogen, cyano, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl, and any combination thereof). Further, when $L_3$ is substituted aryl and the substituent of aryl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spirocycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof. Further, when $L_3$ is substituted aryl and the substituent of aryl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.) (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $L_3$ is substituted aryl and the substituent of aryl is unsubstituted or substituted heteroaryl, the unsubstituted or substituted heteroaryl is selected from the group consisting of unsubstituted or substituted 5- to 25-membered heteroaryl (e.g., unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted aryl and the substituent of aryl is unsubstituted or substituted heterocyclyl, and the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4-to 15-membered bridged heterocyclyl, 5- to 15-membered bridged heterocyclyl, or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl, 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one

or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 106): Pertaining to the compounds of Formula I-1, Formula I-2 or salts, enantiomers, stereoisomers, polymorphs or solvates thereof as described in embodiment 104), wherein $L_3$ is selected from the group consisting of unsubstituted or substituted 5- to 25-membered heteroaryl (preferably, unsubstituted or substituted 5- to 20-membered heteroaryl, or unsubstituted or substituted 5- to 15-membered heteroaryl); more preferably, $L_3$ is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl. Wherein the heteroaryl may be unsubstituted or substituted. The substituted heteroaryl refers to a heteroaryl substituted one or more times (e.g., 1-4, 1-3, or 1-2 times) with a substituent optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, or any combination thereof (preferably, the substituent is optionally selected from the group consisting of $C_1$-$C_3$ alkyl, $C_3$-$C_6$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, unsubstituted or substituted cycloalkyl, unsubstituted or substituted aryl, unsubstituted or substituted heterocyclyl, unsubstituted or substituted heteroaryl, unsubstituted or substituted heterocyclyl or any combination thereof). Further, when $L_3$ is substituted heteroaryl and the substituent of heteroaryl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH- and any combination thereof. Further, when $L_3$ is substituted heteroaryl and the substituent of heteroaryl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted heteroaryl and the substituent of heteroaryl is unsubstituted or substituted heteroaryl, the unsubstituted or substituted heteroaryl is selected from the group consisting of unsubstituted or substituted 5- to 25-membered heteroaryl (preferably, unsubstituted or substituted 5- to 20-membered heteroaryl, unsubstituted or substituted 5- to 15-membered heteroaryl or unsubstituted or substituted 5- to 10-membered heteroaryl); (optionally, the unsubstituted or substituted heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the unsubstituted or substituted heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $L_3$ is substituted heteroaryl and the substituent of heteroaryl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, azacyclopentyl, azacyclohexyl, azacycloheptanyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, diazacycloheptanyl, diazacyclooctyl, bridged heterocyclyl (e.g., 4- to 15-membered bridged heterocyclyl, 5- to 15-membered bridged heterocyclyl, or 7- to 15-membered bridged heterocyclyl, such as azabicyclohexyl, azabicycloheptanyl, azabicycloheptanyl, azabicyclooctyl), or spiro-heterocyclyl (e.g., 4- to 15-membered spiro-heterocyclyl, 5- to 15-membered spiro-heterocyclyl, or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 107): Pertaining to the compounds of Formula I-1, Formula I-2 or salts, enantiomers, stereoisomers, polymorphs or solvates thereof as described in embodiment 104), wherein $L_3$ is selected from the group consisting of unsubstituted or substituted 4- to 20-membered heterocyclyl (e.g., unsubstituted or substituted 4- to 15-membered heterocyclyl); (preferably, $L_3$ is selected from the group consisting of azetidinyl, azacyclopentyl, azacyclohexyl, azacycloheptanyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, diazacycloheptanyl, diazacyclooctyl, bridged heterocyclyl (e.g., 4- to 15-membered bridged heterocyclyl, such as azabicyclohexyl, azabicycloheptanyl, azabicycloheptanyl, azabicyclooctyl), or spiro-heterocyclyl (e.g., 4- to 15-membered spiro-heterocyclyl, such as 3-azaspiro[5.5]undecanyl or 7-azaspiro[3.5]nonanyl)). The heterocyclyl may be unsubstituted or substituted as explicitly defined (e.g., mono-, di-, tri-, or poly-substituted) by a substituent optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_{1-6}$ alkylene, $C_1$-$C_4$ alkyl-NHC(O)-, $C_1$-$C_4$ alkyl-C(O)NH-, alkenyl, unsubstituted or substituted cycloalkyl, unsubstituted or substituted aryl, aralkyl, arylalkenyl, alkylaryl, aralkylamino, heteroarylamino, arylamino, arylphosphino, unsubstituted or substituted heteroaryl, unsubstituted or substituted heterocyclyl, acenaphthenyl, and any combination thereof (preferably, the substituent is selected from the group consisting of deuterium, hydroxy, amino, mercapto, nitro, halogen, cyano, oxo, optionally deuterated $C_1$-$C_6$ alkyl, halogenated $C_1$-$C_6$ alkyl, optionally deuterated $C_3$-$C_6$ cycloalkyl, optionally deuterated $C_1$-$C_6$ alkoxy, $C_1$-$C_4$ alkyl-NH-, $NH_2$-$C_{1-6}$ alkylene, $C_1$-$C_4$ alkyl-NHC(O)-, $C_1$-$C_4$ alkyl-C(O)NH-, unsubstituted or substituted cycloalkyl, unsubstituted or substituted aryl, unsubstituted or substituted heteroaryl, unsubstituted or substituted heterocyclyl and any combination thereof). Further, when $L_3$ is substituted heterocyclyl and the substituent of heterocyclyl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, unsubstituted or substituted $C_3$-$C_6$ cycloalkyl etc.); (optionally, the cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro [4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and

iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted heterocyclyl and the substituent of heterocyclyl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted heterocyclyl and the substituent of heterocyclyl is unsubstituted or substituted heteroaryl, the unsubstituted or substituted heteroaryl is selected from the group consisting of unsubstituted or substituted 5- to 25-membered heteroaryl (preferably, unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted heterocyclyl and the substituent of heterocyclyl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.) (optionally, the unsubstituted or substituted heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4-to 15-membered bridged heterocyclyl, or 5- to 15-membered bridged heterocyclyl, or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl, or 5- to 15-membered spiro-heterocyclyl, or 7- to 15-membered spiro-heterocyclyl, such as 3-azaspiro[5.5]undecanyl or 7-azaspiro [3.5]nonanyl)); and the unsubstituted or substituted heterocyclyl is optionally substituted heterocyclyl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof).

Embodiment 108): Pertaining to the compounds of Formula I-1, Formula I-2 or salts, enantiomers, stereoisomers, polymorphs or solvates thereof as described in embodiment 104), wherein $L_3$ is selected from the group consisting of unsubstituted or substituted $C_3$-$C_{20}$ cycloalkyl (e.g., unsubstituted or substituted $C_3$-$C_{15}$ cycloalkyl). Preferably, $L_3$ is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo [2.2.1]heptyl, 2-oxobicyclo[2.2.1]heptyl or bicyclo[2.2.1]heptentyl). Wherein, the "cycloalkyl" is optionally substituted, and the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, unsubstituted or substituted cycloalkyl, deuterated cycloalkyl, alkenyl, unsubstituted or substituted aryl, unsubstituted or substituted heteroaryl, unsubstituted or substituted heterocyclyl, aralkyl, arylalkenyl, alkyl aryl, alkylamino, aralkylamino, heteroarylamino, arylamino, arylphosphino, acenaphthenyl, oxo or any combination thereof. Further, the substituents are preferably one or more (e.g., 1-5, 1-4, 1-3, 1-2, or 1) selected from the group consisting of trifluoromethyl, mercapto, hydroxy, amino, halogen, cyano, $C_1$-$C_3$ alkyl, $C_1$-$C_3$ alkoxy, trifluoromethyl, unsubstituted or substituted cycloalkyl, unsubstituted or substituted aryl, unsubstituted or substituted heteroaryl, unsubstituted or substituted heterocyclyl or any combination thereof. Further, when $L_3$ is substituted cycloalkyl and the substituent of cycloalkyl is unsubstituted or substituted cycloalkyl, the unsubstituted or substituted cycloalkyl is selected from the group consisting of unsubstituted or substituted $C_3$-$C_{20}$ cycloalkyl (e.g., unsubstituted or substituted $C_3$-$C_{15}$ cycloalkyl, unsubstituted or substituted $C_3$-$C_{10}$ cycloalkyl, unsubstituted or substituted $C_3$-$C_6$ cycloalkyl etc.) (optionally, the unsubstituted or substituted cycloalkyl is selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl, $C_5$-$C_{15}$ spiro-cycloalkyl, or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_3$-$C_{15}$ bridged cycloalkyl, $C_5$-$C_{15}$ bridged cycloalkyl, or $C_7$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl)); and the unsubstituted or substituted cycloalkyl is optionally substituted cycloalkyl, and the number of substituents is preferably one or more (e.g., 1-5, 1-4, 1-3, 1-2, or 1), and said substituents are selected from the group consisting of e.g., $C_1$-$C_6$ alkyl, $C_1$-$C_6$ alkoxy, $C_3$-$C_6$ cycloalkyl, hydroxy, amino, mercapto, halogen (e.g., fluorine, chlorine, bromine, and iodine etc.), cyano, oxo, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof). Further, when $L_3$ is substituted cycloalkyl and the substituent of cycloalkyl is unsubstituted or substituted aryl, the unsubstituted or substituted aryl is selected from the group consisting of unsubstituted or substituted $C_5$-$C_{20}$ aryl (e.g., unsubstituted or substituted $C_5$-$C_{15}$ aryl, unsubstituted or substituted $C_5$-$C_{10}$ aryl etc.); (optionally, the aryl is selected from the group consisting of biphenyl, terphenyl, naphthyl, anthryl, phenanthryl, fluorenyl, or 9,9'-dimethylfluorenyl etc.); and the aryl is optionally substituted aryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted cycloalkyl and the substituent of cycloalkyl is unsubstituted or substituted heteroaryl, the unsubstituted or substituted heteroaryl is selected from the group consisting of unsubstituted or substituted 5- to 25-membered heteroaryl (preferably, unsubstituted or substituted 5- to 20-membered heteroaryl, unsubstituted or substituted 5- to 15-membered heteroaryl or unsubstituted or substituted 5- to 10-membered heteroaryl); (optionally, the heteroaryl is selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, dithiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, naphthyridinyl, acridinyl, xanthenyl, phenanthridinyl, phthalazinyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl); and the heteroaryl is optionally substituted heteroaryl, and the number of substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof. Further, when $L_3$ is substituted cycloalkyl and the substituent of cycloalkyl is unsubstituted or substituted heterocyclyl, the unsubstituted or substituted heterocyclyl is selected from the group consisting of unsubstituted or substituted 4- to 20-membered heterocyclyl (e.g., unsubstituted or substituted 4- to 15-membered heterocyclyl, unsubstituted or substituted 4- to 10-membered heterocyclyl, or unsubstituted or substituted 4- to 8-membered heterocyclyl etc.); (optionally, the heterocyclyl is selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl, diazacyclooctyl, bridged heterocyclyl (e.g., 4- to 15-membered bridged heterocyclyl or 5- to 15-membered bridged heterocyclyl or 7- to 15-membered bridged heterocyclyl, such as azabicyclohexyl, azabicycloheptanyl, diazabicycloheptanyl, azabicyclooctyl), or spiro-heterocyclyl (e.g., 4- to 15-membered spiro-heterocyclyl or 5- to 15-membered spiro-heterocyclyl or 7- to 15-membered spiro-heterocyclyl, such as 3-azaspiro[5.5]undecanyl or 7-azaspiro[3.5]nonanyl)); and the heterocyclyl is optionally substituted heterocyclyl, and the number of

substituents is preferably one or more (e.g., 1-5, 1-4, 1-3, 1-2, or 1), and said substituents are selected from the group consisting of e.g., $C_1$-$C_6$ alkyl, $C_3$-$C_6$ cycloalkyl, hydroxy, amino, mercapto, halogen (e.g., fluorine, chlorine, bromine, and iodine etc.), cyano, oxo, $C_1$-$C_6$ alkoxy, deuterated $C_1$-$C_6$ alkoxy, $C_1$-$C_4$ alkylamino, halogenated $C_1$-$C_6$ alkyl, halogenated $C_3$-$C_6$ cycloalkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment 109): Pertaining to the compounds of Formula I-1, Formula I-2 or salts, enantiomers, stereoisomers, polymorphs or solvates thereof as described in embodiment 104), wherein the substituent of the substituted $L_3$ is optionally one or more selected from the group consisting of deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, linear or branched $C_1$-$C_5$ alkyl, or halogenated $C_1$-$C_5$ alkyl.

Embodiment 110): Pertaining to the compounds of Formula I-1, Formula I-2 or salts, enantiomers, stereoisomers, polymorphs or solvates thereof as described in any one of embodiments 104)-109), wherein $L_3$ is selected from the group consisting of:

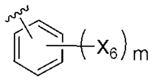 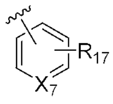 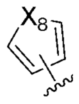 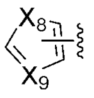 or 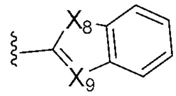 ;

wherein $X_6$ is selected from the group consisting of deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, halogenated $C_1$-$C_6$ alkyl or linear or branched $C_1$-$C_6$ alkyl. Optionally, the halogen may be e.g., F, Cl, Br, and I. Optionally, $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like. Optionally, the halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino, and the like. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_6$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl etc., and the linear or branched $C_1$-$C_6$ alkyl is optionally substituted by one or more optionally selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl) or any combination thereof;

m is an integer of 0, 1, 2, 3, 4 or 5, and when m is an integer of 2, 3, 4 or 5, each $X_6$ is independently identical to or different from each other;

$X_7$ and $X_9$ are each independently selected from the group consisting of CH or N;

each $X_8$ is independently selected from the group consisting of O, S or NH;

$R_{17}$ is selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, halogenated $C_1$-$C_5$ alkyl or linear or branched $C_1$-$C_5$ alkyl, wherein the halogen may optionally be e.g., F, Cl, Br, and I. Optionally, the $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, 2-ethylbutoxy, and the like. Optionally, the halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino, and the like. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_5$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl etc., and the linear or branched $C_1$-$C_5$ alkyl is optionally substituted by one or more optionally selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl), or any combination thereof.

Embodiment 111): Pertaining to the compounds of Formula I-1, Formula I-2 or salts, enantiomers, stereoisomers, polymorphs or solvates thereof as described in any one of embodiments 97)-110), wherein the further selection of $R_{a2}$ is as

described in Formula I. $R_{a2}$ may be the same as or different from $L_3$; and $R_{a2}$ must be present.

Especially preferably the compounds of the present disclosure and their salts (especially pharmaceutically acceptable salts, such as hydrochloride, etc.), enantiomers, stereoisomers, polymorphs, or solvates thereof in Table 1 below are provided:

Table 1. The compounds of the present disclosure

| Compound No. | Structure of the compounds | The compounds' name | Chinese translation of the compounds' name |
|---|---|---|---|
| 1 GT-03464 | 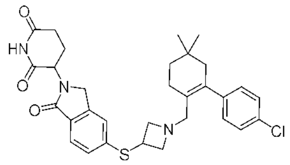 | 3-(5-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione |
| 2 | 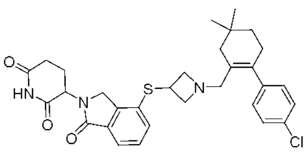 | 3-(4-((1-((4'-chloro-4,4-dimethyl-3,4,5,6-tetrahydro-[1,1'-biphenyl]-2-yl)methyl)azetidin-3-yl)thio)-1-oxoisoindolin-2-yl)piperidine-2,6-dione | 3-(4-((1-((4'-chloro-4,4-dimethyl-3,4,5,6-tetrahydro-[1,1'-biphenyl]-2-yl)methyl)azetidin-3-yl)thio)-1-oxoisoindolin-2-yl)piperidine-2,6-dione |
| 3 GT-06757 | 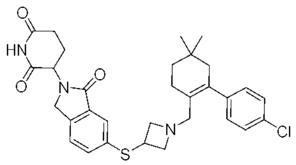 | 3-(6-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione | 3-(6-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione |
| 4 | 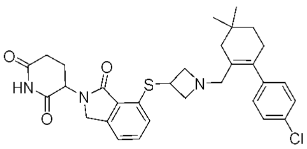 | 3-(7-((1-((4'-chloro-4,4-dimethyl-3,4,5,6-tetrahydro-[1,1'-biphenyl]-2-yl)methyl)azetidin-3-yl)thio)-1-oxoisoindolin-2-yl)piperidine-2,6-dione | 3-(7-((1-((4'-chloro-4,4-dimethyl-3,4,5,6-tetrahydro-[1,1'-biphenyl]-2-yl)methyl)azetidin-3-yl)thio)-1-oxoisoindolin-2-yl)piperidine-2,6-dione |
| 5 GT-04245 | 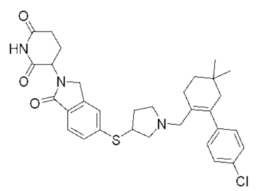 | 3-(5-((1-((4'-chloro-5,5-dimethyl-3,4,5,6-tetrahydro-[1,1'-biphenyl]-2-yl)methyl)pyrrolidin-3-yl)thio)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)pyrrolidin-3-yl)thio)-1-oxoisoindolin-2-yl)piperidine-2,6-dione |
| 6 GT-03462 | 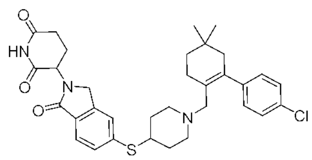 | 3-(5-((1-((4'-chloro-5,5-dimethyl-3,4,5,6-tetrahydro-[1,1'-biphenyl]-2-yl)methyl)piperidin-4-yl)thio)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)piperidin-4-yl)thio)-1-oxoisoindolin-2-yl)piperidine-2,6-dione |
| 7 GT-06641 | 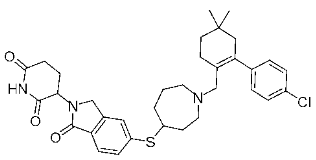 | 3-(5-((1-((4'-chloro-5,5-dimethyl-3,4,5,6-tetrahydro-[1,1'-biphenyl]-2-yl)methyl)azepan-4-yl)thio)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)azepan-4-yl)thio)-1-oxoisoindolin-2-yl)piperidine-2,6-dione |
| 8 | 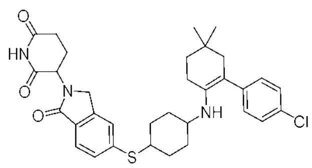 | 3-(5-((4-((4'-chloro-5,5-dimethyl-3,4,5,6-tetrahydro-[1,1'-biphenyl]-2-yl)amino)cyclohexyl)thio)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)amino)cyclohexyl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 9 GT-06813 | 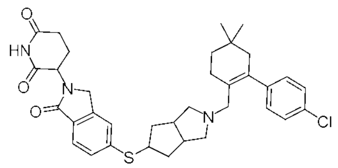 | 3-(5-((2-((4'-chloro-5,5-dimethyl-3,4,5,6-tetrahydro-[1,1'-biphenyl]-2-yl)methyl)octahydrocyclopenta[c]pyrrol-5-yl)thio)-1-oxoisoindolin-2-yl)piperidine-2,6-dione | 3-(5-((2-((4'-chloro-5,5-dimethyl-3,4,5,6-tetrahydro-[1,1'-biphenyl]-2-yl)methyl)octahydrocyclopenta[c]pyrrol-5-yl)thio)-1-oxoisoindolin-2-yl)piperidine-2,6-dione |
| 10 | 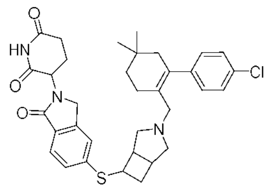 | 3-(5-((3-((4'-chloro-5,5-dimethyl-3,4,5,6-tetrahydro-[1,1'-biphenyl]-2-yl)methyl)-3-azabicyclo[3.2.0]heptan-6-yl)thio)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azabicyclo[3.2.0]heptan-6-yl)thio)-1-oxoisoindolin-2-yl)piperidine-2,6-dione |
| 11 | 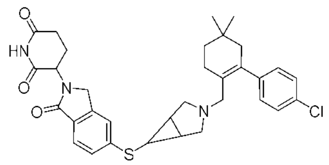 | 3-(5-((3-((4'-chloro-5,5-dimethyl-3,4,5,6-tetrahydro-[1,1'-biphenyl]-2-yl)methyl)-3-azabicyclo[3.1.0]hexan-6-yl)thio)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azabicyclo[3.1.0]hexan-6-yl)thio)-1-oxoisoindolin-2-yl)piperidine-2,6-dione |
| 12 GT-06873 | 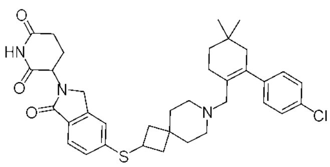 | 3-(5-((7-((4'-chloro-5,5-dimethyl-3,4,5,6-tetrahydro-[1,1'-biphenyl]-2-yl)methyl)-7-azaspiro[3.5]nonan-2-yl)thio)-1-oxoisoindolin-2-yl)piperidine-2,6-dione | 3-(5-((7-((4'-chloro-5,5-dimethyl-3,4,5,6-tetrahydro-[1,1'-biphenyl]-2-yl)methyl)-7-azaspiro[3.5]nonan-2-yl)thio)-1-oxoisoindolin-2-yl)piperidine-2,6-dione |
| 13 GT-06874 | 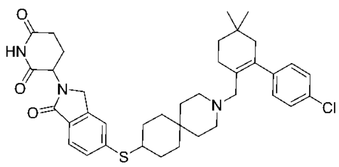 | 3-(5-((3-((4'-chloro-5,5-dimethyl-3,4,5,6-tetrahydro-[1,1'-biphenyl]-2-yl)methyl)-3-azaspiro[5 .5]undecan-9-yl)thio)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azaspiro[5.5]undecan-9-yl)thio)-1-oxoisoindolin-2-yl)piperidine-2,6-dione |
| 14 | 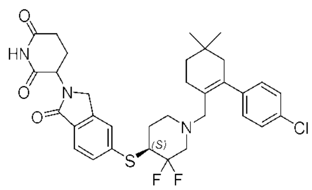 | 3-(5-(((S)-1-((4'-chloro-5,5-dimethyl-3,4,5,6-tetrahydro-[1,1'-biphenyl]-2-yl)methyl)-3,3-difluoropiperidin-4-yl)thio)-1-oxoisoindolin-2-yl)piperidine-2,6-dione | 3-(5-(((S)-1-((4'-chloro-5,5-dimethyl-3,4,5,6-tetrahydro-[1,1'-biphenyl]-2-yl)methyl)-3,3-difluoropiperidin-4-yl)thio)-1-oxoisoindolin-2-yl)piperidine-2,6-dione |
| 15 | 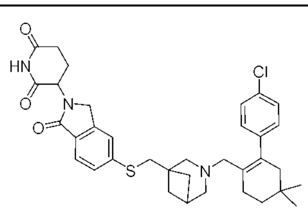 | 3-(5-(((3-((4'-chloro-5,5-dimethyl-3,4,5,6-tetrahydro-[1,1'-biphenyl]-2-yl)methyl)-3-azabicyclo[3.1.1]heptan-1-yl)methyl)thio)-1-oxoisoindolin-2-yl)piperidine-2,6-dione | 3-(5-(((3-((4'-chloro-5,5-dimethyl-3,4,5,6-tetrahydro-[1,1'-biphenyl]-2-yl)methyl)-3-azabicyclo[3.1.1]heptan-1-yl)methyl)thio)-1-oxoisoindolin-2-yl)piperidine-2,6-dione |
| 16 | 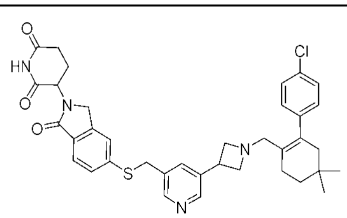 | 3-(5-(((5-(1-((4'-chloro-5,5-dimethyl-3,4,5,6-tetrahydro-[1,1'-biphenyl]-2-yl)methyl)azetidin-3-yl)pyridin-3-yl)methyl)thio)-1-oxoisoindolin-2-yl)piperidine-2,6-dione | 3-(5-(((5-(1-((4'-chloro-5,5-dimethyl-3,4,5,6-tetrahydro-[1,1'-biphenyl]-2-yl)methyl)azetidin-3-yl)pyridin-3-yl)methyl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 17 GT-06137 | 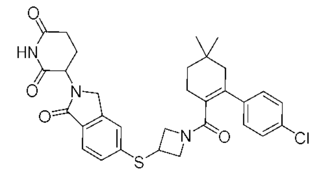 | 3-(5-((1-(4'-chloro-5,5-dimethyl-3,4,5,6-tetrahydro-[1,1'-biphenyl]-2-carbonyl)azetidin-3-yl)thio)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)azetidin-3-yl)thio)-1-oxoisoindolin-2-yl)piperidine-2,6-dione |
| 18 GT-06139 | 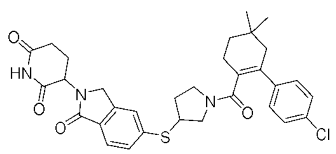 | 3-(5-((1-(4'-chloro-5,5-dimethyl-3,4,5,6-tetrahydro-[1,1'-biphenyl]-2-carbonyl)pyrrolidin-3-yl)thio)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)pyrrolidin-3-yl)thio)-1-oxoisoindolin-2-yl)piperidine-2,6-dione |
| 19 GT-06140 | 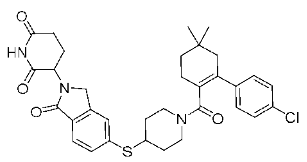 | 3-(5-((1-(4'-chloro-5,5-dimethyl-3,4,5,6-tetrahydro-[1,1'-biphenyl]-2-carbonyl)piperidin-4-yl)thio)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)piperidin-4-yl)thio)-1-oxoisoindolin-2-yl)piperidine-2,6-dione |
| 20 GT-06642 | 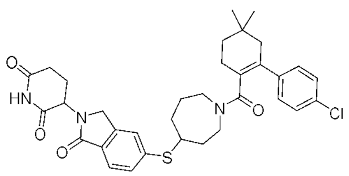 | 3-(5-((1-(4'-chloro-5,5-dimethyl-3,4,5,6-tetrahydro-[1,1'-biphenyl]-2-carbonyl)azepan-4-yl)thio)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)azepan-4-yl)thio)-1-oxoisoindolin-2-yl)piperidine-2,6-dione |
| 21 | 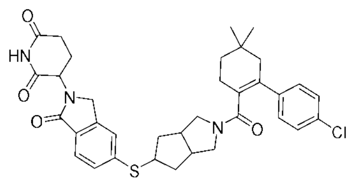 | 3-(5-((2-(4'-chloro-5,5-dimethyl-3,4,5,6-tetrahydro-[1,1'-biphenyl]-2-carbonyl)octahydrocyclope nta[c]pyrrol-5-yl)thio)-1-oxoisoindolin-2-yl)piperidine-2,6-dione | 3-(5-((2-(4'-chloro-5,5-dimethyl-3,4,5,6-tetrahydro-[1,1'-biphenyl]-2-carbonyl)octahydrocyclope nta[c]pyrrol-5-yl)thio)-1-oxoisoindolin-2-yl)piperidine-2,6-dione |
| 22 | 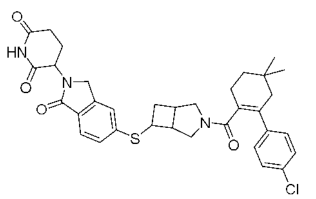 | 3-(5-((3-(4'-chloro-5,5-dimethyl-3,4,5,6-tetrahydro-[1,1'-biphenyl]-2-carbonyl)-3-azabicyclo[3.2.0]heptan-6-yl)thio)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3-azabicyclo[3.2.0]heptan-6-yl)thio)-1-oxoisoindolin-2-yl)piperidine-2,6-dione |
| 23 | 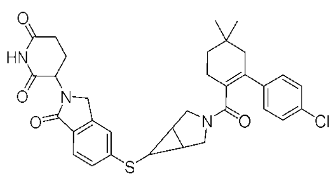 | 3-(5-((3-(4'-chloro-5,5-dimethyl-3,4,5,6-tetrahydro-[1,1'-biphenyl]-2-carbonyl)-3-azabicyclo[3.1.0]hexan-6-yl)thio)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3-azabicyclo[3.1.0]hexan-6-yl)thio)-1-oxoisoindolin-2-yl)piperidine-2,6-dione |
| 24 GT-06918 | 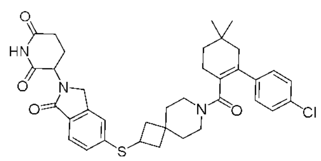 | 3-(5-((7-(4'-chloro-5,5-dimethyl-3,4,5,6-tetrahydro-[1,1'-biphenyl]-2-carbonyl)-7-azaspiro[3.5]nonan-2-yl)thio)-1-oxoisoindolin-2-yl)piperidine-2,6-dione | 3-(5-((7-(4'-chloro-5,5-dimethyl-3,4,5,6-tetrahydro-[1,1'-biphenyl]-2-carbonyl)-7-azaspiro[3.5]nonan-2-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 25 GT-07016 | 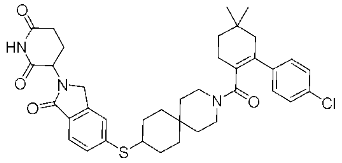 | 3-(5-((3-(4'-chloro-5,5-dimethyl-3,4,5,6-tetrahydro-[1,1'-biphenyl]-2-carbonyl)-3-azaspiro[5 .5]undecan-9-yl)thio)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3-azaspiro[5.5]undecan-9-yl)thio)-1-oxoisoindolin-2-yl)piperidine-2,6-dione |
| 26 | 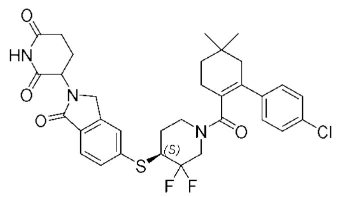 | 3-(5-(((S)-1-(4'-chloro-5,5-dimethyl-3,4,5,6-tetrahydro-[1,1'-biphenyl]-2-carbonyl)-3,3-difluoropiperidin-4-yl)thio)-1-oxoisoindolin-2-yl)piperidine-2,6-dione | 3-(5-(((S)-1-(4'-chloro-5,5-dimethyl-3,4,5,6-tetrahydro-[1,1'-biphenyl]-2-carbonyl)-3,3-difluoropiperidin-4-yl)thio)-1-oxoisoindolin-2-yl)piperidine-2,6-dione |
| 27 | 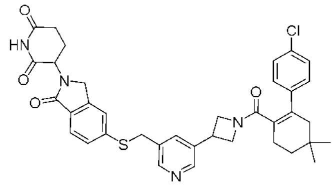 | 3-(5-(((5-(1-(4'-chloro-5,5-dimethyl-3,4,5,6-tetrahydro-[1,1'-biphenyl]-2-carbonyl)azetidin-3-yl)pyridin-3-yl)methyl)thio)-1-oxoisoindolin-2-yl)piperidine-2,6-dione | 3-(5-(((5-(1-(4'-chloro-5,5-dimethyl-3,4,5,6-tetrahydro-[1,1'-biphenyl]-2-carbonyl)azetidin-3-yl)pyridin-3-yl)methyl)thio)-1-oxoisoindolin-2-yl)piperidine-2,6-dione |
| 28 GT-03360 | 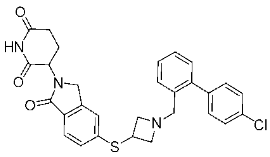 | 3-(5-((1-((4'-chloro-[1,1'-biphenyl]-2-yl)methyl)azetidin-3-yl)thio)-1-oxoisoindolin-2-yl)piperidine-2,6-dione | 3-(5-((1-((4'-chloro-[1,1'-biphenyl]-2-yl)methyl)azetidin-3-yl)thio)-1-oxoisoindolin-2-yl)piperidine-2,6-dione |
| 29 GT-04195 | 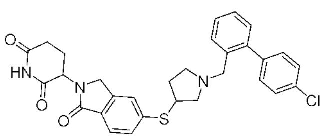 | 3-(5-((1-((4'-chloro-[1,1'-biphenyl]-2-yl)methyl)pyrrolidin-3-yl)thio)-1-oxoisoindolin-2-yl)piperidine-2,6-dione | 3-(5-((1-((4'-chloro-[1,1'-biphenyl]-2-yl)methyl)pyrrolidin-3-yl)thio)-1-oxoisoindolin-2-yl)piperidine-2,6-dione |
| 30 GT-03357 | 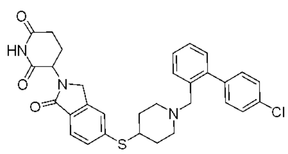 | 3-(5-((1-((4'-chloro-[1,1'-biphenyl]-2-yl)methyl)piperidin-4-yl)thio)-1-oxoisoindolin-2-yl)piperidine-2,6-dione | 3-(5-((1-((4'-chloro-[1,1'-biphenyl]-2-yl)methyl)piperidin-4-yl)thio)-1-oxoisoindolin-2-yl)piperidine-2,6-dione |
| 31 GT-06643 | 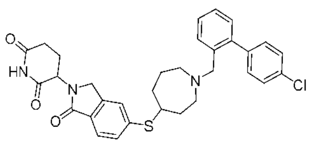 | 3-(5-((1-((4'-chloro-[1,1'-biphenyl]-2-yl)methyl)azepan-4-yl)thio)-1-oxoisoindolin-2-yl)piperidine-2,6-dione | 3-(5-((1-((4'-chloro-[1,1'-biphenyl]-2-yl)methyl)azepan-4-yl)thio)-1-oxoisoindolin-2-yl)piperidine-2,6-dione |
| 32 GT-06914 | 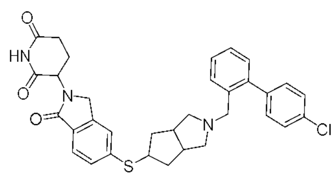 | 3-(5-((2-((4'-chloro-[1,1'-biphenyl]-2-yl)methyl)octahydrocyclope nta[c]pyrrol-5-yl)thio)-1-oxoisoindolin-2-yl)piperidine-2,6-dione | 3-(5-((2-((4'-chloro-[1,1'-biphenyl]-2-yl)methyl)octahydrocyclop enta[c]pyrrol-5-yl)thio)-1-oxoisoindolin-2-yl)piperidine-2,6-dione |
| 33 | 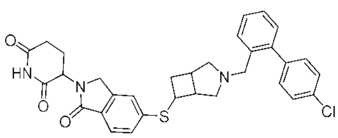 | 3-(5-((3-((4'-chloro-[1,1'-biphenyl]-2-yl)methyl)-3-azabicyclo [3.2.0]heptan-6-yl)thio)-1-oxoisoindolin-2-yl)piperidine-2,6-dione | 3-(5-((3-((4'-chloro-[1,1'-biphenyl]-2-yl)methyl)-3-azabicyclo [3.2.0]heptan-6-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 34 | 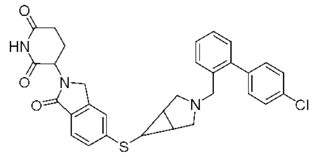 | 3-(5-((3-((4'-chloro-[1,1'-biphenyl]-2-yl)methyl)-3-azabicyclo [3.1.0]hexan-6-yl)thio)-1-oxoisoindolin-2-yl)piperidine-2,6-dione | 3-(5-((3-((4'-chloro-[1,1'-biphenyl]-2-yl)methyl)-3-azabicyclo [3.1.0]hexan-6-yl)thio)-1-oxoisoindolin-2-yl)piperidine-2,6-dione |
| 35 | 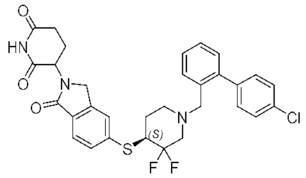 | 3-(5-(((S)-1-((4'-chloro-[1,1'-biphenyl]-2-yl)methyl)-3,3-difluoropiperidin-4-yl)thio)-1-oxoisoindolin-2-yl)piperidine-2,6-dione | 3-(5-(((S)-1-((4'-chloro-[1,1'-biphenyl]-2-yl)methyl)-3,3-difluoropiperidin-4-yl)thio)-1-oxoisoindolin-2-yl)piperidine-2,6-dione |
| 36 GT-06515 | 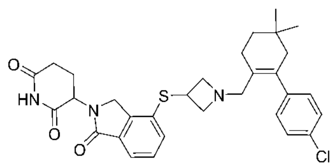 | 3-(4-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione | 3-(4-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione |
| 37 GT-03892 | 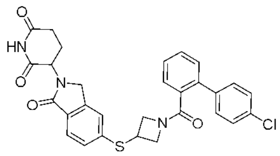 | 3-(5-((1-(4'-chloro-[1,1'-biphenyl]-2-carbonyl)azetidin-3-yl) thio)-1-oxoisoindolin-2-yl)piperidine-2,6-dione | 3-(5-((1-(4'-chloro-[1,1'-biphenyl]-2-carbonyl)azetidin-3-yl) thio)-1-oxoisoindolin-2-yl)piperidine-2,6-dione |
| 38 GT-06181 | 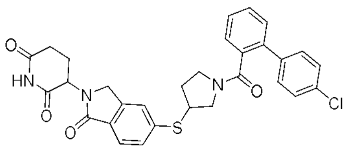 | 3-(5-((1-(4'-chloro-[1,1'-biphenyl]-2-carbonyl)pyrrolidin-3-yl) thio)-1-oxoisoindolin-2-yl)piperidine-2,6-dione | 3-(5-((1-(4'-chloro-[1,1'-biphenyl]-2-carbonyl)pyrrolidin-3-yl) thio)-1-oxoisoindolin-2-yl)piperidine-2,6-dione |
| 39 GT-06182 | 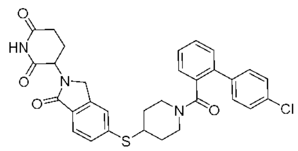 | 3-(5-((1-(4'-chloro-[1,1'-biphenyl]-2-carbonyl)piperidin-4-yl) thio)-1-oxoisoindolin-2-yl)piperidine-2,6-dione | 3-(5-((1-(4'-chloro-[1,1'-biphenyl]-2-carbonyl)piperidin-4-yl) thio)-1-oxoisoindolin-2-yl)piperidine-2,6-dione |
| 40 GT-06644 | 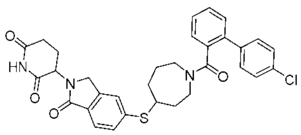 | 3-(5-((1-(4'-chloro-[1,1'-biphenyl]-2-carbonyl)azepan-4-yl) thio)-1-oxoisoindolin-2-yl)piperidine-2,6-dione | 3-(5-((1-(4'-chloro-[1,1'-biphenyl]-2-carbonyl)azepan-4-yl) thio)-1-oxoisoindolin-2-yl)piperidine-2,6-dione |
| 41 | 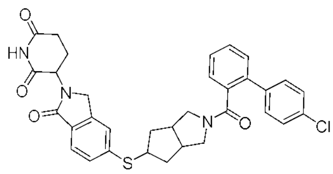 | 3-(5-((2-(4'-chloro-[1,1'-biphenyl]-2-carbonyl)octahydrocyclope nta[c]pyrrol-5-yl)thio)-1-oxoisoindolin-2-yl)piperidine-2,6-dione | 3-(5-((2-(4'-chloro-[1,1'-biphenyl]-2-carbonyl)octahydrocyclope nta[c]pyrrol-5-yl)thio)-1-oxoisoindolin-2-yl)piperidine-2,6-dione |
| 42 | 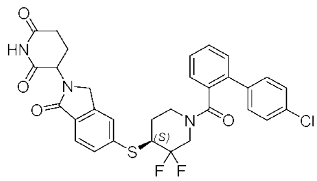 | 3-(5-(((S)-1-(4'-chloro-[1,1'-biphenyl]-2-carbonyl)-3,3-difluoropiperidin-4-yl)thio)-1-oxoisoindolin-2-yl)piperidine-2,6-dione | 3-(5-(((S)-1-(4'-chloro-[1,1'-biphenyl]-2-carbonyl)-3,3-difluoropiperidin-4-yl)thio)-1-oxoisoindolin-2-yl)piperidine-2,6-dione |

(continued)

| Compound No. | Structure of the compounds | The compounds' name | Chinese translation of the compounds' name |
|---|---|---|---|
| 43 GT-06225 | 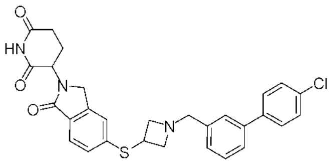 | 3-(5-((1-((4'-chloro-[1,1'-biphenyl]-3-yl)methyl)azetidin-3-yl)thio)-1-oxoisoindolin-2-yl)piperidine-2,6-dione | 3-(5-((1-((4'-chloro-[1,1'-biphenyl]-3-yl)methyl)azetidin-3-yl)thio)-1-oxoisoindolin-2-yl)piperidine-2,6-dione |
| 44 GT-06226 | 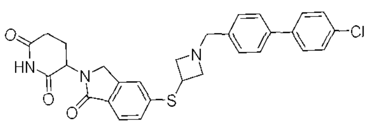 | 3-(5-((1-((4'-chloro-[1,1'-biphenyl]-4-yl)methyl)azetidin-3-yl)thio)-1-oxoisoindolin-2-yl)piperidine-2,6-dione | 3-(5-((1-((4'-chloro-[1,1'-biphenyl]-4-yl)methyl)azetidin-3-yl)thio)-1-oxoisoindolin-2-yl)piperidine-2,6-dione |
| 45 GT-06350 | 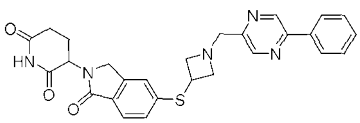 | 3-(1-oxo-5-((1-((5-phenylpyrazin-2-yl)methyl)azetidin-3-yl)thio)isoindolin-2-yl)piperidine-2,6-dione | 3-(1-oxo-5-((1-((5-phenylpyrazin-2-yl)methyl)azetidin-3-yl)thio)isoindolin-2-yl)piperidine-2,6-dione |
| 46 GT-06808 | 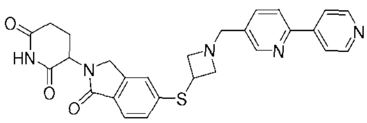 | 3-(5-((1-([2,4'-bipyridin]-5-ylmethyl)azetidin-3-yl)thio)-1-oxoisoindolin-2-yl)piperidine-2,6-dione | 3-(5-((1-([2,4'-bipyridin]-5-ylmethyl)azetidin-3-yl)thio)-1-oxoisoindolin-2-yl)piperidine-2,6-dione |
| 47 GT-06517 | 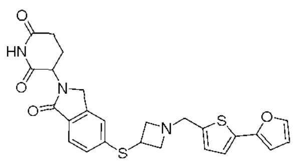 | 3-(5-((1-((5-(furan-2-yl)thiophen-2-yl)methyl)azetidin-3-yl)thio)-1-oxoisoindolin-2-yl)piperidine-2,6-dione | 3-(5-((1-((5-(furan-2-yl)thiophen-2-yl)methyl)azetidin-3-yl)thio)-1-oxoisoindolin-2-yl)piperidine-2,6-dione |
| 48 GT-03457 | 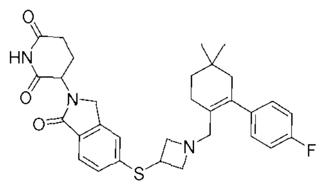 | 3-(5-((1-((4'-fluoro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione | 3-(5-((1-((4'-fluoro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione |
| 49 GT-06301 | 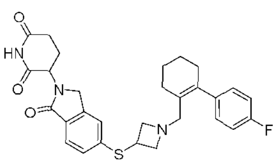 | 3-(5-((1-((4'-fluoro-3,4,5,6-tetrahydro-[1,1'-biphenyl]-2-yl)methyl)azetidin-3-yl)thio)-1-oxoisoindolin-2-yl)piperidine-2,6-dione | 3-(5-((1-((4'-fluoro-3,4,5,6-tetrahydro-[1,1'-biphenyl]-2-yl)methyl)azetidin-3-yl)thio)-1-oxoisoindolin-2-yl)piperidine-2,6-dione |
| 50 GT-06351 | 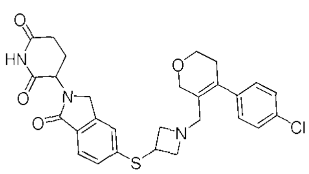 | 3-(5-((1-((4-(4-chlorophenyl)-5,6-dihydro-2H-pyran-3-yl)methyl)azetidin-3-yl)thio)-1-oxoisoindolin-2-yl)piperidine-2,6-dione | 3-(5-((1-((4-(4-chlorophenyl)-5,6-dihydro-2H-pyran-3-yl)methyl)azetidin-3-yl)thio)-1-oxoisoindolin-2-yl)piperidine-2,6-dione |
| 51 GT-06513 | 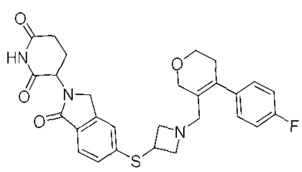 | 3-(5-((1-((4-(4-fluorophenyl)-5,6-dihydro-2H-pyran-3-yl)methyl)azetidin-3-yl)thio)-1-oxoisoindolin-2-yl)piperidine-2,6-dione | 3-(5-((1-((4-(4-fluorophenyl)-5,6-dihydro-2H-pyran-3-yl)methyl)azetidin-3-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 52 GT-06516 | 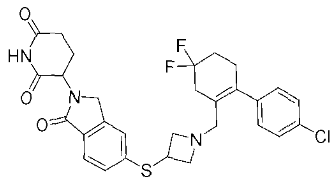 | 3-(5-((1-((4'-chloro-4,4-difluoro-3,4,5,6-tetrahydro-[1,1'-biphenyl]-2-yl)methyl)azetidin-3-yl)thio)-1-oxoisoindolin-2-yl)piperidine-2,6-dione | 3-(5-((1-((4'-chloro-4,4-difluoro-3,4,5,6-tetrahydro-[1,1'-biphenyl]-2-yl)methyl)azetidin-3-yl)thio)-1-oxoisoindolin-2-yl)piperidine-2,6-dione |
| 53 GT-07064 | 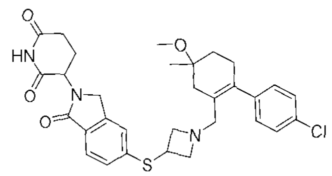 | 3-(5-((1-((4'-chloro-4-methoxy-4-methyl-3,4,5,6-tetrahydro-[1,1'-biphenyl]-2-yl)methyl)azetidin-3-yl)thio)-1-oxoisoindolin-2-yl)piperidine-2,6-dione | 3-(5-((1-((4'-chloro-4-methoxy-4-methyl-3,4,5,6-tetrahydro-[1,1'-biphenyl]-2-yl)methyl)azetidin-3-yl)thio)-1-oxoisoindolin-2-yl)piperidine-2,6-dione |
| 54 GT-06514 | 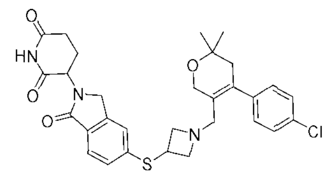 | 3-(5-((1-((4-(4-chlorophenyl)-6,6-dimethyl-5,6-dihydro-2H-pyran-3-yl)methyl)azetidin-3-yl)thio)-1-oxoisoindolin-2-yl)piperidine-2,6-dione | 3-(5-((1-((4-(4-chlorophenyl)-6,6-dimethyl-5,6-dihydro-2H-pyran-3-yl)methyl)azetidin-3-yl)thio)-1-oxoisoindolin-2-yl)piperidine-2,6-dione |
| 55 | 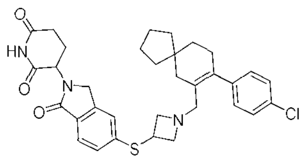 | 3-(5-((1-((8-(4-chlorophenyl)spiro[4.5]dec-7-en-7-yl)methyl)azetidin-3-yl)thio)-1-oxoisoindolin-2-yl)piperidine-2,6-dione | 3-(5-((1-((8-(4-chlorophenyl)spiro[4.5]dec -7-en-7-yl)methyl)azetidin-3-yl)thio)-1-oxoisoindolin-2-yl)piperidine-2,6-dione |
| 56 | 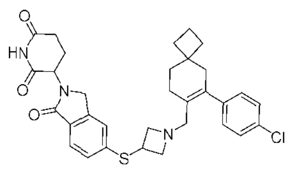 | 3-(5-((1-((6-(4-chlorophenyl)spiro[3.5]non-6-en-7-yl)methyl)azetidin-3-yl)thio)-1-oxoisoindolin-2-yl)piperidine-2,6-dione | 3-(5-((1-((6-(4-chlorophenyl)spiro[3.5]non -6-en-7-yl)methyl)azetidin-3-yl)thio)-1-oxoisoindolin-2-yl)piperidine-2,6-dione |
| 57 GT-06138 | 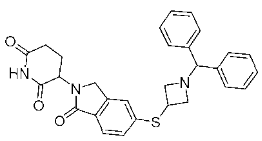 | 3-(5-((1-benzhydrylazetidin-3-yl)thio)-1-oxoisoindolin-2-yl)piperidine-2,6-dione | 3-(5-((1-benzhydrylazetidin-3-yl)thio)-1-oxoisoindolin-2-yl)piperidine-2,6-dione |
| 58 GT-06196 | 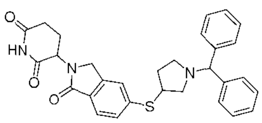 | 3-(5-((1-benzhydrylpyrrolidin-3-yl)thio)-1-oxoisoindolin-2-yl)piperidine-2,6-dione | 3-(5-((1-benzhydrylpyrrolidin-3-yl)thio)-1-oxoisoindolin-2-yl)piperidine-2,6-dione |
| 59 GT-06184 | 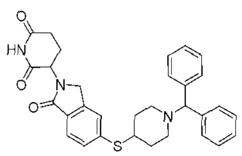 | 3-(5-((1-benzhydrylpiperidin-4-yl)thio)-1-oxoisoindolin-2-yl)piperidine-2,6-dione | 3-(5-((1-benzhydrylpiperidin-4-yl)thio)-1-oxoisoindolin-2-yl)piperidine-2,6-dione |
| 60 GT-06645 | 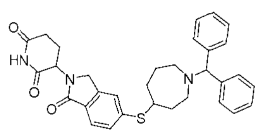 | 3-(5-((1-benzhydrylazepan-4-yl)thio)-1-oxoisoindolin-2-yl)piperidine-2,6-dione | 3-(5-((1-benzhydrylazepan-4-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 61 | 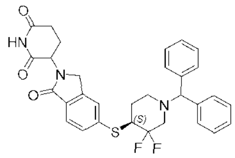 | 3-(5-(((S)-1-benzhydryl-3,3-difluoropiperidin-4-yl)thio)-1-oxoisoindolin-2-yl)piperidine-2,6-dione | 3-(5-(((S)-1-benzhydryl-3,3-difluoropiperidin-4-yl)thio)-1-oxoisoindolin-2-yl)piperidine-2,6-dione |
| 62 GT-06920 | 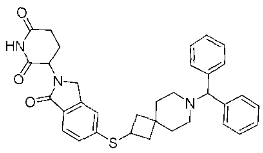 | 3-(5-((7-benzhydryl-7-azaspiro [3.5]nonan-2-yl)thio)-1-oxoisoindolin-2-yl)piperidine-2,6-dione | 3-(5-((7-benzhydryl-7-azaspiro [3.5]nonan-2-yl)thio)-1-oxoisoindolin-2-yl)piperidine-2,6-dione |
| 63 GT-06958 | 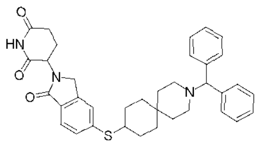 | 3-(5-((3-benzhydryl-3-azaspiro [5 .5]undecan-9-yl)thio)-1-oxoisoindolin-2-yl)piperidine-2,6-dione | 3-(5-((3-benzhydryl-3-azaspiro [5.5]undecan-9-yl)thio)-1-oxoisoindolin-2-yl)piperidine-2,6-dione |
| 64 GT-06922 | 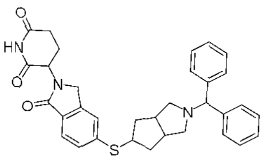 | 3-(5-((2-benzhydryloctahydrocyclop enta[c]pyrrol-5-yl)thio)-1-oxoisoindolin-2-yl)piperidine-2,6-dione | 3-(5-((2-benzhydryloctahydrocyclop enta[c]pyrrol-5-yl)thio)-1-oxoisoindolin-2-yl)piperidine-2,6-dione |
| 65 | 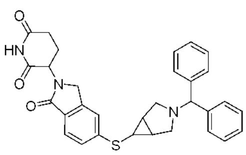 | 3-(5-((3-benzhydryl-3-azabicyclo[3.1.0]hexan-6-yl)thio)-1-oxoisoindolin-2-yl)piperidine-2,6-dione | 3-(5-((3-benzhydryl-3-azabicyclo[3.1.0]hexan-6-yl)thio)-1-oxoisoindolin-2-yl)piperidine-2,6-dione |
| 66 | 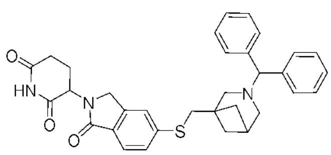 | 3-(5-(((3-benzhydryl-3-azabicyclo[3.1.1]heptan-1-yl)methyl) thio)-1-oxoisoindolin-2-yl)piperidine-2,6-dione | 3-(5-(((3-benzhydryl-3-azabicyclo[3.1.1]heptan-1-yl)methyl) thio)-1-oxoisoindolin-2-yl)piperidine-2,6-dione |
| 67 GT-06758 | 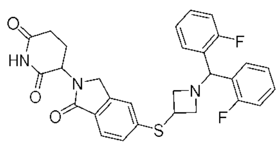 | 3-(5-((1-(bis(2-fluorophenyl) methyl)azetidi n-3-yl)thio)-1-oxoisoindolin-2-yl)piperidine-2,6-dione | 3-(5-((1-(bis(2-fluorophenyl) methyl)azetid in-3-yl)thio)-1-oxoisoindolin-2-yl)piperidine-2,6-dione |
| 68 GT-06349 | 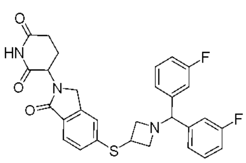 | 3-(5-((1-(bis(3-fluorophenyl) methyl)azetidi n-3-yl)thio)-1-oxoisoindolin-2-yl)piperidine-2,6-dione | 3-(5-((1-(bis(3-fluorophenyl) methyl)azetid in-3-yl)thio)-1-oxoisoindolin-2-yl)piperidine-2,6-dione |
| 69 GT-06503 | 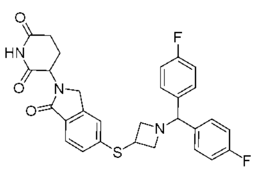 | 3-(5-((1-(bis(4-fluorophenyl) methyl)azetidi n-3-yl)thio)-1-oxoisoindolin-2-yl)piperidine-2,6-dione | 3-(5-((1-(bis(4-fluorophenyl) methyl)azetid in-3-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 70 GT-06504 | 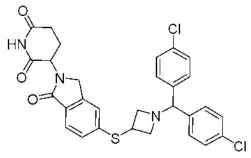 | 3-(5-((1-(bis(4-chlorophenyl) methyl)azetidi n-3-yl)thio)-1-oxoisoindolin-2-yl)piperidine-2,6-dione | 3-(5-((1-(bis(4-chlorophenyl) methyl)azetid in-3-yl)thio)-1-oxoisoindolin-2-yl)piperidine-2,6-dione |
| 71 | 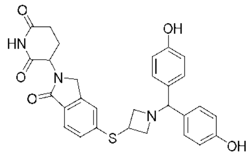 | 3-(5-((1-(bis(4-hydroxyphenyl) methyl)azeti din-3-yl)thio)-1-oxoisoindolin-2-yl)piperidine-2,6-dione | 3-(5-((1-(bis(4-hydroxyphenyl) methyl)azet idin-3-yl)thio)-1-oxoisoindolin-2-yl)piperidine-2,6-dione |
| 72 GT-06923 | 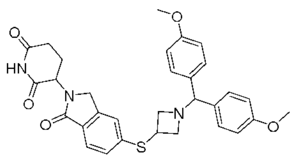 | 3-(5-((1-(bis(4-methoxyphenyl) methyl)azet idin-3-yl)thio)-1-oxoisoindolin-2-yl)piperidine-2,6-dione | 3-(5-((1-(bis(4-methoxyphenyl) methyl)aze tidin-3-yl)thio)-1-oxoisoindolin-2-yl)piperidine-2,6-dione |
| 73 | 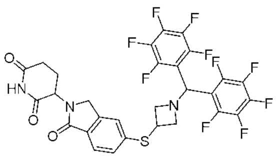 | 3-(5-((1-(bis(perfluorophenyl) methyl )azetidin-3-yl)thio)-1-oxoisoindolin-2-yl)piperidine-2,6-dione | 3-(5-((1-(bis(perfluorophenyl) methy 1)azetidin-3-yl)thio)-1-oxoisoindolin-2-yl)piperidine-2,6-dione |
| 74 GT-06518 | 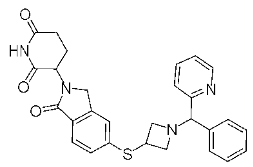 | 3-(1-oxo-5-((1-(phenyl(pyridin-2-yl)methyl)azetidin-3-yl) thio)isoindolin-2-yl)piperidine-2,6-dione | 3-(1-oxo-5-((1-(phenyl(pyridin-2-yl)methyl)azetidin-3-yl) thio)isoindolin-2-yl)piperidine-2,6-dione |
| 75 GT-06807 | 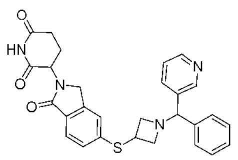 | 3-(1-oxo-5-((1-(phenyl(pyridin-3-yl)methyl)azetidin-3-yl) thio)isoindolin-2-yl)piperidine-2,6-dione | 3-(1-oxo-5-((1-(phenyl(pyridin-3-yl)methyl)azetidin-3-yl) thio)isoindolin-2-yl)piperidine-2,6-dione |
| 76 | 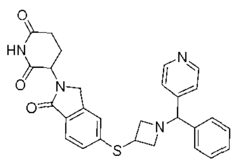 | 3-(1-oxo-5-((1-(phenyl(pyridin-4-yl)methyl)azetidin-3-yl) thio)isoindolin-2-yl)piperidine-2,6-dione | 3-(1-oxo-5-((1-(phenyl(pyridin-4-yl)methyl)azetidin-3-yl) thio)isoindolin-2-yl)piperidine-2,6-dione |
| 77 GT-06811 | 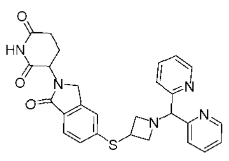 | 3-(5-((1-(di(pyridin-2-yl)methyl) azetidin-3-yl)thio)-1-oxoisoindolin-2-yl)piperidine-2,6-dione | 3-(5-((1-(di(pyridin-2-yl)methyl) azetidin-3-yl)thio)-1-oxoisoindolin-2-yl)piperidine-2,6-dione |
| 78 | 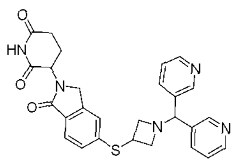 | 3-(5-((1-(di(pyridin-3-yl)methyl) azetidin-3-yl)thio)-1-oxoisoindolin-2-yl)piperidine-2,6-dione | 3-(5-((1-(di(pyridin-3-yl)methyl) azetidin-3-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 79 | 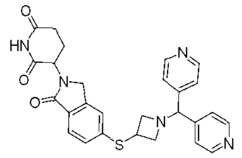 | 3-(5-((1-(di(pyridin-4-yl)methyl) azetidin-3-yl)thio)-1-oxoisoindolin-2-yl)piperidine-2,6-dione | 3-(5-((1-(di(pyridin-4-yl)methyl) azetidin-3-yl)thio)-1-oxoisoindolin-2-yl)piperidine-2,6-dione |
| 80 | 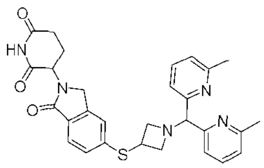 | 3-(5-((1-(bis(6-methylpyridin-2-yl)methyl)azetidin-3-yl)thio)-1-oxoisoindolin-2-vl)piperidine-2,6-dione | 3-(5-((1-(bis(6-methylpyridin-2-yl)methyl)azetidin-3-yl)thio)-1-oxoisoindolin-2-yl)piperidine-2,6-dione |
| 81 | 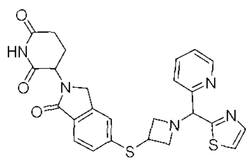 | 3-(1-oxo-5-((1-(pyridin-2-yl(thiazol-2-yl)methyl)azetidin-3-yl) thio)isoindolin-2-yl)piperidine-2,6-dione | 3-(1-oxo-5-((1-(pyridin-2-yl(thiazol-2-yl)methyl)azetidin-3-yl) thio)isoindolin-2-yl)piperidine-2,6-dione |
| 82 | 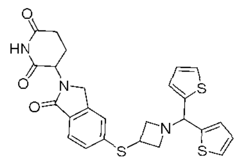 | 3-(5-((1-(di(thiophen-2-yl) methyl)azetidin-3-yl)thio)-1-oxoisoindolin-2-yl)piperidine-2,6-dione | 3-(5-((1-(di(thiophen-2-yl) methyl)azetidin-3-yl)thio)-1-oxoisoindolin-2-yl)piperidine-2,6-dione |
| 83 | 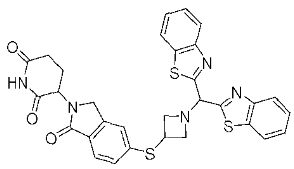 | 3-(5-((1-(bis(benzo[d]thiazol-2-yl)methyl)azetidin-3-yl)thio)-1-oxoisoindolin-2-yl)piperidine-2,6-dione | 3-(5-((1-(bis(benzo[d]thiazol-2-yl)methyl)azetidin-3-yl)thio)-1-oxoisoindolin-2-yl)piperidine-2,6-dione |
| 84 GT-03369 | 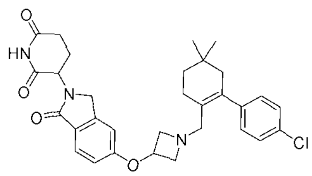 | 3-(5-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione |
| *85* | 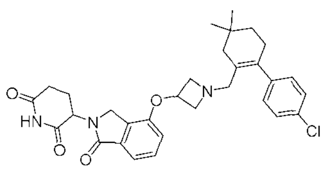 | 3-(4-((1-((4'-chloro-4,4-dimethyl-3,4,5,6-tetrahydro-[1,1'-biphenyl]-2-yl)methyl)azetidin-3-yl)oxy)-1-oxoisoindolin-2-yl)piperidine-2,6-dione | 3-(4-((1-((4'-chloro-4,4-dimethyl-3,4,5,6-tetrahydro-[1,1'-biphenyl]-2-yl)methyl)azetidin-3-yl)oxy)-1-oxoisoindolin-2-yl)piperidine-2,6-dione |
| 86 GT-06872 | 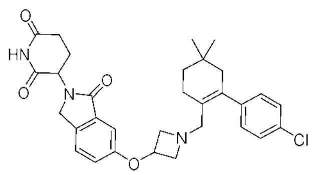 | 3-(6-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione | 3-(6-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione |
| 87 | 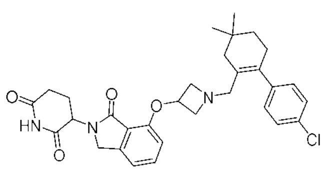 | 3-(7-((1-((4'-chloro-4,4-dimethyl-3,4,5,6-tetrahydro-[1,1'-biphenyl]-2-yl)methyl)azetidin-3-yl)oxy)-1-oxoisoindolin-2-yl)piperidine-2,6-dione | 3-(7-((1-((4'-chloro-4,4-dimethyl-3,4,5,6-tetrahydro-[1,1'-biphenyl]-2-yl)methyl)azetidin-3-yl)oxy)-1-oxoisoindolin-2-yl)piperidine-2,6-dione |

(continued)

| Compound No. | Structure of the compounds | The compounds' name | Chinese translation of the compounds' name |
|---|---|---|---|
| 88 GT-06815 | 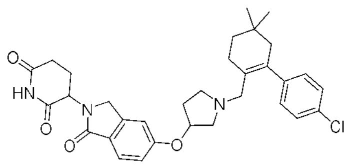 | 3-(5-((1-((4'-chloro-5,5-dimethyl-3,4,5,6-tetrahydro-[1,1'-biphenyl]-2-yl)methyl)pyrrolidin-3-yl)oxy)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)pyrrolidin-3-yl)oxy)-1-oxoisoindolin-2-yl)piperidine-2,6-dione |
| 89 GT-07061 | 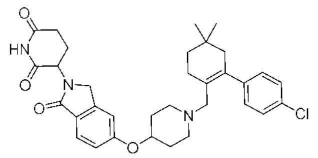 | 3-(5-((1-((4'-chloro-5,5-dimethyl-3,4,5,6-tetrahydro-[1,1'-biphenyl]-2-yl)methyl)piperidin-4-yl)oxy)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)piperidin-4-yl)oxy)-1-oxoisoindolin-2-yl)piperidine-2,6-dione |
| 90 GT-06929 | 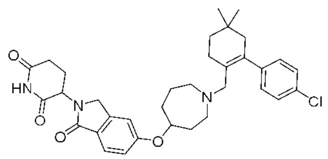 | 3-(5-((1-((4'-chloro-5,5-dimethyl-3,4,5,6-tetrahydro-[1,1'-biphenyl]-2-yl)methyl)azepan-4-yl)oxy)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)azepan-4-yl)oxy)-1-oxoisoindolin-2-yl)piperidine-2,6-dione |
| 91 | 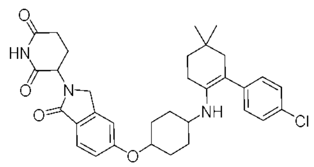 | 3-(5-((4-((4'-chloro-5,5-dimethyl-3,4,5,6-tetrahydro-[1,1'-biphenyl]-2-yl)amino)cyclohexyl)oxy)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)amino)cyclohexyl)oxy)-1-oxoisoindolin-2-yl)piperidine-2,6-dione |
| 92 | 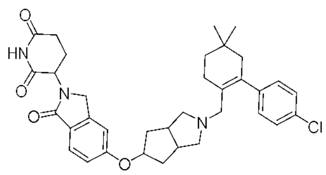 | 3-(5-((2-((4'-chloro-5,5-dimethyl-3,4,5,6-tetrahydro-[1,1'-biphenyl]-2-yl)methyl)octahydrocyclope nta[c]pyrrol-5-yl) oxy)-1-oxoisoindolin-2-yl)piperidine-2,6-dione | 3-(5-((2-((4'-chloro-5,5-dimethyl-3,4,5,6-tetrahydro-[1,1'-biphenyl]-2-yl)methyl)octahydrocyclop enta[c]pyrrol-5-yl) oxy)-1-oxoisoindolin-2-yl)piperidine-2,6-dione |
| 93 | 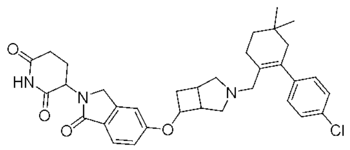 | 3-(5-((3-((4'-chloro-5,5-dimethyl-3,4,5,6-tetrahydro-[1,1'-biphenyl]-2-yl)methyl)-3-azabicyclo[3.2.0]heptan-6-yl)oxy)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azabicyclo[3.2.0]heptan-6-yl)oxy)-1-oxoisoindolin-2-yl)piperidine-2,6-dione |
| 94 | 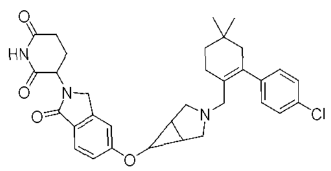 | 3-(5-((3-((4'-chloro-5,5-dimethyl-3,4,5,6-tetrahydro-[1,1'-biphenyl]-2-yl)methyl)-3-azabicyclo[3.1.0]hexan-6-yl)oxy)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azabicyclo[3.1.0]hexan-6-yl)oxy)-1-oxoisoindolin-2-yl)piperidine-2,6-dione |
| 95 GT-06933 | 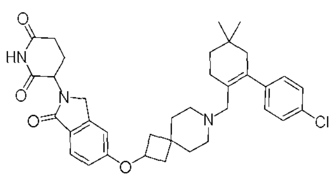 | 3-(5-((7-((4'-chloro-5,5-dimethyl-3,4,5,6-tetrahydro-[1,1'-biphenyl]-2-yl)methyl)-7-azaspiro[3.5]nonan-2-yl)oxy)-1-oxoisoindolin-2-yl)piperidine-2,6-dione | 3-(5-((7-((4'-chloro-5,5-dimethyl-3,4,5,6-tetrahydro-[1,1'-biphenyl]-2-yl)methyl)-7-azaspiro[3.5]nonan-2-yl)oxy)-1-oxoisoindolin-2-yl)piperidine-2,6-dione |

(continued)

| Compound No. | Structure of the compounds | The compounds' name | Chinese translation of the compounds' name |
|---|---|---|---|
| 96 | 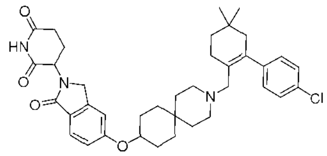 | 3-(5-((3-((4'-chloro-5,5-dimethyl-3,4,5,6-tetrahydro-[1,1'-biphenyl]-2-yl)methyl)-3-azaspiro[5.5]undecan-9-yl)oxy)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azaspiro[5.5]undecan-9-yl)oxy)-1-oxoisoindolin-2-yl)piperidine-2,6-dione |
| 97 | 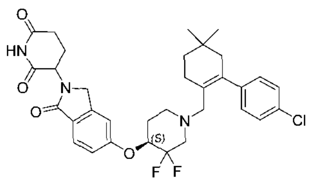 | 3-(5-(((S)-1-((4'-chloro-5,5-dimethyl-3,4,5,6-tetrahydro-[1,1'-biphenyl]-2-yl)methyl)-3,3-difluoropiperidin-4-yl)oxy)-1-oxoisoindolin-2-yl)piperidine-2,6-dione | 3-(5-(((S)-1-((4'-chloro-5,5-dimethyl-3,4,5,6-tetrahydro-[1,1'-biphenyl]-2-yl)methyl)-3,3-difluoropiperidin-4-yl)oxy)-1-oxoisoindolin-2-yl)piperidine-2,6-dione |
| 98 | 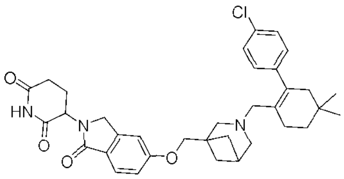 | 3-(5-((3-((4'-chloro-5,5-dimethyl-3,4,5,6-tetrahydro-[1,1'-biphenyl]-2-yl)methyl)-3-azabicyclo[3.1.1]heptan-1-yl)methoxy)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azabicyclo[3.1.1]heptan-1-yl)methoxy)-1-oxoisoindolin-2-yl)piperidine-2,6-dione |
| 99 | 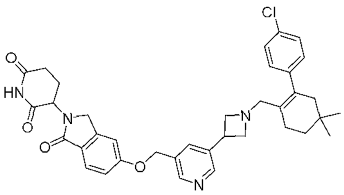 | 3-(5-((5-(1-((4'-chloro-5,5-dimethyl-3,4,5,6-tetrahydro-[1,1'-biphenyl]-2-yl)methyl)azetidin-3-yl)pyridin-3-yl)methoxy)-1-oxoisoindolin-2-yl)piperidine-2,6-dione | 3-(5-((5-(1-((4'-chloro-5,5-dimethyl-3,4,5,6-tetrahydro-[1,1'-biphenyl]-2-yl)methyl)azetidin-3-yl)pyridin-3-yl)methoxy)-1-oxoisoindolin-2-yl)piperidine-2,6-dione |
| 100 GT-06180 | 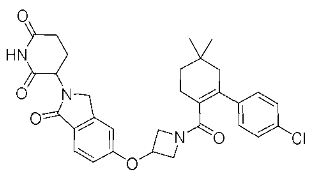 | 3-(5-((1-(4'-chloro-5,5-dimethyl-3,4,5,6-tetrahydro-[1,1'-biphenyl]-2-carbonyl)azetidin-3-yl)oxy)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)azetidin-3-yl)oxy)-1-oxoisoindolin-2-yl)piperidine-2,6-dione |
| 101 GT-06816 | 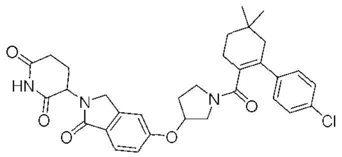 | 3-(5-((1-(4'-chloro-5,5-dimethyl-3,4,5,6-tetrahydro-[1,1'-biphenyl]-2-carbonyl)pyrrolidin-3-yl)oxy)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)pyrrolidin-3-yl)oxy)-1-oxoisoindolin-2-yl)piperidine-2,6-dione |
| 102 GT-06192 | 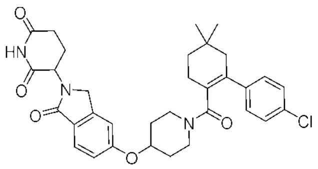 | 3-(5-((1-(4'-chloro-5,5-dimethyl-3,4,5,6-tetrahydro-[1,1'-biphenyl]-2-carbonyl)piperidin-4-yl)oxy)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)piperidin-4-yl)oxy)-1-oxoisoindolin-2-yl)piperidine-2,6-dione |
| 103 GT-07018 | 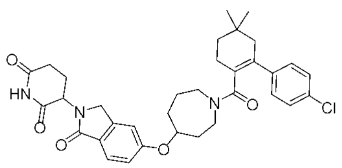 | 3-(5-((1-(4'-chloro-5,5-dimethyl-3,4,5,6-tetrahydro-[1,1'-biphenyl]-2-carbonyl)azepan-4-yl)oxy)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)azepan-4-yl)oxy)-1-oxoisoindolin-2-yl)piperidine-2,6-dione |

(continued)

| Compo und No. | Structure of the compounds | The compounds' name | Chinese translation of the compounds' name |
|---|---|---|---|
| 104 | 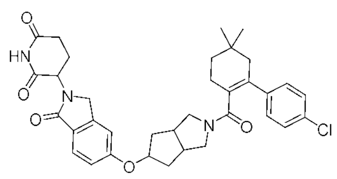 | 3-(5-((2-(4'-chloro-5,5-dimethyl-3,4,5,6-tetrahydro-[1,1'-biphenyl]-2-carbonyl)octahydrocyclope nta[c]pyrrol-5-yl) oxy)-1-oxoisoindolin-2-yl)piperidine-2,6-dione | 3-(5-((2-(4'-chloro-5,5-dimethyl-3,4,5,6-tetrahydro-[1,1'-biphenyl]-2-carbonyl)octahydrocyclope nta[c]pyrrol-5-yl)oxy)-1-oxoisoindolin-2-yl)piperidine-2,6-dione |
| 105 | 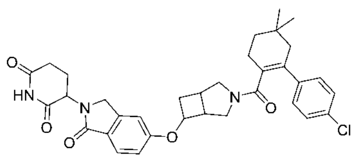 | 3-(5-((3-(4'-chloro-5,5-dimethyl-3,4,5,6-tetrahydro-[1,1'-biphenyl]-2-carbonyl)-3-azabicyclo[3.2.0]heptan-6-yl)oxy)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3-azabicyclo[3.2.0]heptan-6-yl)oxy)-1-oxoisoindolin-2-yl)piperidine-2,6-dione |
| 106 | 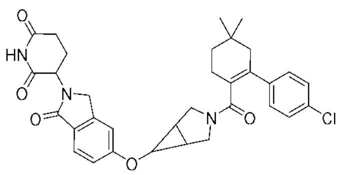 | 3-(5-((3-(4'-chloro-5,5-dimethyl-3,4,5,6-tetrahydro-[1,1'-biphenyl]-2-carbonyl)-3-azabicyclo[3.1.0]hexan-6-yl)oxy)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3-azabicyclo[3.1.0]hexan-6-yl)oxy)-1-oxoisoindolin-2-yl)piperidine-2,6-dione |
| 107 GT-07020 | 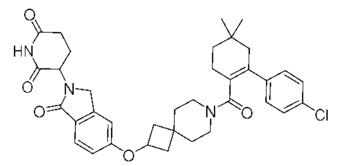 | 3-(5-((7-(4'-chloro-5,5-dimethyl-3,4,5,6-tetrahydro-[1,1'-biphenyl]-2-carbonyl)-7-azaspiro[3.5]nonan-2-yl)oxy)-1-oxoisoindolin-2-yl)piperidine-2,6-dione | 3-(5-((7-(4'-chloro-5,5-dimethyl-3,4,5,6-tetrahydro-[1,1'-biphenyl]-2-carbonyl)-7-azaspiro[3.5]nonan-2-yl)oxy)-1-oxoisoindolin-2-yl)piperidine-2,6-dione |
| 108 | 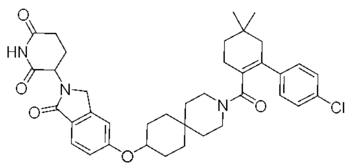 | 3-(5-((3-(4'-chloro-5,5-dimethyl-3,4,5,6-tetrahydro-[1,1'-biphenyl]-2-carbonyl)-3-azaspiro[5 .5]undecan-9-yl)oxy)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3-azaspiro[5.5]undecan-9-yl)oxy)-1-oxoisoindolin-2-yl)piperidine-2,6-dione |
| 109 | 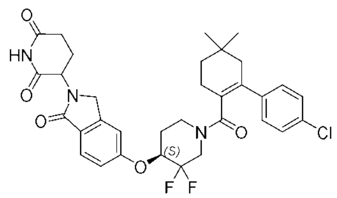 | 3-(5-(((S)-1-(4'-chloro-5,5-dimethyl-3,4,5,6-tetrahydro-[1,1'-biphenyl]-2-carbonyl)-3,3-difluoropiperidin-4-yl)oxy)-1-oxoisoindolin-2-yl)piperidine-2,6-dione | 3-(5-(((S)-1-(4'-chloro-5,5-dimethyl-3,4,5,6-tetrahydro-[1,1'-biphenyl]-2-carbonyl)-3,3-difluoropiperidin-4-yl)oxy)-1-oxoisoindolin-2-yl)piperidine-2,6-dione |
| 110 | 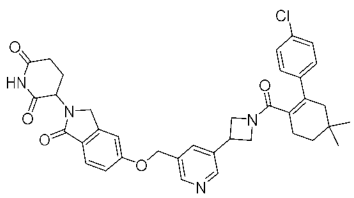 | 3-(5-((5-(1-(4'-chloro-5,5-dimethyl-3,4,5,6-tetrahydro-[1,1'-biphenyl]-2-carbonyl)azetidin-3-yl)pyridin-3-yl)methoxy)-1-oxoisoindolin-2-yl)piperidine-2,6-dione | 3-(5-((5-(1-(4'-chloro-5,5-dimethyl-3,4,5,6-tetrahydro-[1,1'-biphenyl]-2-carbonyl)azetidin-3-yl)pyridin-3-yl)methoxy)-1-oxoisoindolin-2-yl)piperidine-2,6-dione |
| 111 GT-03363 | 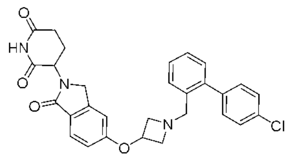 | 3-(5-((1-((4'-chloro-[1,1'-biphenyl]-2-yl)methyl)azetidin-3-yl) oxy)-1-oxoisoindolin-2-yl)piperidine-2,6-dione | 3-(5-((1-((4'-chloro-[1,1'-biphenyl]-2-yl)methyl)azetidin-3-yl) oxy)-1-oxoisoindolin-2-yl)piperidine-2,6-dione |

(continued)

| Compo und No. | Structure of the compounds | The compounds' name | Chinese translation of the compounds' name |
|---|---|---|---|
| 112 GT-06817 | 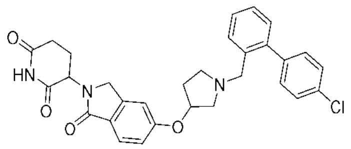 | 3-(5-((1-((4'-chloro-[1,1'-biphenyl]-2-yl)methyl)pyrrolidin-3-yl) oxy)-1-oxoisoindolin-2-yl)piperidine-2,6-dione | 3-(5-((1-((4'-chloro-[1,1'-biphenyl]-2-yl)methyl)pyrrolidin-3-yl) oxy)-1-oxoisoindolin-2-yl)piperidine-2,6-dione |
| 113 GT-06193 | 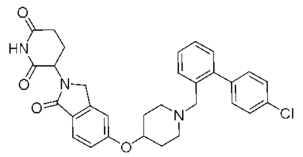 | 3-(5-((1-((4'-chloro-[1,1'-biphenyl]-2-yl)methyl)piperidin-4-yl) oxy)-1-oxoisoindolin-2-yl)piperidine-2,6-dione | 3-(5-((1-((4'-chloro-[1,1'-biphenyl]-2-yl)methyl)piperidin-4-yl) oxy)-1-oxoisoindolin-2-yl)piperidine-2,6-dione |
| 114 GT-06934 | 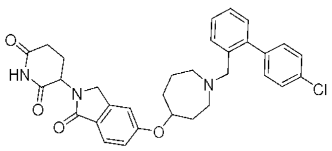 | 3-(5-((1-((4'-chloro-[1,1'-biphenyl]-2-yl)methyl)azepan-4-yl) oxy)-1-oxoisoindolin-2-yl)piperidine-2,6-dione | 3-(5-((1-((4'-chloro-[1,1'-biphenyl]-2-yl)methyl)azepan-4-yl) oxy)-1-oxoisoindolin-2-yl)piperidine-2,6-dione |
| 115 GT-06953 | 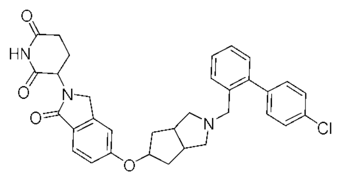 | 3-(5-((2-((4'-chloro-[1,1'-biphe-nyl]-2-yl)methyl)octahydrocy-clope nta[c]pyrrol-5-yl)oxy)-1-oxoisoindolin-2-yl)piperidine-2,6-dione | 3-(5-((2-((4'-chloro-[1,1'-biphe-nyl]-2-yl)methyl)octahydrocy-clop enta[c]pyrrol-5-yl)oxy)-1-oxoisoindolin-2-yl)piperidine-2,6-dione |
| 116 | 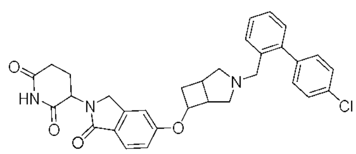 | 3-(5-((3-((4'-chloro-[1,1'-biphenyl]-2-yl)methyl)-3-azabicyclo [3.2.0]heptan-6-yl)oxy)-1-oxoisoindolin-2-yl)piperidine-2,6-dione | 3-(5-((3-((4'-chloro-[1,1'-biphenyl]-2-yl)methyl)-3-azabicyclo [3.2.0]heptan-6-yl)oxy)-1-oxoisoindolin-2-yl)piperidine-2,6-dione |
| 117 | 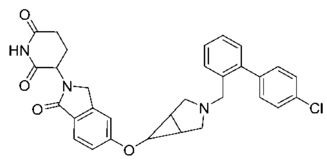 | 3-(5-((3-((4'-chloro-[1,1'-biphenyl]-2-yl)methyl)-3-azabicyclo [3.1.0]hexan-6-yl)oxy)-1-oxoisoindolin-2-yl)piperidine-2,6-dione | 3-(5-((3-((4'-chloro-[1,1'-biphenyl]-2-yl)methyl)-3-azabicyclo [3.1.0]hexan-6-yl)oxy)-1-oxoisoindolin-2-yl)piperidine-2,6-dione |
| 118 | 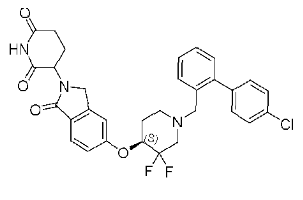 | 3-(5-(((S)-1-((4'-chloro-[1,1'-biphenyl]-2-yl)methyl)-3,3-difluoropiperidin-4-yl)oxy)-1-oxoisoindolin-2-yl)piperidine-2,6-dione | 3-(5-(((S)-1-((4'-chloro-[1,1'-biphenyl]-2-yl)methyl)-3,3-difluoropiperidin-4-yl)oxy)-1-oxoisoindolin-2-yl)piperidine-2,6-dione |
| 119 GT-06183 | 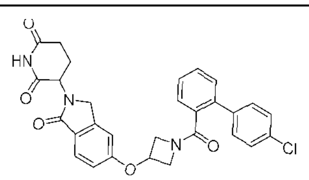 | 3-(5-((1-(4'-chloro-[1,1'-biphenyl]-2-carbonyl)azetidin-3-yl) oxy)-1-oxoisoindolin-2-yl)piperidine-2,6-dione | 3-(5-((1-(4'-chloro-[1,1'-biphenyl]-2-carbonyl)azetidin-3-yl) oxy)-1-oxoisoindolin-2-yl)piperidine-2,6-dione |
| 120 GT-06818 | 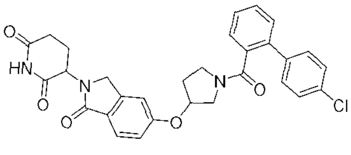 | 3-(5-((1-(4'-chloro-[1,1'-biphenyl]-2-carbonyl)pyrrolidin-3-yl) oxy)-1-oxoisoindolin-2-yl)piperidine-2,6-dione | 3-(5-((1-(4'-chloro-[1,1'-biphenyl]-2-carbonyl)pyrrolidin-3-yl) oxy)-1-oxoisoindolin-2-yl)piperi-dine-2,6-dione |

(continued)

| Compo und No. | Structure of the compounds | The compounds' name | Chinese translation of the compounds' name |
|---|---|---|---|
| 121 GT-06204 | 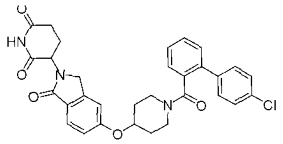 | 3-(5-((1-(4'-chloro-[1,1'-biphenyl]-2-carbonyl)piperidin-4-yl) oxy)-1-oxoisoindolin-2-yl)piperidine-2,6-dione | 3-(5-((1-(4'-chloro-[1,1'-biphenyl]-2-carbonyl)piperidin-4-yl) oxy)-1-oxoisoindolin-2-yl)piperidine-2,6-dione |
| 122 GT-06935 | 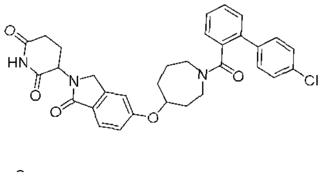 | 3-(5-((1-(4'-chloro-[1,1'-biphenyl]-2-carbonyl)azepan-4-yl) oxy)-1-oxoisoindolin-2-yl)piperidine-2,6-dione | 3-(5-((1-(4'-chloro-[1,1'-biphenyl]-2-carbonyl)azepan-4-yl) oxy)-1-oxoisoindolin-2-yl)piperidine-2,6-dione |
| 123 GT-06954 | 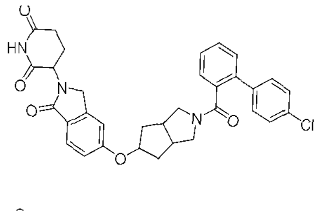 | 3-(5-((2-(4'-chloro-[1,1'-biphenyl]-2-carbonyl)octahydrocyclope nta[c]pyrrol-5-yl)oxy)-1-oxoisoindolin-2-yl)piperidine-2,6-dione | 3-(5-((2-(4'-chloro-[1,1'-biphenyl]-2-carbonyl)octahydrocyclope nta[c]pyrrol-5-yl)oxy)-1-oxoisoindolin-2-yl)piperidine-2,6-dione |
| 124 | 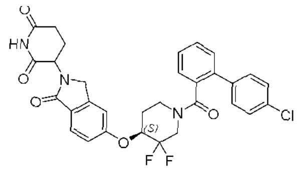 | 3-(5-(((S)-1-(4'-chloro-[1,1'-biphenyl]-2-carbonyl)-3,3-difluoropiperidin-4-yl)oxy)-1-oxoisoindolin-2-yl)piperidine-2,6-dione | 3-(5-(((S)-1-(4'-chloro-[1,1'-biphenyl]-2-carbonyl)-3,3-difluoropiperidin-4-yl)oxy)-1-oxoisoindolin-2-yl)piperidine-2,6-dione |
| 125 GT-06227 | 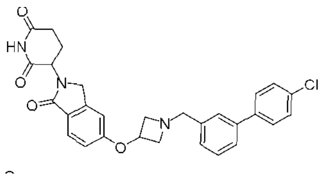 | 3-(5-((1-((4'-chloro-[1,1'-biphenyl]-3-yl)methyl)azetidin-3-yl) oxy)-1-oxoisoindolin-2-yl)piperidine-2,6-dione | 3-(5-((1-((4'-chloro-[1,1'-biphenyl]-3-yl)methyl)azetidin-3-yl) oxy)-1-oxoisoindolin-2-yl)piperidine-2,6-dione |
| 126 GT-06230 | 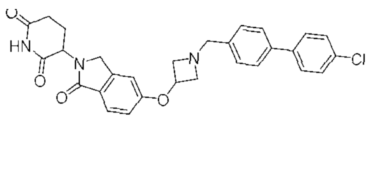 | 3-(5-((1-((4'-chloro-[1,1'-biphenyl]-4-yl)methyl)azetidin-3-yl) oxy)-1-oxoisoindolin-2-yl)piperidine-2,6-dione | 3-(5-((1-((4'-chloro-[1,1'-biphenyl]-4-yl)methyl)azetidin-3-yl) oxy)-1-oxoisoindolin-2-yl)piperidine-2,6-dione |
| 127 GT-06809 | 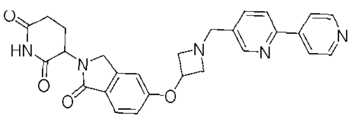 | 3-(5-((1-([2,4'-bipyridin]-5-ylmethyl)azetidin-3-yl)oxy)-1-oxoisoindolin-2-yl)piperidine-2,6-dione | 3-(5-((1-([2,4'-bipyridin]-5-ylmethyl)azetidin-3-yl)oxy)-1-oxoisoindolin-2-yl)piperidine-2,6-dione |
| 128 GT-06353 | 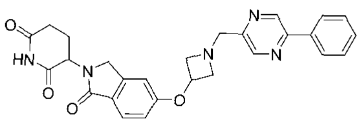 | 3-(1-oxo-5-((1-((5-phenylpyrazin-2-yl)methyl)azetidin-3-yl) oxy)isoindolin-2-yl)piperidine-2,6-dione | 3-(1-oxo-5-((1-((5-phenylpyrazin-2-yl)methyl)azetidin-3-yl) oxy)isoindolin-2-yl)piperidine-2,6-dione |
| 129 GT-06562 | 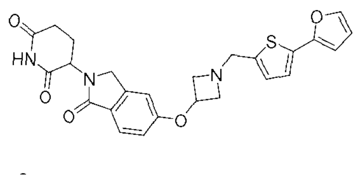 | 3-(5-((1-((5-(furan-2-yl)thiophen-2-yl)methyl)azetidin-3-yl) oxy)-1-oxoisoindolin-2-yl)piperidine-2,6-dione | 3-(5-((1-((5-(furan-2-yl)thiophen-2-yl)methyl)azetidin-3-yl) oxy)-1-oxoisoindolin-2-yl)piperidine-2,6-dione |

(continued)

| Compo und No. | Structure of the compounds | The compounds’ name | Chinese translation of the compounds’ name |
|---|---|---|---|
| 130 GT-03459 | 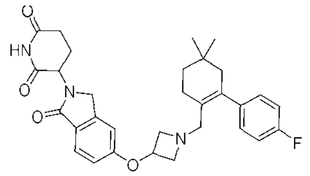 | 3-(5-((1-((4’-fluoro-5,5-dimethyl-3,4,5,6-tetrahydro-[1,1’-biphenyl]-2-yl)methyl)azetidin-3-yl)oxy)-1-oxoisoindolin-2-yl)piperidine-2,6-dione | 3-(5-((1-((4’-fluoro-5,5-dimethyl-3,4,5,6-tetrahydro-[1,1’-biphenyl]-2-yl)methyl)azetidin-3-yl)oxy)-1-oxoisoindolin-2-yl)piperidine-2,6-dione |
| 131 GT-07062 | 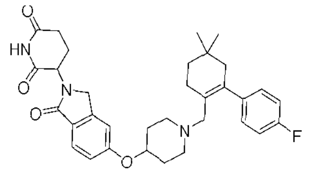 | 3-(5-((1-((4’-fluoro-5,5-dimethyl-3,4,5,6-tetrahydro-[1,1’-biphenyl]-2-yl)methyl)piperidin-4-yl)oxy)-1-oxoisoindolin-2-yl)piperidine-2,6-dione | 3-(5-((1-((4’-fluoro-5,5-dimethyl-3,4,5,6-tetrahydro-[1,1’-biphenyl]-2-yl)methyl)piperidin-4-yl)oxy)-1-oxoisoindolin-2-yl)piperidine-2,6-dione |
| 132 | 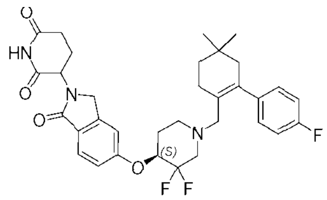 | 3-(5-(((S)-3,3-difluoro-1-((4’-fluoro-5,5-dimethyl-3,4,5,6-tetrahydro-[1,1’-biphenyl]-2-yl) methyl)piperidin-4-yl)oxy)-1-oxoisoindolin-2-yl)piperidine-2,6-dione | 3-(5-(((S)-3,3-difluoro-1-((4’-fluoro-5,5-dimethyl-3,4,5,6-tetrahydro-[1,1’-biphenyl]-2-yl) methyl)piperidin-4-yl)oxy)-1-oxoisoindolin-2-yl)piperidine-2,6-dione |
| 133 GT-06302 | 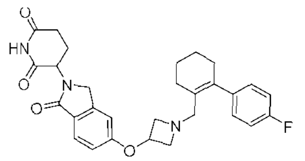 | 3-(5-((1-((4’-fluoro-3,4,5,6-tetrahydro-[1,1’-biphenyl]-2-yl) methyl)azetidin-3-yl)oxy)-1-oxoisoindolin-2-yl)piperidine-2,6-dione | 3-(5-((1-((4’-fluoro-3,4,5,6-tetrahydro-[1, 1’-biphenyl]-2-yl) methyl)azetidin-3-yl)oxy)-1-oxoisoindolin-2-yl)piperidine-2,6-dione |
| 134 GT-06354 | 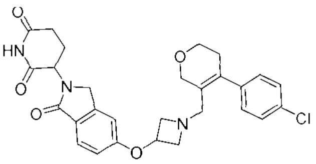 | 3-(5-((1-((4-(4-chlorophenyl)-5,6-dihydro-2H-pyran-3-yl) methyl)azetidin-3-yl)oxy)-1-oxoisoindolin-2-yl)piperidine-2,6-dione | 3-(5-((1-((4-(4-chlorophenyl)-5,6-dihydro-2H-pyran-3-yl) methyl)azetidin-3-yl)oxy)-1-oxoisoindolin-2-yl)piperidine-2,6-dione |
| 135 GT-06560 | 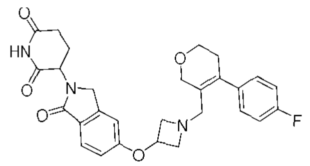 | 3-(5-((1-((4-(4-fluorophenyl)-5,6-dihydro-2H-pyran-3-yl) methyl)azetidin-3-yl)oxy)-1-oxoisoindolin-2-yl)piperidine-2,6-dione | 3-(5-((1-((4-(4-fluorophenyl)-5,6-dihydro-2H-pyran-3-yl) methyl)azetidin-3-yl)oxy)-1-oxoisoindolin-2-yl)piperidine-2,6-dione |
| 136 GT-06563 | 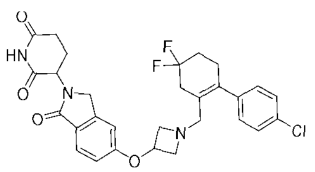 | 3-(5-((1-((4’-chloro-4,4-difluoro-3,4,5,6-tetrahydro-[1,1-biphenyl]-2-yl)methyl)azetidin-3-yl)oxy)-1-oxoisoindolin-2-yl)piperidine-2,6-dione | 3-(5-((1-((4’-chloro-4,4-difluoro-3,4,5,6-tetrahydro-[1,1’-biphenyl]-2-yl)methyl)azetidin-3-yl)oxy)-1-oxoisoindolin-2-yl)piperidine-2,6-dione |

(continued)

| Compound No. | Structure of the compounds | The compounds' name | Chinese translation of the compounds' name |
|---|---|---|---|
| 137 GT-07065 | 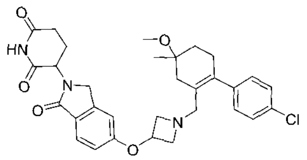 | 3-(5-((1-((4'-chloro-4-methoxy-4-methyl-3,4,5,6-tetrahydro-[1,1'-biphenyl]-2-yl)methyl) azetidin-3-yl)oxy)-1-oxoisoindolin-2-yl)piperidine-2,6-dione | 3-(5-((1-((4'-chloro-4-methoxy-4-methyl-3,4,5,6-tetrahydro-[1,1'-biphenyl]-2-yl)methyl) azetidin-3-yl)oxy)-1-oxoisoindolin-2-yl)piperidine-2,6-dione |
| 138 GT-06561 | 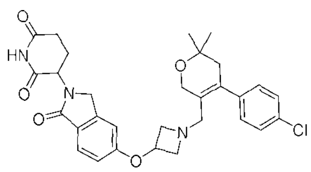 | 3-(5-((1-((4-(4-chlorophenyl)-6,6-dimethyl-5,6-dihydro-2H-pyran-3-yl)methyl)azetidin-3-yl)oxy)-1-oxoisoindolin-2-yl)piperidine-2,6-dione | 3-(5-((1-((4-(4-chlorophenyl)-6,6-dimethyl-5,6-dihydro-2H-pyran-3-yl)methyl)azetidin-3-yl)oxy)-1-oxoisoindolin-2-yl)piperidine-2,6-dione |
| 139 | 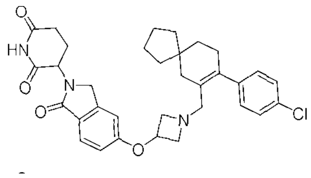 | 3-(5-((1-((8-(4-chlorophenyl) spiro[4.5]dec-7-en-7-yl)methyl) azetidin-3-yl)oxy)-1-oxoisoindolin-2-yl)piperidine-2,6-dione | 3-(5-((1-((8-(4-chlorophenyl) spiro[4.5]dec -7-en-7-yl)methyl) azetidin-3-yl)oxy)-1-oxoisoindolin-2-yl)piperidine-2,6-dione |
| 140 | 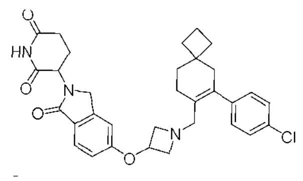 | 3-(5-((1-((6-(4-chlorophenyl) spiro[3.5]non-6-en-7-yl)methyl) azetidin-3-yl)oxy)-1-oxoisoindolin-2-yl)piperidine-2,6-dione | 3-(5-((1-((6-(4-chlorophenyl) spiro[3.5]non -6-en-7-yl)methyl) azetidin-3-yl)oxy)-1-oxoisoindolin-2-yl)piperidine-2,6-dione |
| 141 GT-06185 | 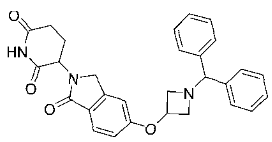 | 3-(5-((1-benzhydrylazetidin-3-yl)oxy)-1-oxoisoindolin-2-yl)piperidine-2,6-dione | 3-(5-((1-benzhydrylazetidin-3-yl)oxy)-1-oxoisoindolin-2-yl)piperidine-2,6-dione |
| 142 GT-06819 | 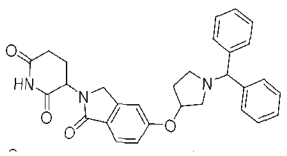 | 3-(5-((1-benzhydrylpyrrolidin-3-yl)oxy)-1-oxoisoindolin-2-yl)piperidine-2,6-dione | 3-(5-((1-benzhydrylpyrrolidin-3-yl)oxy)-1-oxoisoindolin-2-yl)piperidine-2,6-dione |
| 143 GT-06195 | 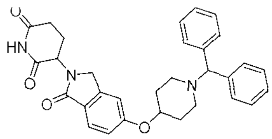 | 3-(5-((1-benzhydrylpiperidin-4-yl)oxy)-1-oxoisoindolin-2-yl)piperidine-2,6-dione | 3-(5-((1-benzhydrylpiperidin-4-yl)oxy)-1-oxoisoindolin-2-yl)piperidine-2,6-dione |
| 144 GT-06928 | 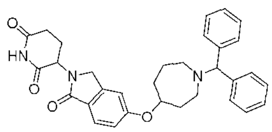 | 3-(5-((1-benzhydrylazepan-4-yl)oxy)-1-oxoisoindolin-2-yl)piperidine-2,6-dione | 3-(5-((1-benzhydrylazepan-4-yl) oxy)-1-oxoisoindolin-2-yl)piperidine-2,6-dione |
| 145 GT-06956 | 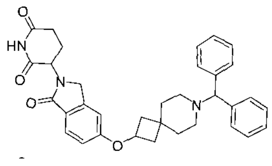 | 3-(5-((7-benzhydryl-7-azaspiro [3.5]nonan-2-yl)oxy)-1-oxoisoindolin-2-yl)piperidine-2,6-dione | 3-(5-((7-benzhydryl-7-azaspiro [3.5]nonan-2-yl)oxy)-1-oxoisoindolin-2-yl)piperidine-2,6-dione |

(continued)

| Compo und No. | Structure of the compounds | The compounds' name | Chinese translation of the compounds' name |
|---|---|---|---|
| 146 | 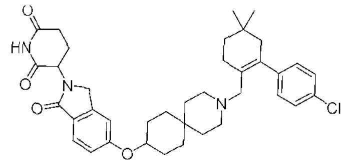 | 3-(5-((3-((4'-chloro-5,5-dimethyl-3,4,5,6-tetrahydro-[1,1'-biphenyl]-2-yl)methyl)-3-azaspiro[5.5]undecan-9-yl)oxy)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3-azaspiro[5.5]undecan-9-yl)oxy)-1-oxoisoindolin-2-yl)piperidine-2,6-dione |
| 147 GT-06955 | 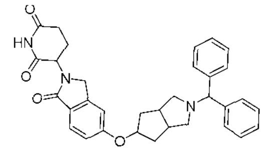 | 3-(5-((2-benzhydryloctahydro-cyclop enta[c]pyrrol-5-yl)oxy)-1-oxoisoindolin-2-yl)piperidine-2,6-dione | 3-(5-((2-benzhydryloctahydrocyclop enta[c]pyrrol-5-yl)oxy)-1-oxoisoindolin-2-yl)piperidine-2,6-dione |
| 148 | 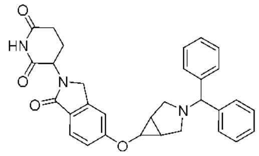 | 3-(5-((3-benzhydryl-3-azabicyclo[3.1.0]hexan-6-yl)oxy)-1-oxoisoindolin-2-yl)piperidine-2,6-dione | 3-(5-((3-benzhydryl-3-azabicyclo[3.1.0]hexan-6-yl)oxy)-1-oxoisoindolin-2-yl)piperidine-2,6-dione |
| 149 | 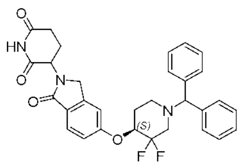 | 3-(5-(((S)-1-benzhydryl-3,3-difluoropiperidin-4-yl)oxy)-1-oxoisoindolin-2-yl)piperidine-2,6-dione | 3-(5-(((S)-1-benzhydryl-3,3-difluoropiperidin-4-yl)oxy)-1-oxoisoindolin-2-yl)piperidine-2,6-dione |
| 150 | 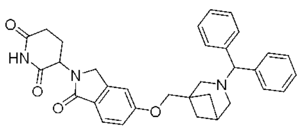 | 3-(5-((3-benzhydryl-3-azabicyclo[3.1.1]heptan-1-yl)methoxy)-1-oxoisoindolin-2-yl)piperidine-2,6-dione | 3-(5-((3-benzhydryl-3-azabicyclo[3.1.1]heptan-1-yl)methoxy)-1-oxoisoindolin-2-yl)piperidine-2,6-dione |
| 151 GT-06759 | 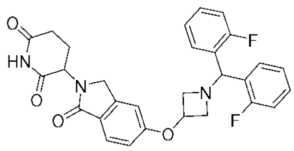 | 3-(5-((1-(bis(2-fluorophenyl) methyl)azetidi n-3-yl)oxy)-1-oxoisoindolin-2-yl)piperidine-2,6-dione | 3-(5-((1-(bis(2-fluorophenyl) methyl)azetid in-3-yl)oxy)-1-oxoisoindolin-2-yl)piperidine-2,6-dione |
| 152 GT-06352 | 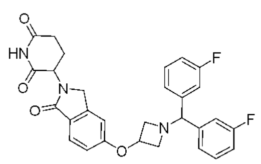 | 3-(5-((1-(bis(3-fluorophenyl) methyl)azetidi n-3-yl)oxy)-1-oxoisoindolin-2-yl)piperidine-2,6-dione | 3-(5-((1-(bis(3-fluorophenyl) methyl)azetid in-3-yl)oxy)-1-oxoisoindolin-2-yl)piperidine-2,6-dione |
| 153 GT-06519 | 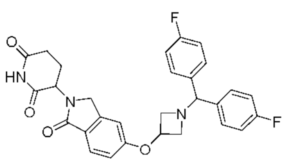 | 3-(5-((1-(bis(4-fluorophenyl) methyl)azetidi n-3-yl)oxy)-1-oxoisoindolin-2-yl)piperidine-2,6-dione | 3-(5-((1-(bis(4-fluorophenyl) methyl)azetid in-3-yl)oxy)-1-oxoisoindolin-2-yl)piperidine-2,6-dione |
| 154 GT-06520 | 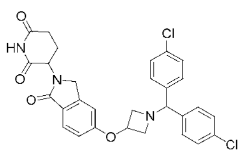 | 3-(5-((1-(bis(4-chlorophenyl) methyl)azetidi n-3-yl)oxy)-1-oxoisoindolin-2-yl)piperidine-2,6-dione | 3-(5-((1-(bis(4-chlorophenyl) methyl)azetid in-3-yl)oxy)-1-oxoisoindolin-2-yl)piperidine-2,6-dione |

(continued)

| Compo und No. | Structure of the compounds | The compounds' name | Chinese translation of the compounds' name |
|---|---|---|---|
| 155 | 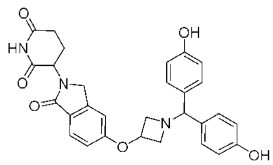 | 3-(5-((1-(bis(4-hydroxyphenyl) methyl)azeti din-3-yl)oxy)-1-oxoisoindolin-2-yl)piperidine-2,6-dione | 3-(5-((1-(bis(4-hydroxyphenyl) methyl)azet idin-3-yl)oxy)-1-oxoisoindolin-2-yl)piperidine-2,6-dione |
| 156 GT-06959 | 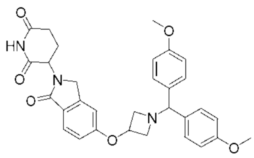 | 3-(5-((1-(bis(4-methoxyphenyl) methyl)azet idin-3-yl)oxy)-1-oxoisoindolin-2-yl)piperidine-2,6-dione | 3-(5-((1-(bis(4-methoxyphenyl) methyl)aze tidin-3-yl)oxy)-1-oxoisoindolin-2-yl)piperidine-2,6-dione |
| 157 | 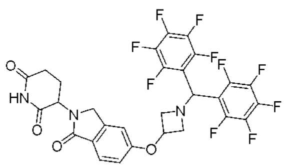 | 3-(5-((1-(bis(perfluorophenyl) methyl )azetidin-3-yl)oxy)-1-oxoisoindolin-2-yl)piperidine-2,6-dione | 3-(5-((1-(bis(perfluorophenyl) methy 1)azetidin-3-yl)oxy)-1-oxoisoindolin-2-yl)piperidine-2,6-dione |
| 158 GT-06559 | 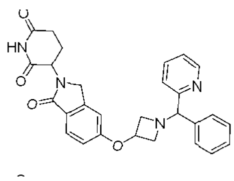 | 3-(1-oxo-5-((1-(phenyl(pyridin-2-yl)methyl)azetidin-3-yl) oxy)isoindolin-2-yl)piperidine-2,6-dione | 3-(1-oxo-5-((1-(phenyl(pyridin-2-yl)methyl)azetidin-3-yl) oxy)isoindolin-2-yl)piperidine-2,6-dione |
| 159 GT-06760 | 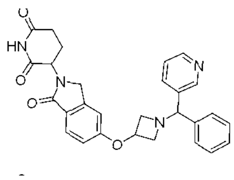 | 3-(1-oxo-5-((1-(phenyl(pyridin-3-yl)methyl)azetidin-3-yl) oxy)isoindolin-2-yl)piperidine-2,6-dione | 3-(1-oxo-5-((1-(phenyl(pyridin-3-yl)methyl)azetidin-3-yl) oxy)isoindolin-2-yl)piperidine-2,6-dione |
| 160 | 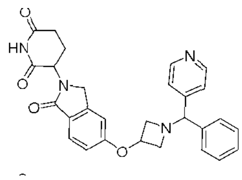 | 3-(1-oxo-5-((1-(phenyl(pyridin-4-yl)methyl)azetidin-3-yl) oxy)isoindolin-2-yl)piperidine-2,6-dione | 3-(1-oxo-5-((1-(phenyl(pyridin-4-yl)methyl)azetidin-3-yl) oxy)isoindolin-2-yl)piperidine-2,6-dione |
| 161 GT-06814 | 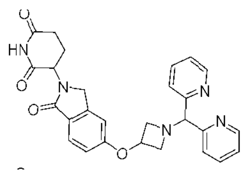 | 3-(5-((1-(di(pyridin-2-yl)methyl) azetidin-3-yl)oxy)-1-oxoisoindolin-2-yl)piperidine-2,6-dione | 3-(5-((1-(di(pyridin-2-yl)methyl) azetidin-3-yl)oxy)-1-oxoisoindolin-2-yl)piperidine-2,6-dione |
| 162 | 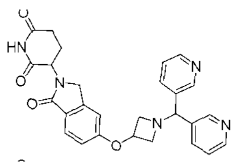 | 3-(5-((1-(di(pyridin-3-yl)methyl) azetidin-3-yl)oxy)-1-oxoisoindolin-2-yl)piperidine-2,6-dione | 3-(5-((1-(di(pyridin-3-yl)methyl) azetidin-3-yl)oxy)-1-oxoisoindolin-2-yl)piperidine-2,6-dione |
| 163 | 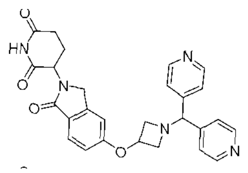 | 3-(5-((1-(di(pyridin-4-yl)methyl) azetidin-3-yl)oxy)-1-oxoisoindolin-2-yl)piperidine-2,6-dione | 3-(5-((1-(di(pyridin-4-yl)methyl) azetidin-3-yl)oxy)-1-oxoisoindolin-2-yl)piperidine-2,6-dione |

(continued)

| Compo und No. | Structure of the compounds | The compounds' name | Chinese translation of the compounds' name |
|---|---|---|---|
| 164 | 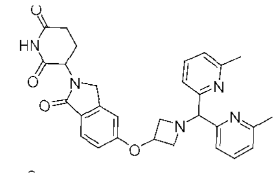 | 3-(5-((1-(bis(6-methylpyridin-2-yl)methyl)azetidin-3-yl)oxy)-1-oxoisoindolin-2-yl)piperidine-2,6-dione | 3-(5-((1-(bis(6-methylpyridin-2-yl)methyl)azetidin-3-yl)oxy)-1-oxoisoindolin-2-yl)piperidine-2,6-dione |
| 165 | 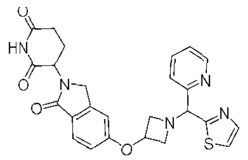 | 3-(1-oxo-5-((1-(pyridin-2-yl(thiazol-2-yl)methyl)azetidin-3-yl)oxy)isoindolin-2-yl)piperidine-2,6-dione | 3-(1-oxo-5-((1-(pyridin-2-yl(thiazol-2-yl)methyl)azetidin-3-yl)oxy)isoindolin-2-yl)piperidine-2,6-dione |
| 166 | 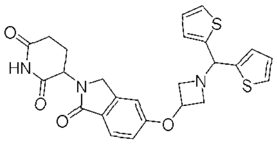 | 3-(5-((1-(di(thiophen-2-yl)methyl)azetidin-3-yl)oxy)-1-oxoisoindolin-2-yl)piperidine-2,6-dione | 3-(5-((1-(di(thiophen-2-yl)methyl)azetidin-3-yl)oxy)-1-oxoisoindolin-2-yl)piperidine-2,6-dione |
| 167 | 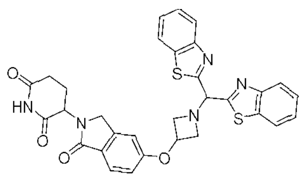 | 3-(5-((1-(bis(benzo[d]thiazol-2-yl)methyl)azetidin-3-yl)oxy)-1-oxoisoindolin-2-yl)piperidine-2,6-dione | 3-(5-((1-(bis(benzo[d]thiazol-2-yl)methyl)azetidin-3-yl)oxy)-1-oxoisoindolin-2-yl)piperidine-2,6-dione |
| 168 GT-06812 | 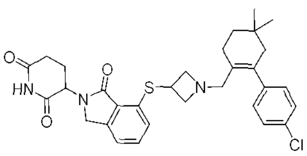 | 3-(7-((1-((4'-chloro-5,5-dimethyl-3,4,5,6-tetrahydro-[1,1 '-biphenyl]-2-yl)methyl)azetidin-3-yl)thio)-1-oxoisoindolin-2-yl)piperidine-2,6-dione | 3-(7-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione |
| 169 GT-06960 | 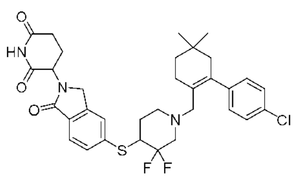 | 3-(5-((1-((4'-chloro-5,5-dimethyl-3,4,5,6-tetrahydro-[1,1'-biphenyl]-2-yl)methyl)-3,3-difluoropiperidin-4-yl)thio)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)-3,3-difluoropiperidin-4-yl)thio)-1-oxoisoindolin-2-yl)piperidine-2,6-dione |
| 170 GT-07022 | 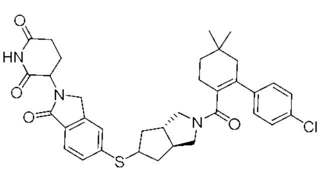 | 3-(5-(((3aS,6aS)-2-(4'-chloro-5,5-dimethyl-3,4,5,6-tetrahydro-[1,1'-biphenyl]-2-carbonyl)octahydrocyclope nta[c]pyrrol-5-yl)thio)-1-oxoisoindolin-2-yl)piperidine-2,6-dione | 3-(5-(((3aS,6aS)-2-(4'-chloro-5,5-dimethyl-3,4,5,6-tetrahydro-[1,1'-biphenyl]-2-carbonyl)octahydrocyclope nta[c]pyrrol-5-yl)thio)-1-oxoisoindolin-2-yl)piperidine-2,6-dione |
| 171 GT-07015 | 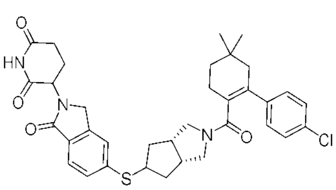 | 3-(5-(((3aR,6aS)-2-(4'-chloro-5,5-dimethyl-3,4,5,6-tetrahydro-[1,1'-biphenyl]-2-carbonyl)octahydrocyclope nta[c]pyrrol-5-yl)thio)-1-oxoisoindolin-2-yl)piperidine-2,6-dione | 3-(5-(((3aR,6aS)-2-(4'-chloro-5,5-dimethyl-3,4,5,6-tetrahydro-[1,1'-biphenyl]-2-carbonyl)octahydrocyclope nta[c]pyrrol-5-yl)thio)-1-oxoisoindolin-2-yl)piperidine-2,6-dione |
| 172 GT-07017 | 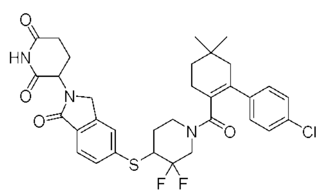 | 3-(5-((1-(4'-chloro-5,5-dimethyl-3,4,5,6-tetrahydro-[1,1'-biphenyl]-2-carbonyl)-3,3-difluoropiperidin-4-yl)thio)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)-3,3-difluoropiperidin-4-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| 173 GT-06924 | 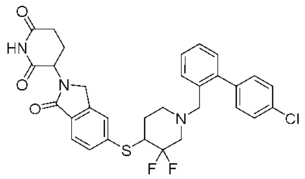 | 3-(5-((1-((4'-chloro-[1,1'-biphenyl]-2-yl)methyl)-3,3-difluoropiperidin-4-yl)thio)-1-oxoisoindolin-2-yl)piperidine-2,6-dione | 3-(5-((1-((4'-chloro-[1,1'-biphenyl]-2-yl)methyl)-3,3-difluoropiperidin-4-yl)thio)-1-oxoisoindolin-2-yl)piperidine-2,6-dione |
| 174 GT-07023 | 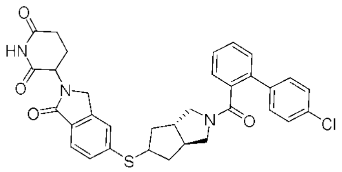 | 3-(5-(((3aS,6aS)-2-(4'-chloro-[1,1'-biphenyl]-2-carbonyl)octahydrocyclope nta[c]pyrrol-5-yl)thio)-1-oxoisoindolin-2-yl)piperidine-2,6-dione | 3-(5-(((3aS,6aS)-2-(4'-chloro-[1,1'-biphenyl]-2-carbonyl)octahydrocyclope nta[c]pyrrol-5-yl)thio)-1-oxoisoindolin-2-yl)piperidine-2,6-dione |
| 175 GT-07024 | 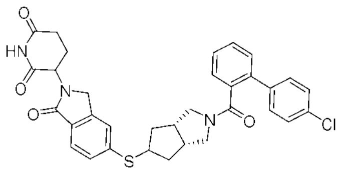 | 3-(5-(((3aR,6aS)-2-(4'-chloro-[1,1'-biphenyl]-2-carbonyl)octahydrocyclope nta[c]pyrrol-5-yl)thio)-1-oxoisoindolin-2-yl)piperidine-2,6-dione | 3-(5-(((3aR,6aS)-2-(4'-chloro-[1,1'-biphenyl]-2-carbonyl)octahydrocyclope nta[c]pyrrol-5-yl)thio)-1-oxoisoindolin-2-yl)piperidine-2,6-dione |
| 176 GT-06925 | 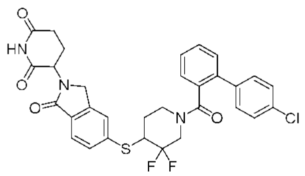 | 3-(5-((1-(4'-chloro-[1,1'-biphenyl]-2-carbonyl)-3,3-difluoropiperidin-4-yl)thio)-1-oxoisoindolin-2-yl)piperidine-2,6-dione | 3-(5-((1-(4'-chloro-[1,1'-biphenyl]-2-carbonyl)-3,3-difluoropiperidin-4-yl)thio)-1-oxoisoindolin-2-yl)piperidine-2,6-dione |
| 177 GT-06926 | 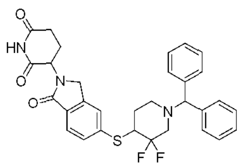 | 3-(5-((1-benzhydryl-3,3-difluoropiperidin-4-yl)thio)-1-oxoisoindolin-2-yl)piperidine-2,6-dione | 3-(5-((1-benzhydryl-3,3-difluoropiperidin-4-yl)thio)-1-oxoisoindolin-2-yl)piperidine-2,6-dione |
| 178 GT-06952 | 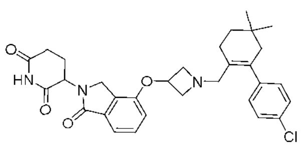 | 3-(4-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione | 3-(4-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione |
| 179 GT-07063 | 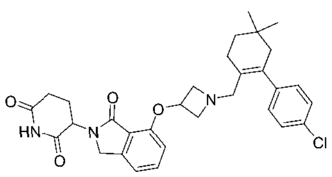 | 3-(7-((1-((4'-chloro-5,5-dimethyl-3,4,5,6-tetrahydro-[1,1'-biphenyl]-2-yl)methyl))zetidine-3-yl)oxy)-1-oxoisoindolin-2-yl)piperidine-2,6-dione | 3-(7-((1-((4'-chloro-5,5-dimethyl-3,4,5,6-tetrahydro-[1,1'-biphenyl]-2-yl)methyl))zetidine-3-yl)oxy)-1-oxoisoindolin-2-yl)piperidine-2,6-dione |
| 180 GT-06930 | 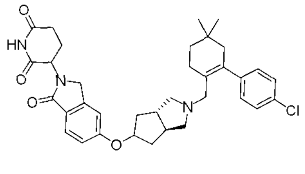 | 3-(5-(((3aS,6aS)-2-((4'-chloro-5,5-dimethyl-3,4,5,6-tetrahydro-[1,1'-biphenyl]-2-yl) methyl)octahydrocyclope nta[c] pyrrol-5-yl)oxy)-1-oxoisoindolin-2-yl)piperidine-2,6-dione | 3-(5-(((3aS,6aS)-2-((4'-chloro-5,5-dimethyl-3,4,5,6-tetrahydro-[1,1'-biphenyl]-2-yl) methyl)octahydrocyclop enta[c] pyrrol-5-yl)oxy)-1-oxoisoindolin-2-yl)piperidine-2,6-dione |

(continued)

| Compo und No. | Structure of the compounds | The compounds' name | Chinese translation of the compounds' name |
|---|---|---|---|
| 181 GT-06931 | 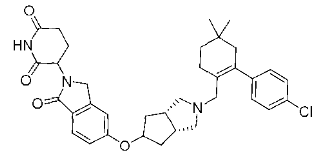 | 3-(5-(((3aR,6aS)-2-((4'-chloro-5,5-dimethyl-3,4,5,6-tetrahydro-[1,1'-biphenyl]-2-yl) methyl)octahydrocyclope nta[c] pyrrol-5-yl)oxy)-1-oxoisoindolin-2-yl)piperidine-2,6-dione | 3-(5-(((3aR,6aS)-2-((4'-chloro-5,5-dimethyl-3,4,5,6-tetrahydro-[1,1'-biphenyl]-2-yl) methyl)octahydrocyclop enta[c] pyrrol-5-yl)oxy)-1-oxoisoindolin-2-yl)piperidine-2,6-dione |
| 182 GT-07025 | 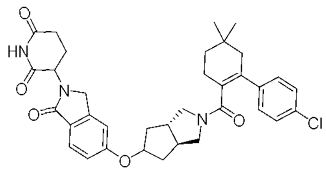 | 3-(5-(((3aS,6aS)-2-(4'-chloro-5,5-dimethyl-3,4,5,6-tetrahydro-[1,1'-biphenyl]-2-carbonyl)octahydrocyclope nta[c]pyrrol-5-yl)oxy)-1-oxoisoindolin-2-yl)piperidine-2,6-dione | 3-(5-(((3aS,6aS)-2-(4'-chloro-5,5-dimethyl-3,4,5,6-tetrahydro-[1,1'-biphenyl]-2-carbonyl)octahydrocyclope nta[c]pyrrol-5-yl)oxy)-1-oxoisoindolin-2-yl)piperidine-2,6-dione |
| 183 GT-07019 | 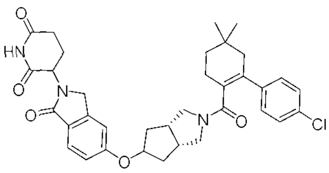 | 3-(5-(((3aR,6aS)-2-(4'-chloro-5,5-dimethyl-3,4,5,6-tetrahydro-[1,1'-biphenyl]-2-carbonyl)octahydrocyclope nta[c]pyrrol-5-yl)oxy)-1-oxoisoindolin-2-yl)piperidine-2,6-dione | 3-(5-(((3aR,6aS)-2-(4'-chloro-5,5-dimethyl-3,4,5,6-tetrahydro-[1,1'-biphenyl]-2-carbonyl)octahydrocyclope nta[c]pyrrol-5-yl)oxy)-1-oxoisoindolin-2-yl)piperidine-2,6-dione |
| 184 GT-06228 | 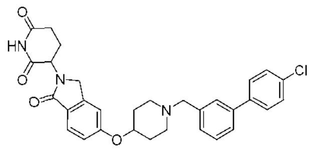 | 3-(5-((1-((4'-chloro-[1,1'-biphenyl]-3-yl)methyl)piperidin-4-yl) oxy)-1-oxoisoindolin-2-yl)piperidine-2,6-dione | 3-(5-((1-((4'-chloro-[1,1'-biphenyl]-3-yl)methyl)piperidin-4-yl) oxy)-1-oxoisoindolin-2-yl)piperidine-2,6-dione |

Specifically, the aforementioned structure may be further substituted by one or more substituents selected from the group consisting of: deuterium, halogen, cyano, nitro, hydroxy, carbonyl, ester group, imide group, phosphine oxide group, alkoxy, aryloxy, alkylthio, arylthio, alkylsulfonyl, arylsulfonyl, silyl, boryl, alkyl, cycloalkyl, alkenyl, aryl, aralkyl, arylalkenyl, alkylaryl, alkylamino, aralkylamino, heteroarylamino, arylamino, arylphosphino, and heteroaryl etc.

In another aspect, the present disclosure provides an embodiment A1): the compound of Formula A:

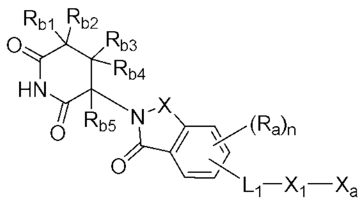

Formula A

or a salt thereof (including pharmaceutically acceptable salts), enantiomers, stereoisomers, polymorphs or solvates thereof,

wherein X is selected from the group consisting of CO or $CH_2$;
$L_1$ is selected from the group consisting of S or O;
$X_1$ is selected from the group consisting of unsubstituted or substituted linear or branched alkylene, or $X_1$ is absent;
$X_a$ represents the structure of Formula A1:

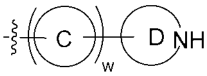

Formula A1

wherein ring C is selected from the group consisting of unsubstituted or substituted arylene, unsubstituted or substituted heteroarylene, unsubstituted or substituted heterocyclylene, or unsubstituted or substituted cycloalkylene, and w represents an integer of 0 or 1; and
ring D represents unsubstituted or substituted nitrogen-containing heterocyclyl;

$R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl;
$R_{b1}$, $R_{b2}$, $R_{b3}$, $R_{b4}$ and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl; and
n is an integer of 0, 1, 2 or 3, and when n is an integer of 2 or 3, each $R_a$ is independently identical to or different from each other.

The present disclosure provides a compound of Formula A or a salt thereof (including pharmaceutically acceptable salts), stereoisomers (including enantiomers), polymorphs, or solvates thereof, which exhibit CRBN E3 ubiquitin ligase binding activity to facilitate substrate protein recruitment, thereby demonstrating immunomodulatory, anti-inflammatory, and anti-tumor activities or favorable pharmacokinetic properties, and which may serve as intermediates for preparing the compounds of Formula I or salts, stereoisomers (including enantiomers), polymorphs, or solvates thereof of the present disclosure.

Embodiment A2): Pertaining to the compound of Formula A or salts, enantiomers, stereoisomers, polymorphs or solvates thereof as described in embodiment A1), wherein X in Formula A is CO.

Embodiment A3): Pertaining to the compound of Formula A or salts, enantiomers, stereoisomers, polymorphs or solvates thereof as described in embodiment A1), wherein X in Formula is $CH_2$.

Embodiment A4): Pertaining to the compound of Formula A or salts, enantiomers, stereoisomers, polymorphs or solvates thereof as described in embodiment A1), wherein $L_1$ in Formula A is S.

Embodiment A5): Pertaining to the compound of Formula A or salts, enantiomers, stereoisomers, polymorphs or solvates thereof as described in embodiment A1), wherein $L_1$ in Formula A is O.

Embodiment A6): Pertaining to the compound of Formula A or salts, enantiomers, stereoisomers, polymorphs or solvates thereof as described in any one of embodiments A1)-A5), wherein $X_1$ is unsubstituted or substituted linear or branched alkylene. When $X_1$ is unsubstituted or substituted linear or branched alkylene, the $X_1$ represents unsubstituted or substituted linear or branched $C_1$-$C_5$ alkylene, or unsubstituted or substituted linear or branched $C_1$-$C_3$ alkylene etc.

Embodiment A7): Pertaining to the compound of Formula A or salts, enantiomers, stereoisomers, polymorphs or solvates thereof as described in any one of embodiments A1)-A6), wherein when $X_1$ is unsubstituted or substituted linear or branched $C_1$-$C_5$ alkylene, the linear or branched $C_1$-$C_5$ alkylene may be methylene, ethylene, propylene, n-propylene, isopropylene, butylene, n-butylene, isobutylene, tert-butylene, sec-butylene, 1-methyl-butylene, 1-ethyl-butylene, pentylene, n-pentylene, isopentylene, neopentylene, or tert-pentylene etc.

Embodiment A8): Pertaining to the compound of Formula A or salts, enantiomers, stereoisomers, polymorphs or solvates thereof as described in any one of embodiments A1)-A7), wherein when $X_1$ is unsubstituted or substituted linear or branched $C_1$-$C_3$ alkylene, the linear or branched alkylene may be e.g., methylene, ethylene, propylene, or isopropylene.

Embodiment A9): Pertaining to the compound of Formula A or salts, enantiomers, stereoisomers, polymorphs or solvates thereof as described in any one of embodiments A1)-A8), wherein the $X_1$ is selected from the group consisting of methylene.

Embodiment A10): Pertaining to the compound of Formula A or salts, enantiomers, stereoisomers, polymorphs or solvates thereof as described in any one of embodiments A1)-A8), wherein the substituents of the substituted $X_1$ is optionally selected from the group consisting of deuterium, halogen, cyano, nitro, hydroxy, mercapto, carbonyl, ester group, imide group, amino, phosphine oxide group, alkoxy, halogenated alkoxy, aryloxy, alkylthio, arylthio, alkylsulfonyl, arylsulfonyl, silyl, boryl, alkyl, cycloalkyl, alkenyl, aryl, aralkyl, arylalkenyl, alkyl aryl, alkylamino, aralkylamino, heteroaryl amino, aryl amino, arylphosphino, heteroaryl, and acenaphthenyl etc., or any combination thereof. Optionally, the substituents of the substituted $X_1$ may be e.g., one or more substituents selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl), nitro, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo or any combination thereof. Further optionally, the substituents of the substituted $X_1$ may be e.g., selected

from the group consisting of deuterium, halogen, nitro, mercapto, hydroxy, cyano, $C_1$-$C_3$ alkyl, $C_1$-$C_3$ alkoxy, halogenated $C_1$-$C_3$ alkoxy, halogenated $C_1$-$C_3$ alkyl, amino, silyl, boryl, or any combination thereof. Further optionally, the substituents of the substituted $X_1$ may be e.g., selected from the group consisting of deuterium, F, Cl, methoxy, methyl, hydroxy, or any combination thereof.

Embodiment A11): Pertaining to the compound of Formula A or salts, enantiomers, stereoisomers, polymorphs or solvates thereof as described in any one of embodiments A1)-A5), wherein $X_1$ is absent.

Embodiment A12): Pertaining to the compound of Formula A or salts, enantiomers, stereoisomers, polymorphs or solvates thereof as described in any one of embodiments A1)-A11), wherein $X_a$ represents the structure of Formula A1:

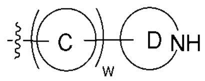

Formula A1

wherein ring C is selected from the group consisting of unsubstituted or substituted $C_5$-$C_{30}$ arylene, unsubstituted or substituted 5- to 30-membered heteroarylene, unsubstituted or substituted 4- to 30-membered heterocyclylene, or unsubstituted or substituted $C_3$-$C_{30}$ cycloalkylene; preferably, ring C is selected from the group consisting of unsubstituted or substituted $C_5$-$C_{20}$ arylene, unsubstituted or substituted 5- to 20-membered heteroarylene, unsubstituted or substituted 4- to 20-membered heterocyclylene, or unsubstituted or substituted $C_3$-$C_{20}$ cycloalkylene;
w represents an integer of 0 or 1; and
ring D represents unsubstituted or substituted 4- to 30-membered nitrogen-containing heterocyclyl; preferably, ring D represents unsubstituted or substituted 4- to 20-membered nitrogen-containing heterocyclyl.

Embodiment A13): Pertaining to the compound of Formula A or salts, enantiomers, stereoisomers, polymorphs or solvates thereof as described in any one of embodiments A1)-A12), wherein $X_a$ represents the structure of Formula A1:

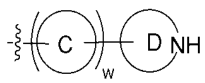

Formula A1

wherein ring C is selected from the group consisting of unsubstituted or substituted $C_5$-$C_{15}$ arylene, unsubstituted or substituted 5- to 15-membered heteroarylene, unsubstituted or substituted 4- to 15-membered heterocyclylene, or unsubstituted or substituted $C_3$-$C_{15}$ cycloalkylene, wherein the substituents of the substituted ring C are optionally one or more substituents selected from the group consisting of halogen, mercapto, hydroxy, amino, cyano, $C_1$-$C_6$ alkyl, $C_1$-$C_6$ alkoxy, halogenated $C_1$-$C_6$ alkyl, or any combination thereof;
w represents an integer of 0 or 1; and
ring D represents unsubstituted or substituted 4- to 15-membered nitrogen-containing heterocyclyl.

Embodiment A14): Pertaining to the compound of Formula A or salts, enantiomers, stereoisomers, polymorphs or solvates thereof as described in any one of embodiments A1)-A13), wherein the ring C is selected from the group consisting of the following groups:

azetidinylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, azacyclooctylene, diazacycloheptanylene, diazacyclooctylene, bridged heterocyclylene (e.g., 5- to 15-membered bridged heterocyclylene, such as azabicyclohexylene, azabicycloheptanylene or azabicyclooctylene), or spiro-heterocyclylene (e.g., 5- to 15-membered spiro-heterocyclylene, such as 3-azaspiro[5.5]undecanylene or 7-azaspiro[3.5]nonanylene); or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo [2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene; or

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{15}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), or bridged cycloalkylene (e.g., $C_5$-$C_{15}$ bridged cycloalkylene, adamantanylene, noradamantanylene, bornylene, bicyclo[2.2.1]heptylene, 2-oxobicyclo[2.2.1]heptylene, or bicyclo[2.2.1]heptentylene); or

phenylene or naphthylene;

wherein ring C is optionally substituted by one or more substituents selected from the group consisting of deuterium, halogen, nitro, mercapto, hydroxy, cyano, $C_1$-$C_6$ alkyl, $C_1$-$C_6$ alkoxy, halogenated $C_1$-$C_6$ alkoxy, halogenated $C_1$-$C_6$ alkyl, unsubstituted or substituted amino, unsubstituted or substituted silyl, unsubstituted or substituted boryl, or any combination thereof.

Embodiment A15): Pertaining to the compound of Formula A or salts, enantiomers, stereoisomers, polymorphs or solvates thereof as described in any one of embodiments A1)-A14), wherein the ring D is selected from the group consisting of the following groups:

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptanyl, azacyclooctyl, diazacycloheptanyl, diazacyclooctyl, nitrogen-containing bridged heterocyclyl (e.g., 5- to 15-membered bridged heterocyclyl, such as azabicyclohexyl, azabicycloheptanyl or azabicyclooctyl), or nitrogen-containing spiro-heterocyclyl (e.g., 5- to 15-membered spiro-heterocyclyl, such as 3-azaspiro[5.5]undecanyl or 7-azaspiro[3.5]nonanyl);

wherein the ring D is optionally substituted by one or more substituents selected from the group consisting of deuterium, halogen, nitro, mercapto, hydroxy, cyano, oxo, $C_1$-$C_6$ alkyl, $C_1$-$C_6$ alkoxy, halogenated $C_1$-$C_6$ alkoxy, halogenated $C_1$-$C_6$ alkyl, unsubstituted or substituted amino, unsubstituted or substituted silyl, unsubstituted or substituted boryl, or any combination thereof.

Embodiment A16): Pertaining to the compound of Formula A or salts, enantiomers, stereoisomers, polymorphs or solvates thereof as described in any one of embodiments A1)-A15), wherein $X_a$ is selected from the group consisting of:

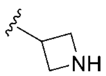 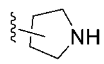 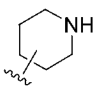 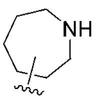 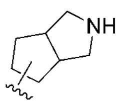 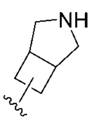 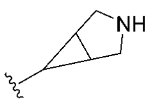 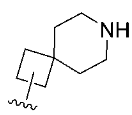 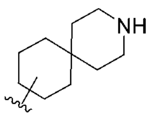

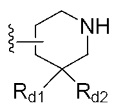 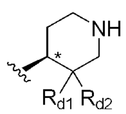 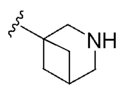 or 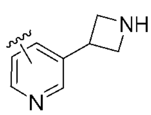 ;

wherein $R_{d1}$ and $R_{d2}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, and unsubstituted or substituted linear or branched $C_1$-$C_5$ alkyl; and $R_{d1}$ and $R_{d2}$ are not hydrogen at the same time.

Embodiment A17): Pertaining to the compound of Formula A or salts, enantiomers, stereoisomers, polymorphs or solvates thereof as described in embodiment A16), wherein $R_{d1}$ and $R_{d2}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, and unsubstituted or substituted linear or branched $C_1$-$C_5$ alkyl; and $R_{d1}$ and $R_{d2}$ are not hydrogen at the same time. In specific embodiments, optionally, the halogen may be e.g., F, Cl, Br or I. Optionally, the $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, and 2-ethylbutoxy etc. Optionally, the halogenated $C_1$-$C_6$ alkoxy may be e.g., trifluoromethoxy etc. Optionally, the unsubstituted or substituted amino includes amino, N-methylamino, N-ethylamino, N-phenylamino, N,N-dimethylamino, and N,N-diethylamino etc. Optionally, the unsubstituted or substituted silyl may be e.g., trimethylsilyl, triethylsilyl, tert-butyldimethylsilyl, vinyldimethylsilyl, propyldimethylsilyl, triphenylsilyl, diphenylsilyl, and phenylsilyl etc. Optionally, the unsubstituted or substituted boryl may include e.g., trimethylboryl, triethylboryl, tert-butyldimethylboryl, triphenylboryl, and phenylboryl etc. Optionally, the linear or branched $C_1$-$C_5$ alkyl includes but is not limited to methyl, ethyl, propyl, isopropyl, butyl, n-butyl, isobutyl, tert-butyl, sec-butyl, 1-methylbutyl, 1-ethylbutyl,

pentyl, n-pentyl, isopentyl, neopentyl, and tert-pentyl etc. Wherein said linear or branched $C_1$-$C_5$ alkyl is optionally substituted with one or more substituents optioanlly selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl), or any combination thereof.

Embodiment A18): Pertaining to the compound of Formula A or salts, enantiomers, stereoisomers, polymorphs or solvates thereof as described in any one of embodiments A1)-A17), wherein $R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl.

Embodiment A19): Pertaining to the compound of Formula A or salts, enantiomers, stereoisomers, polymorphs or solvates thereof as described in embodiment A18), wherein $R_a$ is selected from the group consisting of deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl.

Embodiment A20): Pertaining to the compound of Formula A or salts, enantiomers, stereoisomers, polymorphs or solvates thereof as described in embodiment A18) or A19), wherein $R_a$ is selected from the group consisting of deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl.

Embodiment A21): Pertaining to the compound of Formula A or salts, enantiomers, stereoisomers, polymorphs or solvates thereof as described in embodiment A20), wherein $R_a$ is deuterium.

Embodiment A22): Pertaining to the compound of Formula A or salts, enantiomers, stereoisomers, polymorphs or solvates thereof as described in embodiment A20), wherein $R_a$ is halogen, and the halogen is optionally F, Cl, Br, or I.

Embodiment A23): Pertaining to the compound of Formula A or salts, enantiomers, stereoisomers, polymorphs or solvates thereof as described in embodiment A20), wherein $R_a$ is nitro.

Embodiment A24): Pertaining to the compound of Formula A or salts, enantiomers, stereoisomers, polymorphs or solvates thereof as described in embodiment A20), wherein $R_a$ is cyano.

Embodiment A25): Pertaining to the compound of Formula A or salts, enantiomers, stereoisomers, polymorphs or solvates thereof as described in embodiment A20), wherein $R_a$ is amino.

Embodiment A26): Pertaining to the compound of Formula A or salts, enantiomers, stereoisomers, polymorphs or solvates thereof as described in embodiment A20), wherein $R_a$ is hydroxy.

Embodiment A27): Pertaining to the compound of Formula A or salts, enantiomers, stereoisomers, polymorphs or solvates thereof as described in embodiment A20), wherein $R_a$ is mercapto.

Embodiment A28): Pertaining to the compound of Formula A or salts, enantiomers, stereoisomers, polymorphs or solvates thereof as described in embodiment A20), wherein $R_a$ is alkoxy. Optionally, when $R_a$ is alkoxy, the alkoxy is $C_1$-$C_6$ alkoxy. $C_1$-$C_6$ alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, and 2-ethylbutoxy etc.

Embodiment A29): Pertaining to the compound of Formula A or salts, enantiomers, stereoisomers, polymorphs or solvates thereof as described in embodiment A20), wherein $R_a$ is halogenated alkoxy. Optionally, when $R_a$ is halogenated alkoxy, the halogenated alkoxy is halogenated $C_1$-$C_6$ alkoxy; Further optionally, the halogenated $C_1$-$C_6$ alkoxy is trifluoromethoxy etc.

Embodiment A30): Pertaining to the compound of Formula A or salts, enantiomers, stereoisomers, polymorphs or solvates thereof as described in embodiment A20), wherein $R_a$ is unsubstituted or substituted amino. Optionally, the unsubstituted or substituted amino includes amino, N-methylamino, N-ethylamino, N-phenylamino, N,N-dimethylamino, and N,N-diethylamino etc.

Embodiment A31): Pertaining to the compound of Formula A or salts, enantiomers, stereoisomers, polymorphs or solvates thereof as described in embodiment A20), wherein $R_a$ is unsubstituted or substituted silyl. Optionally, the unsubstituted or substituted silyl is specifically trimethylsilyl, triethylsilyl, tert-butyldimethylsilyl, vinyldimethylsilyl, propyldimethylsilyl, triphenylsilyl, diphenylsilyl, and phenylsilyl etc.

Embodiment A32): Pertaining to the compound of Formula A or salts, enantiomers, stereoisomers, polymorphs or solvates thereof as described in embodiment A20), wherein $R_a$ is unsubstituted or substituted boryl. Optionally, the unsubstituted or substituted boryl is specifically trimethylboryl, triethylboryl, tert-butyldimethylboryl, triphenylboryl, and phenylboryl etc.

Embodiment A33): Pertaining to the compound of Formula A or salts, enantiomers, stereoisomers, polymorphs

or solvates thereof as described in embodiment A20), wherein $R_a$ is unsubstituted or substituted linear or branched alkyl, optionally the number of carbon atoms may be for example $C_1$-$C_{30}$, $C_1$-$C_{25}$, $C_1$-$C_{20}$, $C_1$-$C_{15}$, $C_1$-$C_{10}$, $C_1$-$C_5$, and $C_1$-$C_3$ etc, preferably linear or branched $C_1$-$C_{10}$ alkyl. Further optionally, the linear or branched $C_1$-$C_{10}$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, nonyl, 2,2-dimethylheptyl, 1-ethylpropyl, 1,1-dimethylpropyl, isohexyl, 4-methylhexyl, or 5-methylhexyl etc. Optionally, the linear or branched $C_1$-$C_{10}$ alkyl includes but is not limited to methyl, ethyl, and propyl etc. Wherein the alkyl is optionally substituted by one or more substituents optionally selected from the group consisting of deuterium, halogen, cyano, nitro, hydroxy, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo, or any combination thereof. The substituent are preferably one or more selected from the group consisting of D (i.e., deuterium), halogen, hydroxy, cyano, $C_{1-3}$ alkyl, $C_{1-3}$ alkoxy, trifluoromethyl, heterocyclyl (e.g., 4- to 8-membered heterocyclyl) or any combination thereof.

Embodiment A34): Pertaining to the compound of Formula A or salts, enantiomers, stereoisomers, polymorphs or solvates thereof as described in embodiment A20), wherein $R_a$ is unsubstituted or substituted cycloalkyl. Optionally, when the $R_a$ is unsubstituted or substituted cycloalkyl, it has 3 to 20 carbon atoms (i.e., $C_{3-20}$ cycloalkyl), 3 to 15 carbon atoms (i.e., $C_{3-15}$ cycloalkyl), 3 to 12 carbon atoms (i.e., $C_{3-12}$ cycloalkyl), 3 to 11 carbon atoms (i.e., $C_{3-11}$ cycloalkyl), 3 to 10 carbon atoms (i.e., $C_{3-10}$ cycloalkyl), 3 to 8 carbon atoms (i.e., $C_{3-8}$ cycloalkyl), 3 to 7 carbon atoms (i.e., $C_{3-7}$ cycloalkyl), or 3 to 6 carbon atoms (i.e., $C_{3-6}$ cycloalkyl). Preferably, $R_1$ is unsubstituted or substituted $C_3$-$C_{15}$ cycloalkyl. Optionally, $C_3$-$C_{15}$ cycloalkyl includes but is not limited to cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_5$-$C_{15}$ spiro-cycloalkyl or $C_7$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl) etc. Further optionally, $C_3$-$C_{15}$ cycloalkyl includes but is not limited to cyclopropyl, cyclobutyl, and cyclopentyl etc. Wherein said cycloalkyl is optionally substituted with one or more (e.g., 1-5, 1-4, 1-3, 1-2, or 1) substituents optioanlly selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, deuterium, nitro, carbonyl, ester group, imide group, amino, phosphine oxide group, deuterated alkoxy, trifluoromethoxy, aryloxy, alkylthio, arylthio, alkylsulfonyl, arylsulfonyl, silyl, boryl, alkyl, deuterated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, acenaphthenyl, oxo or any combination thereof. The substituents are preferably one or more selected from the group consisting of trifluoromethyl, mercapto, hydroxy, amino, halogen, cyano, $C_1$-$C_3$ alkyl, $C_1$-$C_3$ alkoxy, trifluoromethyl, heterocyclyl, or any combination thereof.

Embodiment A35): Pertaining to the compound of Formula A or salts, enantiomers, stereoisomers, polymorphs or solvates thereof as described in embodiment A20), wherein $R_a$ is unsubstituted or substituted heterocyclyl. Optionally, when the $R_a$ is unsubstituted or substituted heterocyclyl, the unsubstituted or substituted heterocyclyl is unsubstituted or substituted 4- to 30-membered heterocyclyl, 4- to 25-membered heterocyclyl, 4- to 20-membered heterocyclyl, 4- to 15-membered heterocyclyl, 4- to 10-membered heterocyclyl, 10- to 15-membered heterocyclyl, 5- to 15-membered heterocyclyl, or 7- to 15-membered heterocyclyl etc. Optionally, the heterocyclyl contains 1-3 heteroatoms, or 1-2 heteroatoms, or 1 heteroatom etc, with each heteroatom independently selected from the group consisting of O, N, and S etc. Further optionally, the heterocyclyl includes but is not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, diazacycloheptanyl (e.g., 1, 4-diazacycloheptanyl, 4, 5-diazacycloheptanyl, or 1, 3-diazacycloheptanyl etc.), azabicyclohexyl (e.g., 3-azabicyclo[3.1.0]hexyl etc.), azabicycloheptanyl (e.g., 3-azabicyclo[3.1.1]heptanyl, 3-azabicyclo[3.2.0]heptanyl etc), azabicyclooctyl (e.g., cis-7-azabicyclo[3.3.0]octyl etc.), and spiro-heterocyclyl (e.g., 4- to 15-membered spiro-heterocyclyl, or 5- to 15-membered spiro-heterocyclyl, or 7-to 15-membered spiro-heterocyclyl, such as 7-azaspiro[3.5]nonanyl, 3-azaspiro[5,5]undecanyl etc.). The heterocyclyl may be unsubstituted or substituted as explicitly defined (e.g., mono-, di-, tri-, or polysubstituted) by a substituent optionally selected from the group consisting of deuterium, hydroxy, amino, mercapto, nitro, halogen, cyano, carbonyl, ester group, imide group, phosphine oxide group, trifluoromethoxy, oxo, optionally deuterated $C_1$-$C_6$ alkyl, halogenated $C_1$-$C_6$ alkyl, optionally deuterated $C_{3-6}$ cycloalkyl, optionally deuterated $C_1$-$C_6$ alkoxy, $C_1$-$_4$ alkyl-NH-, $NH_2$-$C_1$-$_6$ alkylene, $C_1$-$_4$ alkyl-NHC(O)-, $C_1$-$_4$ alkyl-C(O)NH-, alkenyl, aryl, aralkyl, arylalkenyl, alkylaryl, aralkylamino, heteroarylamino, arylamino, arylphosphino, heteroaryl, acenaphthenyl, and any combination thereof (preferably, the substituent is selected from the group consisting of deuterium, hydroxy, amino, mercapto, nitro, halogen, cyano, oxo, optionally deuterated $C_1$-$C_6$ alkyl, halogenated $C_1$-$C_6$ alkyl, optionally deuterated $C_{3-6}$ cycloalkyl, optionally deuterated $C_1$-$C_6$ alkoxy, $C_1$-$_4$ alkyl-NH-, $NH_2$-$C_1$-$_6$ alkylene, $C_1$-$_4$ alkyl-NHC(O)-, $C_1$-$_4$ alkyl-C(O)NH-, and any

combination thereof).

Embodiment A36): Pertaining to the compound of Formula A or salts, enantiomers, stereoisomers, polymorphs or solvates thereof as described in embodiment A20), wherein $R_a$ is unsubstituted or substituted aryl. Optionally, the unsubstituted or substituted aryl is unsubstituted or substituted $C_{5}$-$_{30}$ aryl. Optionally, the unsubstituted or substituted $C_{5}$-$_{30}$ aryl is unsubstituted or substituted $C_{5}$-$_{25}$, $C_{5}$-$_{20}$, or $C_{5}$-$_{15}$ aryl. Optionally, $C_{5}$-$_{30}$ aryl may be monocyclic or polycyclic aryl. In some embodiments, the monocyclic aryl includes but is not limited to phenyl, biphenyl, terphenyl, quaterphenyl, and quinquephenyl etc. Polycyclic aryl includes but is not limited to naphthyl, anthryl, phenanthryl, pyrenyl, perylenyl, and fluorenyl etc. The fluorenyl may be substituted (e.g., 9,9'-dimethylfluorenyl and 9,9'-diphenylfluorenyl etc.). Furthermore, two of the substituents may combine with each other to form a spirocyclic structure such as 9,9'-spirobifluorenyl etc. The aryl is optionally substituted aryl. The substituted aryl refers to an aryl group substituted one or more times (e.g., 1-4, 1-3, or 1-2 times) with a substituent. For example, aryl is mono-, di-, or tri-substituted with a substituent optionally selected from the group consisting of e.g., deuterated $C_1$-$C_6$ alkyl, deuterated $C_{3-6}$ cycloalkyl, deuterium, hydroxy, amino, mercapto, halogen, cyano, nitro, carbonyl, ester group, imide group, phosphine oxide group, trifluoromethoxy, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, alkyl, cycloalkyl, alkenyl, aryl, aralkyl, arylalkenyl, alkylaryl, aralkylamino, heteroarylamino, arylamino, arylphosphino, heteroaryl, acenaphthenyl, and any combination thereof. Preferably, the substituent is selected from the group consisting of deuterated $C_1$-$C_5$ alkyl, deuterated $C_{3-6}$ cycloalkyl, hydroxy, amino, mercapto, halogen, cyano, deuterated $C_1$-$C_6$ alkoxy, $C_1$-$C_4$ alkylamino, halogenated $C_1$-$C_6$ alkyl, amino-substituted $C_1$-$C_6$ alkylene, $C_1$-$C_4$ alkyl-NHC(O)-, $C_1$-$C_4$ alkyl-C(O)NH-, and any combination thereof.

Embodiment A37): Pertaining to the compound of Formula A or salts, enantiomers, stereoisomers, polymorphs or solvates thereof as described in embodiment A20), wherein $R_a$ is unsubstituted or substituted heteroaryl. When the $R_a$ is unsubstituted or substituted heteroaryl, the unsubstituted or substituted heteroaryl is unsubstituted or substituted 5- to 30-membered heteroaryl. Optionally, the unsubstituted or substituted 5- to 30-membered heteroaryl is unsubstituted or substituted 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered heteroaryl. Optionally, the heteroaryl includes but is not limited to pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, tetrazolyl, pyranyl, thiopyranyl, pyrazinyl, thiazinyl, triazinyl, tetrazinyl, quinolinyl, isoquinolinyl, quinazolinyl, quinoxalinyl, cinnolinyl, naphthyridinyl, acridinyl, xanthenyl, phenanthridinyl, phthalazinyl, triazaindenyl, indolyl, indolinyl, indolizinyl, phthalazinyl, pyrazolopyridyl, pyrazolopyrimidinyl, pyridopyrimidinyl, pyridopyrazinyl, pyrazinopyrazinyl, benzothiazolyl, benzoxazolyl, benzimidazolyl, benzothienyl, benzofuranyl, isobenzofuranyl, dibenzothienyl, dibenzofuranyl, indazolyl, carbazolyl, benzocarbazolyl, dibenzocarbazolyl, indolocarbazolyl, indenocarbazolyl, phenazinyl, imidazopyridinyl, phenanthridinyl, phenanthrolinyl, phenothiazinyl, imidazopyridinyl, imidazophenanthridinyl, benzimidazoquinazolinyl, benzimidazophenanthridinyl, pyrrolopyridinyl, pyrrolothiazolyl, imidazothiazolyl, pyrenyl, dinaphthofuranyl, naphthobenzofuranyl, dinaphthothienyl, and naphthobenzothienyl etc. Wherein the heteroaryl is unsubstituted or substituted. The substituted heteroaryl refers to a heteroaryl substituted one or more times (e.g., 1-4, 1-3, or 1-2 times) with a substituent optionally selected from the group consisting of $C_1$-$C_3$ alkyl, $C_3$-$C_6$ cycloalkyl, hydroxy, amino, mercapto, halogen, deuterium, nitro, carbonyl, ester group, imide group, phosphine oxide group, trifluoromethoxy, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, alkenyl, aryl, aralkyl, arylalkenyl, alkylaryl, aralkylamino, heteroarylamino, arylamino, arylphosphino, heteroaryl, acenaphthenyl, or any combination thereof. Preferably, the substituent is optionally selected from the group consisting of $C_1$-$C_3$ alkyl, $C_3$-$C_6$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_1$-$C_3$ alkylene, $C_1$-$C_3$ alkyl-NHC(O)-, $C_1$-$C_3$ alkyl-C(O)NH-, cyano, or any combination thereof.

Embodiment A38): Pertaining to the compound of Formula A or salts, enantiomers, stereoisomers, polymorphs or solvates thereof as described in any one of embodiments A1)-A37), wherein $R_{b1}$, $R_{b2}$, $R_{b3}$, $R_{b4}$, and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl.

Embodiment A39): Pertaining to the compound of Formula A or salts, enantiomers, stereoisomers, polymorphs or solvates thereof as described in any one of embodiments A1)-A37), wherein $R_{b1}$, $R_{b2}$, $R_{b3}$, $R_{b4}$, and $R_{b5}$ are each independently selected from the group consisting of hydrogen, deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, or unsubstituted or substituted linear or branched $C_1$-$C_5$ alkyl.

Embodiment A40): Pertaining to the compound of Formula A or salts, enantiomers, stereoisomers, polymorphs or solvates thereof as described in any one of embodiments A1)-A37), wherein $R_{b1}$, $R_{b2}$, $R_{b3}$, $R_{b4}$, and $R_{b5}$ are each independently selected from the group consisting of hydrogen, deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, or unsubstituted or substituted linear or branched $C_1$-$C_5$ alkyl.

Embodiment A41): Pertaining to the compound of Formula A or salts, enantiomers, stereoisomers, polymorphs or solvates thereof as described in any one of embodiments A1)-A37), wherein $R_{b1}$, $R_{b2}$, $R_{b3}$, $R_{b4}$, and $R_{b5}$ are each independently selected from the group consisting of hydrogen.

Embodiment A42): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of deuterium.

Embodiment A43): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of halogen. Optionally, when the $R_{b1}$, $R_{b2}$, $R_{b3}$, $R_{b4}$, and $R_{b5}$ are each independently selected from the group consisting of halogen, the halogen is F, Cl, Br, or I.

Embodiment A44): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of nitro.

Embodiment A45): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of cyano.

Embodiment A46): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of amino.

Embodiment A47): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of hydroxy.

Embodiment A48): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of mercapto.

Embodiment A49): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of alkoxy. Optionally, when the $R_{b1}$, $R_{b2}$, $R_{b3}$, $R_{b4}$, and $R_{b5}$ are each independently selected from the group consisting of alkoxy, the alkoxy is $C_1$-$C_6$ alkoxy. Further optionally, the alkoxy may include but is not limited to e.g., methoxy, ethoxy, n-propoxy, isopropoxy, n-butoxy, isobutoxy, tert-butoxy, sec-butoxy, n-pentoxy, neopentoxy, isopentoxy, n-hexoxy, 3,3-dimethylbutoxy, and 2-ethylbutoxy etc.

Embodiment A50): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of halogenated alkoxy. Optionally, when the $R_{b1}$, $R_{b2}$, $R_{b3}$, $R_{b4}$, and $R_{b5}$ are each independently selected from the group consisting of halogenated alkoxy, the halogenated alkoxy is halogenated $C_1$-$C_6$ alkoxy. Further the halogenated $C_1$-$C_6$ alkoxy is trifluoromethoxy etc.

Embodiment A51): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of unsubstituted or substituted amino. Optionally, the unsubstituted or substituted amino includes amino, N-methylamino, N-ethylamino, N-phenylamino, N,N-dimethylamino, and N,N-diethylamino etc.

Embodiment A52): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of unsubstituted or substituted silyl. Optionally, the unsubstituted or substituted silyl is specifically trimethylsilyl, triethylsilyl, tert-butyldimethylsilyl, vinyldimethylsilyl, propyldimethylsilyl, triphenylsilyl, diphenylsilyl, and phenylsilyl etc.

Embodiment A53): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of unsubstituted or substituted boryl. Optionally, the unsubstituted or substituted boryl is specifically trimethylboryl, triethylboryl, tert-butyldimethylboryl, triphenylboryl, and phenylboryl etc.

Embodiment A54): Pertaining to the compound of Formula A or salts, enantiomers, stereoisomers, polymorphs or solvates thereof as described in embodiment A38), wherein $R_{b1}$, $R_{b2}$, $R_{b3}$, $R_{b4}$, and $R_{b5}$ are each independently selected from the group consisting of unsubstituted or substituted linear or branched alkyl. Optionally, the number of carbon atoms may be for example $C_1$-$C_{30}$, $C_1$-$C_{25}$, $C_1$-$C_{20}$, $C_1$-$C_{15}$, $C_1$-$C_{10}$, $C_1$-$C_5$ or $C_1$-$C_3$ etc, with linear or branched $C_1$-$C_{10}$ alkyl being preferred. Further optionally, linear or branched $C_1$-$C_{10}$ alkyl includes but is not limited to methyl, ethyl, propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, nonyl, 2,2-dimethylheptyl, 1-ethylpropyl, 1,1-dimethylpropyl, isohexyl, 4-methylhexyl, and 5-methylhexyl etc. Optionally, the linear or branched $C_1$-$C_{10}$ alkyl includes but is not limited to methyl, ethyl, and propyl etc. Wherein the alkyl is optionally substituted, and the substituent may be e.g., selected from the group consisting of deuterium, halogen, cyano, nitro, hydroxy, mercapto, carbonyl, ester group, imide group, amino, phosphine oxide group, alkoxy, deuterated alkoxy, trifluoromethoxy, aryloxy, alkylthio, arylthio, alkylsulfonyl, arylsulfonyl, silyl, boryl, alkyl, deuterated alkyl, halogenated alkyl, amino-substituted alkylene, alkyl-NHC(O)-, alkyl-C(O)NH-, cycloalkyl, deuterated cycloalkyl, alkenyl, aryl, aralkyl, arylalkenyl, alkyl aryl, alkylamino, aralkylamino, heteroarylamino, arylamino, arylphosphino, heteroaryl, acenaphthenyl, oxo or any combination thereof (preferably one or more selected from the group consisting of D (i.e., deuterium), halogen, hydroxy, cyano, $C_1$-$C_3$ alkyl, $C_1$-$C_3$ alkoxy, trifluoromethyl, heterocyclyl (e.g., 4- to 8-membered heterocyclyl), or any combination thereof).

Embodiment A55): Pertaining to the compound of Formula A or salts, enantiomers, stereoisomers, polymorphs or solvates thereof as described in any one of embodiment A1)-A54), wherein n is an integer of 0, 1, 2 or 3, and when n is an integer of 2 or 3, each $R_a$ is independently identical to or different from each other. In some embodiments, n is an integer of 0, 1 or 2.

Especially preferably the compounds of the present disclosure and their salts (especially pharmaceutically acceptable salts, such as hydrochloride, etc.), enantiomers, stereoisomers, polymorphs, or solvates thereof in Table 2 below are provided:

Table 2. The compounds of the present disclosure

| Compound No. | Structure of the compounds | The compounds' name | Chinese translation of the compounds' name |
|---|---|---|---|
| GT-03613 | 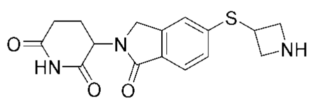 | 3-(5-(azetidin-3-ylthio)-1-oxoisoindolin-2-yl)piperidine-2,6-dione | 3-(5-(azetidin-3-ylthio)-1-oxoisoindolin-2-yl)piperidine-2,6-dione |
| GT-04309 | 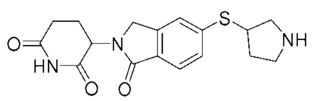 | 3-(1-oxo-5-(pyrrolidin-3-ylthio) isoindolin-2-yl)piperidine-2,6-dione | 3-(1-oxo-5-(pyrrolidin-3-ylthio) isoindolin-2-yl)piperidine-2,6-dione |
| GT-03616 | 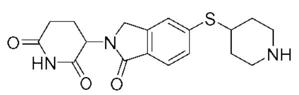 | 3-(1-oxo-5-(piperidin-4-ylthio) isoindolin-2-yl)piperidine-2,6-dione | 3-(1-oxo-5-(piperidin-4-ylthio) isoindolin-2-yl)piperidine-2,6-dione |
| GT-06995 | 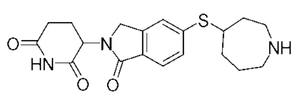 | 3-(5-(azepan-4-ylthio)-1-oxoisoindolin-2-yl)piperidine-2,6-dione | 3-(5-(azepan-4-ylthio)-1-oxoisoindolin-2-yl)piperidine-2,6-dione |
| GT-06992 | 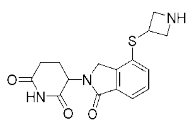 | 3-(4-(azetidin-3-ylthio)-1-oxoisoindolin-2-yl)piperidine-2,6-dione | 3-(4-(azetidin-3-ylthio)-1-oxoisoindolin-2-yl)piperidine-2,6-dione |
| GT-06993 | 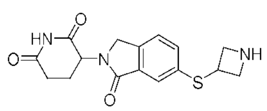 | 3-(6-(azetidin-3-ylthio)-1-oxoisoindolin-2-yl)piperidine-2,6-dione | 3-(6-(azetidin-3-ylthio)-1-oxoisoindolin-2-yl)piperidine-2,6-dione |
| GT-06994 | 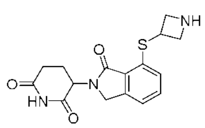 | 3-(7-(azetidin-3-ylthio)-1-oxoisoindolin-2-yl)piperidine-2,6-dione | 3-(7-(azetidin-3-ylthio)-1-oxoisoindolin-2-yl)piperidine-2,6-dione |
| GT-06997 | 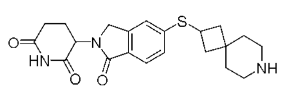 | 3-(5-((7-azaspiro[3.5]nonan-2-yl)thio)-1-oxoisoindolin-2-yl)piperidine-2,6-dione | 3-(5-((7-azaspiro[3.5]nonan-2-yl)thio)-1-oxoisoindolin-2-yl)piperidine-2,6-dione |
| GT-06998 | 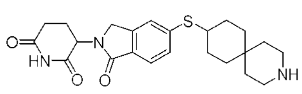 | 3-(5-((3-azaspiro[5.5]undecan-9-yl)thio)-1-oxoisoindolin-2-yl)piperidine-2,6-dione | 3-(5-((3-azaspiro[5.5]undecan-9-yl)thio)-1-oxoisoindolin-2-yl)piperidine-2,6-dione |

(continued)

| Compo und No. | Structure of the compounds | The compounds' name | Chinese translation of the compounds' name |
|---|---|---|---|
| GT-06996 | 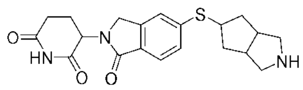 | 3-(5-((octahydrocyclopenta[c] pyrrol-5-yl)thio)-1-oxoisoindolin-2-yl)piperidine-2,6-dione | 3-(5-((octahydrocyclopenta[c] pyrro 1-5-yl)thio)-1-oxoisoindolin-2-yl)piperidine-2,6-dione |
| GT-06999 | 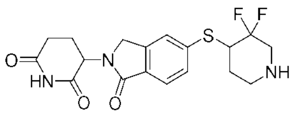 | 3-(5-((3,3-difluoropiperidin-4-yl) thio)-1-oxoisoindolin-2-yl)piperidine-2,6-dione | 3-(5-((3,3-difluoropiperidin-4-yl) thio)-1-oxoisoindolin-2-yl)piperidine-2,6-dione |
| | 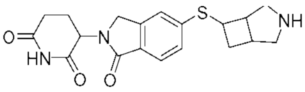 | 3-(5-((3-azabicyclo[3.2.0]heptan-6-yl)thio)-1-oxoisoindolin-2-yl)piperidine-2,6-dione | 3-(5-((3-azabicyclo[3.2.0]heptan-6-yl)thio)-1-oxoisoindolin-2-yl)piperidine-2,6-dione |
| | 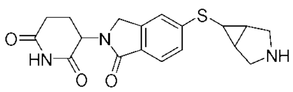 | 3-(5-((3-azabicyclo[3.1.0]hexan-6-yl)thio)-1-oxoisoindolin-2-yl)piperidine-2,6-dione | 3-(5-((3-azabicyclo[3.1.0]hexan-6-yl)thio)-1-oxoisoindolin-2-yl)piperidine-2,6-dione |
| | 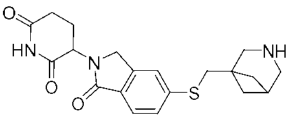 | 3-(5-(((3-azabicyclo[3.1.1]heptan-1-yl)methyl)thio)-1-oxoisoindolin-2-yl)piperidine-2,6-dione | 3-(5-(((3-azabicyclo[3.1.1]heptan-1-yl)methyl)thio)-1-oxoisoindolin-2-yl)piperidine-2,6-dione |
| | 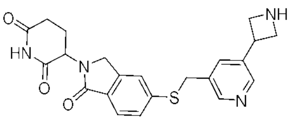 | 3-(5-(((5-(azetidin-3-yl)pyridin-3-yl)methyl)thio)-1-oxoisoindolin-2-yl)piperidine-2,6-dione | 3-(5-(((5-(azetidin-3-yl)pyridin-3-yl)methyl)thio)-1-oxoisoindolin-2-yl)piperidine-2,6-dione |
| GT-03614 | 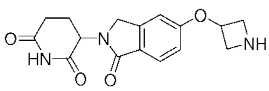 | 3-(5-(azetidin-3-yloxy)-1-oxoisoindolin-2-yl)piperidine-2,6-dione | 3-(5-(azetidin-3-yloxy)-1-oxoisoindolin-2-yl)piperidine-2,6-dione |
| GT-07002 | 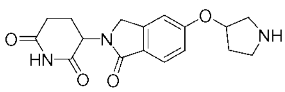 | 3-(1-oxo-5-(pyrrolidin-3-yloxy) isoindolin-2-yl)piperidine-2,6-dione | 3-(1-oxo-5-(pyrrolidin-3-yloxy) isoindolin-2-yl)piperidine-2,6-dione |
| GT-03612 | 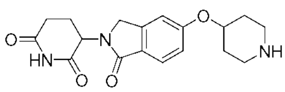 | 3-(1-oxo-5-(piperidin-4-yloxy) isoindolin-2-yl)piperidine-2,6-dione | 3-(1-oxo-5-(piperidin-4-yloxy) isoindolin-2-yl)piperidine-2,6-dione |
| GT-07003 | 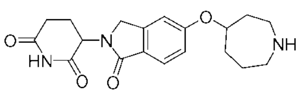 | 3-(5-(azepan-4-yloxy)-1-oxoisoindolin-2-yl)piperidine-2,6-dione | 3-(5-(azepan-4-yloxy)-1-oxoisoindolin-2-yl)piperidine-2,6-dione |
| GT-07000 | 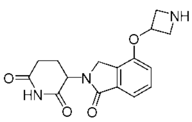 | 3-(4-(azetidin-3-yloxy)-1-oxoisoindolin-2-yl)piperidine-2,6-dione | 3-(4-(azetidin-3-yloxy)-1-oxoisoindolin-2-yl)piperidine-2,6-dione |
| GT-07001 | 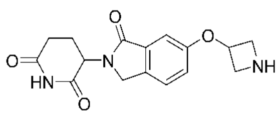 | 3-(6-(azetidin-3-yloxy)-1-oxoisoindolin-2-yl)piperidine-2,6-dione | 3-(6-(azetidin-3-yloxy)-1-oxoisoindolin-2-yl)piperidine-2,6-dione |
| GT-07052 | 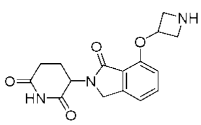 | 3-(7-(azetidin-3-yloxy)-1-oxoisoindolin-2-yl)piperidine-2,6-dione | 3-(7-(azetidin-3-yloxy)-1-oxoisoindolin-2-yl)piperidine-2,6-dione |
| GT-07005 | 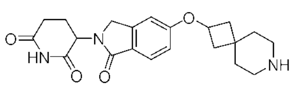 | 3-(5-((7-azaspiro[3.5]nonan-2-yl)oxy)-1-oxoisoindolin-2-yl)piperidine-2,6-dione | 3-(5-((7-azaspiro[3.5]nonan-2-yl)oxy)-1-oxoisoindolin-2-yl)piperidine-2,6-dione |

(continued)

| Compo und No. | Structure of the compounds | The compounds' name | Chinese translation of the compounds' name |
|---|---|---|---|
| GT-07004 | 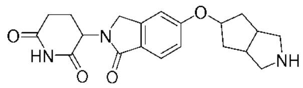 | 3-(5-((octahydrocyclopenta[c] pyrrol-5-yl)oxy)-1-oxoisoindolin-2-yl)piperidine-2,6-dione | 3-(5-((octahydrocyclopenta[c] pyrro 1-5-yl)oxy)-1-oxoisoindolin-2-yl)piperidine-2,6-dione |
| | 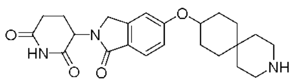 | 3-(5-((3-azaspiro[5.5]undecan-9-yl)oxy)-1-oxoisoindolin-2-yl)piperidine-2,6-dione | 3-(5-((3-azaspiro[5.5]undecan-9-yl)oxy)-1-oxoisoindolin-2-yl)piperidine-2,6-dione |
| | 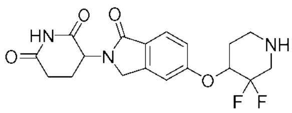 | 3-(5-((3,3-difluoropiperidin-4-yl) oxy)-1-oxoisoindolin-2-yl)piperidine-2,6-dione | 3-(5-((3,3-difluoropiperidin-4-yl) oxy)-1-oxoisoindolin-2-yl)piperidine-2,6-dione |
| | 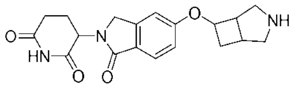 | 3-(5-((3-azabicyclo[3.2.0]heptan-6-yl)oxy)-1-oxoisoindolin-2-yl)piperidine-2,6-dione | 3-(5-((3-azabicyclo[3.2.0]heptan-6-yl)oxy)-1-oxoisoindolin-2-yl)piperidine-2,6-dione |
| | 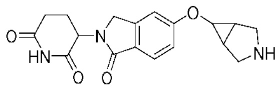 | 3-(5-((3-azabicyclo[3.1.0]hexan-6-yl)oxy)-1-oxoisoindolin-2-yl)piperidine-2,6-dione | 3-(5-((3-azabicyclo[3.1.0]hexan-6-yl)oxy)-1-oxoisoindolin-2-yl)piperidine-2,6-dione |
| | 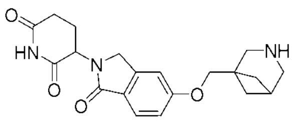 | 3-(5-((3-azabicyclo[3.1.1]heptan-1-yl)methoxy)-1-oxoisoindolin-2-yl)piperidine-2,6-dione | 3-(5-((3-azabicyclo[3.1.1]heptan-1-yl)methoxy)-1-oxoisoindolin-2-yl)piperidine-2,6-dione |
| | 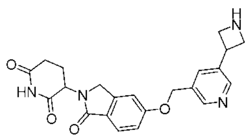 | 3-(5-((5-(azetidin-3-yl)pyridin-3-yl)methoxy)-1-oxoisoindolin-2-yl)piperidine-2,6-dione | 3-(5-((5-(azetidin-3-yl)pyridin-3-yl)methoxy)-1-oxoisoindolin-2-yl)piperidine-2,6-dione |

In a further aspect, the present disclosure provides the compounds of Formula I, Formula A, or salts, enantiomers, stereoisomers, polymorphs or solvates thereof of the present disclosure, for use as a medicament.

## Pharmaceutically compositions

In a further aspect, the present disclosure provides a pharmaceutical composition comprising the compounds of the present disclosure (the compounds of Formula I or Formula A) or salts, enantiomers, stereoisomers, polymorphs or solvates thereof, and at least one pharmaceutically acceptable salt, and at least one pharmaceutically acceptable carrier or excipient.

The composition may comprise one or more pharmaceutically acceptable carriers, which generally refer to carriers used for the administration of therapeutic agents and do not themselves induce the production of antibodies harmful to the individual receiving the composition and exhibit no excessive toxicity upon administration. These carriers are well-known to those skilled in the art, e.g., relevant information on pharmaceutically acceptable carriers disclosed in Remington's Pharmaceutical Sciences (Mack Pub. Co., N.J., 1991). Specifically, the carrier may comprise, but is not limited to, one or more selected from the group consisting of saline, buffers, glucose, water, glycerol, ethanol, adjuvants, and combinations thereof. Each carrier must be "acceptable" in the sense of being compatible with the other ingredients of the formulation (including the compounds useful in the present disclosure) and not injurious to the patient.

The composition may comprise one or more pharmaceutically acceptable excipients, which refer to a pharmaceutically acceptable material, such as a filler, stabilizer, dispersant, suspending agent, diluent, vehicle, and thickener, solvent, or encapsulating material, with which the useful compounds according to the present disclosure are carried or transported into or administered to a patient so that they may perform their intended function. Generally, such constructs are carried or transported from one organ or part of the body to another organ or part of the body. The excipient is compatible with the other ingredients of the formulation, including the compounds useful in the present disclosure, and is not harmful to the patient, and the excipient must be "acceptable". Some examples of materials that may be used as pharmaceutically acceptable excipients include sugars such as lactose, glucose, and sucrose; starches such as corn

starch and potato starch; cellulose and its derivatives such as sodium carboxymethyl cellulose, ethyl cellulose and cellulose acetate; powdered tragacanth; malt; gelatin; talc; vehicles such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer, and other common non-toxic compatible substances used in pharmaceutical formulations.

The pharmaceutical composition of the present disclosure comprising, as an active ingredient, the compounds of the present disclosure (the compounds of Formula I or Formula A) or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of the present disclosure (the compounds of Formula I or Formula A) into conventional, dispersible, chewable, orally disintegrating or rapidly dissolving formulations as needed.

The compounds of the present disclosure (the compounds of Formula I or Formula A), as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of the present disclosure (the compounds of Formula I or Formula A) or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

The pharmaceutical composition provided in the present disclosure further comprises at least one additional therapeutic agent for treating or preventing diseases associated with cereblon protein. In one embodiment, the disease or disorder associated with cereblon protein is selected from the group consisting of: tumor, infectious disease, inflammatory disease, immune system diseases, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. Specifically, the disease or disorder associated with cereblon protein is selected from the group consisting of: myeloma (e.g., multiple myeloma, plasma cell myeloma, smoldering myeloma, smoldering multiple myeloma); myelofibrosis; bone marrow disease; myelodysplastic syndrome (MDS); previously treated myelodysplastic syndrome; transplantation-related cancer; neutropenia; leukemia (e.g., acute myeloid leukemia, chronic myelogenous leukemia, B-cell chronic lymphocytic leukemia, leukemia-associated anemia, acute myeloid leukemia (AML), monocytic leukemia, acute lymphoblastic leukemia (ALL), acute B-cell lymphoblastic leukemia, acute T-cell lymphoblastic leukemia, chronic lymphocytic leukemia, lymphoma cell leukemia, T-cell lymphoblastic leukemia, myelomonocytic leukemia); lymphoma (e.g., diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma); thyroid cancer; melanoma; lung cancer (e.g., lung adenocarcinoma, lung squamous cell carcinoma, non-small cell lung cancer, and small cell lung cancer); inflammatory myofibroblastoma; colorectal cancer; intestinal cancer; brain glioma; astroblastoma; neuroglioma; peripheral neuroepithelioma; ovarian cancer; bronchial cancer; prostate cancer; breast cancer (e.g., triple negative breast cancer, sporadic breast cancer, and patients with Cowden syndrome); pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma (e.g., rhabdomyosarcoma, various lipogenic tumors, Ewing's sarcoma/primitive neuroectodermal tumors (Ewing/PNETs), and leiomyosarcoma); urothelial carcinoma; basal cell carcinoma; oral

squamous cell carcinoma; cholangiocarcinoma; bone cancer; cervical cancer; skin cancer; bone tumors; brain tumors; lung or mesothelium tumor; colonic tumors; ureteral tumors; Richter syndrome (RS); sepsis syndrome; autoimmune diseases (e.g., rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, atopic dermatitis, vitiligo, experimental autoimmune encephalomyelitis, and Arthus reaction); keratoconjunctivitis; inflammatory diseases (e.g., Crohn's disease and ulcerative colitis, pneumonia, osteoarthritis, synovitis, systemic inflammatory response syndrome, airway inflammation, bronchitis, rheumatoid spondylitis, gouty arthritis, eczema, psoriasis, dermatitis, uveitis, conjunctivitis, acute respiratory distress syndrome, bacteremia, endotoxemia, aphthous ulcer, gingivitis, pancreatitis, regional ileitis, atrophic gastritis, peritonitis, pepticulcer, or irritable bowel syndrome); cerebral malaria; infectious diseases (e.g., viral pneumonia, Acquired immunodeficiency syndrome (AIDS), COVID-19 novel coronavirus infection, gram-negative bacteria infection, gram-positive bacteria infection, tuberculosis); septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**Kits/Packaged Products**

The present disclosure further provides a kit comprising the pharmaceutical composition, which comprises a pharmaceutically acceptable carrier or excipient, and the compounds of Formula I as defined in any one of embodiments 1)-111) or the compounds of Formula A as defined in any one of embodiments A1)-A55); instructions for use describing how to administer the pharmaceutical composition in combination with chemotherapy, radiotherapy, and/or targeted therapy for the prevention or treatment of cancer.

The present disclosure provides kits comprising the compounds, pharmaceutical compositions, or other agents described herein, which may be used to perform the methods described herein. In some embodiments, the kits contain at least one purified compound in one or more containers. In some embodiments, the kits include all components necessary and/or sufficient to perform detection assays, including all controls, instructions for conducting the assays, and any necessary software for analyzing and presenting results. The kits may also include labels or indications describing the kit components or a set of instructions for their use in the ligand detection methods of the present disclosure. Such instructions may be associated with the package insert and/or packaging of the kit or its components. Those skilled in the art will readily recognize that the compounds, pharmaceutical compositions, or other agents of the present disclosure can be easily incorporated into established kit formats well-known in the field. Such kits may further include, for example, other compounds and/or compositions, one or more devices for administering said compounds and/or compositions, and written instructions in a form prescribed by governmental agencies regulating the manufacture, use, or sale of pharmaceutical or biological products.

**Uses and Therapeutic Methods**

In a further aspect, the present disclosure provides use of the compounds or salts, enantiomers, stereoisomers, polymorphs or solvates thereof of the present disclosure, or the pharmaceutical composition of the present disclosure for the manufacture of a medicament for the treatment of a disease or disorder associated with cereblon protein.

Specifically, in the use provided by the present disclosure, the disease or disorder associated with cereblon protein is selected from the group consisting of: tumor, infectious disease, inflammatory disease, immune system diseases, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

More specifically, in the use provided by the present disclosure, the disease or disorder associated with cereblon protein is selected from the group consisting of: myeloma (e.g., multiple myeloma, plasma cell myeloma, smoldering myeloma, smoldering multiple myeloma); myelofibrosis; bone marrow disease; myelodysplastic syndrome (MDS); previously treated myelodysplastic syndrome; transplantation-related cancer; neutropenia; leukemia (e.g., acute myeloid leukemia, chronic myelogenous leukemia, B-cell chronic lymphocytic leukemia, leukemia-associated anemia, acute myeloid leukemia (AML), monocytic leukemia, acute lymphoblastic leukemia (ALL), acute B-cell lymphoblastic leukemia, acute T-cell lymphoblastic leukemia, chronic lymphocytic leukemia, lymphoma cell leukemia, T-cell lymphoblastic leukemia, myelomonocytic leukemia); lymphoma (e.g., diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma); thyroid cancer; melanoma; lung cancer (e.g., lung adenocarcinoma, lung squamous cell carcinoma, non-small cell lung cancer, and small cell lung cancer); inflammatory myofibroblastoma; colorectal cancer; intestinal cancer; brain glioma; astroblastoma; neuroglioma; peripheral neuroepithelioma; ovarian cancer; bronchial cancer; prostate cancer; breast cancer (e.g., triple negative breast cancer, sporadic breast cancer, and patients with Cowden syndrome); pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma (e.g., rhabdomyosarcoma, various lipogenic tumors, Ewing's sarcoma/primitive neuroectodermal tumors (Ewing/PNETs), and leiomyosarcoma); urothelial carcinoma; basal cell carcinoma; oral squamous cell carcinoma; cholangiocarcinoma; bone cancer; cervical cancer; skin cancer; bone tumors; brain tumors; lung or mesothelium tumor; colonic tumors; ureteral tumors; Richter syndrome (RS); sepsis syndrome; autoimmune diseases (e.g., rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, atopic dermatitis, vitiligo, experimental autoimmune encephalomyelitis, and Arthus reaction); keratoconjunctivitis; inflammatory diseases (e.g., Crohn's disease and ulcerative colitis, pneumonia, osteoarthritis, synovitis, systemic inflammatory response syndrome, airway inflammation, bronchitis, rheumatoid spondylitis, gouty arthritis, eczema, psoriasis, dermatitis, uveitis, conjunctivitis, acute respiratory distress syndrome, bacteremia, endotoxemia, aphthous ulcer, gingivitis, pancreatitis, regional ileitis, atrophic gastritis, peritonitis, pepticulcer, or irritable bowel syndrome); cerebral malaria; infectious diseases (e.g., viral pneumonia, Acquired immunodeficiency syndrome (AIDS), COVID-19 novel coronavirus infection, gram-negative bacteria infection, gram-positive bacteria infection, tuberculosis); septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

In a further aspect, the present disclosure provides a method for treating or preventing a disease or disorder associated with cereblon protein, comprising administering to a subject a therapeutically effective amount of the compounds or salts, enantiomers, stereoisomers, polymorphs or solvates thereof of the present disclosure, or the pharmaceutical composition of the present disclosure.

Specifically, in the therapeutic method provided by the present disclosure, the disease or disorder associated with cereblon protein is selected from the group consisting of: tumor, infectious disease, inflammatory disease, immune system diseases, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

More specifically, in the method provided by the present disclosure, the disease or disorder associated with cereblon protein is selected from the group consisting of: myeloma (e.g., multiple myeloma, plasma cell myeloma, smoldering myeloma, smoldering multiple myeloma); myelofibrosis; bone marrow disease; myelodysplastic syndrome (MDS); previously treated myelodysplastic syndrome; transplantation-related cancer; neutropenia; leukemia (e.g., acute myeloid leukemia, chronic myelogenous leukemia, B-cell chronic lymphocytic leukemia, leukemia-associated anemia, acute myeloid leukemia (AML), monocytic leukemia, acute lymphoblastic leukemia (ALL), acute B-cell lymphoblastic leukemia, acute T-cell lymphoblastic leukemia, chronic lymphocytic leukemia, lymphoma cell leukemia, T-cell lymphoblastic leukemia, myelomonocytic leukemia); lymphoma (e.g., diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma); thyroid cancer; melanoma; lung cancer (e.g., lung adenocarcinoma, lung squamous cell carcinoma, non-small cell lung cancer, and small cell lung cancer); inflammatory myofibroblastoma; colorectal cancer; intestinal cancer; brain glioma; astroblastoma; neuroglioma; peripheral neuroepithelioma; ovarian cancer; bronchial cancer; prostate cancer; breast cancer (e.g., triple negative breast cancer, sporadic breast cancer, and patients with Cowden syndrome); pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma (e.g., rhabdomyosarcoma, various lipogenic tumors, Ewing's sarcoma/primitive neuroectodermal tumors (Ewing/PNETs), and leiomyosarcoma); urothelial carcinoma; basal cell carcinoma; oral squamous cell carcinoma; cholangiocarcinoma; bone cancer;

cervical cancer; skin cancer; bone tumors; brain tumors; lung or mesothelium tumor; colonic tumors; ureteral tumors; Richter syndrome (RS); sepsis syndrome; autoimmune diseases (e.g., rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, atopic dermatitis, vitiligo, experimental autoimmune encephalomyelitis, and Arthus reaction); keratoconjunctivitis; inflammatory diseases (e.g., Crohn's disease and ulcerative colitis, pneumonia, osteoarthritis, synovitis, systemic inflammatory response syndrome, airway inflammation, bronchitis, rheumatoid spondylitis, gouty arthritis, eczema, psoriasis, dermatitis, uveitis, conjunctivitis, acute respiratory distress syndrome, bacteremia, endotoxemia, aphthous ulcer, gingivitis, pancreatitis, regional ileitis, atrophic gastritis, peritonitis, pepticulcer, or irritable bowel syndrome); cerebral malaria; infectious diseases (e.g., viral pneumonia, Acquired immunodeficiency syndrome (AIDS), COVID-19 novel coronavirus infection, gram-negative bacteria infection, gram-positive bacteria infection, tuberculosis); septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

In the method for treating or preventing a disease or disorder associated with cereblon protein, the compounds of Formula I or Formula A or salts, stereoisomers, polymorphs or solvates thereof, or the pharmaceutical composition of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

In another aspect, the present disclosure provides use of the compound of Formula A or salts, enantiomers, stereoisomers, polymorphs or solvates thereof of the present disclosure for the manufacture of a Cereblon protein (CRBN)-binding agent.

In another aspect, the present disclosure provides use of the compound of Formula A or salts, enantiomers, stereoisomers, polymorphs or solvates thereof of the present disclosure for the preparation of the compound of Formula I or salts, enantiomers, polymorphs or solvates thereof of the present disclosure.

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| $AlCl_3$ | aluminum chloride |
| dioxane | 1,4-dioxane |
| DMF | N,N-dimethylformamide |
| DCM | dichloromethane |
| DIEA or DIPEA | N,N-diisopropylethylamine |
| DMSO | dimethyl sulfoxide |
| EDCI | 1-(3-dimethylaminopropyl)-3-ethylcarbodiimide hydrochloride |
| ESI | electrospray ionization |
| EtOAc | ethyl acetate |
| eq or equiv | equivalent |
| HATU | 2-(7-Azabenzotriazol-1-yl)-N,N,N',N'-tetramethyluronium hexafluorophosphate |
| $^1$H NMR | proton nuclear magnetic resonance |
| HOAt | 1-hydroxy-7-azabenzotriazole |
| KOAc | potassium acetate |
| LC/MS | liquid chromatography-mass spectrometry |
| TEA | triethylamine |
| -OMs | methanesulfonyloxy |
| -ONs | 4-nitrobenzenesulfonyl |
| -OTs | 4-methylbenzenesulfonyloxy |
| $Pd(OAc)_2$ | palladium(II) acetate |

| | |
|---|---|
| TLC | thin-layer chromatography |
| Toluene | toluene |
| Xantphos | 4,5-bis(diphenylphosphino)-9,9-dimethylxanthene |

The preparation methods for the compounds of Formula I and Formula A of the present disclosure are described in greater detail below. However, these specific methods do not limit the invention in any way. The compounds of the present invention may also be conveniently prepared by optionally combining various synthetic methods described in this specification or known in the art. Such combinations can be readily accomplished by those skilled in the art to which this invention pertains.

All temperatures are reported in degrees Celsius (°C). Compounds were characterized by $^1$H-NMR (400 MHz, Bruker Advance 400 spectrometer). Chemical shifts ($\delta$) are reported in parts per million (ppm) relative to the solvent used. Multiplicity is reported as: s = singlet, d = doublet, t = triplet, q = quartet, p = pentet, hex = hextet, hep = heptet, m = multiplet, br. = broad; coupling constants (J) are reported in Hertz (Hz). Alternatively, compounds were characterized by LC/MS using either: (1) a Sciex API 2000 system with Agilent 1100 binary pump, DAD and ELSD detectors, or (2) an Agilent 6125B quadrupole MS system with Agilent 1260 quaternary pump, DAD and ELSD detectors.

Solvents and reagents are processed as follows:
the solvents used in the reactions such as N,N-dimethylformamide, ethyl acetate, anhydrous toluene, dichloromethane, methanol, ethanol, and hydrochloric acid were all purchased from Sinopharm Group; Other reaction reagents and drugs can be purchased from commercial suppliers and used without special instructions.

General synthetic methods of the compounds of Formula I

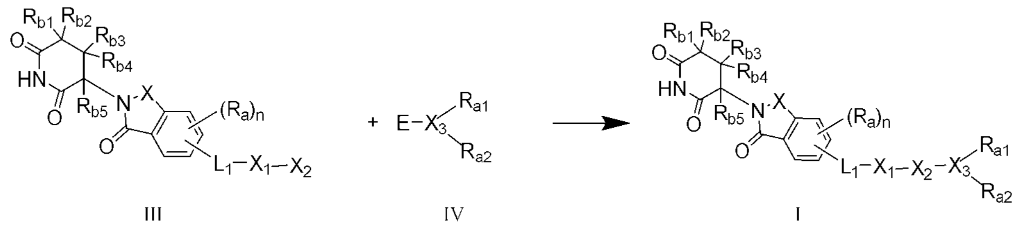

III IV I

Wherein (1) E-$X_3$ represents CO, $R_{a2}$ represents hydrogen; or (2) E-$X_3$ represents halogen-CH, MsO-CH, TsO-CH, or NsO-CH; or (3) E-$X_3$ represents CO, $R_{a2}$ represents hydroxy.

Synthetic method I:

In one embodiment of the present application, when E-$X_3$ represents CO and $R_{a2}$ represents hydrogen, the synthesis of the compounds of Formula I comprises:

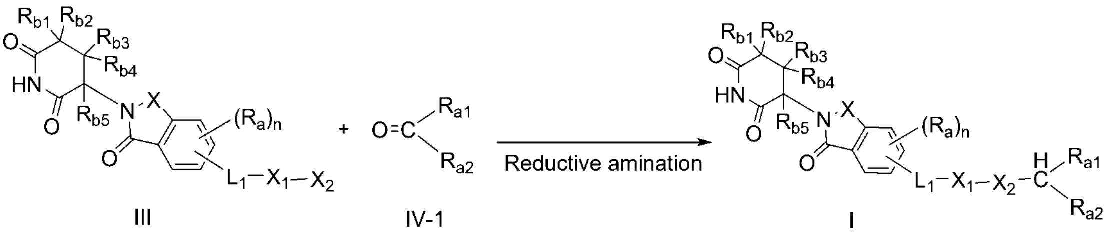


III IV-1 I

In synthetic method I, $(R_a)_n$, X, $L_1$, $X_1$, $R_{b1}$, $R_{b2}$, $R_{b3}$, $R_{b4}$, $R_{b5}$, $R_{a1}$, and $R_{a2}$ are as defined in the compounds of Formula (I) and its various sub-embodiments of the present disclosure. In the compound of Formula (III), $X_2$ represents optionally substituted nitrogen-containing heterocyclyl, optionally substituted aryl-$NH_2$, optionally substituted heteroaryl-$NH_2$, or optionally substituted cycloalkyl-$NH_2$.

Depending on the target compound, modifications and adjustments can be made to the substrates, reaction conditions (including stoichiometry, temperature, time, etc.), and workup procedures in synthetic method I using techniques and methods well-known to those skilled in the art to obtain the desired target compound. The reductive amination reaction in synthetic method I may employ conventional techniques and methods familiar to those skilled in the art. For example, the reductive amination may be conducted in the presence of sodium triacetoxyborohydride and 1,2-dichloroethane, or sodium cyanoborohydride and 1,2-dichloroethane, at temperatures ranging from room temperature to 80 °C (e.g., 40 °C -60°C, 40°C -50 °C, or 50 °C -60 °C). The molar ratio of the compound of Formula III to the compound of Formula IV-1 may be, for instance, 1:1.1-2, 1:1.1-1.5, 1:1.1-1.2, or 1:1.2-1.3 etc.

General Synthetic Procedure: To a solution of the amine substrate (Formula III) (1.0 equiv.) and the aldehyde substrate (Formula IV-1) (1.2 eq.) in 1,2-dichloroethane was added acetic acid (1 drop). The reaction solution was stirred at 50 °C for 2 hours, followed by the addition of sodium triacetoxyborohydride (2.0 eq.) to the solution. The reaction solution was stirred for another 17 hours. After monitoring the reaction via LCMS and confirming its completion, the reaction solution was filtered. The filtrate was subjected to HPLC for separation and purification to afford the target compound.

Synthetic method II:

When E-$X_3$ represents halogen-CH, MsO-CH, TsO-CH, or NsO-CH, the synthesis of the compounds of Formula I comprises:

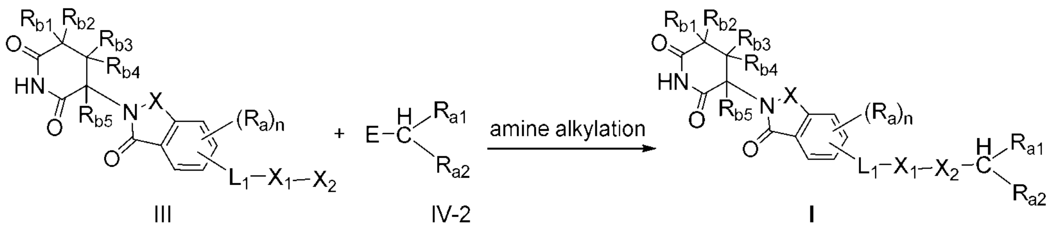


III IV-2 I

Wherein E is Cl, Br, I, OMs, OTs, or ONs.
In synthetic method II, $(R_a)_n$, X, $L_1$, $X_1$, $R_{b1}$, $R_{b2}$, $R_{b3}$, $R_{b4}$, $R_{b5}$, $R_{a1}$, and $R_{a2}$ are as defined in the compounds of Formula (I) and its various sub-embodiments of the present disclosure. In the compound of Formula (III), $X_2$ represents optionally substituted nitrogen-containing heterocyclyl, optionally substituted aryl-$NH_2$, optionally substituted heteroaryl-$NH_2$, or optionally substituted cycloalkyl-$NH_2$.
The amine alkylation reaction in synthetic method II may employ conventional techniques and methods familiar to those skilled in the art. For example, the amine alkylation may be conducted in the presence of either DIEA and sodium iodide, or triethylamine and sodium iodide at temperatures ranging from room temperature to 80 °C (typically 40 °C -60 °C, 40 °C -50°C, or 50 °C -60 °C), with the molar ratio of the compound of Formula III to the compound of Formula IV-2 being 1:1.1-2, 1:1.1-1.5, 1:1.1-1.2, or 1:1.2-1.3, etc.
General Synthetic Procedure: To a solution of the amine substrate (Formula III) (1.0 equiv) and the bromide substrate (Formula IV-2) (1.2 equiv) in N,N-dimethylformamide was added triethylamine (2.0 equiv) and sodium iodide (1.0 equiv). The reaction solution was stirred at 50 °C for 2 hours. After monitoring the reaction via LCMS and confirming its completion, the reaction solution was filtered. The filtrate was subjected to HPLC for separation and purification to afford the target compound.

Synthetic method III:

When E-$X_3$ represents CO and $R_{a2}$ represents hydroxy, the synthesis of the compounds of Formula I comprises:

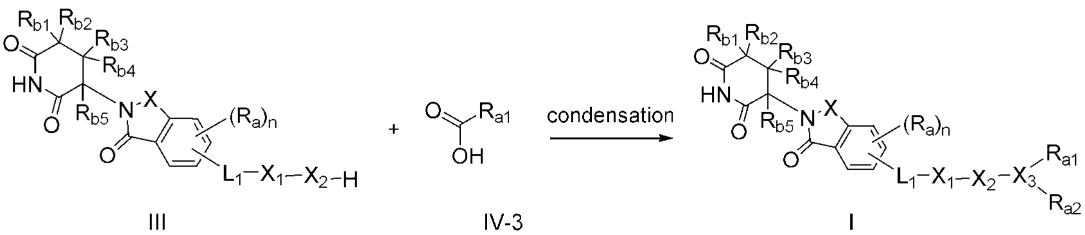


III IV-3 I

In synthetic method III, $(R_a)_n$, X, $L_1$, $X_1$, $R_{b1}$, $R_{b2}$, $R_{b3}$, $R_{b4}$, $R_{b5}$, $R_{a1}$, and $R_{a2}$ are as defined in the compounds of Formula (I) and its various sub-embodiments of the present disclosure. In the compound of Formula (III), $X_2$ represents optionally substituted nitrogen-containing heterocyclyl, optionally substituted aryl-$NH_2$, optionally substituted heteroaryl-$NH_2$, or optionally substituted cycloalkyl-$NH_2$.
The amide condensation reaction in synthetic method III may employ conventional techniques and methods familiar to those skilled in the art. For example, the amide condensation may be conducted in the presence of either HATU, DIEA and DMF, or HOAt, EDCI, TEA and DCM at temperatures ranging from room temperature to 80 °C (e.g., from room temperature to 30°C, 40 °C-60 °C, 40 °C-50 °C, or 50 °C-60°C), with the molar ratio of the compound of Formula III to the compound of Formula IV-3 being 1:1.1-2, 1:1.1-1.5, 1:1.1-1.2, or 1:1.2-1.3, etc.
General Synthetic Procedure: To a solution of the amine substrate (1.0 equiv) and the carboxylic acid substrate

(1.2 equiv) in N,N-dimethylformamide were added HATU (1.5 equiv) and triethylamine (3.0 equiv). The reaction solution was stirred at 50 °C for 2 hours. After monitoring the reaction via LCMS and confirming its completion, the reaction solution was filtered. The filtrate was subjected to HPLC for separation and purification to afford the target compound.

General synthetic method A of the compounds of Formula A

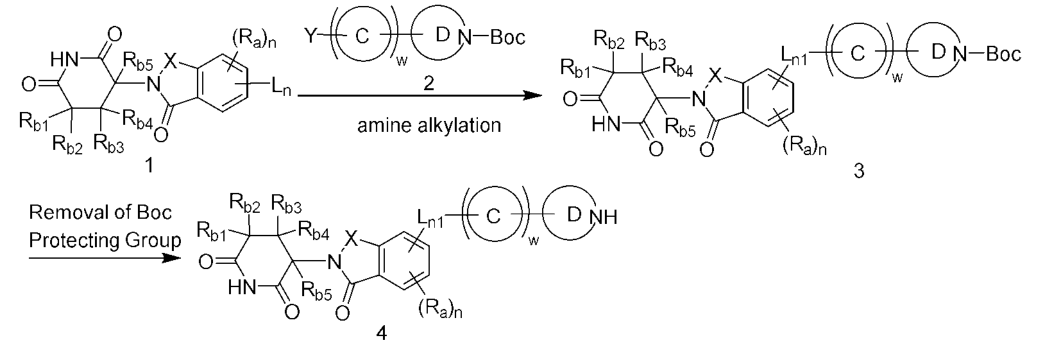


Wherein $L_n$ is OH or SH, Y is Cl, Br, I, OMs, OTs, or ONs etc, and $L_{n1}$ is O or S.

In synthetic method A, $(R_a)_n$, X, ring C, w, $R_{b1}$, $R_{b2}$, $R_{b3}$, $R_{b4}$ and $R_{b5}$ are as defined in the compounds of Formula A and its various sub-embodiments of the present disclosure. The nitrogen-containing heterocycle D in Reaction Substrate 2 of step 1 may be an optionally substituted heterocyclic group including, but not limited to, azetidine, pyrrolidine, imidazolidine, pyrazolidine, piperidine, piperazine, azacycloheptane, azacyclooctane, 7-azaspiro[3.5]nonane, 3-azaspiro[5.5]undecane, octahydrocyclopenta[c]pyrrole, 3-azabicyclo[3.2.0]heptane, 3-azabicyclo[3.1.0]hexane, and 3-azabicyclo[3.1.1]heptane etc.

The amine alkylation reaction in synthetic method A may employ conventional techniques and methods familiar to those skilled in the art. For example, the amine alkylation may be conducted in the presence of potassium carbonate and DMF, or DIEA and sodium iodide, or triethylamine and sodium iodide at temperatures ranging from room temperature to 95°C (e.g., 40 °C-60 °C, 40 °C-50 °C, 50 °C-60 °C, 60 °C-80 °C, or 60 °C-90 °C). The molar ratio of the reaction substrate 1 to the reaction substrate 2 in step 1 may be, for instance, 1:1.1-2, 1:1.1-1.5, 1:1.1-1.2, or 1:1.2-1.3 etc.

General Synthetic Procedure: Step 1: to a solution of the reaction substrate 1 (1.0 equiv) and reaction substrate 2 (1.2 equiv) in N,N-dimethylformamide was added potassium carbonate (2.0 equiv). The reaction flask was purged with $N_2$ three times to remove air, and the reaction mixture was heated to 80 °C with stirring for 16 hours. After monitoring the reaction via TLC and confirming its completion, the reaction mixture was cooled to room temperature, diluted with water and extracted with dichloromethane. The combined organic phases were dried over anhydrous sodium sulfate, filtered, and the filtrate was concentrated under reduced pressure. The crude residue was purified by silica gel column chromatography to afford Boc-protected product 3.

Step 2: To a solution of the Boc-protected product 3 from Step 1 in dichloromethane was added a solution of HCl in ethyl acetate (2.0 equiv). The reaction mixture was stirred at room temperature for 1 hour. After monitoring the reaction via LCMS and confirming its completion, the reaction mixture was filtered to afford the target product 4 as a solid.

Example A1: Preparation of 3-(5-(azetidin-3-yloxy)-1-oxoisoindolin-2-yl)piperidine-2,6-dione (GT-03614)

Scheme 1

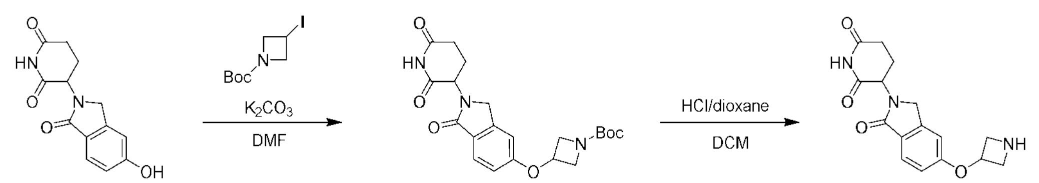


Step 1): To a solution of 3-(5-hydroxy-1-oxoisoindolin-2-yl)piperidine-2,6-dione (7.0 g, 26.9 mmol) in N,N-dimethylformamide (100 mL) were successively added tert-butyl 3-iodoazetidine-1-carboxylate (7.6 g, 26.9 mmol) and potassium carbonate (7.4 g, 53.8 mmol). The reaction mixture was stirred at 90 °C for 18 hours. After monitoring the reaction via TLC and confirming its completion, the reaction mixture was cooled to room temperature and concentrated under reduced pressure. The residue was washed with water (100 mL) and extracted with ethyl acetate (100 mL × 3). The

combined organic phases were washed with saturated brine (100 mL), filtered, and the filtrate was concentrated under reduced pressure. The residue was purified by column chromatography to afford tert-butyl 3-((2-(2,6-dioxopiperidin-3-yl)-1-oxoisoindolin-5-yl)oxy)azetidine-1-carboxylate (2.0 g, yield: 18%, white solid). $^1$H NMR (400 MHz, DMSO) δ 10.96 (s, 1H), 7.65 (d, J = 8.4 Hz, 1H), 7.04 (s, 1H), 6.98 (d, J = 8.4, Hz, 1H), 5.12 - 5.03 (m, 2H), 4.43 - 4.24 (m, 4H), 3.82 (d, J = 9.2 Hz, 2H), 2.96 - 2.85 (m, 1H), 2.61 - 2.57 (m, 1H), 2.41 - 2.36 (m, 1H), 2.03 - 1.93 (m, 1H), 1.39 (s, 9H). LC/MS (ESI) m/z: calcd for $C_{21}H_{25}N_3NaO_6$+ [M+Na]$^+$, 438.16; found, 438.2.

Step 2): To a solution of tert-butyl 3-((2-(2,6-dioxopiperidin-3-yl)-1-oxoisoindolin-5-yl)oxy)azetidine-1-carboxylate (2.0 g, 4.8 mmol) in dichloromethane (20 mL) was added hydrochloric acid/1,4-dioxane solution (80 mL, 3 M). The reaction mixture was stirred at room temperature for 1 hour. After monitoring the reaction via TLC and confirming its completion, the reaction mixture was filtered. The filter cake was washed with ethyl acetate (10 mL × 2) to afford 3-(5-(azetidin-3-yloxy)-1-oxoisoindolin-2-yl)piperidine-2,6-dione (GT-03614) (1.6 g, yield: 92%, white solid). $^1$H NMR (400 MHz, DMSO) δ 10.97 (s, 1H), 9.32 (brs, 1H), 9.30 (brs, 1H), 7.67 (d, *J* = 8.4 Hz, 1H), 7.10 (s, 1H), 7.02 (d, *J* = 8.4, 1H), 5.20 - 5.16 (m, 1H), 5.10 - 5.06 (m, 1H), 4.48 - 4.46 (m, 2H), 4.34 (dd, *J* = 48.6, 17.2 Hz, 2H), 4.01 (brs, 2H), 2.97 - 2.85 (m, 1H), 2.62 - 2.60 (m, 1H), 2.39 - 2.37 (m, 1H), 2.03 - 1.95 (m, 1H), 1.24 (s, 1H). LC/MS (ESI) m/z: calcd for $C_{16}H_{18}N_3O_4$+ [M+H]$^+$, 316.13; found, 316.2.

Example A2: Preparation of 3-(5-(azetidin-3-ylthio)-1-oxoisoindolin-2-yl)piperidine-2,6-dione (GT-03613)

Scheme 2

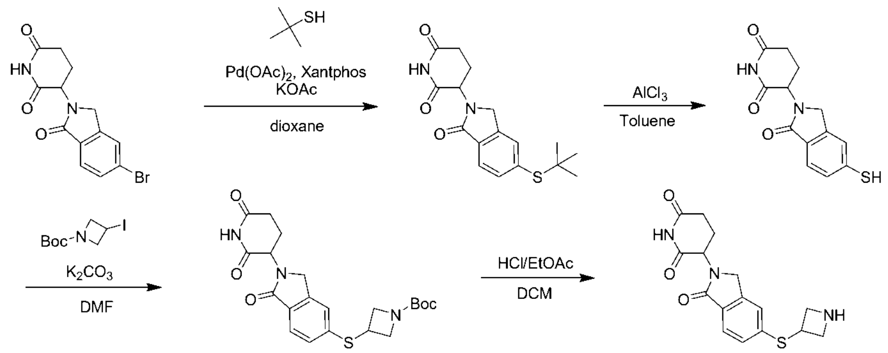


A mixture of 3-(5-bromo-1-oxoisoindolin-2-yl)piperidine-2,6-dione (10.0 g, 30.9 mmol), Xantphos (0.54 g, 0.93 mmol), palladium acetate (0.21 g, 0.93 mmol), potassium acetate (6.07 g, 61.9 mmol), and tert-butylthiol (5.2 mL, 46.4 mmol) in 1,4-dioxane (120 mL) was degassed with nitrogen three times and heated to 90 °C for 18 hours. After monitoring the reaction via LCMS and confirming its completion, the reaction mixture was cooled to room temperature and concentrated under reduced pressure. The residue was washed with water (50 mL) and extracted with dichloromethane (100 mL × 3). The combined organic phases were dried over anhydrous sodium sulfate, filtered, and the filtrate was concentrated under reduced pressure. The residue was triturated with ethanol (40 mL) and filtered to afford 3-(5-(tert-butylthio)-1-oxoisoindolin-2-yl)piperidine-2,6-dione (9.4 g, 91%, brown solid). LC/MS (ESI) m/z: calcd for $C_{17}H_{21}N_2O_3S^+$ [M+H]$^+$, 333.13; found, 333.1.

To a solution of 3-(5-(tert-butylthio)-1-oxoisoindolin-2-yl)piperidine-2,6-dione (9.4 g, 28.3 mmol) in anhydrous toluene (120 mL) was added aluminum chloride (11.3 g, 84.8 mmol) portionwise. The reaction mixture was heated to 35 °C for 18 hours. After monitoring the reaction via LCMS and confirming its completion, the reaction mixture was cooled to room temperature, quenched with 20% aqueous citric acid (50 mL), and stirred for 2 hours. The resulting precipitate was filtered and washed with ethyl acetate (50 mL × 2) to give 3-(5-mercapto-1-oxoisoindolin-2-yl)piperidine-2,6-dione (7.2 g, 92%, brown solid). LC/MS (ESI) m/z: calcd for $C_{13}H_{13}N_2O_3S^+$ [M+H]$^+$, 277.06; found, 277.0.

To a solution of 3-(5-mercapto-1-oxoisoindolin-2-yl)piperidine-2,6-dione (3.3 g, 11.9 mmol) in N,N-dimethylformamide (33 mL) were successively added tert-butyl 3-iodoazetidine-1-carboxylate (3.38 g, 11.9 mmol) and potassium carbonate (4.13 g, 29.8 mmol). The reaction mixture was heated to 80 °C for 18 hours. After monitoring the reaction via LCMS and confirming its completion, the reaction mixture was diluted with ethyl acetate (200 mL) and washed with water (50 mL × 3). The organic phases were dried over anhydrous sodium sulfate, filtered, and the filtrate was concentrated under reduced pressure to give tert-butyl 3-((2-(2,6-dioxopiperidin-3-yl)-1-oxoisoindolin-5-yl)thio)azetidine-1-carboxylate (4.5 g, yield: 87%, yellow solid). LC/MS (ESI) m/z: calcd for $C_{21}H_{25}N_3NaO_5S^+$ [M+Na]$^+$, 454.14; found, 454.1.

To a solution of tert-butyl 3-((2-(2,6-dioxopiperidin-3-yl)-1-oxoisoindolin-5-yl)thio)azetidine-1-carboxylate (8.8 g, 20.4 mmol) in dichloromethane (60 mL) was added HCl/ethyl acetate solution (88 mL, 3M). The reaction mixture was

stirred to room temperature for 3 hours. After monitoring the reaction via TLC and confirming its completion, the reaction mixture was filtered. The filter cake was washed with ethyl acetate (10 mL × 2) to afford 3-(5-(azetidin-3-ylthio)-1-oxoisoindolin-2-yl)piperidine-2,6-dione (GT-03613) (5 g, yield: 67%, yellow solid). $^1H$ NMR (400 MHz, DMSO) δ 10.99 (s, 1H), 9.27 (brs, 2H), 7.69 (d, *J* = 8.0 Hz, 1H), 7.52 (s, 1H), 7.40 (d, *J* = 8.0 Hz, 1H), 5.11 (dd, *J* = 13.2, 5.2 Hz, 1H), 4.68 - 4.29 (m, 5H), 3.88 (s, 2H), 2.92 - 2.89 (m, 1H), 2.62 - 2.59 (m, 1H), 2.45 - 2.33 (m, 1H), 2.01 - 1.98 (m, 1H). LC/MS (ESI) m/z: calcd for $C_{16}H_{18}N_3O_3S^+$ [M+H]$^+$, 332.11; found, 332.0.

Example A3: Preparation of 3-(1-oxo-5-(pyrrolidin-3-ylthio)isoindolin-2-yl)piperidine-2,6-dione (GT-04309)

The target compound (GT-04309) was prepared as a yellow solid (3.7 g, yield: 99%) according to the method described in Scheme 2. $^1H$ NMR (400 MHz, DMSO) δ 10.99 (s, 1H), 9.18 (brs, 2H), 7.69 (d, J = 8.0 Hz, 1H), 7.65(s, 1H), 7.51 (d, J = 8.0 Hz, 1H), 5.13 - 5.08 (m, 1H), 4.39 (dd, J = 48.6, 17.2 Hz, 2H), 4.26 - 4.12 (m, 1H), 3.71 - 3.63 (m, 1H), 3.28 (s, 1H), 3.12 - 3.10 (m, 1H), 2.95 - 2.83 (m, 1H), 2.61 - 2.59 (m, 1H), 2.46 - 2.25 (m, 2H), 2.04 - 1.82 (m, 2H), 1.28 - 1.23 (m, 1H). LC/MS (ESI) m/z: calcd for $C_{17}H_{19}N_3O_3S^+$ [M+H]$^+$, 346.12; found, 346.0.

Example A4: Preparation of 3-(1-oxo-5-(piperidin-4-ylthio)isoindolin-2-yl)piperidine-2,6-dione (GT-03616)

The target compound (GT-03616) was prepared as a white solid (2.7 g, yield: 51%) according to the method described in Scheme 2. $^1H$ NMR (400 MHz, DMSO) δ 11.00 (s, 1H), 8.67 (s, 1H), 8.59 (s, 1H), 7.73 - 7.63 (m, 2H), 7.52 (dd, J = 8.0 Hz, 1H), 5.11 (dd, J = 13.2, 5.2 Hz, 1H), 4.38 (dd, J = 48.6, 17.2 Hz, 2H), 3.75 - 3.61 (m, 1H), 3.33 - 3.30 (m, 2H), 3.06 - 2.85 (m, 3H), 2.62 - 2.59 (m, 1H), 2.42 - 2.39 (m, 1H), 2.12 - 2.09 (m, 2H), 2.04 - 1.95 (m, 1H), 1.74 - 1.59 (m, 2H). LC/MS (ESI) m/z: calcd for $C_{18}H_{22}N_3O_3S^+$ [M+H]$^+$, 360.14; found, 360.1.

Example A5: Preparation of 3-(5-(azepan-4-ylthio)-1-oxoisoindolin-2-yl)piperidine-2,6-dione (GT-06995)

The target compound (GT-06995) was prepared as a white solid (2.0 g, yield: 86%) according to the method described in synthetic method A. $^1H$ NMR (400 MHz, MeOD-d4) δ 7.75 (d, *J* = 8.0 Hz, 1H), 7.64 (s, 1H), 7.53 (d, *J* = 8.0 Hz, 1H), 5.14 (dd, *J* = 13.3, 5.2 Hz, 1H), 4.60 - 4.41 (m, 2H), 3.79 (tt, *J* = 8.7, 4.3 Hz, 1H), 3.42 (ddd, *J* = 14.0, 7.6, 2.0 Hz, 1H), 3.32 - 3.20 (m, 3H), 3.00 - 2.73 (m, 2H), 2.51 (qd, *J*= 13.2, 4.8 Hz, 1H), 2.41 - 2.16 (m, 3H), 2.15 - 1.88 (m, 3H), 1.86 - 1.73 (m, 1H). LCMS (ESI) calcd for $C_{19}H_{24}N_3O_3S^+$ [M+H]$^+$: 374.15, found, 374.1.

Example A6: Preparation of 3-(4-(azetidin-3-ylthio)-1-oxoisoindolin-2-yl)piperidine-2,6-dione (GT-06992)

The target compound (GT-06992) was prepared as a white solid (4.5 g, yield: 76%) according to the method described in synthetic method A. $^1H$ NMR (400 MHz, DMSO-d6) δ 11.01 (s, 1H), 9.08 (s, 2H), 7.69 (dd, *J* = 6.2, 2.2 Hz, 1H), 7.62 - 7.52 (m, 2H), 5.15 (dd, *J* = 13.3, 5.1 Hz, 1H), 4.56 - 4.44 (m, 3H), 4.40 (s, 1H), 4.27 (d, *J* = 17.5 Hz, 1H), 3.95 - 3.85 (m, 2H), 3.00 - 2.87 (m, 1H), 2.60 (d, *J* = 17.6 Hz, 1H), 2.45 (dd, *J* = 13.1, 4.4 Hz, 1H), 2.01 (dd, *J* = 11.0, 5.9 Hz, 1H). LCMS (ESI) calcd for $C_{16}H_{18}N_3O_3S^+$ [M+H]$^+$: 332.11, found, 332.1.

Example A7: Preparation of 3-(6-(azetidin-3-ylthio)-1-oxoisoindolin-2-yl)piperidine-2,6-dione (GT-06993)

The target compound (GT-06993) was prepared as a white solid (2.5 g, yield: 70%) according to the method described in synthetic method A. $^1H$ NMR (400 MHz, MeOD-d4) δ 7.76 (s, 1H), 7.66 (dd, J = 8.0, 1.5 Hz, 1H), 7.61 (d, *J* = 8.0 Hz, 1H), 5.16 (dd, *J* = 13.3, 5.2 Hz, 1H), 4.58 - 4.40 (m, 5H), 4.02 - 3.97 (m, 2H), 2.92 - 2.86 (m, 1H), 2.82 - 2.78 (m, 1H), 2.56 - 2.44 (m, 1H), 2.21 - 2.15 (m, 1H). LCMS (ESI) calcd for $C_{16}H_{18}N_3O_3S^+$ [M+H]$^+$: 332.11, found, 332.1.

Example A8: Preparation of 3-(7-(azetidin-3-ylthio)-1-oxoisoindolin-2-yl)piperidine-2,6-dione (GT-06994)

The target compound (GT-06994) was prepared as a white solid (3.1 g, yield: 78%) according to the method described in synthetic method A. $^1H$ NMR (400 MHz, MeOD) δ 7.58 (t, *J* = 7.7 Hz, 1H), 7.49 - 7.38 (m, 1H), 7.15 (d, *J* = 7.7 Hz, 1H), 5.10 (dd, *J* = 13.3, 5.2 Hz, 1H), 4.70 - 4.60 (m, 2H), 4.58 - 4.53 (m, 1H), 4.49 - 4.43 (m, 2H), 4.00 (dd, *J*= 11.8, 5.9 Hz, 2H), 2.97 - 2.85 (m, 1H), 2.82 - 2.76 (m, 1H), 2.54 - 2.46 (m, 1H), 2.20 - 2.14 (m, 1H). LCMS (ESI) calcd for $C_{16}H_{18}N_3O_3S^+$ [M+H]$^+$: 332.11, found, 332.1.

Example A9: Preparation of 3-(5-((7-azaspiro[3.5]nonan-2-yl)thio)-1-oxoisoindolin-2-yl)piperidine-2,6-dione (GT-06997)

The target compound (GT-06997) was prepared as a white solid (480 mg, yield: 69%) according to the method

described in synthetic method A. $^1$H NMR (400 MHz, $D_2O$) δ 7.59 (d, *J* = 8.0 Hz, 1H), 7.26 - 7.24 (m, 2H), 5.04 (dd, *J* = 13.2, 5.2 Hz, 1H), 4.45 - 4.32 (m, 2H), 4.05 - 3.85 (m, 1H), 3.14 - 3.06 (m, 2H), 3.06 - 2.98 (m, 2H), 2.86 - 2.82 (m, 2H), 2.52 - 2.35 (m, 3H), 2.23 - 2.11 (m, 1H), 1.89 - 1.84 (m, 4H), 1.80 - 1.73 (m, 2H). LCMS (ESI) calcd for $C_{21}H_{26}N_3O_3S^+$ $[M+H]^+$: 400.17, found, 400.1.

Example A10: Preparation of 3-(5-((3-azaspiro[5.5]undecan-9-yl)thio)-1-oxoisoindolin-2-yl)piperidine-2,6-dione (GT-06998)

The target compound (GT-06998) was prepared as a white solid (350 mg, yield: 90%) according to the method described in synthetic method A. $^1$H NMR (400 MHz, MeOD-d4) δ 7.70 (d, *J* = 8.0 Hz, 1H), 7.56 (s, 1H), 7.49 (dd, *J* = 8.0, 1.4 Hz, 1H), 5.14 (dd, *J* = 13.3, 5.2 Hz, 1H), 4.53 - 4.39 (m, 2H), 3.49 - 3.38(m, 1H), 3.19 - 3.11 (m, 4H), 2.91 - 2.86 (m, 1H), 2.81 - 2.76 (m, 1H), 2.50 - 2.46 (m, 1H), 2.22 - 2.13 (m, 1H), 1.99 - 1.91 (m, 2H), 1.85 - 1.71 (m, 4H), 1.65 - 1.54 (m, 4H), 1.47 - 1.37 (m, 2H). LCMS (ESI) calcd for $C_{23}H_{30}N_3O_3S^+$ $[M+H]^+$: 428.20, found, 428.2.

Example A11: Preparation of 3-(5-((octahydrocyclopenta[c]pyrrol-5-yl)thio)-1-oxoisoindolin-2-yl)piperidine-2,6-dione (GT-06996)

The target compound (GT-06996) was prepared as a white solid (960 mg, yield: 88%) according to the method described in synthetic method A. $^1$H NMR (400 MHz, DMSO-d6) δ 10.99 (s, 1H), 9.37 (s, 2H), 7.78 - 7.53 (m, 2H), 7.52 - 7.34 (m, 1H), 5.10 (dd, *J*= 13.2, 4.9 Hz, 1H), 4.49 (d, J=17.5 Hz, 1H), 4.30 (d, *J* = 17.5 Hz, 1H), 4.14 - 3.64 (m, 1H), 3.31 - 3.26 (m, 1H), 3.24 - 3.04 (m, 3H), 3.02 - 2.82 (m, 4H), 2.60 (d, *J* = 16.5 Hz, 1H), 2.43 - 2.32 (m, 2H), 2.18 - 2.04 (m, 1H), 2.02 - 1.94 (m, 1H), 1.84 - 1.73 (m, 1H), 1.58 - 1.47 (m, 1H). LCMS (ESI) calcd for $C_{20}H_{24}N_3O_3S^+$ $[M+H]^+$: 386.15, found, 386.1.

Example A12: Preparation of 3-(5-((3,3-difluoropiperidin-4-yl)thio)-1-oxoisoindolin-2-yl)piperidine-2,6-dione (GT-06999)

The target compound (GT-06999) was prepared as a white solid (1.65g, yield: 80%) according to the method described in synthetic method A. $^1$H NMR (400 MHz, DMSO-d6) δ 11.00 (s, 1H), 10.36 (s, 1H), 9.46 (s, 1H), 7.80 - 7.67 (m, 2H), 7.61 (d, *J* = 8.0 Hz, 1H), 5.12 (dd, *J*= 13.3, 4.9 Hz, 1H), 4.46 (d, *J*= 17.5 Hz, 1H), 4.33 (d, *J* = 17.5 Hz, 1H), 4.31 - 4.18 (m, 1H), 3.86 - 3.81 (m, 1H), 3.64 - 3.59 (m, 1H), 3.29 - 3.26 (m, 1H), 3.12 (t, *J* = 12.1 Hz, 1H), 2.97 - 2.84 (m, 1H), 2.61 (d, *J* = 16.8 Hz, 1H), 2.46 - 2.31 (m, 2H), 2.02 - 1.94 (m, 2H). LCMS (ESI) calcd for $C_{18}H_{20}F_2N_3O_3S^+$ $[M+H]^+$: 396.12, found, 396.1.

Example A13: Preparation of 3-(1-oxo-5-(pyrrolidin-3-yloxy)isoindolin-2-yl)piperidine-2,6-dione (GT-07002)

The target compound (GT-07002) was prepared as a white solid (1.0 g, yield: 86%) according to the method described in synthetic method A. $^1$H NMR (400 MHz, DMSO-d6) δ 10.98 (s, 1H), 9.84 (s, 1H), 9.61 (s, 1H), 7.66 (d, *J* = 8.4 Hz, 1H), 7.23 (s, 1H), 7.09 (dd, *J* = 8.4, 1.9 Hz, 1H), 5.29 - 5.22 (m, 1H), 5.08 (dd, *J* = 13.3, 5.0 Hz, 1H), 4.41 (d, *J* = 17.3 Hz, 1H), 4.29 (d, *J* = 17.3 Hz, 1H), 3.55 - 3.49 (m, 1H), 3.38 - 3.34 (m, 2H), 3.30 - 3.17 (m, 1H), 2.96 - 2.87 (m, 1H), 2.60 (d, *J* = 17.1 Hz, 1H), 2.42 - 2.37 (m, 1H), 2.30 - 2.11 (m, 2H), 2.01 - 1.97 (m, 1H). LCMS (ESI) calcd for $C_{17}H_{20}N_3O_4^+$ $[M+H]^+$: 330.14, found, 330.1.

Example A14: Preparation of 3-(1-oxo-5-(piperidin-4-yloxy)isoindolin-2-yl)piperidine-2,6-dione (GT-03612)

The target compound (GT-03612) was prepared as a white solid (2.3 g, yield: 98%) according to the method described in synthetic method A. $^1$H NMR (400 MHz, DMSO) δ 10.96 (s, 1H), 9.15 (s, 2H), 7.65 (d, J = 8.4 Hz, 1H), 7.25 (s, 1H), 7.13 (dd, J = 8.4, 1.9 Hz, 1H), 5.07 (dd, J = 13.3, 5.0 Hz, 1H), 4.84 - 4.75 (m, 1H), 4.40 (d, J = 17.2 Hz, 1H), 4.27 (d, J = 17.2 Hz, 1H), 3.22 (s, 2H), 3.07 (d, J = 6.0 Hz, 2H), 2.91 (ddd, J = 13.5, 12.5, 5.4 Hz, 1H), 2.60 (d, J = 16.9 Hz, 1H), 2.39 (qd, J = 13.2, 4.3 Hz, 1H), 2.15 (s, 2H), 2.03 - 1.95 (m, 1H), 1.90 (d, J = 11.9 Hz, 2H). LCMS (ESI) calcd for $C_{18}H_{22}N_3O_4^+$ $[M+H]^+$: 344.16, found, 344.1.

Example A15: Preparation of 3-(5-(azepan-4-yloxy)-1-oxoisoindolin-2-yl)piperidine-2,6-dione (GT-07003)

The target compound (GT-07003) was prepared as a white solid (440 mg, yield: 91%) according to the method described in synthetic method A. $^1$H NMR (400 MHz, DMSO-d6) δ 10.97 (s, 1H), 8.76 (s, 2H), 7.64 (d, *J* = 8.4 Hz, 1H), 7.20 (s, 1H), 7.07 (d, *J* = 8.5 Hz, 1H), 5.08 (dd, *J* = 13.3, 5.0 Hz, 1H), 4.87 - 4.78 (m, 1H), 4.39 (d, *J* = 17.1 Hz, 1H), 4.27 (d, *J* = 17.2 Hz, 1H), 3.30 - 3.24 (m, 1H), 3.21 - 3.04 (m, 3H), 2.99 - 2.83 (m, 1H), 2.60 (d, *J* = 17.1 Hz, 1H), 2.43 - 2.34 (m, 1H), 2.27 - 2.14 (m, 1H), 2.13 - 1.83 (m, 5H), 1.78 - 1.71 (m, 1H). LCMS (ESI) calcd for $C_{19}H_{24}N_3O_4^+$ $[M+H]^+$: 358.18, found, 358.1.

Example A16: Preparation of 3-(4-(azetidin-3-yloxy)-1-oxoisoindolin-2-yl)piperidine-2,6-dione (GT-07000)

The target compound (GT-07000) was prepared as a white solid (4.2 g, yield: 95%) according to the method described in synthetic method A. $^1$H NMR (400 MHz, MeOD) δ 7.59 - 7.40 (m, 2H), 6.99 - 6.97 (m, 1H), 5.37 - 5.27 (m, 1H), 5.17 (dd, *J* = 13.3, 5.1 Hz, 1H), 4.64 - 4.59 (m, 2H), 4.56 - 4.44 (m, 2H), 4.23 - 4.19 (m, 2H), 2.97 - 2.88 (m, 1H), 2.83 - 2.77 (m, 1H), 2.56 - 2.47 (m, 1H), 2.23 - 2.17 (m, 1H).LCMS (ESI) calcd for $C_{16}H_{18}N_3O_4^+$ $[M+H]^+$: 316.13, found, 316.1.

Example A17: Preparation of 3-(6-(azetidin-3-yloxy)-1-oxoisoindolin-2-yl)piperidine-2,6-dione (GT-07001)

The target compound (GT-07001) was prepared as a white solid (1.1 g, yield: 93%) according to the method described in synthetic method A. $^1$H NMR (400 MHz, DMSO-d6) δ 10.99 (s, 1H), 9.23 (s, 1H), 9.06(s, 1H), 7.58 (d, *J* = 8.2 Hz, 1H), 7.20 (dd, *J* = 8.3, 2.5 Hz, 1H), 7.11 (d, *J* = 2.4 Hz, 1H), 5.28 - 5.17 (m, 1H), 5.11 (dd, *J*= 13.3, 5.1 Hz, 1H), 4.55 - 4.45 (m, 2H), 4.44 - 4.35 (m, 1H), 4.26 (d, *J*= 17.1 Hz, 1H), 4.05 - 4.01 (m, 2H), 2.96 - 2.87 (m, 1H), 2.60 (d, *J* = 17.2 Hz, 1H), 2.45 - 2.34 (m, 1H), 2.03 - 1.97 (m, 1H). LCMS (ESI) calcd for $C_{16}H_{18}N_3O_4^+$ $[M+H]^+$: 316.13, found, 316.1.

Example A18: Preparation of 3-(7-(azetidin-3-yloxy)-1-oxoisoindolin-2-yl)piperidine-2,6-dione (GT-07052)

The target compound (GT-07052) was prepared as a white solid (95 mg, yield: 100%) according to the method described in synthetic method A. $^1$H NMR (400 MHz, DMSO-d6) δ 10.98 (s, 1H), 9.07 (s, 1H), 8.89 (s, 1H), 7.56 (t, *J*= 7.9 Hz, 1H), 7.25 (d, *J* = 7.5 Hz, 1H), 6.84 (d, *J*= 8.2 Hz, 1H), 5.28 - 5.12 (m, 1H), 5.01 (dd, *J* = 13.2, 5.0 Hz, 1H), 4.57 - 4.37 (m, 3H), 4.30 (d, *J* = 17.5 Hz, 1H), 4.07 (s, 2H), 2.95 - 2.84 (m, 1H), 2.68 - 2.59 (m, 1H), 2.42 - 2.34 (m, 1H), 2.02 - 1.96 (m, 1H). LCMS (ESI) calcd for $C_{16}H_{18}N_3O_4^+$ $[M+H]^+$: 316.13, found, 316.1.

Example A19: Preparation of 3-(5-((7-azaspiro[3.5]nonan-2-yl)oxy)-1-oxoisoindolin-2-yl)piperidine-2,6-dione (GT-07005)

The target compound (GT-07005) was prepared as a white solid (2.1 g, yield: 95%) according to the method described in synthetic method A. $^1$H NMR (400 MHz, DMSO-d6) δ 10.97 (s, 1H), 8.97 (s, 2H), 7.62 (d, *J* = 8.4 Hz, 1H), 7.06 (s, 1H), 6.97 (dd, *J* = 8.4, 1.9 Hz, 1H), 5.07 (dd, *J* = 13.3, 5.0 Hz, 1H), 4.90 - 4.79 (m, 1H), 4.38 (d, *J* = 17.3 Hz, 1H), 4.26 (d, *J* = 17.2 Hz, 1H), 3.07 - 3.00 (m, 2H), 2.98 - 2.92 (m, 2H), 2.87 (d, *J* = 5.3 Hz, 1H), 2.64 - 2.52 (m, 2H), 2.50 - 2.47 (m, 1H), 2.39 - 2.33 (m, 1H), 2.03 - 1.95 (m, 1H), 1.93 - 1.85 (m, 2H), 1.83 - 1.80 (m, 2H), 1.79 - 1.71 (m, 2H). LCMS (ESI) calcd for $C_{19}H_{26}N_3O_4^+$ $[M+H]^+$: 384.19, found, 384.2.

Example A20: Preparation of 3-(5-((octahydrocyclopenta[c]pyrrol-5-yl)oxy)-1-oxoisoindolin-2-yl)piperidine-2,6-dione (GT-07004)

The target compound (GT-07004) was prepared as a white solid (1.5 g, yield: 86%) according to the method described in synthetic method A. $^1$H NMR (400 MHz, DMSO) δ 11.03 (s, 1H), 9.50 (s, 1H), 9.39 (s, 1H), 7.71 - 7.66 (m, 1H), 7.29 - 7.26 (m, 1H), 7.16 - 7.09 (m, 1H), 5.19 - 5.12 (m, 1H), 5.08 - 5.06(m, 1H), 4.45 (d, *J* = 17.2 Hz, 1H), 4.32 (d, *J* = 17.2 Hz, 1H), 3.49 - 3.22 (m, 2H), 3.16 - 2.87 (m, 5H), 2.65 (d, *J* = 16.9 Hz, 1H), 2.47 - 2.43 (m, 1H), 2.25 - 2.22 (m, 1H), 2.14 - 2.01 (m, 2H), 1.94 - 1.83 (m, 1H). LCMS (ESI) calcd for $C_{20}H_{24}N_3O_4^+$ $[M+H]^+$: 370.18, found, 370.1.

**Example 1: Preparation of 3-(5-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione (GT-03464; compound 1)**

The synthesis was performed according to synthetic method I using compound (GT-03613) as the starting material.

The target compound (GT-03464) was prepared as a white solid (10 mg, yield: 20%). $^1$H NMR (400 MHz, MeOD) δ 7.66 (d, *J* = 8.0 Hz, 1H), 7.33 - 7.28 (m, 3H), 7.24 - 7.15 (m, 1H), 7.05 - 6.99 (m, 2H), 5.05 (dd, *J* = 13.2, 5.2 Hz, 2H), 4.59 - 4.44 (m, 4H), 4.41 - 4.30 (m, 4H), 3.74 (s, 2H), 2.95 - 2.75 (m, 2H), 2.73 - 2.67 (m, 1H), 2.40 - 2.36 (m, 1H), 2.17 - 2.02 (m, 5H), 1.49 - 1.43 (m 2H), 0.92 (s, 6H). LC/MS (ESI) m/z: calcd for $C_{31}H_{35}ClN_3O_3S^+$ $[M+H]^+$, 564.21; found, 564.2.

**Example 2: Preparation of 3-(5-((1-((4'-chloro-5,5-dimethyl-3,4,5,6-tetrahydro-[1,1'-biphenyl]-2-yl)methyl)pyrrolidin-3-yl)thio)-1-oxoisoindolin-2-yl)piperidine-2,6-dione (GT-04245; compound 5)**

The synthesis was performed according to synthetic method I using compound (GT-04309) as the starting material.

The target compound (GT-04245) was prepared as a white solid (12 mg, yield: 18%). $^1$H NMR (400 MHz, MeOD)

δ 7.67 (d, $J$ = 8.0 Hz, 1H), 7.53 - 7.33 (m, 2H), 7.29 (d, $J$ = 8.4 Hz, 2H), 7.00 (d, $J$ = 8.4 Hz, 2H), 5.06 (dd, $J$ = 13.2, 5.2 Hz, 1H), 4.46 - 4.33 (m, 2H), 4.15 - 3.90 (m, 1H), 3.85 - 3.77 (m, 1H), 3.72 - 3.65 (m, 1H), 3.56 - 3.48 (m, 1H), 3.37 - 3.28 (m, 1H), 3.14 - 3.08 (m, 1H), 2.87 - 2.77 (m, 2H), 2.74 - 2.63 (m, 1H), 2.50 - 2.29 (m, 2H), 2.22 - 2.15 (m, 2H), 2.09 - 2.03 (m, 3H), 1.96 - 1.79 (m, 1H), 1.47 (t, $J$ = 6.0 Hz, 2H), 0.92 (s, 6H). LC/MS (ESI) m/z: calcd for $C_{32}H_{37}ClN_3O_3S^+$ $[M+H]^+$, 578.22; found, 578.3.

**Example 3: Preparation of 3-(5-((1-((4'-chloro-5,5-dimethyl-3,4,5,6-tetrahydro-[1,1'-biphenyl]-2-yl)methyl)piperidin-4-yl)thio)-1-oxoisoindolin-2-yl)piperidine-2,6-dione (GT-03462; compound 6)**

The synthesis was performed according to synthetic method I using compound (GT-03616) as the starting material.

The target compound (GT-03462) was prepared as a white solid (6 mg, yield: 16%). $^1$H NMR (400 MHz, MeOD) δ 7.65 (d, $J$ = 8.0 Hz, 1H), 7.51 (brs, 1H), 7.46 - 7.41 (m, 1H), 7.35 - 7.29 (m, 2H), 7.06 - 6.99 (s, 2H), 5.05 (dd, $J$ = 13.2, 5.2 Hz, 1H), 4.60 - 4.50(m, 2H), 4.45 - 4.30 (m, 2H), 3.53 - 3.46 (m, 2H), 3.39 - 3.31 (m, 3H), 2.89 - 2.74 (m, 1H), 2.74 - 2.54 (m, 2H), 2.47 - 2.28 (m, 1H), 2.26 - 1.96 (m, 7H), 1.78 - 1.67 (m, 1H), 1.53 - 1.42 (m, 2H), 0.92 (s, 6H). LC/MS (ESI) m/z: calcd for $C_{33}H_{39}ClN_3O_3S^+$ $[M+H]^+$, 592.24; found, 592.3.

**Example 4: Preparation of 3-(5-((1-((4'-chloro-[1,1'-biphenyl]-2-yl)methyl)azetidin-3-yl)thio)-1-oxoisoindolin-2-yl)piperidine-2,6-dione (GT-03360; compound 28)**

The synthesis was performed according to synthetic method II using compound (GT-03613) as the starting material.

The target compound (GT-03360) was prepared as a white solid (20 mg, yield: 47%). $^1$H NMR (400 MHz, MeOD) δ 7.64 (d, J = 8.0 Hz, 1H), 7.44-7.40 (m, 4H), 7.30 (d, J = 12.8 Hz, 2H), 7.24-7.22 (m, 3H), 7.21 (d, J = 8.4 Hz, 1H), 5.07-5.03 (m, 1H), 4.41-4.36 (m, 5H), 4.28-4.17 (m, 2H), 3.76-3.70 (m, 2H), 2.86-2.76 (m, 1H), 2.71-2.66 (m, 1H), 2.41-2.37 (m, 1H), 2.10-2.05 (m, 1H). LC/MS (ESI) m/z: calcd for $C_{29}H_{27}ClN_3O_3S^+$ $[M+H]^+$, 532.15; found, 532.1.

**Example 5: Preparation of 3-(5-((1-((4'-chloro-[1,1'-biphenyl]-2-yl)methyl)pyrrolidin-3-yl)thio)-1-oxoisoindolin-2-yl)piperidine-2,6-dione (GT-04195; compound 29)**

The synthesis was performed according to synthetic method II using compound (GT-04309) as the starting material.

The target compound (GT-04195) was prepared as a white solid (27 mg, yield: 45%). $^1$H NMR (400 MHz, MeOD) δ 7.79 (d, $J$ = 8.0 Hz, 1H), 7.72 - 7.69 (m, 1H), 7.62 - 7.55 (m, 2H), 7.53 - 7.48 (m, 3H), 7.44 - 7.41 (m, 2H), 7.34 (d, $J$ = 8.4 Hz, 2H), 5.18 (dd, $J$ = 13.2, 5.2 Hz, 1H), 4.57 - 4.47 (m, 4H), 4.20 - 4.04 (m, 1H), 3.64 - 3.44 (m, 1H), 3.10 - 2.86 (m, 2H), 2.84 - 2.82 (m, 1H), 2.54 - 2.49 (m, 2H), 2.22 - 2.20 (m, 1H), 2.02 - 1.91 (m, 1H), 1.46 - 1.37 (m, 3H). LC/MS (ESI) m/z: calcd for $C_{30}H_{29}ClN_3O_3S^+$ $[M+H]^+$, 546.16; found, 546.2.

**Example 6: Preparation of 3-(5-((1-((4'-chloro-[1,1'-biphenyl]-2-yl)methyl)piperidin-4-yl)thio)-1-oxoisoindolin-2-yl)piperidine-2,6-dione (GT-03357; compound 30)**

The synthesis was performed according to synthetic method II using compound (GT-03616) as the starting material.

The target compound (GT-03357) was prepared as a white solid (18 mg, yield: 51%). $^1$H NMR (400 MHz, MeOD) δ 7.78 - 7.73 (m, 2H), 7.65 - 7.48 (m, 6H), 7.47 - 7.33 (m, 3H), 5.16 (dd, $J$ = 13.2, 5.2 Hz, 1H), 4.54 - 4.41 (m, 2H), 4.39 (s, 1H), 3.51 - 3.42 (m, 1H), 3.39 - 3.35(m, 2H), 3.29 - 3.01 (m, 1H), 3.00 - 2.86 (m, 1H), 2.83 - 2.77 (m, 2H), 2.59 - 2.40 (m, 1H), 2.23 - 2.11 (m, 3H), 1.82 - 1.73 (m, 2H), 1.41 - 1.38 (m, 1H).LC/MS (ESI) m/z: calcd for $C_{31}H_{31}ClN_3O_3S^+$ $[M+H]^+$, 560.18; found, 560.2.

**Example 7: Preparation of 3-(5-((1-(4'-chloro-[1,1'-biphenyl]-2-carbonyl)azetidin-3-yl)thio)-1-oxoisoindolin-2-yl)piperidine-2,6-dione (GT-03892; compound 37)**

The synthesis was performed according to synthetic method III using compound (GT-03613) as the starting material.

The target compound (GT-03892) was prepared as a white solid (15 mg, yield: 20%). $^1$H NMR (400 MHz, MeOD) δ 7.73-7.71 (m, 1H), 7.59-7.54 (m, 1H), 7.52-7.43 (m, 7H), 7.33 (s, 1H), 7.29-7.24 (m, 1H), 5.17-5.10 (m, 1H), 4.53-4.43 (m, 3H), 4.22-4.13 (m, 2H), 3.83-3.77 (m, 1H), 3.04-2.86 (m, 2H), 2.84-2.79 (m, 1H), 2.55-2.44 (m, 1H), 2.23-2.13 (m, 1H). LC/MS (ESI) m/z: calcd for $C_{29}H_{25}ClN_3O_4S^+$ $[M+H]^+$, 546.12; found, 546.1.

**Example 8: Preparation of 3-(5-((1-((4'-fluoro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione (GT-03457; compound 48)**

The synthesis was performed according to synthetic method I using compound GT-03613 as the starting material.

The target compound (GT-03457) was prepared as a white solid (10 mg, yield: 20%). $^1$H NMR (400 MHz, MeOD) δ 7.77 (d, *J* = 8.0 Hz, 1H), 7.42 (s, 1H), 7.33 (s, 1H), 7.15 (d, *J* = 11.2 Hz, 4H), 5.14 (dd, *J* = 13.2, 5.2 Hz, 1H), 4.66 - 4.55 (m, 5H), 4.50 (d, *J* = 6.0 Hz, 3H), 4.41 - 4.31 (m, 1H), 3.92 - 3.82 (m, 2H), 2.99 - 2.89 (m, 1H), 2.85 - 2.75 (m, 1H), 2.56 - 2.48 (m, 1H), 2.32 - 2.07 (m, 5H), 1.56 (t, *J* = 6.4 Hz, 2H), 1.02 (s, 6H). LC/MS (ESI) m/z: calcd for $C_{31}H_{35}FN_3O_3S^+$ [M+H]$^+$, 548.24; found, 548.3.

**Example 9: Preparation of 3-(5-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione (GT-03369; compound 84)**

The synthesis was performed according to synthetic method I using compound GT-03614 as the starting material.

The target compound (GT-03369) was prepared as a white solid (20 mg, yield: 40%). $^1$H NMR (400 MHz, MeOD) *δ* 7.65 (d, *J* = 8.4 Hz, 1H), 7.39-7.31 (m, 1H), 7.26-7.16 (m, 1H), 7.08-6.97 (m, 2H), 6.92-6.85 (m, 1H), 6.77-6.63 (m, 1H), 5.05-5.01 (m,1H), 4.98-4.95 (m, 1H), 4.67-4.57 (m, 1H), 4.42-4.32 (m, 2H), 4.22 (brs, 1H), 3.91-3.66 (m, 3H), 2.84-2.76 (m, 1H), 2.70-2.66 (m, 1H), 2.41-2.36 (m, 1H), 2.14-2.09 (m, 2H), 2.03 (brs, 3H), 1.46 (t, *J* = 6.4 Hz, 2H), 0.92 (s, 6H). LC/MS (ESI) m/z: calcd for $C_{31}H_{35}ClN_3O_4^+$ [M+H]$^+$, 548.23; found, 548.2.

**Example 10: Preparation of 3-(5-((1-((4'-chloro-[1,1'-biphenyl]-2-yl)methyl)azetidin-3-yl)oxy)-1-oxoisoindolin-2-yl)piperidine-2,6-dione (GT-03363; compound 111)**

The synthesis was performed according to synthetic method II using compound (GT-03614) as the starting material.

The target compound (GT-03363) was prepared as a white solid (20 mg, yield: 45%). $^1$H NMR (400 MHz, MeOD) *δ* 7.63 (d, *J*= 8.4 Hz, 1H), 7.53-7.47 (m, 1H), 7.46-7.42 (m, 2H), 7.36 (d, *J* = 8.0 Hz, 2H), 7.30-7.27 (m, 1H), 7.25-7.23 (m, 2H), 6.81 (brs, 1H), 6.77 (d, *J* = 8.2 Hz, 1H), 5.05-5.00 (m, 1H), 4.98-4.94 (m, 1H), 4.50 (brs, 2H), 4.45 (brs, 2H), 4.35 (d, *J* = 5.8 Hz, 2H), 3.91 (brs, 2H), 2.85-2.77 (m, 1H), 2.71-2.65 (m, 1H), 2.43-2.32 (m, 1H), 2.09-2.03 (m, 1H). LC/MS (ESI) m/z: calcd for $C_{29}H_{27}ClN_3O_4^+$ [M+H]$^+$, 516.17; found, 516.2.

**Example 11: Preparation of 3-(5-((1-((4'-fluoro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione (GT-03459; compound 130)**

The synthesis was performed according to synthetic method I using compound (GT-03614) as the starting material.

The target compound (GT-03459) was prepared as a white solid (10 mg, yield: 20%). $^1$H NMR (400 MHz, MeOD) δ 7.76 (d, *J* = 8.4 Hz, 1H), 7.19 - 7.15 (m, 2H), 7.12 - 6.99 (m, 2H), 6.98 - 6.75 (m, 2H), 5.14 (dd, *J*= 13.2, 5.2 Hz, 1H), 5.10 - 5.06 (m, 1H), 4.70 - 4.58 (m, 2H), 4.53 - 4.40 (m, 2H), 4.18 - 3.75 (m, 2H), 3.94 (s, 2H), 2.99 - 2.87 (m, 1H), 2.86 - 2.75 (m, 1H), 2.59 - 2.41 (m, 1H), 2.32 - 2.17 (m, 3H), 2.16 (s, 2H), 1.57 (t, *J* = 6.4 Hz, 2H), 1.03 (s, 6H). LC/MS (ESI) m/z: calcd for $C_{31}H_{35}FN_3O_4^+$ [M+H]$^+$, 532.26; found, 532.3.

**Example 12: Preparation of 3-(5-((1-((4-(4-chlorophenyl)-5,6-dihydro-2H-pyran-3-yl)methyl)azetidin-3-yl)oxy)-1-oxoisoindolin-2-yl)piperidine-2,6-dione (GT-06354; compound 134)**

The synthesis was performed according to synthetic method I using compound (GT-03614) as the starting material.

The target compound (GT-06354) was prepared as a white solid (12 mg, yield: 19%). $^1$H NMR (400 MHz, DMSO-d6) δ 10.98 (s, 1H), 7.69 - 7.63 (m, 1H), 7.59 - 7.35 (m, 3H), 7.31 - 7.27 (m, 2H), 6.99 - 6.92 (m, 1H), 5.09 (dd, J = 13.2, 5.0 Hz, 1H), 4.99 - 4.93 (m, 1H), 4.71 - 4.58 (m, 1H), 4.40 (d, J = 17.4 Hz, 1H), 4.32 - 4.18 (m, 4H), 4.07 - 3.97 (m, 1H), 3.96 - 3.69 (m, 4H), 3.66 - 3.51 (m, 1H), 2.99 - 2.84 (m, 1H), 2.60 (d, J = 17.1 Hz, 1H), 2.55 - 2.52 (m, 1H), 2.45 - 2.32 (m, 2H), 2.05 - 1.93 (m, 1H). LCMS (ESI) calcd for $C_{28}H_{29}ClN_3O_5^+$ [M+H]$^+$: 522.19, found, 522.2.

**Example 13: Preparation of 3-(4-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione (GT-06515)**

The target compound (GT-07004) was prepared as a white solid (1.5 g, yield: 86%) according to the method described in synthetic method I (GT-06515) (white solid, 16 mg, yield: 24%). $^1$H NMR (400 MHz, DMSO-d6) δ 11.04 (s, 1H), 10.86 (s, 1H), 7.66 (d, J = 7.1 Hz, 1H), 7.61 - 7.49 (m, 1H), 7.46 - 7.40 (m, 3H), 7.21 - 7.13 (m, 2H), 5.15 (dd, J = 15.9, 10.6 Hz, 1H), 4.69 - 4.53 (m, 1H), 4.42 - 4.06 (m, 4H), 3.75 (s, 1H), 3.69 - 3.61 (m, 1H), 2.95 - 2.89(m, 1H), 2.68 - 2.64 (m, 1H), 2.55 - 2.53(m, 2H), 2.46 - 2.42 (m, 1H), 2.27 - 2.11 (m, 2H), 2.10 - 1.95 (m, 3H), 1.53 - 1.36 (m, 2H), 0.96 (d, J = 7.3 Hz, 6H). LCMS (ESI) calcd for $C_{31}H_{35}ClN_3O_3S^+$ $[M+H]^+$: 564.21, found, 564.3.

**Example 14: Preparation of 3-(6-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione (GT-06757)**

The target compound (GT-06757) was prepared as a white solid (14 mg, yield: 17%) according to the method described in synthetic method I. $^1$H NMR (400 MHz, DMSO-d6) δ 10.93 (s, 1H), 7.54 (d, J = 7.8 Hz, 1H), 7.48 (s, 1H), 7.39 (d, J = 7.9 Hz, 3H), 7.13 - 7.09 (m, 2H), 5.05 (dd, J = 13.3, 5.1 Hz, 1H), 4.56 - 4.47 (m, 1H), 4.39 (d, J = 17.6 Hz, 1H), 4.28 - 4.17 (m, 3H), 4.07 - 4.00 (m 1H), 3.80 - 3.54 (m, 3H), 2.94 - 2.78 (m, 1H), 2.56 - 2.51 (m, 1H), 2.40 - 2.26 (m, 1H), 2.12- 2.04 (m, 2H), 1.95 (s, 3H), 1.35 (t, J = 6.1 Hz, 2H), 0.87 (s, 6H). LCMS (ESI) calcd for $C_{31}H_{35}ClN_3O_3S^+$ $[M+H]^+$: 564.21, found, 564.3.

**Example 15: Preparation of 3-(7-((1-((4'-chloro-5,5-dimethyl-3,4,5,6-tetrahydro-[1,1'-biphenyl]-2-yl)methyl)azetidin-3-yl)thio)-1-oxoisoindolin-2-yl)piperidine-2,6-dione (GT-06812)**

The target compound (GT-06812) was prepared as a white solid (13 mg, yield: 20%) according to the method described in synthetic method I. $^1$H NMR (400 MHz, DMSO-d6) δ 11.00 (s, 1H), 7.55 (t, J = 7.6 Hz, 1H), 7.49 - 7.44 (m, 1H), 7.42 - 7.39 (m, 1H), 7.35 (d, J = 8.0 Hz, 1H), 7.21 (d, J = 8.1 Hz, 1H), 7.14 (d, J = 7.8 Hz, 1H), 7.01 (d, J = 8.1 Hz, 1H), 5.08 - 5.03 (m, 1H), 4.76 - 4.68 (m, 1H), 4.53 - 4.21 (m, 4H), 4.14 - 4.08 (m, 1H), 3.86 - 3.74 (m, 2H), 3.00 - 2.82 (m, 1H), 2.61 (d, J = 17.6 Hz, 1H), 2.56 - 2.51(m, 1H), 2.45 - 2.32 (m, 1H), 2.18 - 2.12 (m, 2H), 2.05 - 1.98 (m, 3H), 1.44 - 1.42(m, 2H), 0.95 (s, 6H). LCMS (ESI) calcd for $C_{31}H_{35}ClN_3O_3S^+$ $[M+H]^+$: 564.21, found, 564.3.

**Example 16: Preparation of 3-(5-((1-((4'-chloro-5,5-dimethyl-3,4,5,6-tetrahydro-[1,1'-biphenyl]-2-yl)methyl)azepan-4-yl)thio)-1-oxoisoindolin-2-yl)piperidine-2,6-dione (GT-06641)**

The target compound (GT-06641) was prepared as a white solid (41 mg, yield: 52%) according to the method described in synthetic method I. $^1$H NMR (400 MHz, DMSO-d6) δ 11.00 (s, 1H), 7.69 - 7.66 (m, 1H), 7.57 (d, J = 6.7 Hz, 1H), 7.47 - 7.41 (m, 3H), 7.14 (dd, J = 8.4, 3.0 Hz, 2H), 5.11 (d, J = 13.3 Hz, 1H), 4.52 - 4.39 (m, 1H), 4.39 - 4.25 (m, 1H), 3.78 - 3.65 (m, 1H), 3.58 - 3.51 (m, 2H), 3.27 - 3.18 (m, 1H), 3.10 - 2.81 (m, 3H), 2.61 (d, J = 16.7 Hz, 1H), 2.44 - 2.15 (m, 3H), 2.14 - 1.94 (m, 6H), 1.92 - 1.69 (m, 2H), 1.68 - 1.37 (m, 4H), 0.96 (s, 3H), 0.95(s, 3H). LCMS (ESI) calcd for $C_{34}H_{41}ClN_3O_3S^+$ $[M+H]^+$: 606.26, found, 606.3.

**Example 17: Preparation of 3-(5-((2-((4'-chloro-5,5-dimethyl-3,4,5,6-tetrahydro-[1,1'-biphenyl]-2-yl)methyl)octahydrocyclopenta[c]pyrrol-5-yl)thio)-1-oxoisoindolin-2-yl)piperidine-2,6-dione (GT-06813)**

The target compound (GT-06813) was prepared as a white solid (20 mg, yield: 32%) according to the method described in synthetic method I. $^1$H NMR (400 MHz, DMSO-d6) δ 10.99 (s, 1H), 7.68 - 7.62 (m, 1H), 7.59 - 7.50 (m, 1H), 7.47 - 7.42 (m, 2H), 7.41 - 7.36 (m, 1H), 7.19 - 7.13 (m, 2H), 5.10 (dd, J = 13.2, 5.1 Hz, 1H), 4.44 (d, J = 17.6 Hz, 1H), 4.31 (d, J = 17.6 Hz, 1H), 3.99 - 3.90 (m, 1H), 3.74 - 3.51 (m, 4H), 3.31 - 3.20 (m, 2H), 3.19 - 3.02 (m, 1H), 3.01 - 2.84 (m, 3H), 2.81 - 2.67 (m, 1H), 2.61 (d, J = 16.3 Hz, 1H), 2.39 (d, J = 12.9 Hz, 1H), 2.35 - 2.22 (m, 4H), 2.09 - 1.90 (m, 4H), 1.71 - 1.57 (m, 1H), 0.97 (s, 6H). LCMS (ESI) calcd for $C_{35}H_{41}ClN_3O_3S^+$ $[M+H]^+$: 618.26, found, 618.3.

**Example 18: Preparation of 3-(5-((7-((4'-chloro-5,5-dimethyl-3,4,5,6-tetrahydro-[1,1'-biphenyl]-2-yl)methyl)-7-azaspiro[3.5]nonan-2-yl)thio)-1-oxoisoindolin-2-yl)piperidine-2,6-dione (GT-06873)**

The target compound (GT-06873) was prepared as a white solid (14 mg, yield: 22%) according to the method described in synthetic method I. $^1$H NMR (400 MHz, DMSO-d6) δ 10.91 (s, 1H), 9.26 (s, 1H), 7.55 (d, J = 8.0 Hz, 1H), 7.37 (d, J = 8.3 Hz, 2H), 7.32 (s, 1H), 7.21 (d, J = 8.3 Hz, 1H), 7.06 (d, J = 8.3 Hz, 2H), 5.02 (dd, J = 13.3, 5.1 Hz, 1H), 4.36 - 4.17 (m, 2H), 4.04 - 3.90 (m, 1H), 3.48 - 3.41 (m, 2H), 3.12 - 3.02 (m, 2H), 2.92 - 2.76 (m, 1H), 2.58 - 2.51 (m, 2H), 2.39 - 2.27 (m, 3H), 2.25 - 2.14 (m, 2H), 1.96 (brs, 2H), 1.95 - 1.88 (m, 2H), 1.88 - 1.72 (m, 4H), 1.71 - 1.59 (m, 2H), 1.41 - 1.38 (m, 2H), 0.89

(s, 6H). LCMS (ESI) calcd for $C_{36}H_{43}ClN_3O_3S^+$ $[M+H]^+$: 632.27, found, 632.3.

**Example 19: Preparation of 3-(5-((3-((4'-chloro-5,5-dimethyl-3,4,5,6-tetrahydro-[1,1'-biphenyl]-2-yl)methyl)-3-azaspiro[5.5]undecan-9-yl)thio)-1-oxoisoindolin-2-yl)piperidine-2,6-dione (GT-06874)**

The target compound (GT-06874) was prepared as a white solid (19 mg, yield: 30%) according to the method described in synthetic method I. $^1H$ NMR (400 MHz, DMSO-d6) δ 10.92 (s, 1H), 9.18 (s, 1H), 7.57 (d, J = 8.0 Hz, 1H), 7.50 (s, 1H), 7.37 (d, J = 8.1 Hz, 3H), 7.06 (d, J = 8.3 Hz, 2H), 5.03 (dd, J = 13.3, 5.1 Hz, 1H), 4.35 (d, J = 17.5 Hz, 1H), 4.23 (d, J = 17.4 Hz, 1H), 3.59 - 3.43 (m, 2H), 3.02 - 2.99 (m, 2H), 2.92 - 2.77 (m, 1H), 2.66 - 2.55(m, 2H), 2.53 (d, J = 16.8 Hz, 1H), 2.31 - 2.27 (m, 1H), 2.20 (brs, 2H), 2.06 - 1.86 (m, 3H), 1.85 - 1.59 (m, 5H), 1.52 - 1.40(m, 1H), 1.46 - 1.19 (m, 8H), 1.09 - 1.04 (m, 1H), 0.89 (s, 6H). LCMS (ESI) calcd for $C_{38}H_{47}ClN_3O_3S^+$ $[M+H]^+$: 660.30, found, 660.3.

**Example 20: Preparation of 3-(5-((1-((4'-chloro-5,5-dimethyl-3,4,5,6-tetrahydro-[1,1'-biphenyl]-2-yl)methyl)-3,3-difluoropiperidin-4-yl)thio)-1-oxoisoindolin-2-yl)piperidine-2,6-dione (GT-06960)**

The target compound (GT-06960) was prepared as a white solid (9 mg, yield: 12%) according to the method described in synthetic method I. $^1H$ NMR (400 MHz, DMSO-d6) δ 10.92 (s, 1H), 7.60 - 7.59 (m, 2H), 7.47 (d, J = 8.1 Hz, 1H), 7.34 (d, J = 8.1 Hz, 2H), 7.04 (d, J = 8.1 Hz, 2H), 5.03 (dd, J = 13.3, 5.0 Hz, 1H), 4.36 (d, J = 17.4 Hz, 1H), 4.23 (d, J = 17.5 Hz, 1H), 3.95 - 3.83 (m, 1H), 3.27 - 3.19 (m, 4H), 2.91 - 2.77 (m, 2H), 2.53 (d, J = 17.2 Hz, 1H), 2.37 - 2.29 (m, 2H), 2.12 (brs, 2H), 2.06 - 1.86 (m, 4H), 1.82 - 1.54 (m, 1H), 1.37 (t, J = 6.1 Hz, 2H), 0.89 (s, 3H), 0.88(s, 3H). LCMS (ESI) calcd for $C_{33}H_{37}ClF_2N_3O_3S^+$ $[M+H]^+$: 628.22, found, 628.3.

**Example 21: Preparation of 3-(5-((1-(4'-chloro-5,5-dimethyl-3,4,5,6-tetrahydro-[1,1'-biphenyl]-2-carbonyl)azetidin-3-yl)thio)-1-oxoisoindolin-2-yl)piperidine-2,6-dione (GT-06137)**

The target compound (GT-06137) was prepared as a white solid (39 mg, yield: 47%) according to the method described in synthetic method III. $^1H$ NMR (400 MHz, DMSO-d6) δ 10.99 (s, 1H), 7.63 (d, J = 8.0 Hz, 1H), 7.40 (dd, J = 8.4, 1.3 Hz, 2H), 7.33 (s, 1H), 7.25 - 7.21 (m, 3H), 5.10 (dd, J = 13.1, 4.7 Hz, 1H), 4.42 (d, J = 17.5 Hz, 1H), 4.36 - 4.27 (m, 1H), 4.27 - 4.04 (m, 3H), 3.44 (dd, J = 10.2, 3.6 Hz, 1H), 3.23 (dd, J = 8.5, 3.8 Hz, 1H), 3.00 - 2.81 (m, 1H), 2.60 (d, J = 17.0 Hz, 1H), 2.39 (dd, J = 13.2, 4.4 Hz, 1H), 2.25 - 2.16 (m, 3H), 2.12 - 1.93 (m, 2H), 1.40 (t, J = 6.3 Hz, 2H), 0.96 (s, 3H), 0.95(s, 3H). LCMS (ESI) calcd for $C_{31}H_{33}ClN_3O_4S^+$ $[M+H]^+$: 578.19, found, 578.2.

**Example 22: Preparation of 3-(5-((1-(4'-chloro-5,5-dimethyl-3,4,5,6-tetrahydro-[1,1'-biphenyl]-2-carbonyl)pyrrolidin-3-yl)thio)-1-oxoisoindolin-2-yl)piperidine-2,6-dione (GT-06139)**

The target compound (GT-06139) was prepared as a white solid (28 mg, yield: 34%) according to the method described in synthetic method III. $^1H$ NMR (400 MHz, DMSO-d6) δ 11.00 (s, 1H), 7.67 - 7.64 (m, 1H), 7.54 - 7.43 (m, 1H), 7.41 - 7.31 (m, 3H), 7.26 (d, J = 8.5 Hz, 1H), 7.21 (d, J = 7.9 Hz, 1H), 5.19 - 5.04 (m, 1H), 4.51 - 4.26 (m, 2H), 4.00 - 3.89 (m, 1H), 3.18 - 3.08 (m, 3H), 2.98 - 2.88 (m, 2H), 2.61 (d, J = 16.8 Hz, 1H), 2.44 - 2.37 (m, 1H), 2.34 - 2.21 (m, 2H), 2.16 - 1.98 (m, 4H), 1.72 - 1.59 (m, 1H), 1.47 - 1.42 (m, 1H), 1.40 - 1.27 (m, 1H), 0.99 (d, J = 4.0 Hz, 6H). LCMS (ESI) calcd for $C_{32}H_{35}ClN_3O_4S^+$ $[M+H]^+$: 592.20, found, 592.2.

**Example 23: Preparation of 3-(5-((1-(4'-chloro-5,5-dimethyl-3,4,5,6-tetrahydro-[1,1'-biphenyl]-2-carbonyl)piperidin-4-yl)thio)-1-oxoisoindolin-2-yl)piperidine-2,6-dione (GT-06140)**

The target compound (GT-06140) was prepared as a white solid (38 mg, yield: 47%) according to the method described in synthetic method III. $^1H$ NMR (400 MHz, DMSO-d6) δ 10.99 (s, 1H), 7.63 (d, J = 8.0 Hz, 1H), 7.52 (s, 1H), 7.48 - 7.35 (m, 3H), 7.30 (d, J = 8.1 Hz, 1H), 7.22 (d, J = 7.8 Hz, 1H), 5.10 (dd, J = 13.1, 4.9 Hz, 1H), 4.42 (d, J = 17.5 Hz, 1H), 4.29 (d, J = 17.3 Hz, 1H), 4.19 - 4.04 (m, 1H), 3.07 - 2.85 (m, 2H), 2.70 - 2.59 (m, 4H), 2.47 - 2.24 (m, 4H), 2.09 - 1.81 (m, 5H), 1.64 - 1.51 (m, 1H), 1.48 - 1.42 (m, 2H), 1.00 (s, 3H), 0.97(s, 3H). LCMS (ESI) calcd for $C_{33}H_{37}ClN_3O_4S^+$ $[M+H]^+$: 606.22, found, 606.2.

**Example 24: Preparation of 3-(5-((1-(4'-chloro-5,5-dimethyl-3,4,5,6-tetrahydro-[1,1'-biphenyl]-2-carbonyl)azepan-4-yl)thio)-1-oxoisoindolin-2-yl)piperidine-2,6-dione (GT-06642)**

The target compound (GT-06642) was prepared as a white solid (28 mg, yield: 35%) according to the method described in synthetic method III. $^1H$ NMR (400 MHz, DMSO-d6) δ 10.74 (s, 1H), 7.46 - 7.35 (m, 1H), 7.26 - 7.21 (m, 1H), 7.17 - 7.04 (m, 5H), 4.85 (d, J = 12.7 Hz, 1H), 4.18 (d, J = 16.3 Hz, 1H), 4.05 (d, J = 17.7 Hz, 1H), 3.09 - 2.98 (m, 4H), 2.78 -

2.56 (m, 2H), 2.35 (d, J = 17.7 Hz, 1H), 2.23 - 2.02 (m, 4H), 2.03 - 1.81 (m, 2H), 1.80 - 1.71 (m, 1H), 1.68 - 1.60 (m, 2H), 1.32 - 1.12 (m, 5H), 0.73 (s, 3H), 0.71(s, 3H). LCMS (ESI) calcd for $C_{34}H_{39}ClN_3O_4S^+$ $[M+H]^+$: 606.23, found, 606.2.

**Example 25: Preparation of 3-(5-(((3aS,6aS)-2-(4'-chloro-5,5-dimethyl-3,4,5,6-tetrahydro-[1,1'-biphenyl]-2-carbonyl)octahydrocyclopenta[c]pyrrol-5-yl)thio)-1-oxoisoindolin-2-yl)piperidine-2,6-dione (GT-07022)**

The target compound (GT-07022) was prepared as a white solid (20 mg, yield: 31%) according to the method described in synthetic method III. $^1$H NMR (400 MHz, DMSO-d6) δ 10.91 (s, 1H), 7.55 (d, J = 8.0 Hz, 1H), 7.42 (s, 1H), 7.35 - 7.26 (m, 3H), 7.22 (d, J = 8.2 Hz, 2H), 5.02 (dd, J = 13.3, 4.9 Hz, 1H), 4.34 (d, J = 17.4 Hz, 1H), 4.22 (d, J = 17.2 Hz, 1H), 3.62 - 3.44 (m, 1H), 3.17 - 3.04 (m, 3H), 2.84 - 2.79 (m, 3H), 2.55 - 2.43 (m, 2H), 2.38 - 2.26 (m, 3H), 2.23 - 2.10 (m, 5H), 2.00 - 1.81 (m, 2H), 1.39 - 1.30 (m, 2H), 0.91 (s, 3H), 0.90(s, 3H). LCMS (ESI) calcd for $C_{35}H_{39}ClN_3O_4S^+$ $[M+H]^+$: 632.23, found, 632.2.

**Example 26: Preparation of 3-(5-(((3aR,6aS)-2-(4'-chloro-5,5-dimethyl-3,4,5,6-tetrahydro-[1,1'-biphenyl]-2-carbonyl)octahydrocyclopenta[c]pyrrol-5-yl)thio)-1-oxoisoindolin-2-yl)piperidine-2,6-dione (GT-07015)**

The target compound (GT-07015) was prepared as a white solid (14 mg, yield: 22%) according to the method described in synthetic method III. $^1$H NMR (400 MHz, DMSO-d6) δ 10.92 (s, 1H), 7.58 (d, J = 7.9 Hz, 1H), 7.38 (s, 1H), 7.31 (d, J = 8.4 Hz, 2H), 7.27 - 7.21 (m, 3H), 5.04 (dd, J = 13.2, 4.8 Hz, 1H), 4.37 (d, J = 17.0 Hz, 1H), 4.25 (d, J = 17.6 Hz, 1H), 3.56 - 3.41 (m, 1H), 3.04 - 2.96 (m, 1H), 2.88 - 2.81 (m, 1H), 2.77 - 2.63 (m, 1H), 2.59 - 2.49 (m, 4H), 2.35 - 2.30 (m, 1H), 2.29 - 2.03 (m, 4H), 1.97 - 1.89 (m, 2H), 1.70 - 1.44 (m, 3H), 1.36 (t, J = 5.9 Hz, 3H), 0.92 (s, 3H), 0.91(s, 3H). LCMS (ESI) calcd for $C_{35}H_{39}ClN_3O_4S^+$ $[M+H]^+$: 632.23, found, 632.2.

**Example 27: Preparation of 3-(5-((7-(4'-chloro-5,5-dimethyl-3,4,5,6-tetrahydro-[1,1'-biphenyl]-2-carbonyl)-7-azaspiro[3.5]nonan-2-yl)thio)-1-oxoisoindolin-2-yl)piperidine-2,6-dione (GT-06918)**

The target compound (GT-06918) was prepared as a white solid (27 mg, yield: 51%) according to the method described in synthetic method III. $^1$H NMR (400 MHz, DMSO-d6) δ 10.97 (s, 1H), 7.61 (d, J = 8.0 Hz, 1H), 7.40 - 7.34 (m, 3H), 7.28 - 7.24 (m, 3H), 5.09 (dd, J = 13.2, 4.9 Hz, 1H), 4.41 (d, J = 17.2 Hz, 1H), 4.28 (d, J = 17.4 Hz, 1H), 4.09 - 3.90 (m, 1H), 3.47 - 4.34 (m, 1H), 3.22 - 2.85 (m, 4H), 2.60 (d, J = 16.6 Hz, 1H), 2.47 - 2.32 (m, 4H), 2.30 - 2.23 (m, 1H), 2.20 - 1.84 (m, 4H), 1.74 - 1.65 (m, 1H), 1.61 - 1.50 (m, 1H), 1.50 - 1.34 (m, 3H), 1.34 - 1.06 (m, 2H), 0.97 (s, 6H). LCMS (ESI) calcd for $C_{36}H_{41}ClN_3O_4S^+$ $[M+H]^+$: 646.25, found, 646.3.

**Example 28: Preparation of 3-(5-((3-(4'-chloro-5,5-dimethyl-3,4,5,6-tetrahydro-[1,1'-biphenyl]-2-carbonyl)-3-azaspiro[5.5]undecan-9-yl)thio)-1-oxoisoindolin-2-yl)piperidine-2,6-dione (GT-07016)**

The target compound (GT-07016) was prepared as a white solid (17 mg, yield: 32%) according to the method described in synthetic method III. $^1$H NMR (400 MHz, DMSO-d6) δ 10.91 (s, 1H), 7.55 (d, J = 8.0 Hz, 1H), 7.48 (s, 1H), 7.38 - 7.32 (m, 1H), 7.30 (d, J = 8.6 Hz, 2H), 7.20 (d, J = 8.6 Hz, 2H), 5.02 (dd, J = 13.3, 5.1 Hz, 1H), 4.35 (d, J = 17.4 Hz, 1H), 4.22 (d, J = 17.4 Hz, 1H), 3.54 - 3.49 (m, 1H), 3.35 - 3.29 (m, 2H), 3.07 - 2.76 (m, 4H), 2.53 (d, J = 16.0 Hz, 1H), 2.40 - 2.21 (m, 3H), 2.02 - 1.82 (m, 3H), 1.74 - 1.61 (m, 3H), 1.43 - 1.32 (m, 4H), 1.17 - 0.98 (m, 4H), 0.92 (s, 3H), 0.90 (s, 3H), 0.88 - 0.84 (m, 1H), 0.78 - 0.72 (m, 1H). LCMS (ESI) calcd for $C_{38}H_{45}ClN_3O_4S^+$ $[M+H]^+$: 674.28, found, 674.3.

**Example 29: Preparation of 3-(5-((1-(4'-chloro-5,5-dimethyl-3,4,5,6-tetrahydro-[1,1'-biphenyl]-2-carbonyl)-3,3-difluoropiperidin-4-yl)thio)-1-oxoisoindolin-2-yl)piperidine-2,6-dione (GT-07017)**

The target compound (GT-07017) was prepared as a white solid (26 mg, yield: 41%) according to the method described in synthetic method III. $^1$H NMR (400 MHz, DMSO-d6) δ 10.93 (s, 1H), 7.59 - 7.55 (m, 2H), 7.45 - 7.41 (m, 1H), 7.33 - 7.27 (m, 2H), 7.23 - 7.09 (m, 2H), 5.03 (dd, J = 13.3, 5.0 Hz, 1H), 4.35 (d, J = 17.4 Hz, 1H), 4.22 (d, J = 17.4 Hz, 1H), 4.16 - 3.94 (m, 1H), 3.94 - 3.69 (m, 1H), 3.61 - 3.40 (m, 1H), 3.08 - 2.97 (m, 1H), 2.92 - 2.77 (m, 1H), 2.52 (d, J = 16.6 Hz, 1H), 2.42 - 2.21 (m, 4H), 2.12 - 1.75 (m, 4H), 1.53 - 1.26 (m, 3H), 0.93 (s, 6H), 0.90(s, 3H). LCMS (ESI) calcd for $C_{33}H_{35}ClF_2N_3O_4S^+$ $[M+H]^+$: 642.20, found, 642.3.

**Example 30: Preparation of 3-(5-((1-((4'-chloro-[1,1'-biphenyl]-2-yl)methyl)azepan-4-yl)thio)-1-oxoisoindolin-2-yl)piperidine-2,6-dione (GT-06643)**

The target compound (GT-06643) was prepared as a white solid (45 mg, yield: 60%) according to the method described in synthetic method I. $^1$H NMR (400 MHz, DMSO-d6) δ 11.00 (s, 1H), 8.05 - 8.03 (m, 1H), 7.66 (d, J = 7.9 Hz, 1H),

7.57 - 7.52 (m, 5H), 7.47 - 7.35 (m, 3H), 7.35 - 7.27 (m, 1H), 5.11 (dd, J = 13.2, 4.9 Hz, 1H), 4.46 - 4.26 (m, 4H), 3.83 - 3.58 (m, 1H), 3.24 - 3.07 (m, 1H), 2.96 - 2.87 (m, 3H), 2.61 (d, J = 16.9 Hz, 1H), 2.45 - 2.38 (m, 1H), 2.11 - 1.99 (m, 4H), 1.86 - 1.74 (m, 2H), 1.70 - 1.39 (m, 2H). LCMS (ESI) calcd for $C_{32}H_{33}ClN_3O_3S^+$ [M+H]$^+$: 574.19, found, 574.2.

**Example 31: Preparation of 3-(5-((2-((4'-chloro-[1,1'-biphenyl]-2-yl)methyl)octahydrocyclopenta[c]pyrrol-5-yl) thio)-1-oxoisoindolin-2-yl)piperidine-2,6-dione (GT-06914)**

The target compound (GT-06914) was prepared as a white solid (30 mg, yield: 51%) according to the method described in synthetic method I. $^1$H NMR (400 MHz, DMSO-d6) δ 10.92 (s, 1H), 8.00 - 7.84 (m, 1H), 7.60 - 7.51(m, 1H), 7.52 - 7.39 (m, 5H), 7.39 - 7.29 (m, 3H), 7.28 - 7.20 (m, 1H), 5.05 - 5.01 (m, 1H), 4.46 - 4.15 (m, 4H), 3.51 - 3.48 (m, 1H), 3.44 - 3.40 (m, 1H), 3.10 - 2.85 (m, 1H), 2.96 - 2.74 (m, 3H), 2.73 - 2.58 (m, 2H), 2.53 (d, J = 17.4 Hz, 1H), 2.39 - 2.26 (m, 1H), 2.25 - 2.13 (m, 1H), 2.03 - 1.82 (m, 2H), 1.78 - 1.45 (m, 1H), 1.41 - 1.35 (m, 1H). LCMS (ESI) calcd for $C_{33}H_{33}ClN_3O_3S^+$ [M+H]$^+$: 586.19, found, 586.3.

**Example 32: Preparation of 3-(5-((1-((4'-chloro-[1,1'-biphenyl]-2-yl)methyl)-3,3-difluoropiperidin-4-yl)thio)-1-oxoisoindolin-2-yl)piperidine-2,6-dione (GT-06924)**

The target compound (GT-06924) was prepared as a white solid (5 mg, yield: 7%) according to the method described in synthetic method I. $^1$H NMR (400 MHz, DMSO-d6) δ 10.90 (s, 1H), 7.58 (d, J = 7.9 Hz, 2H), 7.50 - 7.40 (m, 6H), 7.35 - 7.28 (m, 3H), 5.02 (dd, J = 13.1, 4.8 Hz, 1H), 4.70 - 4.48 (m, 1H), 4.35 (d, J = 16.9 Hz, 1H), 4.22 (d, J = 17.3 Hz, 1H), 4.13 - 4.02 (m, 1H), 3.18 - 3.03 (m, 4H), 2.88 - 2.79 (m, 1H), 2.73 - 2.57 (m, 1H), 2.52 (d, J = 17.3 Hz, 1H), 2.39 - 2.22 (m, 1H), 1.98 - 1.84 (m, 1H), 1.78 - 1.66 (m, 1H), 1.58 - 1.45 (m, 1H). LCMS (ESI) calcd for $C_{31}H_{29}ClFN_3O_3S^+$ [M+H]$^+$: 596.16, found, 596.3.

**Example 33: Preparation of 3-(5-((1-(4'-chloro-[1,1'-biphenyl]-2-carbonyl)pyrrolidin-3-yl)thio)-1-oxoisoindolin-2-yl)piperidine-2,6-dione (GT-06181)**

The target compound (GT-06181) was prepared as a white solid (32 mg, yield: 41%) according to the method described in synthetic method III. $^1$H NMR (400 MHz, DMSO-d6) δ 10.92 (s, 1H), 7.60 (d, J = 8.0 Hz, 1H), 7.47 - 7.42 (m, 3H), 7.42 - 7.36 (m, 4H), 7.35 - 7.29 (m, 3H), 5.06 - 5.01 (m, 1H), 4.41 - 4.20 (m, 2H), 4.01 (s, 1H), 3.66 - 3.64(m, 1H), 2.95 - 2.90 (m, 1H), 2.85 - 2.77 (m, 1H), 2.53 (d, J = 17.2 Hz, 1H), 2.36 - 2.27 (m, 1H), 2.29 - 2.04 (m, 1H), 1.95 - 1.90 (m, 1H), 1.74 - 1.66 (m, 1H), 1.64 - 1.48 (m, 1H). LCMS (ESI) calcd for $C_{30}H_{27}ClN_3O_4S^+$ [M+H]$^+$: 560.14, found, 560.2.

**Example 34: Preparation of 3-(5-((1-(4'-chloro-[1,1'-biphenyl]-2-carbonyl)piperidin-4-yl)thio)-1-oxoisoindolin-2-yl)piperidine-2,6-dione (GT-06182)**

The target compound (GT-06182) was prepared as a white solid (48 mg, yield: 62%) according to the method described in synthetic method III. $^1$H NMR (400 MHz, DMSO-d6) δ 10.91 (s, 1H), 7.56 (d, J = 8.0 Hz, 1H), 7.53 - 7.37 (m, 8H), 7.32 (d, J = 8.1 Hz, 2H), 5.02 (dd, J = 13.3, 5.1 Hz, 1H), 4.40 - 4.08 (m, 3H), 3.51 - 3.37 (m, 1H), 3.02 - 2.92 (m, 1H), 2.91 - 2.84 (m, 1H), 2.82 - 2.72 (m, 2H), 2.57 - 2.46 (m, 2H), 2.36 - 2.26 (m, 1H), 2.00 - 1.87 (m, 1H), 1.83 - 1.80 (m, 1H), 1.55 - 1.25 (m, 2H). LCMS (ESI) calcd for $C_{31}H_{29}ClN_3O_4S^+$ [M+H]$^+$: 574.16, found, 574.2.

**Example 35: Preparation of 3-(5-((1-(4'-chloro-[1,1'-biphenyl]-2-carbonyl)azepan-4-yl)thio)-1-oxoisoindolin-2-yl)piperidine-2,6-dione (GT-06644)**

The target compound (52 mg, yield: 68%) was prepared as a white solid (52 mg, yield: 68%) according to the method described in synthetic method III. $^1$H NMR (400 MHz, DMSO-d6) δ 10.99 (s, 1H), 7.71 - 7.60 (m, 1H), 7.52 - 7.41 (m, 9H), 7.34 (d, J = 7.5 Hz, 1H), 5.11 (dd, J = 13.2, 4.7 Hz, 1H), 4.47 - 4.28 (m, 2H), 3.70 - 3.46 (m, 2H), 3.21 - 3.06 (m, 1H), 2.91 - 2.79 (m, 2H), 2.79 - 2.74 (m, 1H), 2.60 (d, J = 16.6 Hz, 1H), 2.44 - 2.34 (m, 1H), 2.08 - 1.99 (m, 2H), 1.93 - 1.80 (m, 1H), 1.79 - 1.39 (m, 4H). LCMS (ESI) calcd for $C_{32}H_{31}ClN_3O_4S^+$ [M+H]$^+$: 588.17, found, 588.2.

**Example 36: Preparation of 3-(5-(((3aS,6aS)-2-(4 ' -chloro-[1,1 ' -biphenyl]-2-carbonyl)octahydrocyclopenta[c] pyrrol-5-yl)thio)-1-oxoisoindolin-2-yl)piperidine-2,6-dione (GT-**07023)

The target compound (GT-07023) was prepared as a white solid (13 mg, yield: 22%) according to the method described in synthetic method III. $^1$H NMR (400 MHz, DMSO-d6) δ 10.91 (s, 1H), 7.55 (d, J = 8.0 Hz, 1H), 7.46 - 7.42 (m, 3H), 7.42 - 7.38 (m, 5H), 7.32 - 7.29(m, 1H), 7.28 - 7.26(m, 1H), 5.02 (dd, J = 13.3, 5.1 Hz, 1H), 4.35 (d, J = 16.1 Hz, 1H), 4.22 (d, J = 16.9 Hz, 1H), 3.63 - 3.47 (m, 1H), 3.35 - 3.28 (m, 3H), 3.02 - 2.97 (m, 1H), 2.90 - 2.76 (m, 1H), 2.64 - 2.45 (m, 4H), 2.37

- 2.26 (m, 3H), 2.12 - 2.05 (m, 1H), 2.00 - 1.85 (m, 1H). LCMS (ESI) calcd for $C_{33}H_{31}ClN_3O_4S^+$ [M+H]$^+$: 600.17, found, 600.2.

**Example 37: Preparation of 3-(5-(((3aR,6aS)-2-(4'-chloro-[1,1'-biphenyl]-2-carbonyl)octahydrocyclopenta[c]pyrrol-5-yl)thio)-1-oxoisoindolin-2-yl)piperidine-2,6-dione (GT-07024)**

The target compound (GT-07024) was prepared as a white solid (15 mg, yield: 25%) according to the method described in synthetic method III. $^1$H NMR (400 MHz, DMSO-d6) δ 10.91 (s, 1H), 7.55 (d, J = 8.0 Hz, 1H), 7.46 - 7.42 (m, 3H), 7.42 - 7.38 (m, 5H), 7.32 - 7.29(m, 1H), 7.28 - 7.26(m, 1H), 5.02 (dd, J = 13.3, 5.1 Hz, 1H), 4.35 (d, J = 16.1 Hz, 1H), 4.22 (d, J = 16.9 Hz, 1H), 3.63 - 3.47 (m, 1H), 3.35 - 3.28 (m, 3H), 3.02 - 2.97 (m, 1H), 2.90 - 2.76 (m, 1H), 2.64 - 2.45 (m, 4H), 2.37 - 2.26 (m, 3H), 2.12 - 2.05 (m, 1H), 2.00 - 1.85 (m, 1H). LCMS (ESI) calcd for $C_{33}H_{31}ClN_3O_4S^+$ [M+H]$^+$: 600.17, found, 600.2.

**Example 38: Preparation of 3-(5-((1-(4'-chloro-[1,1'-biphenyl]-2-carbonyl)-3,3-difluoropiperidin-4-yl)thio)-1-oxoisoindolin-2-yl)piperidine-2,6-dione (GT-06925)**

The target compound (GT-06925) was prepared as a white solid (20 mg, yield: 27%) according to the method described in synthetic method III. $^1$H NMR (400 MHz, DMSO-d6) δ 10.90 (s, 1H), 7.58 (d, J = 7.9 Hz, 2H), 7.50 - 7.40 (m, 6H), 7.35 - 7.28 (m, 3H), 5.02 (dd, J = 13.1, 4.8 Hz, 1H), 4.70 - 4.48 (m, 1H), 4.35 (d, J = 16.9 Hz, 1H), 4.22 (d, J = 17.3 Hz, 1H), 4.13 - 4.02 (m, 1H), 3.18 - 3.03 (m, 2H), 2.88 - 2.79 (m, 1H), 2.73 - 2.57 (m, 1H), 2.52 (d, J = 17.3 Hz, 1H), 2.39 - 2.22 (m, 1H), 1.98 - 1.84 (m, 1H), 1.78 - 1.66 (m, 1H), 1.58 - 1.45 (m, 1H). LCMS (ESI) calcd for $C_{31}H_{27}ClF_2N_3O_4S^+$ [M+H]$^+$: 610.14, found, 610.2.

**Example 39: Preparation of 3-(5-((1-((4'-chloro-[1,1'-biphenyl]-3-yl)methyl)azetidin-3-yl)thio)-1-oxoisoindolin-2-yl)piperidine-2,6-dione (GT-06225)**

The target compound (GT-06225) was prepared as a white solid (10 mg, yield: 13%) according to the method described in synthetic method I. $^1$H NMR (400 MHz, DMSO-d6) δ 10.92 (s, 1H), 7.81 (s, 1H), 7.73 - 7.64 (m, 3H), 7.60 (d, J = 8.2 Hz, 1H), 7.50 - 7.42 (m, 5H), 7.33 (d, J = 7.8 Hz, 1H), 5.03 (dd, J = 13.2, 5.0 Hz, 1H), 4.62 - 4.44 (m, 3H), 4.39 - 4.32 (m, 2H), 4.27 - 4.21 (m, 2H), 4.11 - 3.93 (m, 2H), 2.91 - 2.76 (m, 1H), 2.54 - 2.50 (m, 1H), 2.35 - 2.26 (m, 1H), 1.96 - 1.90 (m, 1H). LCMS (ESI) calcd for $C_{29}H_{27}ClN_3O_3S^+$ [M+H]$^+$: 532.15, found, 532.1.

**Example 40: Preparation of 3-(5-((1-((4'-chloro-[1,1'-biphenyl]-4-yl)methyl)azetidin-3-yl)thio)-1-oxoisoindolin-2-yl)piperidine-2,6-dione (GT-06226)**

The target compound (GT-06226) was prepared as a white solid (12 mg, yield: 16%) according to the method described in synthetic method I. $^1$H NMR (400 MHz, DMSO-d6) δ 10.92 (s, 1H), 7.73 - 7.65 (m, 4H), 7.62 (d, J = 8.0 Hz, 1H), 7.56 (d, J = 8.1 Hz, 2H), 7.47 (d, J = 8.6 Hz, 3H), 7.34 (d, J = 7.6 Hz, 1H), 5.05 - 5.01 (m, 1H), 4.64 - 4.44 (m, 3H), 4.40 - 4.32 (m, 2H), 4.27 - 4.22 (m, 2H), 3.99 (brs, 2H), 2.90 - 2.78 (m, 1H), 2.55 - 2.51 (m, 1H), 2.38 - 2.24 (m, 1H), 1.97 - 1.90 (m, 1H). LCMS (ESI) calcd for $C_{29}H_{27}ClN_3O_3S^+$ [M+H]$^+$: 532.15, found, 532.2.

**Example 41: Preparation of 3-(1-oxo-5-((1-((5-phenylpyrazin-2-yl)methyl)azetidin-3-yl)thio)isoindolin-2-yl)piperidine-2,6-dione (GT-06350)**

The target compound (GT-06350) was prepared as a white solid (15 mg, yield: 26%) according to the method described in synthetic method II. $^1$H NMR (400 MHz, DMSO-d6) δ 11.00 (s, 1H), 9.29 (s, 1H), 8.84 (s, 1H), 8.28 - 8.11 (m, 3H), 7.61 - 7.50 (m, 5H), 5.11 (dd, J = 12.4, 4.1 Hz, 1H), 4.71 (d, J = 16.1 Hz, 4H), 4.64 - 4.50 (m, 1H), 4.45 (d, J = 17.8 Hz, 1H), 4.33 (d, J = 17.3 Hz, 1H), 4.27 - 4.18 (m, 2H), 2.96 - 2.87 (m, 1H), 2.60 (d, J = 17.9 Hz, 1H), 2.46 - 2.33 (m, 1H), 2.05 - 1.97 (m, 1H). LCMS (ESI) calcd for $C_{27}H_{26}N_5O_3S^+$ [M+H]$^+$: 500.18, found, 500.2.

**Example 42: Preparation of 3-(5-((1-([2,4'-bipyridin]-5-ylmethyl)azetidin-3-yl)thio)-1-oxoisoindolin-2-yl)piperidine-2,6-dione (GT-06808)**

The target compound (GT-06808) was prepared as a white solid (27 mg, yield: 46%) according to the method described in synthetic method II. $^1$H NMR (400 MHz, DMSO-d6) δ 11.00 (s, 1H), 9.00 (d, J = 1.7 Hz, 1H), 8.97 (d, J = 6.5 Hz, 2H), 8.55 (d, J = 6.5 Hz, 1H), 8.43 (d, J = 8.2 Hz, 1H), 8.32 (dd, J = 8.3, 2.1 Hz, 1H), 7.68 (dd, J = 8.0, 1.9 Hz, 1H), 7.53 (d, J = 5.0 Hz, 1H), 7.40 (t, J = 6.5 Hz, 1H), 5.16 - 5.05 (m, 1H), 4.67 (s, 2H), 4.56 - 4.27 (m, 5H), 4.27 - 4.14 (m, 1H), 3.89 - 3.83(m, 1H), 2.99 - 2.84 (m, 1H), 2.60 (d, J = 16.6 Hz, 1H), 2.46 - 2.33 (m, 1H), 2.05 - 1.93 (m, 1H). LCMS (ESI) calcd for

$C_{27}H_{26}N_5O_3S^+$ [M+H]$^+$: 500.18, found, 500.2.

**Example 43: Preparation of 3-(5-((1-((5-(furan-2-yl)thiophen-2-yl)methyl)azetidin-3-yl)thio)-1-oxoisoindolin-2-yl)piperidine-2,6-dione (GT-06517)**

The target compound (GT-06517) was prepared as a white solid (27 mg, yield: 46%) according to the method described in synthetic method I. $^1$H NMR (400 MHz, DMSO-d6) δ 11.01 (s, 1H), 7.74 (s, 1H), 7.69 (d, J = 8.0 Hz, 1H), 7.52 (s, 1H), 7.41 (d, J = 7.9 Hz, 1H), 7.37 - 7.28 (m, 2H), 6.83 (d, J = 3.4 Hz, 1H), 6.61 (s, 1H), 5.11 (dd, J = 13.2, 4.9 Hz, 1H), 4.77 - 4.47 (m, 5H), 4.44 (d, J = 17.5 Hz, 1H), 4.32 (d, J = 17.4 Hz, 1H), 4.09 - 3.95 (m, 2H), 3.01 - 2.84 (m, 1H), 2.60 (d, J = 16.5 Hz, 1H), 2.45 - 2.33 (m, 1H), 2.03 - 1.98(m, 1H). LCMS (ESI) calcd for $C_{25}H_{24}N_3O_4S_2^+$ [M+H]$^+$: 494.12, found, 494.2.

**Example 44: Preparation of 3-(5-((1-((4'-fluoro-3,4,5,6-tetrahydro-[1,1'-biphenyl]-2-yl)methyl)azetidin-3-yl)thio)-1-oxoisoindolin-2-yl)piperidine-2,6-dione (GT-06301)**

The target compound (GT-06301) was prepared as a white solid (11 mg, yield: 18%) according to the method described in synthetic method I. $^1$H NMR (400 MHz, DMSO-d6) δ 11.00 (s, 1H), 7.72 - 7.66 (m, 1H), 7.46 - 7.37 (m, 1H), 7.33 - 7.07 (m, 5H), 5.11 (dd, J = 13.2, 5.1 Hz, 1H), 4.64 (brs, 1H), 4.53 - 4.19 (m, 4H), 4.18 - 3.95 (m, 1H), 3.81 - 3.64 (m, 2H), 3.00 - 2.81 (m, 1H), 2.67 - 2.58 (m, 2H), 2.41 (brs, 2H), 2.25 (brs, 2H), 2.13 (brs, 2H), 2.03 - 1.97 (m, 1H), 1.66 (brs, 3H). LCMS (ESI) calcd for $C_{29}H_{31}FN_3O_3S^+$ [M+H]$^+$: 520.21, found, 520.1.

**Example** 45: **Preparation** of **3-(5-((1-((4-(4-chlorophenyl)-5,6-dihydro-2H-pyran-3-yl)methyl)azetidin-3-yl)thio)-1-oxoisoindolin-2-yl)piperidine-2,6-dione (GT-06351)**

The target compound (GT-06351) was prepared as a white solid (10 mg, yield: 16%) according to the method described in synthetic method I. $^1$H NMR (400 MHz, DMSO-d6) δ 10.92 (s, 1H), 7.61 (t, J = 8.7 Hz, 1H), 7.45 - 7.28 (m, 3H), 7.29 - 7.12 (m, 3H), 5.03 (dd, J = 13.3, 5.1 Hz, 1H), 4.67 - 4.41 (m, 1H), 4.36 (d, J = 17.5 Hz, 1H), 4.24 (d, J = 17.5 Hz, 2H), 4.12 (s, 2H), 3.82 - 3.65 (m, 3H), 3.60 - 3.37 (m, 4H), 2.92 - 2.77 (m, 1H), 2.53 (d, J = 17.1 Hz, 1H), 2.41 - 2.31 (m, 1H), 2.32 - 2.23 (m, 2H), 1.99 - 1.87 (m, 1H). LCMS (ESI) calcd for $C_{28}H_{29}ClN_3O_4S^+$ [M+H]$^+$: 538.16, found, 538.2.

**Example 46: Preparation of 3-(5-((1-((4-(4-fluorophenyl)-5,6-dihydro-2H-pyran-3-yl)methyl)azetidin-3-yl)thio)-1-oxoisoindolin-2-yl)piperidine-2,6-dione (GT-06513)**

The target compound (GT-06513) was prepared as a white solid (16 mg, yield: 26%) according to the method described in synthetic method I. $^1$H NMR (400 MHz, DMSO-d6) δ 11.00 (s, 1H), 7.71 - 7.63 (m, 1H), 7.43 - 7.36 (m, 1H), 7.33 - 7.20 (m, 5H), 5.11 (dd, J = 13.3, 5.1 Hz, 1H), 4.67 - 4.60 (m, 1H), 4.45 - 4.18 (m, 6H), 4.13 - 4.05 (m, 1H), 3.91 - 3.76 (m, 3H), 3.68 - 3.61 (m, 1H), 2.99 - 2.85 (m, 1H), 2.65 - 2.53 (m, 2H), 2.46 - 2.31 (m, 3H), 2.08 - 1.95 (m, 1H). LCMS (ESI) calcd for $C_{28}H_{29}FN_3O_4S^+$ [M+H]$^+$: 522.19, found, 522.2.

**Example 47: Preparation of 3-(5-((1-((4'-chloro-4,4-difluoro-3,4,5,6-tetrahydro-[1,1'-biphenyl]-2-yl)methyl)azetidin-3-yl)thio)-1-oxoisoindolin-2-yl)piperidine-2,6-dione (GT-06516)**

The target compound (GT-06516) was prepared as a white solid (13 mg, yield: 20%) according to the method described in synthetic method I. $^1$H NMR (400 MHz, DMSO-d6) δ 10.92 (s, 1H), 7.60 (d, J = 7.8 Hz, 1H), 7.48 - 7.35 (m, 2H), 7.33 - 7.29 (m, 1H), 7.23 - 7.10 (m, 3H), 5.04 (dd, J = 13.2, 5.1 Hz, 1H), 4.66 - 4.50 (m, 1H), 4.45 - 4.15 (m, 4H), 4.11 - 3.96 (m, 1H), 3.82 - 3.73 (m, 2H), 3.64 - 3.56 (m, 1H), 2.94 - 2.68 (m, 3H), 2.53 (d, J = 16.9 Hz, 1H), 2.48 - 2.46 (m, 2H), 2.35 - 2.31 (m, 1H), 2.13 - 2.04 (m, 2H), 1.94 - 1.90 (m, 1H). LCMS (ESI) calcd for $C_{29}H_{29}ClF_2N_3O_3S^+$ [M+H]$^+$: 572.16, found, 572.2.

**Example 48: Preparation of 3-(5-((1-((4'-chloro-4-methoxy-4-methyl-3,4,5,6-tetrahydro-[1,1'-biphenyl]-2-yl)methyl)azetidin-3-yl)thio)-1-oxoisoindolin-2-yl)piperidine-2,6-dione (GT-07064)**

The target compound (GT-07064) was prepared as a white solid (10 mg, yield: 14%) according to the method described in synthetic method I. $^1$H NMR (400 MHz, DMSO) δ 10.92 (s, 1H), 7.60 (d, J = 8.0 Hz, 1H), 7.40 (d, J = 8.4 Hz, 1H), 7.37 - 7.24 (m, 3H), 7.17 (d, J = 8.2 Hz, 1H), 7.13 (d, J = 8.4 Hz, 1H), 5.04 (dd, J = 13.3, 5.0 Hz, 1H), 4.59 - 4.53 (m, 1H), 4.37 (d, J = 17.6 Hz, 1H), 4.27 - 4.22 (m, 2H), 4.04 - 4.00 (m, 1H), 3.71 - 3.64 (m, 2H), 3.57 - 3.51 (m, 1H), 3.09 (s, 3H), 2.92 - 2.77 (m, 1H), 2.53 (d, J = 16.3 Hz, 1H), 2.49 - 2.44 (m, 1H), 2.40 - 2.22 (m, 2H), 2.20 - 2.13 (m, 3H), 1.97 - 1.92 (m, 1H), 1.73 - 1.68 (m, 1H), 1.58 - 1.51 (m, 1H), 1.11 (s, 3H). LCMS (ESI) calcd for $C_{31}H_{35}ClN_3O_4S^+$ [M+H]$^+$: 580.20, found, 580.3.

**Example 49: Preparation of 3-(5-((1-((4-(4-chlorophenyl)-6,6-dimethyl-5,6-dihydro-2H-pyran-3-yl)methyl)azetidin-3-yl)thio)-1-oxoisoindolin-2-yl)piperidine-2,6-dione (GT-06514)**

The target compound (GT-06514) was prepared as a white solid (21 mg, yield: 32%) according to the method described in synthetic method I. $^1$H NMR (400 MHz, DMSO-d6) δ 11.00 (s, 1H), 7.67 (d, J = 8.0 Hz, 1H), 7.50 (d, J = 8.3 Hz, 1H), 7.44 (d, J = 8.4 Hz, 1H), 7.39 (s, 1H), 7.35 - 7.22 (m, 3H), 5.11 (dd, J = 13.3, 5.1 Hz, 1H), 4.65 - 4.60 (m, 1H), 4.50 - 4.15 (m, 6H), 4.13 - 4.10 (m, 1H), 3.85 (s, 2H), 3.73 - 3.65 (m, 1H), 2.99 - 2.85 (m, 1H), 2.61 (d, J = 16.7 Hz, 1H), 2.46 - 2.35 (m, 1H), 2.23 (s, 2H), 2.06 - 1.94 (m, 1H), 1.21 (s, 6H). LCMS (ESI) calcd for $C_{30}H_{33}ClN_3O_4S^+$ [M+H]$^+$: 566.19, found, 566.2.

**Example 50: Preparation of 3-(5-((1-benzhydrylazetidin-3-yl)thio)-1-oxoisoindolin-2-yl)piperidine-2,6-dione (GT-06138)**

The target compound (GT-06138) was prepared as a white solid (24 mg, yield: 33%) according to the method described in synthetic method II. $^1$H NMR (400 MHz, DMSO-d6) δ 10.91 (s, 1H), 7.64 - 7.52 (m, 5H), 7.40 - 7.24 (m, 8H), 5.02 (dd, J = 13.3, 5.1 Hz, 1H), 4.64 - 4.52 (m, 2H), 4.36 - 4.32 (m, 2H), 4.25 - 4.19(m, 2H), 4.10 - 3.95 (m, 2H), 2.91 - 2.77 (m, 1H), 2.52 (d, J = 17.0 Hz, 1H), 2.37 - 2.21 (m, 1H), 1.96 - 1.90 (m, 1H). LCMS (ESI) calcd for $C_{29}H_{28}N_3O_3S^+$ [M+H]$^+$: 498.18, found, 498.2.

**Example 51: Preparation of 3-(5-((1-benzhydrylpyrrolidin-3-yl)thio)-1-oxoisoindolin-2-yl)piperidine-2,6-dione (GT-06196)**

The target compound (GT-06196) was prepared as a white solid (11 mg, yield: 15%) according to the method described in synthetic method II. $^1$H NMR (400 MHz, DMSO-d6) δ 10.92 (s, 1H), 7.81 - 7.70 (m, 3H), 7.61 - 7.55 (m, 2H), 7.41 - 7.24 (m, 8H), 5.69 - 5.60 (m, 1H), 5.02 (dd, J = 13.1, 5.0 Hz, 1H), 4.38 - 4.21 (m, 3H), 3.69 - 3.58 (m, 1H), 3.23 - 3.10 (m, 2H), 3.05 - 2.94 (m, 1H), 2.91 - 2.76 (m, 1H), 2.59 - 2.50 (m, 2H), 2.37 - 2.26 (m, 1H), 2.13 - 1.98 (m, 1H), 1.94 - 1.85 (m, 1H). LCMS (ESI) calcd for $C_{30}H_{30}N_3O_3S^+$ [M+H]$^+$: 512.20, found, 512.2.

**Example 52: Preparation of 3-(5-((1-benzhydrylpiperidin-4-yl)thio)-1-oxoisoindolin-2-yl)piperidine-2,6-dione (GT-06184)**

The target compound (GT-06184) was prepared as a white solid (12 mg, yield: 17%) according to the method described in synthetic method II. $^1$H NMR (400 MHz, DMSO-d6) δ 10.92 (s, 1H), 7.84 (d, J = 7.5 Hz, 1H), 7.79 (d, J = 7.5 Hz, 3H), 7.61 - 7.55 (m, 2H), 7.48 - 7.41 (m, 1H), 7.42 - 7.34 (m, 4H), 7.33 - 7.21 (m, 2H), 5.03 (dd, J = 12.8, 4.5 Hz, 1H), 4.36 (d, J = 17.5 Hz, 1H), 4.23 (d, J = 17.3 Hz, 1H), 3.52 - 3.45 (m, 1H), 3.20 - 3.13 (m, 2H), 3.08 - 2.97 (m, 2H), 2.88 - 2.79 (m, 1H), 2.52 (d, J = 16.4 Hz, 1H), 2.36 - 2.25 (m, 2H), 2.11 - 2.06 (m, 3H), 1.99 - 1.84 (m, 2H). LCMS (ESI) calcd for $C_{31}H_{32}N_3O_3S^+$ [M+H]$^+$: 526.22, found, 526.2.

**Example 53: Preparation of 3-(5-((1-benzhydrylazepan-4-yl)thio)-1-oxoisoindolin-2-yl)piperidine-2,6-dione (GT-06645)**

The target compound (GT-06645) was prepared as a white solid (7 mg, yield: 10%) according to the method described in synthetic method II. $^1$H NMR (400 MHz, DMSO-d6) δ 10.91 (s, 1H), 7.80 - 7.75 (m, 4H), 7.57 (t, J = 7.9 Hz, 1H), 7.52 (s, 1H), 7.41 - 7.35 (m, 5H), 7.32 - 7.23 (m, 2H), 5.61 (dd, J = 28.7, 9.4 Hz, 1H), 5.04 - 5.01 (m, 1H), 4.34 (d, J = 16.8 Hz, 1H), 4.22 (d, J = 17.1 Hz, 1H), 3.84 - 3.71 (m, 1H), 3.10 - 2.98 (m, 1H), 2.95 - 2.80 (m, 2H), 2.52 (d, J = 18.3 Hz, 2H), 2.37 - 2.25 (m, 2H), 2.22 - 2.14 (m, 2H), 2.08 - 2.02 (m, 1H), 1.95 -1.86 (m, 4H), 1.71 - 1.67 (m, 1H), 1.57 - 1.48 (m, 1H). LCMS (ESI) calcd for $C_{32}H_{34}N_3O_3S^+$ [M+H]$^+$: 540.23, found, 540.2.

**Example 54: Preparation of 3-(5-((1-benzhydryl-3,3-difluoropiperidin-4-yl)thio)-1-oxoisoindolin-2-yl)piperidine-2,6-dione (GT-06926)**

The target compound (GT-06926) was prepared as a white solid (12 mg, yield: 17%) according to the method described in synthetic method II. $^1$H NMR (400 MHz, DMSO-d6) δ 10.91 (s, 1H), 7.62 (s, 1H), 7.58 (d, J = 8.0 Hz, 1H), 7.49 (d, J = 8.0 Hz, 1H), 7.33 (d, J = 7.3 Hz, 4H), 7.28 - 7.23 (m, 4H), 7.20 - 7.11 (m, 2H), 5.02 (dd, J = 13.3, 4.9 Hz, 1H), 4.52 (s, 1H), 4.35 (d, J = 17.3 Hz, 1H), 4.22 (d, J = 17.5 Hz, 1H), 3.98 - 3.77 (m, 1H), 2.99 - 2.92 (m, 1H), 2.91 - 2.77 (m, 1H), 2.71 (d, J = 11.5 Hz, 1H), 2.52 (d, J = 16.7 Hz, 1H), 2.40 - 2.23 (m, 2H), 2.15 (t, J = 10.9 Hz, 1H), 2.10 - 1.99 (m, 1H), 1.99 - 1.87 (m, 1H), 1.79 - 1.70 (m, 1H). LCMS (ESI) calcd for $C_{31}H_{30}F_2N_3O_3S^+$ [M+H]$^+$: 562.20, found, 562.2.

**Example 55: Preparation of 3-(5-((7-benzhydryl-7-azaspiro[3.5]nonan-2-yl)thio)-1-oxoisoindolin-2-yl)piperidine-2,6-dione (GT-06920)**

The target compound (GT-06920) was prepared as a white solid (7 mg, yield: 15%) according to the method described in synthetic method II. $^1$H NMR (400 MHz, DMSO-d6) δ 11.37 (s, 1H), 10.91 (s, 1H), 7.87 - 7.73 (m, 4H), 7.58 - 7.55 (m, 1H), 7.45 - 7.32 (m, 5H), 7.33 - 7.27 (m, 2H), 7.25 - 7.22 (m, 1H), 5.50 (d, J = 9.3 Hz, 1H), 5.02 (dd, J = 13.4, 5.0 Hz, 1H), 4.34 (d, J = 17.4 Hz, 1H), 4.21 (d, J = 17.4 Hz, 1H), 4.13 - 3.93 (m, 1H), 3.57 - 3.44 (m, 1H), 3.09 - 2.75 (m, 4H), 2.55 - 2.44 (m, 2H), 2.40 - 2.20 (m, 2H), 2.09 - 2.00 (m, 1H), 1.98 - 1.87 (m, 2H), 1.83 - 1.75 (m, 3H), 1.69 - 1.65 (m, 1H). LCMS (ESI) calcd for $C_{34}H_{36}N_3O_3S^+$ [M+H]$^+$: 566.25, found, 566.3.

**Example 56: Preparation of 3-(5-((3-benzhydryl-3-azaspiro[5.5]undecan-9-yl)thio)-1-oxoisoindolin-2-yl)piperidine-2,6-dione (GT-06958)**

The target compound (GT-06958) was prepared as a white solid (6 mg, yield: 14%) according to the method described in synthetic method II. $^1$H NMR (400 MHz, DMSO) δ 10.92 (s, 1H), 7.72 - 7.70 (m, 3H), 7.60 - 7.56 (m, 1H), 7.52 - 7.50 (m, 1H), 7.42 - 7.36 (m, 5H), 7.32 - 7.28 (m, 2H), 7.20 - 7.11 (m, 1H), 5.04 - 5.01 (m, 1H), 4.36 (d, J = 17.3 Hz, 1H), 4.23 (d, J = 17.8 Hz, 1H), 3.59 - 3.52(m, 1H), 3.44 - 3.36 (m, 1H), 3.01 - 2.95 (m, 2H), 2.88 - 2.81 (m, 1H), 2.53 (d, J = 16.1 Hz, 1H), 2.33 - 2.25 (m, 1H), 1.97 - 1.90 (m, 2H), 1.83 - 1.75 (m, 3H), 1.69 - 1.63 (m, 2H), 1.56 - 1.52 (m, 2H), 1.46 - 1.37 (m, 4H), 1.32 - 1.24 (m, 2H). LCMS (ESI) calcd for $C_{36}H_{40}N_3O_3S^+$ [M+H]$^+$: 594.28, found, 594.3.

**Example 57: Preparation of 3-(5-((2-benzhydryloctahydrocyclopenta[c]pyrrol-5-yl)thio)-1-oxoisoindolin-2-yl) piperidine-2,6-dione (GT-06922)**

The target compound (GT-06922) was prepared as a white solid (6 mg, yield: 12%) according to the method described in synthetic method II. $^1$H NMR (400 MHz, DMSO-d6) δ 10.92 (s, 1H), 7.85 - 7.69 (m, 4H), 7.62 - 7.48 (m, 2H), 7.43 - 7.35 (m, 5H), 7.32 - 7.27 (m, 2H), 5.65 - 5.50 (m, 1H), 5.08 - 4.94 (m, 1H), 4.36 (dd, J = 17.4, 7.6 Hz, 1H), 4.23 (dd, J = 17.4, 8.7 Hz, 1H), 3.99 - 3.86 (m, 1H), 3.12 - 3.09 (m, 1H), 2.94 - 2.78 (m, 4H), 2.53 (d, J = 16.0 Hz, 1H), 2.37 - 2.26 (m, 3H), 2.08 - 1.87 (m, 2H), 1.82 - 1.73 (m, 1H), 1.62 - 1.54 (m, 1H), 1.49 - 1.42 (m, 1H). LCMS (ESI) calcd for $C_{33}H_{34}N_3O_3S^+$ [M+H]$^+$: 552.23, found, 552.3.

**Example 58: Preparation of 3-(5-((1-(bis(2-fluorophenyl)methyl)azetidin-3-yl)thio)-1-oxoisoindolin-2-yl)piperidine-2,6-dione (GT-06758)**

The target compound (GT-06758) was prepared as a white solid (25 mg, yield: 40%) according to the method described in synthetic method II. $^1$H NMR (400 MHz, DMSO-d6) δ 10.91 (s, 1H), 7.69 - 7.49 (m, 3H), 7.48 - 7.24 (m, 4H), 7.25 - 7.04 (m, 4H), 5.02 (dd, J = 13.2, 5.0 Hz, 1H), 4.49 - 4.14 (m, 4H), 3.99 (s, 1H), 3.57 - 3.33 (m, 3H), 2.94 - 2.77 (m, 1H), 2.52 (d, J = 16.3 Hz, 1H), 2.32- 2.29 (m, 1H), 1.99 - 1.87 (m, 1H). LCMS (ESI) calcd for $C_{29}H_{26}F_2N_3O_3S^+$ [M+H]$^+$: 534.17, found, 534.3.

**Example 59: Preparation of 3-(5-((1-(bis(3-fluorophenyl)methyl)azetidin-3-yl)thio)-1-oxoisoindolin-2-yl)piperidine-2,6-dione (GT-06349)**

The target compound (GT-06349) was prepared as a white solid (21 mg, yield: 34%) according to the method described in synthetic method II. $^1$H NMR (400 MHz, DMSO-d6) δ 10.92 (s, 1H), 7.69 - 7.47 (m, 2H), 7.47 - 7.20 (m, 7H), 7.18 - 7.01 (m, 2H), 5.11 - 4.95 (m, 1H), 4.52 - 4.13 (m, 4H), 3.71 - 3.53 (m, 2H), 2.92 - 2.76 (m, 2H), 2.59 - 2.49 (m, 2H), 2.37 - 2.23 (m, 1H), 2.02 - 1.86 (m, 1H). LCMS (ESI) calcd for $C_{29}H_{26}F_2N_3O_3S^+$ [M+H]$^+$: 534.17, found, 534.3.

**Example 60: Preparation of 3-(5-((1-(bis(4-fluorophenyl)methyl)azetidin-3-yl)thio)-1-oxoisoindolin-2-yl)piperidine-2,6-dione (GT-06503)**

The target compound (GT-06503) was prepared as a white solid (17 mg, yield: 27%) according to the method described in synthetic method II. $^1$H NMR (400 MHz, DMSO-d6) δ 10.93 (s, 1H), 7.83 - 7.50 (m, 5H), 7.50 - 7.34 (m, 2H), 7.34 - 7.14 (m, 4H), 5.02 (dd, J = 13.3, 5.0 Hz, 1H), 4.58 - 4.39 (m, 3H), 4.34 (d, J = 17.5 Hz, 1H), 4.22 (d, J = 17.6 Hz, 1H), 4.08 - 3.95 (m, 2H), 2.90 - 2.76 (m, 1H), 2.58 - 2.49 (m, 2H), 2.34 - 2.26 (m, 1H), 1.98 - 1.88 (m, 1H). LCMS (ESI) calcd for $C_{29}H_{26}F_2N_3O_3S^+$ [M+H]$^+$: 534.17, found, 534.2.

**Example 61: Preparation of 3-(5-((1-(bis(4-chlorophenyl)methyl)azetidin-3-yl)thio)-1-oxoisoindolin-2-yl)piperidine-2,6-dione (GT-06504)**

The target compound (GT-06504) was prepared as a white solid (26 mg, yield: 40%) according to the method described in synthetic method II. $^1$H NMR (400 MHz, DMSO-d6) δ 10.92 (d, J = 11.3 Hz, 1H), 7.82 - 7.51 (m, 5H), 7.50 - 7.31 (m, 6H), 5.02 (dd, J = 13.4, 5.0 Hz, 1H), 4.64 - 4.32 (m, 3H), 4.34 (d, J = 17.6 Hz, 1H), 4.21 (d, J = 17.6 Hz, 1H), 4.15 - 3.88 (m, 2H), 2.90 - 2.77 (m, 1H), 2.58 - 2.50 (m, 2H), 2.37 - 2.25 (m, 1H), 1.98 - 1.88 (m, 1H). LCMS (ESI) calcd for $C_{29}H_{26}Cl_2N_3O_3S^+$ [M+H]$^+$: 566.11, found, 566.1.

**Example 62: Preparation of 3-(5-((1-(bis(4-methoxyphenyl)methyl)azetidin-3-yl)thio)-1-oxoisoindolin-2-yl)piperidine-2,6-dione (GT-06923)**

The target compound (GT-06923) was prepared as a white solid (9 mg, yield: 14%) according to the method described in synthetic method II. $^1$H NMR (400 MHz, DMSO-d6) δ 10.92 (s, 1H), 7.61 - 7.58 (m, 1H), 7.52 - 7.47 (m, 4H), 7.42 - 7.24 (m, 2H), 6.95 - 6.90 (m, 4H), 5.69 (dd, J = 81.4, 9.8 Hz, 1H), 5.02 (dd, J = 13.3, 5.1 Hz, 1H), 4.60 - 4.16 (m, 5H), 3.99 - 3.93 (m, 2H), 3.67 (s, 6H), 2.95 - 2.74 (m, 1H), 2.52 (d, J = 16.9 Hz, 1H), 2.39 - 2.23 (m, 1H), 1.95 - 1.90 (m, 1H). LCMS (ESI) calcd for $C_{31}H_{31}N_3NaO_5S^+$ [M+Na]$^+$: 580.19, found, 580.3.

**Example 63: Preparation of 3-(1-oxo-5-((1-(phenyl(pyridin-2-yl)methyl)azetidin-3-yl)thio)isoindolin-2-yl)piperidine-2,6-dione (GT-06518)**

The target compound (GT-06518) was prepared as a white solid (5 mg, yield: 9%) according to the method described in synthetic method II. $^1$H NMR (400 MHz, DMSO-d6) δ 10.99 (s, 1H), 8.73 - 8.60 (m, 1H), 7.92 - 7.81 (m, 1H), 7.75 - 7.62 (m, 2H), 7.62 - 7.49 (m, 3H), 7.49 - 7.29 (m, 5H), 5.21 - 5.05 (m, 1H), 4.64 - 4.13 (m, 4H), 3.71 - 3.67 (m, 1H), 3.60 - 3.40 (m, 3H), 2.97 - 2.88 (m, 1H), 2.61 (d, J = 18.4 Hz, 1H), 2.44 - 2.37 (m, 1H), 2.05 - 1.98 (m, 1H). LCMS (ESI) calcd for $C_{28}H_{27}N_4O_3S^+$ [M+H]$^+$: 499.18, found, 499.2.

**Example 64: Preparation of 3-(1-oxo-5-((1-(phenyl(pyridin-3-yl)methyl)azetidin-3-yl)thio)isoindolin-2-yl)piperidine-2,6-dione (GT-06807)**

The target compound (GT-06807) was prepared as a white solid (18 mg, yield: 31%) according to the method described in synthetic method II. $^1$H NMR (400 MHz, DMSO-d6) δ 10.95 (s, 1H), 8.98 - 8.82 (m, 1H), 8.59 - 8.54 (m, 1H), 8.40 - 8.16 (m, 1H), 8.07 - 8.95 (m, 1H), 7.84 - 7.44 (m, 4H), 7.43 - 7.17 (m, 4H), 5.13 - 4.95 (m, 1H), 4.85 - 4.58 (m, 1H), 4.50 - 3.95 (m, 4H), 3.89 - 3.59 (m, 2H), 2.94 - 2.77 (m, 2H), 2.54 (d, J = 16.5 Hz, 1H), 2.40 - 2.20 (m, 1H), 2.04 - 1.85 (m, 1H). LCMS (ESI) calcd for $C_{28}H_{27}N_4O_3S^+$ [M+H]$^+$: 499.18, found, 499.2.

**Example 65: Preparation of 3-(5-((1-(di(pyridin-2-yl)methyl)azetidin-3-yl)thio)-1-oxoisoindolin-2-yl)piperidine-2,6-dione (GT-06811)**

The target compound (GT-06811) was prepared as a white solid (20 mg, yield: 34%) according to the method described in synthetic method II. $^1$H NMR (400 MHz, DMSO-d6) δ 11.02 (s, 1H), 8.66 - 8.53 (m, 2H), 7.96 - 7.83 (m, 2H), 7.78 - 7.64 (m, 1H), 7.62 - 7.55 (m, 3H), 7.51 - 7.32 (m, 3H), 5.20 - 5.01 (m, 1H), 4.65 - 4.61 (m, 1H), 4.51 - 4.42 (m, 1H), 4.36 - 4.30 (m, 1H), 4.29 - 4.00 (m, 2H), 3.67 - 3.56 (m, 2H), 3.02 - 2.89 (m, 1H), 2.62 (d, J = 16.7 Hz, 1H), 2.45 - 2.38 (m, 1H), 2.07 - 1.94 (m, 1H). LCMS (ESI) calcd for $C_{27}H_{26}N_5O_3S^+$ [M+H]$^+$: 500.18, found, 500.2.

**Example 66: Preparation of 3-(4-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione (GT-06952)**

The target compound (GT-06952) was prepared as a white solid (31 mg, yield: 46%) according to the method described in synthetic method I. $^1$H NMR (400 MHz, DMSO-d6) δ 10.96 (s, 1H), 7.47 - 7.29 (m, 4H), 7.23 (d, J = 8.0 Hz, 1H), 7.09 - 7.06 (m, 2H), 6.91 - 6.83 (m, 1H), 5.23 - 4.90 (m, 2H), 4.68 - 4.57 (m, 1H), 4.34 - 4.01 (m, 3H), 3.86 - 3.56 (m, 3H), 3.46 (d, J = 5.1 Hz, 1H), 2.93 - 2.82 (m, 1H), 2.63 - 2.52 (m, 2H), 2.21 - 2.10 (m, 2H), 2.06 - 1.84 (m, 3H), 1.43 - 1.27 (m, 2H), 0.88 (s, 6H). LCMS (ESI) calcd for $C_{31}H_{35}ClN_3O_4^+$ [M+H]$^+$: 548.23, found, 548.3.

**Example 67: Preparation of 3-(6-((1-((4'-chloro-5,5-dimethyl-3,4,5,6-tetrahydro-[1,1'-biphenyl]-2-yl)methyl)azetidin-3-yl)oxy)-1-oxoisoindolin-2-yl)piperidine-2,6-dione (GT-06872)**

The target compound (GT-06872) was prepared as a white solid (19 mg, yield: 28%) according to the method

described in synthetic method I. $^1H$ NMR (400 MHz, DMSO-d6) δ 10.99 (s, 1H), 7.58 - 7.56 (m, 1H), 7.51 - 7.36 (m, 3H), 7.22 (d, J = 8.0 Hz, 1H), 7.20 - 7.11 (m, 2H), 5.11 (dd, J = 13.2, 4.7 Hz, 1H), 5.07 - 5.00 (m, 1H), 4.73 - 4.68 (m, 1H), 4.42 - 4.25 (m, 3H), 3.84 - 3.76 (m, 2H), 2.95 - 2.87 (m, 1H), 2.61 (d, J = 17.2 Hz, 1H), 2.45 - 2.33 (m, 1H), 2.25 - 2.15 (m, 3H), 2.10 - 1.94 (m, 4H), 1.48 - 1.44 (m, 2H), 0.95 (s, 6H). LCMS (ESI) calcd for $C_{31}H_{35}ClN_3O_4^+$ [M+H]$^+$: 548.23, found, 548.3.

**Example 68: Preparation of 3-(7-((1-((4'-chloro-5,5-dimethyl-3,4,5,6-tetrahydro-[1,1'-biphenyl]-2-yl)methyl))zetidine-3-yl)oxy)-1-oxoisoindolin-2-yl)piperidine-2,6-dione (GT-07063)**

The target compound (GT-07063) was prepared as a white solid (6 mg, yield: 10%) according to the method described in synthetic method I. $^1H$ NMR (400 MHz, DMSO-d6) δ 10.91 (s, 1H), 10.49 (s, 1H), 7.46 (t, J = 7.8 Hz, 1H), 7.39 (t, J = 8.1 Hz, 1H), 7.28 (d, J = 8.1 Hz, 1H), 7.22 - 7.04 (m, 3H), 6.69 (d, J = 8.2 Hz, 1H), 5.03 - 4.85 (m, 2H), 4.58 (t, J = 17.0 Hz, 1H), 4.34 (d, J = 17.6 Hz, 1H), 4.24 - 4.19 (m, 2H), 3.92 - 3.63 (m, 3H), 2.90 - 2.73 (m, 1H), 2.56 - 2.50 (m, 1H), 2.48 - 2.45 (m, 1H), 2.36 - 2.28 (m, 1H), 2.16 - 2.07 (m, 2H), 2.02 - 1.86 (m, 3H), 1.40 - 1.35 (m, 2H), 0.88 (s, 6H). LCMS (ESI) calcd for $C_{31}H_{35}ClN_3O_4^+$ [M+H]$^+$: 548.23, found, 548.3.

**Example 69: Preparation of 3-(5-((1-((4'-chloro-5,5-dimethyl-3,4,5,6-tetrahydro-[1,1'-biphenyl]-2-yl)methyl)pyrrolidin-3-yl)oxy)-1-oxoisoindolin-2-yl)piperidine-2,6-dione (GT-06815)**

The target compound (GT-06815) was prepared as a white solid (41 mg, yield: 63%) according to the method described in synthetic method I. $^1H$ NMR (400 MHz, DMSO-d6) δ 10.98 (s, 1H), 7.46 (t, J = 7.9 Hz, 1H), 7.40 - 7.37 (m, 2H), 7.16 - 7.12 (m, 2H), 6.84 (d, J = 5.9 Hz, 1H), 6.63 (d, J = 8.4 Hz, 1H), 5.17 - 5.08 (m, 2H), 4.41 (d, J = 19.5 Hz, 1H), 4.29 (d, J = 17.3 Hz, 1H), 3.87 - 3.74 (m, 2H), 3.58 - 3.54 (m, 2H), 3.16 (d, J = 14.4 Hz, 1H), 2.95 - 2.89 (m, 2H), 2.61 (d, J = 17.2 Hz, 1H), 2.44 - 2.40 (m, 2H), 2.29 - 2.20 (m, 2H), 2.12 - 1.98 (m, 3H), 1.54 - 1.41 (m, 3H), 0.97 (s, 6H). LCMS (ESI) calcd for $C_{32}H_{37}ClN_3O_4^+$ [M+H]$^+$: 562.25, found, 562.3.

**Example 70: Preparation of 3-(5-((1-((4'-chloro-5,5-dimethyl-3,4,5,6-tetrahydro-[1,1'-biphenyl]-2-yl)methyl)piperidin-4-yl)oxy)-1-oxoisoindolin-2-yl)piperidine-2,6-dione (GT-07061)**

The target compound (GT-07061) was prepared as a white solid (4 mg, yield: 8%) according to the method described in synthetic method I. $^1H$ NMR (400 MHz, DMSO-d6) δ 10.97 (s, 1H), 7.66 - 7.62 (m, 1H), 7.52 - 7.42 (m, 2H), 7.22 - 7.12 (m, 3H), 7.09 - 7.00 (m, 1H), 5.14 - 5.02 (m, 1H), 4.82 - 4.63 (m, 1H), 4.37 (d, J = 16.9 Hz, 1H), 4.25 (d, J = 17.4 Hz, 1H), 3.62 - 3.55 (m, 3H), 2.95 - 2.88 (m, 1H), 2.82 - 2.74 (m, 2H), 2.62 - 2.57 (m, 3H), 2.36 - 2.31 (m, 2H), 2.25 - 2.16 (m, 2H), 2.05 (s, 2H), 2.02 - 1.92 (m, 3H), 1.52 - 1.47 (m, 2H), 0.97 (s, 6H). LCMS (ESI) calcd for $C_{33}H_{39}ClN_3O_4^+$ [M+H]$^+$: 576.26, found, 576.3.

**Example 71: Preparation of 3-(5-((1-((4'-chloro-5,5-dimethyl-3,4,5,6-tetrahydro-[1,1'-biphenyl]-2-yl)methyl)azepan-4-yl)oxy)-1-oxoisoindolin-2-yl)piperidine-2,6-dione (GT-06929)**

The target compound (GT-06929) was prepared as a white solid (17 mg, yield: 27%) according to the method described in synthetic method I. $^1H$ NMR (400 MHz, DMSO-d6) δ 10.90 (s, 1H), 7.55 (d, J = 8.4 Hz, 1H), 7.40 - 7.36 (m, 2H), 7.13 - 7.03 (m, 3H), 6.98 - 6.88 (m, 1H), 5.00 (dd, J = 13.3, 5.0 Hz, 1H), 4.70 - 4.61 (m, 1H), 4.31 (d, J = 17.2 Hz, 1H), 4.19 (d, J = 17.3 Hz, 1H), 3.52 - 3.46 (m, 2H), 3.41 - 3.30 (m, 1H), 3.19 - 3.07 (m, 1H), 3.00 - 2.95 (m, 1H), 2.88 - 2.79 (m, 3H), 2.52 (d, J = 16.7 Hz, 1H), 2.39 - 2.16 (m, 3H), 2.16 - 2.05 (m, 1H), 2.01 - 1.97 (m, 3H), 1.94 - 1.86 (m, 2H), 1.84 - 1.79 (m, 2H), 1.76 - 1.60 (m, 2H), 1.44 - 1.31 (m, 2H), 0.89 (s, 6H). LCMS (ESI) calcd for $C_{34}H_{41}ClN_3O_4^+$ [M+H]$^+$: 590.28, found, 590.3.

**Example 72: Preparation of 3-(5-(((3aS,6aS)-2-((4'-chloro-5,5-dimethyl-3,4,5,6-tetrahydro-[1,1'-biphenyl]-2-yl) methyl)octahydrocyclopenta[c]pyrrol-5-yl)oxy)-1-oxoisoindolin-2-yl)piperidine-2,6-dione (GT-06930)**

The target compound (GT-06930) was prepared as a white solid (28 mg, yield: 44%) according to the method described in synthetic method I. $^1H$ NMR (400 MHz, DMSO-d6) δ 10.99 (s, 1H), 10.78 (s, 1H), 7.59 (d, J = 8.4 Hz, 1H), 7.53 (d, J = 8.4 Hz, 2H), 7.46 (d, J = 8.4 Hz, 1H), 7.23 (d, J = 8.4 Hz, 2H), 7.20 (d, J = 8.5 Hz, 1H), 5.11 - 5.01 (m, 2H), 4.38 (d, J = 17.2 Hz, 1H), 4.27 (d, J = 17.2 Hz, 1H), 3.66 - 2.53 (m, 4H), 3.20 - 3.16 (m, 1H), 2.99 - 2.92 (m, 3H), 2.63 - 2.59 (m, 1H), 2.43 - 2.28 (m, 4H), 2.07 (s, 2H), 2.03 - 1.90 (m, 3H), 1.86 - 1.83 (m, 2H), 1.48 (t, J = 6.0 Hz, 2H), 0.97 (s, 6H). LCMS (ESI) calcd for $C_{35}H_{41}ClN_3O_4^+$ [M+H]$^+$: 602.28, found, 602.3.

**Example 73: Preparation of 3-(5-(((3aR,6aS)-2-((4'-chloro-5,5-dimethyl-3,4,5,6-tetrahydro-[1,1'-biphenyl]-2-yl)methyl)octahydrocyclopenta[c]pyrrol-5-yl)oxy)-1-oxoisoindolin-2-yl)piperidine-2,6-dione (GT-06931)**

The target compound (GT-06931) was prepared as a white solid (11 mg, yield: 17%) according to the method described in synthetic method I. $^1$H NMR (400 MHz, DMSO-d6) δ 10.89 (s, 1H), 7.54 (dd, J = 8.3, 5.4 Hz, 1H), 7.38 (d, J = 8.4 Hz, 2H), 7.11 (d, J = 8.4 Hz, 2H), 7.06 (d, J = 14.5 Hz, 1H), 6.94 (d, J = 8.1 Hz, 1H), 5.00 (dd, J = 12.7, 5.2 Hz, 2H), 4.31 (d, J = 17.2 Hz, 1H), 4.19 (d, J = 17.0 Hz, 1H), 3.57 - 3.52 (m, 3H), 3.15 (d, J = 11.8 Hz, 1H), 2.91 - 2.81 (m, 3H), 2.72 - 2.67 (m, 1H), 2.54 - 2.51 (m, 2H), 2.38 - 2.19 (m, 3H), 2.09 - 1.87 (m, 5H), 1.85 - 1.77 (m, 2H), 1.39 (brs, 2H), 0.90 (s, 6H). LCMS (ESI) calcd for $C_{35}H_{41}ClN_3O_4^+$ $[M+H]^+$: 602.28, found, 602.3.

**Example 74: Preparation of 3-(5-((7-((4'-chloro-5,5-dimethyl-3,4,5,6-tetrahydro-[1,1'-biphenyl]-2-yl)methyl)-7-azaspiro[3.5]nonan-2-yl)oxy)-1-oxoisoindolin-2-yl)piperidine-2,6-dione (GT-06933)**

The target compound (GT-06933) was prepared as a white solid (15 mg, yield: 29%) according to the method described in synthetic method I. $^1$H NMR (400 MHz, DMSO-d6) δ 10.89 (s, 1H), 9.64 (s, 1H), 7.54 (d, J = 8.4 Hz, 1H), 7.37 (d, J = 8.3 Hz, 2H), 7.07 (d, J = 8.3 Hz, 2H), 6.95 (s, 1H), 6.87 (dd, J = 8.4, 2.0 Hz, 1H), 4.99 (dd, J = 13.3, 5.1 Hz, 1H), 4.80 - 4.63 (m, 1H), 4.29 (dd, J = 17.2, 5.6 Hz, 1H), 4.17 (dd, J = 17.2, 4.7 Hz, 1H), 3.46 (brs, 2H), 3.08 (dd, J = 27.5, 11.6 Hz, 2H), 2.93 - 2.74 (m, 1H), 2.54 - 2.47 (m, 3H), 2.38 - 2.16 (m, 5H), 2.07 - 1.81 (m, 6H), 1.77 - 1.61 (m, 2H), 1.58 (d, J = 13.7 Hz, 1H), 1.40 (t, J = 5.9 Hz, 2H), 0.89 (s, 6H). LCMS (ESI) calcd for $C_{36}H_{43}ClN_3O_4^+$ $[M+H]^+$: 616.29, found, 616.3.

**Example 75: Preparation of 3-(5-((1-(4'-chloro-5,5-dimethyl-3,4,5,6-tetrahydro-[1,1'-biphenyl]-2-carbonyl)azetidin-3-yl)oxy)-1-oxoisoindolin-2-yl)piperidine-2,6-dione (GT-06180)**

The target compound (GT-06180) was prepared as a white solid (26 mg, yield: 31%) according to the method described in synthetic method III. $^1$H NMR (400 MHz, DMSO-d6) δ 10.97 (s, 1H), 7.62 (d, J = 8.4 Hz, 1H), 7.37 (dd, J = 8.6, 2.3 Hz, 2H), 7.26 (dd, J = 8.5, 1.7 Hz, 2H), 6.93 (s, 1H), 6.85 (dd, J = 8.4, 1.9 Hz, 1H), 5.07 (dd, J = 13.2, 3.7 Hz, 1H), 4.97 - 4.82 (m, 1H), 4.38 (d, J = 17.3 Hz, 1H), 4.25 (d, J = 17.1 Hz, 1H), 4.22 - 4.04 (m, 2H), 3.60 - 3.50 (m, 2H), 2.99 - 2.83 (m, 1H), 2.59 (d, J = 17.6 Hz, 1H), 2.44 - 2.32 (m, 2H), 2.25 - 2.18 (m, 2H), 2.06 (s, 1H), 2.01 - 1.97 (m, 1H), 1.42 (t, J = 6.4 Hz, 2H), 0.98 (s, 3H), 0.96(s, 3H). LCMS (ESI) calcd for $C_{31}H_{33}ClN_3O_5^+$ $[M+H]^+$: 562.21, found, 562.3.

**Example 76: Preparation of 3-(5-((1-(4'-chloro-5,5-dimethyl-3,4,5,6-tetrahydro-[1,1'-biphenyl]-2-carbonyl)pyrrolidin-3-yl)oxy)-1-oxoisoindolin-2-yl)piperidine-2,6-dione (GT-06816)**

The target compound (GT-06816) was prepared as a white solid (33 mg, yield: 49%) according to the method described in synthetic method III. $^1$H NMR (400 MHz, DMSO-d6) δ 10.98 (s, 1H), 7.63 - 7.60 (m, 1H), 7.37 - 7.34 (m, 1H), 7.28 - 7.25 (m, 2H), 7.22 - 7.17 (m, 1H), 7.01 - 6.93 (m, 1H), 6.91 - 6.76 (m, 1H), 5.11 - 5.07 (m, 1H), 4.96 (d, J = 28.8 Hz, 1H), 4.43 - 4.38 (m, 1H), 4.30 - 4.24 (m, 1H), 3.27 - 3.19 (m, 2H), 2.99 - 2.82 (m, 2H), 2.60 (d, J = 17.1 Hz, 1H), 2.46 - 2.32 (m, 2H), 2.29 - 2.20 (m, 2H), 2.16 - 1.76 (m, 5H), 1.53 - 1.33 (m, 2H), 1.01 (s, 3H), 0.99 (s, 3H). LCMS (ESI) calcd for $C_{32}H_{35}ClN_3O_5^+$ $[M+H]^+$: 576.23, found, 576.3.

**Example 77: Preparation of 3-(5-((1-(4'-chloro-5,5-dimethyl-3,4,5,6-tetrahydro-[1,1'-biphenyl]-2-carbonyl)piperidin-4-yl)oxy)-1-oxoisoindolin-2-yl)piperidine-2,6-dione (GT-06192)**

The target compound (GT-06192) was prepared as a white solid (27 mg, yield: 33%) according to the method described in synthetic method III. $^1$H NMR (400 MHz, DMSO-d6) δ 10.96 (s, 1H), 7.59 (d, J = 8.4 Hz, 1H), 7.41 (d, J = 6.4 Hz, 2H), 7.28 (d, J = 6.4 Hz, 2H), 7.18 - 7.10 (m, 1H), 7.05 - 6.95 (m, 1H), 5.06 (dd, J = 13.3, 4.9 Hz, 1H), 4.54 (s, 1H), 4.36 (d, J = 17.2 Hz, 1H), 4.23 (d, J = 17.1 Hz, 1H), 3.90 - 3.87 (m, 1H), 3.73 - 3.62 (m, 2H), 2.97 - 2.82 (m, 2H), 2.59 (d, J = 17.8 Hz, 2H), 2.46 - 2.34 (m, 3H), 2.18 - 2.03 (m, 1H), 2.03 - 1.81 (m, 3H), 1.69 - 1.56 (m, 1H), 1.56 - 1.41 (m, 3H), 1.00 (s, 3H), 0.97(s, 3H). LCMS (ESI) calcd for $C_{33}H_{37}ClN_3O_5^+$ $[M+H]^+$: 590.24, found, 590.3.

**Example 78: Preparation of 3-(5-((1-(4'-chloro-5,5-dimethyl-3,4,5,6-tetrahydro-[1,1'-biphenyl]-2-carbonyl)azepan-4-yl)oxy)-1-oxoisoindolin-2-yl)piperidine-2,6-dione (GT-07018)**

The target compound (GT-07018) was prepared as a white solid (23 mg, yield: 35%) according to the method described in synthetic method III. $^1$H NMR (400 MHz, DMSO-d6) δ 10.98 (s, 1H), 7.63 - 7.59 (m, 1H), 7.46 - 7.26 (m, 4H), 7.08 - 7.02 (m, 1H), 6.98 - 6.92 (m, 1H), 5.07 (dd, J = 13.2, 5.0 Hz, 1H), 4.38 (d, J = 17.3 Hz, 1H), 4.33 - 4.15 (m, 1H), 3.29 - 3.22 (m, 2H), 3.12 - 3.03 (m, 2H), 3.01 - 2.81 (m, 1H), 2.59 (d, J = 16.2 Hz, 1H), 2.47 - 2.29 (m, 3H), 2.21 - 2.11 (m, 1H), 2.01 - 1.88(m, 2H), 1.84 - 1.67 (m, 2H), 1.67 - 1.51 (m, 2H), 1.51 - 1.31 (m, 3H), 1.18 - 1.12 (m, 1H), 1.01 (s, 3H), 0.98(s, 3H). LCMS

(ESI) calcd for $C_{34}H_{39}ClN_3O_5^+$ [M+H]$^+$: 604.26, found, 604.3.

**Example 79: 3-(5-(((3aS,6aS)-2-(4'-chloro-5,5-dimethyl-3,4,5,6-tetrahydro-[1,1'-biphenyl]-2-carbonyl)octahydrocyclopenta[c]pyrrol-5-yl)oxy)-1-oxoisoindolin-2-yl)piperidine-2,6-dione (GT-07025) Preparation of**

The target compound (GT-07025) was prepared as a white solid (22 mg, yield: 34%) according to the method described in synthetic method III. $^1H$ NMR (400 MHz, DMSO-d6) δ 10.91 (s, 1H), 7.54 (d, J = 8.4 Hz, 1H), 7.27 (d, J = 8.4 Hz, 2H), 7.21 (d, J = 8.5 Hz, 2H), 6.96 (s, 1H), 6.83 - 6.81 (m, 1H), 5.00 (dd, J = 13.4, 5.0 Hz, 1H), 4.74 (s, 1H), 4.31 (d, J = 17.2 Hz, 1H), 4.19 (d, J = 17.2 Hz, 1H), 3.27 - 2.96 (m, 4H), 2.92 - 2.64 (m, 3H), 2.52 (d, J = 17.1 Hz, 1H), 2.40 - 2.25 (m, 3H), 2.25 - 2.13 (m, 2H), 2.13 - 1.97 (m, 3H), 1.92 - 1.88 (m, 2H), 1.35 (t, J = 6.2 Hz, 2H), 0.91 (s, 6H), 0.90(s, 3H). LCMS (ESI) calcd for $C_{35}H_{39}ClN_3O_5^+$ [M+H]$^+$: 616.26, found, 616.3.

**Example 80: Preparation of 3-(5-(((3aR,6aS)-2-(4'-chloro-5,5-dimethyl-3,4,5,6-tetrahydro-[1,1'-biphenyl]-2-carbonyl)octahydrocyclopenta[c]pyrrol-5-yl)oxy)-1-oxoisoindolin-2-yl)piperidine-2,6-dione (GT-07019)**

The target compound (GT-07019) was prepared as a white solid (11 mg, yield: 17%) according to the method described in synthetic method III. $^1H$ NMR (400 MHz, DMSO-d6) δ 10.90 (s, 1H), 7.53 (d, J = 8.7 Hz, 1H), 7.34 (d, J = 8.4 Hz, 2H), 7.23 (d, J = 8.4 Hz, 2H), 6.94 (s, 1H), 6.84 (d, J = 8.7 Hz, 1H), 5.00 (dd, J = 13.3, 5.1 Hz, 1H), 4.78 - 4.46 (m, 1H), 4.32 (d, J = 17.2 Hz, 1H), 4.19 (d, J = 17.3 Hz, 1H), 3.21 - 3.15 (m, 2H), 3.06 - 2.99 (m, 2H), 2.92 - 2.77 (m, 2H), 2.77 - 2.62 (m, 1H), 2.54 - 2.50 (m, 3H), 2.38 - 2.25 (m, 2H), 2.20 - 2.08 (m, 3H), 1.94 - 1.90 (m, 1H), 1.86 - 1.62 (m, 2H), 1.37 (t, J = 6.2 Hz, 2H), 0.92 (s, 3H), 0.91(s, 3H). LCMS (ESI) calcd for $C_{35}H_{39}ClN_3O_5^+$ [M+H]$^+$: 616.26, found, 616.3.

**Example 81: Preparation of 3-(5-((7-(4'-chloro-5,5-dimethyl-3,4,5,6-tetrahydro-[1,1'-biphenyl]-2-carbonyl)-7-azaspiro[3.5]nonan-2-yl)oxy)-1-oxoisoindolin-2-yl)piperidine-2,6-dione (GT-07020)**

The target compound (GT-07020) was prepared as a white solid (42 mg, yield: 66%) according to the method described in synthetic method III. $^1H$ NMR (400 MHz, DMSO-d6) δ 10.89 (s, 1H), 7.52 (dd, J = 8.4, 1.3 Hz, 1H), 7.30 (dd, J = 8.4, 5.9 Hz, 2H), 7.19 (dd, J = 8.5, 6.6 Hz, 2H), 6.93 (d, J = 5.1 Hz, 1H), 6.85 (d, J = 8.4 Hz, 1H), 4.99 (dd, J = 13.3, 5.1 Hz, 1H), 4.76 - 4.60 (m, 1H), 4.29 (d, J = 17.2 Hz, 1H), 4.16 (d, J = 17.2 Hz, 1H), 3.50 - 3.33 (m, 2H), 3.17 - 2.91 (m, 3H), 2.88 - 2.78 (m, 1H), 2.52 (d, J = 16.7 Hz, 1H), 2.40 - 2.23 (m, 4H), 2.09 - 1.98 (m, 2H), 1.95 - 1.79 (m, 2H), 1.71 - 1.62 (m, 2H), 1.45 - 1.31 (m, 3H), 1.27 - 1.15 (m, 1H), 1.15 - 1.02 (m, 1H), 0.92 (s, 3H), 0.90(s, 3H). LCMS (ESI) calcd for $C_{36}H_{41}ClN_3O_5^+$ [M+H]$^+$: 630.27, found, 630.3.

**Example 82: Preparation of 3-(5-((1-((4'-chloro-[1,1'-biphenyl]-2-yl)methyl)pyrrolidin-3-yl)oxy)-1-oxoisoindolin-2-yl)piperidine-2,6-dione (GT-06817)**

The target compound (GT-06817) was prepared as a white solid (40 mg, yield: 65%) according to the method described in synthetic method I. $^1H$ NMR (400 MHz, DMSO-d6) δ 11.58 (s, 1H), 10.99 (s, 1H), 8.04 (d, J = 7.0 Hz, 1H), 7.64 (t, J = 8.0 Hz, 1H), 7.61 - 7.45 (m, 5H), 7.42 (d, J = 8.3 Hz, 1H), 7.36 - 7.33 (m, 3H), 5.10 - 5.06 (m, 2H), 4.52 - 4.43 (m, 1H), 4.49 - 4.35 (m, 2H), 4.29 - 6.26 (m, 1H), 3.83 - 3.71 (m, 1H), 3.61 - 3.41 (m, 1H), 3.19 - 3.16 (m, 1H), 2.97 - 2.88 (m, 2H), 2.61 (d, J = 17.5 Hz, 1H), 2.46 - 2.37 (m, 1H), 2.30 - 2.14 (m, 1H), 2.06 - 1.91 (m, 2H). LCMS (ESI) calcd for $C_{30}H_{29}ClN_3O_4^+$ [M+H]$^+$: 530.18, found, 530.2.

**Example 83: Preparation of 3-(5-((1-((4'-chloro-[1,1'-biphenyl]-2-yl)methyl)piperidin-4-yl)oxy)-1-oxoisoindolin-2-yl)piperidine-2,6-dione (GT-06193)**

The target compound (GT-06193) was prepared as a white solid (52 mg, yield: 68%) according to the method described in synthetic method I. $^1H$ NMR (400 MHz, DMSO-d6) δ 10.89 (s, 1H), 8.00 - 7.95 (m, 1H), 7.55 - 7.47 (m, 5H), 7.33 (d, J = 8.3 Hz, 2H), 7.28 - 7.25 (m, 1H), 7.09 (s, 1H), 6.97 - 6.94 (m, 1H), 5.01 - 4.97 (m, 1H), 4.33 - 4.12 (m, 4H), 3.19 (d, J = 12.2 Hz, 1H), 3.07 (d, J = 10.1 Hz, 1H), 2.93 - 2.87 (m, 1H), 2.86 - 2.68 (m, 3H), 2.52 (d, J = 16.3 Hz, 1H), 2.38 - 2.24 (m, 1H), 2.20 - 2.08 (m, 1H), 2.08 - 1.98 (m, 1H), 1.98 - 1.79 (m, 3H). LCMS (ESI) calcd for $C_{31}H_{31}ClN_3O_4^+$ [M+H]$^+$: 544.20, found, 544.2.

**Example 84: Preparation of 3-(5-((1-((4'-chloro-[1,1'-biphenyl]-2-yl)methyl)azepan-4-yl)oxy)-1-oxoisoindolin-2-yl)piperidine-2,6-dione (GT-06934)**

The target compound (GT-06934) was prepared as a white solid (30 mg, yield: 50%) according to the method described in synthetic method I. $^1H$ NMR (400 MHz, DMSO-d6) δ 10.89 (s, 1H), 8.01 - 7.94 (m, 1H), 7.54 (dd, J = 8.4, 2.3 Hz,

1H), 7.51 - 7.43 (m, 4H), 7.38 - 7.29 (m, 2H), 7.27 - 7.25 (m, 1H), 7.04 (s, 1H), 6.97 - 6.82 (m, 1H), 5.00 (dd, J = 13.2, 5.0 Hz, 1H), 4.66 (d, J = 30.3 Hz, 1H), 4.32 - 4.15 (m, 4H), 3.06 - 2.98 (m, 1H), 2.94 - 2.73 (m, 3H), 2.52 (d, J = 16.9 Hz, 1H), 2.36 - 2.26 (m, 1H), 2.16 - 1.69 (m, 5H), 1.66 - 1.58 (m, 2H), 1.54 - 1.47 (m, 1H). LCMS (ESI) calcd for $C_{32}H_{33}ClN_3O_4^+$ $[M+H]^+$: 558.22, found, 558.2.

**Example** 85: **Preparation** of **3-(5-((2-((4'-chloro-[1,1'-biphenyl]-2-yl)methyl)octahydrocyclopenta[c]pyrrol-5-yl) oxy)-1-oxoisoindolin-2-yl)piperidine-2,6-dione (GT-06953)**

The target compound (GT-06953) was prepared as a white solid (44 mg, yield: 59%) according to the method described in synthetic method I. $^1$H NMR (400 MHz, DMSO-d6) δ 10.92 (s, 1H), 7.84 - 7.82 (m, 1H), 7.54 (m, 3H), 7.50 - 7.45 (m, 3H), 7.36 - 7.33 (m, 3H), 7.32 - 7.27 (m, 1H), 5.02 (dd, J = 13.3, 5.1 Hz, 1H), 4.99 - 4.94 (m, 1H), 4.33 - 4.20 (m, 4H), 3.46 - 3.34 (m, 2H), 3.16 - 2.98 (m, 1H), 2.94 - 2.78 (m, 3H), 2.54 (d, J = 16.3 Hz, 2H), 2.41 - 2.28 (m, 3H), 1.94 - 1.81 (m, 3H). LCMS (ESI) calcd for $C_{33}H_{33}ClN_3O_4^+$ $[M+H]^+$: 570.22, found, 570.2.

**Example 86: Preparation of 3-(5-((1-(4'-chloro-[1,1'-biphenyl]-2-carbonyl)azetidin-3-yl)oxy)-1-oxoisoindolin-2-yl)piperidine-2,6-dione (GT-06183)**

The target compound (GT-06183) was prepared as a white solid (37 mg, yield: 46%) according to the method described in synthetic method I. $^1$H NMR (400 MHz, MeOD) δ 7.60 (d, *J* = 8.9 Hz, 1H), 7.50 - 7.44 (m, 1H), 7.42 - 7.36 (m, 3H), 7.34 (d, *J* = 1.9 Hz, 4H), 6.78 - 6.75 (m, 2H), 5.01 (dd, *J* = 13.3, 5.2 Hz, 1H), 4.85 - 480 (m, 1H), 4.41 - 4.24 (m, 3H), 4.00 - 3.96 (m, 1H), 3.85 - 3.74 (m, 1H), 3.46 - 3.35 (m, 1H), 2.87 - 2.74 (m, 1H), 2.73 - 2.62 (m, 1H), 2.41 - 2.32 (m, 1H), 2.12 - 2.00 (m, 1H). LCMS (ESI) calcd for $C_{29}H_{25}ClN_3O_5^+$ $[M+H]^+$: 530.15, found, 530.2.

**Example 87: Preparation of 3-(5-((1-(4'-chloro-[1,1'-biphenyl]-2-carbonyl)pyrrolidin-3-yl)oxy)-1-oxoisoindolin-2-yl)piperidine-2,6-dione (GT-06818)**

The target compound (GT-06818) was prepared as a white solid (33 mg, yield: 52%) according to the method described in synthetic method I. $^1$H NMR (400 MHz, DMSO-d6) δ 10.97 (s, 1H), 7.65 - 7.59 (m, 1H), 7.55 - 7.53 (m, 1H), 7.52 - 7.46 (m, 3H), 7.46 - 7.41 (m, 2H), 7.41 - 7.31 (m, 2H), 7.03 - 6.99 (m, 1H), 6.90 (d, J = 7.7 Hz, 1H), 5.17 - 4.90 (m, 2H), 4.45 - 4.36 (m, 1H), 4.28 (t, J = 15.8 Hz, 1H), 3.71 - 3.49 (m, 2H), 3.06 - 3.00 (m, 1H), 2.88 - 2.78 (m, 1H), 2.60 (d, J = 15.3 Hz, 1H), 2.47 - 2.30 (m, 1H), 2.13 - 1.80 (m, 3H). LCMS (ESI) calcd for $C_{30}H_{27}ClN_3O_5^+$ $[M+H]^+$: 544.16, found, 544.2.

**Example 88: Preparation of 3-(5-((1-(4'-chloro-[1,1'-biphenyl]-2-carbonyl)piperidin-4-yl)oxy)-1-oxoisoindolin-2-yl)piperidine-2,6-dione (GT-06204)**

The target compound (GT-06204) was prepared as a white solid (36 mg, yield: 46%) according to the method described in synthetic method I. $^1$H NMR (400 MHz, DMSO-d6) δ 10.89 (s, 1H), 7.52 - 7.44 (m, 4H), 7.42 - 7.36 (m, 4H), 7.31 (t, J = 7.7 Hz, 1H), 7.11 - 7.04 (m, 1H), 6.98 - 6.90 (m, 1H), 5.01 - 4.96 (m, 1H), 4.55 - 4.49 (m, 1H), 4.28 (d, J = 16.6 Hz, 1H), 4.16 (d, J = 17.3 Hz, 1H), 3.93 - 3.77 (m, 1H), 2.96 - 2.89 (m, 1H), 2.85 - 2.78 (m, 1H), 2.72 - 2.54 (m, 3H), 2.32 - 2.26 (m, 1H), 1.94 - 1.86 (m, 2H), 1.60 - 1.52 (m, 1H), 1.39 - 1.30 (m, 1H), 1.24 - 1.17 (m, 1H). LCMS (ESI) calcd for $C_{31}H_{29}ClN_3O_5^+$ $[M+H]^+$: 558.18, found, 558.3.

**Example 89: Preparation of 3-(5-((1-(4'-chloro-[1,1'-biphenyl]-2-carbonyl)azepan-4-yl)oxy)-1-oxoisoindolin-2-yl)piperidine-2,6-dione (GT-06935)**

The target compound (GT-06935) was prepared as a white solid (39 mg, yield: 63%) according to the method described in synthetic method I. $^1$H NMR (400 MHz, DMSO-d6) δ 10.89 (s, 1H), 7.54 (d, J = 8.4 Hz, 1H), 7.51 - 7.39 (m, 7H), 7.31 - 7.28 (m, 1H), 7.02 (d, J = 16.4 Hz, 1H), 6.95 - 6.88 (m, 1H), 5.00 (dd, J = 13.2, 4.9 Hz, 1H), 4.64 - 3.92 (m, 4H), 3.47 - 3.39 (m, 1H), 3.12 - 2.94 (m, 1H), 2.93 - 2.71 (m, 2H), 2.52 (d, J = 16.4 Hz, 1H), 2.36 - 2.25 (m, 1H), 1.98 - 1.86 (m, 1H), 1.83 - 1.73 (m, 1H), 1.69 - 1.52 (m, 3H), 1.47 - 1.27 (m, 1H), 1.23 - 1.11 (m, 1H). LCMS (ESI) calcd for $C_{32}H_{31}ClN_3O_5^+$ $[M+H]^+$: 572.19, found, 572.2.

**Example 90: Preparation of 3-(5-((2-(4'-chloro-[1,1'-biphenyl]-2-carbonyl)octahydrocyclopenta[c]pyrrol-5-yl) oxy)-1-oxoisoindolin-2-yl)piperidine-2,6-dione (GT-06954)**

The target compound (GT-06954) was prepared as a white solid (43 mg, yield: 56%) according to the method described in synthetic method I. $^1$H NMR (400 MHz, DMSO-d6) δ 10.90 (s, 1H), 7.56 (d, J = 8.5 Hz, 1H), 7.49 - 7.41 (m, 2H), 7.41 - 7.34 (m, 5H), 7.29 (dd, J = 6.7, 5.3 Hz, 1H), 6.99 (s, 1H), 6.86 (d, J = 8.3 Hz, 1H), 5.01 (dd, J = 13.3, 5.0 Hz, 1H), 4.84 -

4.71 (m, 1H), 4.32 (d, J = 17.2 Hz, 1H), 4.20 (d, J = 17.2 Hz, 1H), 3.53 - 3.43 (m, 1H), 3.22 - 3.15(m, 1H), 3.04 - 2.95 (m, 1H), 2.91 - 2.77 (m, 1H), 2.71 - 2.62 (m, 1H), 2.57 - 2.50 (m, 2H), 2.39 - 2.26 (m, 2H), 2.17 - 2.10 (m, 1H), 2.08 - 2.01 (m, 1H), 1.99 - 1.85 (m, 1H), 1.48 - 1.28(m, 1H), 1.16 - 1.03 (m, 1H). LCMS (ESI) calcd for $C_{33}H_{31}ClN_3O_5^+$ [M+H]$^+$: 584.19, found, 584.2.

**Example 91: Preparation of 3-(5-((1-((4'-chloro-[1,1'-biphenyl]-3-yl)methyl)azetidin-3-yl)oxy)-1-oxoisoindolin-2-yl)piperidine-2,6-dione (GT-06227)**

The target compound (GT-06227) was prepared as a white solid (17 mg, yield: 22%) according to the method described in synthetic method I. $^1$H NMR (400 MHz, DMSO-d6) δ 10.90 (s, 1H), 7.75 - 7.65 (m, 4H), 7.60 (d, J = 8.4 Hz, 1H), 7.50 - 7.44 (m, 4H), 7.03 (bts, 1H), 6.97 - 6.94 (m, 1H), 5.00 (dd, J = 13.3, 5.0 Hz, 1H), 4.49 - 4.41 (m, 3H), 4.35 - 4.29 (m, 2H), 4.26 - 4.17 (m, 2H), 4.14 - 3.99 (m, 2H), 2.90 - 2.77 (m, 1H), 2.54 - 2.49 (m, 1H), 2.38 - 2.24 (m, 1H), 1.97 - 1.85 (m, 1H). LCMS (ESI) calcd for $C_{20}H_{27}ClN_3O_4^+$ [M+H]$^+$: 516.17, found, 516.2.

**Example 92: Preparation of 3-(5-((1-((4'-chloro-[1,1'-biphenyl]-4-yl)methyl)azetidin-3-yl)oxy)-1-oxoisoindolin-2-yl)piperidine-2,6-dione (GT-06230)**

The target compound (GT-06230) was prepared as a white solid (16 mg, yield: 20%) according to the method described in synthetic method I. $^1$H NMR (400 MHz, DMSO-d6) δ 10.90 (s, 1H), 7.70 - 7.65 (m, 4H), 7.62 - 7.57 (m, 3H), 7.47 (dd, J = 9.0, 2.4 Hz, 2H), 7.03 (s, 1H), 6.96 (dd, J = 8.4, 2.1 Hz, 1H), 5.01 (dd, J = 13.3, 5.0 Hz, 1H), 4.53 - 4.22 (m, 7H), 4.06 (brs, 2H), 2.90 - 2.77 (m, 1H), 2.55 - 2.50 (m, 1H), 2.33 - 2.29 (m, 1H), 1.99 - 1.85 (m, 1H). LCMS (ESI) calcd for $C_{29}H_{27}ClN_3O_4^+$ [M+H]$^+$: 516.17, found, 516.2.

**Example 93: Preparation of 3-(5-((1-([2,4'-bipyridin]-5-ylmethyl)azetidin-3-yl)oxy)-1-oxoisoindolin-2-yl)piperidine-2,6-dione (GT-06809)**

The target compound (GT-06809) was prepared as a white solid (18 mg, yield: 30%) according to the method described in synthetic method II. $^1$H NMR (400 MHz, DMSO-d6) δ 10.98 (s, 1H), 9.04 (s, 1H), 8.99 (d, J = 6.3 Hz, 2H), 8.58 (d, J = 6.3 Hz, 2H), 8.45 (d, J = 8.2 Hz, 1H), 8.36 (d, J = 7.4 Hz, 1H), 7.67 (d, J = 8.4 Hz, 1H), 7.12 (s, 1H), 7.04 (d, J = 8.2 Hz, 1H), 5.08 (dd, J = 13.3, 5.0 Hz, 1H), 4.78 - 4.56 (m, 4H), 4.42 - 4.45 (m, 5H), 3.02 - 2.82 (m, 1H), 2.60 (d, J = 16.9 Hz, 1H), 2.41 - 2.37 (m, 1H), 2.03 - 1.97 (m, 1H). LCMS (ESI) calcd for $C_{27}H_{26}N_5O_4^+$ [M+H]$^+$: 484.20, found, 484.2.

**Example 94: Preparation of 3-(1-oxo-5-((1-((5-phenylpyrazin-2-yl)methyl)azetidin-3-yl)oxy)isoindolin-2-yl)piperidine-2,6-dione (GT-06353)**

The target compound (GT-06353) was prepared as a white solid (33 mg, yield: 56%) according to the method described in synthetic method II. $^1$H NMR (400 MHz, DMSO-d6) δ 10.98 (s, 1H), 9.30 (d, J = 1.3 Hz, 1H), 8.86 (d, J = 1.3 Hz, 1H), 8.18 (dd, J = 7.9, 1.6 Hz, 2H), 7.62 - 7.50 (m, 4H), 7.12 (d, J = 1.8 Hz, 1H), 7.03 (dd, J = 8.4, 2.2 Hz, 1H), 5.22 (brs, 1H), 5.08 (dd, J = 13.3, 5.1 Hz, 1H), 4.74 - 4.69 (m, 4H), 4.41 (dd, J = 17.4, 8.6 Hz, 1H), 4.35 - 4.20 (m, 3H), 2.99 - 2.85 (m, 1H), 2.60 (d, J = 16.6 Hz, 1H), 2.44 - 2.33 (m, 1H), 2.04 - 1.96 (m, 1H). LCMS (ESI) calcd for $C_{27}H_{26}N_5O_4^+$ [M+H]$^+$: 484.20, found, 484.2.

**Example 95: Preparation of 3-(5-((1-((5-(furan-2-yl)thiophen-2-yl)methyl)azetidin-3-yl)oxy)-1-oxoisoindolin-2-yl)piperidine-2,6-dione (GT-06562)**

The target compound (GT-06562) was prepared as a white solid (18 mg, yield: 31%) according to the method described in synthetic method II. $^1$H NMR (400 MHz, DMSO-d6) δ 10.90 (s, 1H), 7.67 (d, J = 1.4 Hz, 1H), 7.60 (d, J = 8.4 Hz, 1H), 7.28 (dd, J = 10.5, 3.6 Hz, 2H), 7.03 (s, 1H), 6.96 (dd, J = 8.4, 2.2 Hz, 1H), 6.75 (d, J = 3.3 Hz, 1H), 6.54 (dd, J = 3.4, 1.8 Hz, 1H), 5.17 - 5.07 (m, 1H), 5.01 (dd, J = 13.3, 5.1 Hz, 2H), 4.60 (brs, 2H), 4.49 - 4.43 (m, 2H), 4.32 (d, J = 17.6 Hz, 2H), 4.20 (d, J = 17.4 Hz, 1H), 4.10 - 4.04 (m, 2H), 2.90 - 2.76 (m, 1H), 2.53 (d, J = 16.5 Hz, 1H), 2.34 - 2.27(m, 1H), 1.97 - 1.84 (m, 1H). LCMS (ESI) calcd for $C_{25}H_{24}N_3O_5S^+$ [M+H]$^+$: 478.14, found, 478.1.

**Example 96: Preparation of 3-(5-((1-((4'-fluoro-5,5-dimethyl-3,4,5,6-tetrahydro-[1,1'-biphenyl]-2-yl)methyl)piperidin-4-yl)oxy)-1-oxoisoindolin-2-yl)piperidine-2,6-dione (GT-07062)**

The target compound (GT-07062) was prepared as a white solid (7 mg, yield: 10%) according to the method described in synthetic method I. $^1$H NMR (400 MHz, DMSO-d6) δ 10.97 (s, 1H), 7.66 - 7.60 (m, 1H), 7.30 - 7.21 (m, 2H), 7.21 - 7.15 (m, 3H), 7.09 - 7.02 (m, 1H), 5.14 - 5.02 (m, 1H), 4.82 - 4.63 (m, 1H), 4.37 (d, J = 16.9 Hz, 1H), 4.25 (d, J = 17.4 Hz, 1H), 3.62 - 3.55 (m, 3H), 2.95 - 2.88 (m, 1H), 2.82 - 2.74 (m, 2H), 2.62 - 2.57 (m, 3H), 2.36 - 2.31 (m, 2H), 2.25 - 2.16 (m, 2H), 2.05

(s, 2H), 2.02 - 1.92 (m, 3H), 1.52 - 1.47 (m, 2H), 0.97 (s, 6H). LCMS (ESI) calcd for $C_{33}H_{39}FN_3O_4^+$ [M+H]+: 560.29, found, 560.3.

**Example 97: Preparation of 3-(5-((1-((4'-chloro-[1,1'-biphenyl]-3-yl)methyl)piperidin-4-yl)oxy)-1-oxoisoindolin-2-yl)piperidine-2,6-dione (GT-06228)**

The target compound (GT-06228) was prepared as a white solid (12 mg, yield: 16%) according to the method described in synthetic method I. $^1$H NMR (400 MHz, DMSO-d6) δ 10.90 (s, 1H), 7.96 - 7.93 (m, 1H), 7.75 - 7.67 (m, 4H), 7.51 - 7.46 (m, 4H), 7.22 - 7.13 (m, 1H), 7.09 - 7.03 (m, 1H), 5.00 (dd, J = 13.2, 5.0 Hz, 1H), 4.42 - 4.16 (m, 5H), 3.24 - 3.10 (m, 3H), 3.08 - 2.96 (m, 1H), 2.90 - 2.76 (m, 1H), 2.53 (d, J = 17.5 Hz, 1H), 2.34 - 2.29 (m, 1H), 2.23 - 2.13 (m, 2H), 2.06 - 2.00(m, 1H), 1.99 - 1.84 (m, 2H). LCMS (ESI) calcd for $C_{31}H_{31}ClN_3O_4^+$ [M+H]+: 544.20, found, 544.2.

**Example 98: Preparation of 3-(5-((1-((4'-fluoro-3,4,5,6-tetrahydro-[1,1'-biphenyl]-2-yl)methyl)azetidin-3-yl) oxy)-1-oxoisoindolin-2-yl)piperidine-2,6-dione (GT-06302)**

The target compound (GT-06302) was prepared as a white solid (15 mg, yield: 24%) according to the method described in synthetic method I. $^1$H NMR (400 MHz, DMSO-d6) δ 10.98 (s, 1H), 7.70 - 7.64 (m, 1H), 7.45 - 7.30 (m, 1H), 7.26 - 7.11 (m, 5H), 5.20 - 4.96 (m, 2H), 4.72 - 4.61 (m, 1H), 4.42 - 4.21 (m, 3H), 3.83 - 3.69 (m, 2H), 2.95 - 2.86 (m, 1H), 2.74 - 2.62 (m, 3H), 2.33 - 2.22 (m, 3H), 2.19 - 2.11 (m, 2H), 2.07 - 1.92 (m, 2H), 1.67 (s, 3H). LCMS (ESI) calcd for $C_{29}H_{31}FN_3O_4^+$ [M+H]+: 504.23, found, 504.2.

**Example 99: Preparation of 3-(5-((1-((4-(4-fluorophenyl)-5,6-dihydro-2H-pyran-3-yl)methyl)azetidin-3-yl)oxy)-1-oxoisoindolin-2-yl)piperidine-2,6-dione (GT-06560)**

The target compound (GT-06560) was prepared as a white solid (20 mg, yield: 32%) according to the method described in synthetic method I. $^1$H NMR (400 MHz, DMSO-d6) δ 10.91 (s, 1H), 7.65 - 7.53 (m, 1H), 7.32 - 7.17 (m, 3H), 7.12 - 7.02 (m, 2H), 6.95 - 6.84 (m, 1H), 5.02 (dd, J = 13.3, 5.1 Hz, 1H), 4.93 - 4.90 (m, 1H), 4.5863 - 4.54(m, 1H), 4.32 (d, J = 17.5 Hz, 1H), 4.22 (d, J = 17.5 Hz, 1H), 4.18 - 4.15 (m, 3H), 3.90 - 3.60 (m, 5H), 2.91 - 2.77 (m, 1H), 2.53 (d, J = 16.8 Hz, 1H), 2.46 - 2.44 (m, 1H), 2.39 - 2.25 (m, 3H), 1.99 - 1.86 (m, 1H). LCMS (ESI) calcd for $C_{28}H_{29}FN_3O_5^+$ [M+H]+: 506.21, found, 506.2.

**Example 100: Preparation of 3-(5-((1-((4'-chloro-4,4-difluoro-3,4,5,6-tetrahydro-[1,1'-biphenyl]-2-yl)methyl)azetidin-3-yl)oxy)-1-oxoisoindolin-2-yl)piperidine-2,6-dione (GT-06563)**

The target compound (GT-06563) was prepared as a white solid (15 mg, yield: 22%) according to the method described in synthetic method I. $^1$H NMR (400 MHz, DMSO-d6) δ 11.06 (s, 1H), 10.91 (s, 1H), 7.61 - 7.56 (m, 1H), 7.49 - 7.41 (m, 1H), 7.34 (d, J = 8.1 Hz, 1H), 7.26 - 7.11 (m, 2H), 7.00 - 6.81 (m, 1H), 6.74 (d, J = 7.4 Hz, 1H), 5.01 (dd, J = 13.3, 5.1 Hz, 1H), 4.92 - 4.88(m, 1H), 4.68 - 4.49 (m, 1H), 4.35 - 4.18 (m, 3H), 3.83 - 3.61 (m, 3H), 2.91 - 2.68 (m, 3H), 2.53 (d, J = 16.7 Hz, 1H), 2.46 - 2.44(m, 2H), 2.37 - 2.28 (m, 1H), 2.17 - 2.00 (m, 2H), 1.94 - 1.89 (m, 1H). LCMS (ESI) calcd for $C_{29}H_{29}ClF_2N_3O_4^+$ [M+H]+: 556.18, found, 556.2.

**Example 101: Preparation of 3-(5-((1-((4'-chloro-4-methoxy-4-methyl-3,4,5,6-tetrahydro-[1,1'-biphenyl]-2-yl) methyl)azetidin-3-yl)oxy)-1-oxoisoindolin-2-yl)piperidine-2,6-dione (GT-07065)**

The target compound (GT-07065) was prepared as a white solid (12 mg, yield: 18%) according to the method described in synthetic method I. $^1$H NMR (400 MHz, DMSO-d6) δ 10.98 (s, 1H), 7.64 (d, J = 8.4 Hz, 1H), 7.49 (d, J = 8.4 Hz, 1H), 7.38 (d, J = 8.4 Hz, 2H), 7.26 (d, J = 8.4 Hz, 1H), 7.19 (d, J = 8.4 Hz, 2H), 5.09 (dd, J = 13.2, 5.1 Hz, 1H), 5.04 - 4.95 (m, 1H), 4.71 - 4.56 (m, 1H), 4.40 (d, J = 17.6 Hz, 1H), 4.29 (d, J = 17.6 Hz, 1H), 4.23 - 4.18 (m, 1H), 3.85 - 3.66 (m, 3H), 3.17 (s, 3H), 2.98 - 2.85 (m, 1H), 2.60 (d, J = 17.4 Hz, 1H), 2.55 - 2.51 (m, 1H), 2.45 - 2.32 (m, 2H), 2.32 - 2.17 (m, 3H), 2.02 - 1.98 (m, 1H), 1.81 - 1.76 (m, 1H), 1.67 - 1.60 (m, 1H), 1.20 (s, 3H). LCMS (ESI) calcd for $C_{31}H_{35}ClN_3O_5^+$ [M+H]+: 564.23, found, 564.3.

**Example 102: Preparation of 3-(5-((1-((4-(4-chlorophenyl)-6,6-dimethyl-5,6-dihydro-2H-pyran-3-yl)methyl)azetidin-3-yl)oxy)-1-oxoisoindolin-2-yl)piperidine-2,6-dione (GT-06561)**

The target compound (GT-06561) was prepared as a white solid (21 mg, yield: 31%) according to the method described in synthetic method I. $^1$H NMR (400 MHz, DMSO-d6) δ 10.98 (s, 1H), 7.69 - 7.60 (m, 1H), 7.51 - 7.47 (m, 1H), 7.46 - 7.36 (m, 2H), 7.36 - 7.21 (m, 2H), 7.00 - 6.94 (m, 1H), 5.13 - 5.08 (m, 2H), 4.97 (brs, 1H), 4.72 - 4.60 (m, 1H), 4.44 - 4.17 (m, 5H), 3.98 - 3.81 (m, 2H), 3.78 - 3.72 (m, 1H), 2.96 - 2.87 (m, 1H), 2.65 - 2.57 (m, 1H), 2.46 - 2.35 (m, 1H), 2.24 (s, 2H), 2.04 -

1.98 (m, 1H), 1.22 (s, 6H). LCMS (ESI) calcd for $C_{30}H_{33}ClN_3O_5^+$ [M+H]$^+$: 550.21, found, 550.2.

**Example 103: Preparation of 3-(5-((1-benzhydrylazetidin-3-yl)oxy)-1-oxoisoindolin-2-yl)piperidine-2,6-dione (GT-06185)**

The target compound (GT-06185) was prepared as a white solid (21 mg, yield: 29%) according to the method described in synthetic method II. $^1$H NMR (400 MHz, DMSO-d6) δ 10.89 (s, 1H), 7.66 - 7.53 (m, 5H), 7.43 - 7.33 (m, 6H), 7.05 - 6.92 (m, 2H), 6.00 - 5.82 (m, 1H), 5.04 - 4.96 (m, 1H), 4.62 - 4.39 (m, 2H), 4.36 - 4.04 (m, 5H), 2.87 - 2.75 (m, 1H), 2.51 (d, J = 18.4 Hz, 1H), 2.34 - 2.23 (m, 1H), 1.94 - 1.86 (m, 1H). LCMS (ESI) calcd for $C_{29}H_{28}N_3O_4^+$ [M+H]$^+$: 482.21, found, 482.2.

**Example 104: Preparation of 3-(5-((1-benzhydrylpyrrolidin-3-yl)oxy)-1-oxoisoindolin-2-yl)piperidine-2,6-dione (GT-06819)**

The target compound (GT-06819) was prepared as a white solid (16 mg, yield: 28%) according to the method described in synthetic method II. $^1$H NMR (400 MHz, DMSO-d6) δ 10.90 (s, 1H), 7.93 - 7.68 (m, 4H), 7.58 (t, J = 7.6 Hz, 1H), 7.41 - 7.30 (m, 7H), 7.16 (d, J = 27.1 Hz, 1H), 5.70 - 5.62 (m, 1H), 5.22 - 5.18 (m, 1H), 5.00 (dd, J = 13.2, 4.5 Hz, 1H), 4.32 (d, J = 17.6 Hz, 1H), 4.20 (d, J = 17.2 Hz, 1H), 3.95 - 3.76 (m, 1H), 3.60 - 3.42 (m, 1H), 3.24 - 3.14 (m, 1H), 3.09 - 3.04 (m, 1H), 2.92 - 2.77 (m, 1H), 2.74 - 2.58 (m, 1H), 2.52 (d, J = 17.4 Hz, 1H), 2.35 - 2.26 (m, 1H), 2.25 - 2.07 (m, 1H), 1.92 - 1.86 (m, 1H). LCMS (ESI) calcd for $C_{30}H_{30}N_3O_4^+$ [M+H]$^+$: 496.22, found, 496.2.

**Example 105: Preparation of 3-(5-((1-benzhydrylpiperidin-4-yl)oxy)-1-oxoisoindolin-2-yl)piperidine-2,6-dione (GT-06195)**

The target compound (GT-06195) was prepared as a white solid (15 mg, yield: 21%) according to the method described in synthetic method II. $^1$H NMR (400 MHz, DMSO-d6) δ 10.89 (s, 1H), 7.87 - 7.79 (m, 4H), 7.64 - 7.53 (m, 1H), 7.44 - 7.36 (m, 4H), 7.32 - 7.28 (m, 2H), 7.18 (d, J = 16.3 Hz, 1H), 7.13 - 7.00 (m, 1H), 5.02 - 4.97 (m, 1H), 4.84 (s, 1H), 4.32 (d, J = 17.0 Hz, 1H), 4.20 (d, J = 17.0 Hz, 1H), 3.04 - 3.03 (m, 3H), 2.91 - 2.77 (m, 1H), 2.63 - 2.49 (m, 4H), 2.35 - 2.29 (m, 2H), 2.21 - 2.12 (m, 1H), 2.05 - 1.99 (m, 1H), 1.96 - 1.83 (m, 1H). LCMS (ESI) calcd for $C_{31}H_{32}N_3O_4^+$ [M+H]$^+$: 510.24, found, 510.3.

**Example 106: Preparation of 3-(5-((1-benzhydrylazepan-4-yl)oxy)-1-oxoisoindolin-2-yl)piperidine-2,6-dione (GT-06928)**

The target compound (GT-06928) was prepared as a white solid (12 mg, yield: 26%) according to the method described in synthetic method II. $^1$H NMR (400 MHz, DMSO-d6) δ 11.82 (s, 1H), 10.89 (s, 1H), 7.89 - 7.77 (m, 4H), 7.54 (dd, J = 8.4, 1.9 Hz, 1H), 7.41 - 7.34 (m, 4H), 7.32 - 7.23 (m, 2H), 7.12 (d, J = 12.1 Hz, 1H), 7.03 - 6.95 (m, 1H), 5.70 - 5.62 (m, 1H), 4.99 (dd, J = 13.4, 4.9 Hz, 1H), 4.86 - 4.68 (m, 1H), 4.30 (dd, J = 17.3, 4.1 Hz, 1H), 4.18 (d, J = 17.2 Hz, 1H), 3.48 (q, J = 7.1 Hz, 1H), 3.08 - 2.91 (m, 2H), 2.93 - 2.77 (m, 1H), 2.52 (d, J = 16.7 Hz, 1H), 2.39 - 2.18 (m, 3H), 2.16 - 2.05 (m, 1H), 2.01 - 1.79 (m, 4H), 1.78 - 1.67 (m, 1H). LCMS (ESI) calcd for $C_{32}H_{34}N_3O_4^+$ [M+H]$^+$: 524.25, found, 524.3.

**Example 107: Preparation of 3-(5-((7-benzhydryl-7-azaspiro[3.5]nonan-2-yl)oxy)-1-oxoisoindolin-2-yl)piperidine-2,6-dione (GT-06956)**

The target compound (GT-06956) was prepared as a white solid (14 mg, yield: 20%) according to the method described in synthetic method II. $^1$H NMR (400 MHz, DMSO-d6) δ 10.90 (s, 1H), 7.83 - 7.71 (m, 4H), 7.55 (d, J = 8.4 Hz, 1H), 7.41 - 7.36 (m, 4H), 7.31 - 7.20 (m, 2H), 6.98 (s, 1H), 6.91 - 6.88 (m, 1H), 5.54 (d, J = 9.3 Hz, 1H), 5.00 (dd, J = 13.2, 5.0 Hz, 1H), 4.84 - 4.66 (m, 1H), 4.30 (d, J = 17.3 Hz, 1H), 4.18 (d, J = 17.3 Hz, 1H), 3.06 - 2.94 (m, 3H), 2.91 - 2.79 (m, 2H), 2.55 - 2.50 (m, 2H), 2.38 - 2.23 (m, 2H), 2.07 - 1.99 (m, 2H), 1.92 - 1.83 (m, 5H). LCMS (ESI) calcd for $C_{34}H_{36}N_3O_4^+$ [M+H]$^+$: 550.27, found, 550.3.

**Example 108: Preparation of 3-(5-((2-benzhydryloctahydrocyclopenta[c]pyrrol-5-yl)oxy)-1-oxoisoindolin-2-yl) piperidine-2,6-dione (GT-06955)**

The target compound (GT-06955) was prepared as a white solid (9 mg, yield: 12%) according to the method described in synthetic method II. $^1$H NMR (400 MHz, DMSO) δ 10.90 (s, 1H), 7.77 - 7.74 (m, 4H), 7.61 (d, J = 8.4 Hz, 1H), 7.38 (t, J = 7.4 Hz, 4H), 7.32 - 7.28 (m, 2H), 7.22 (s, 1H), 7.16 - 7.07 (m, 1H), 5.10 - 4.95 (m, 2H), 4.35 (d, J = 17.3 Hz, 1H), 4.23 (d, J = 17.2 Hz, 1H), 3.48 - 3.40 (m, 2H), 3.22 - 3.17 (m, 1H), 2.97 - 2.83 (m, 4H), 2.55 - 2.49 (m, 1H), 2.40 - 2.21 (m, 2H),

2.09 - 1.99 (m, 2H), 1.93 - 1.81 (m, 3H). LCMS (ESI) calcd for $C_{33}H_{34}N_3O_4^+$ $[M+H]^+$: 536.25, found, 536.3.

**Example 109: Preparation of 3-(5-((1-(bis(2-fluorophenyl)methyl)azetidin-3-yl)oxy)-1-oxoisoindolin-2-yl)piperidine-2,6-dione (GT-06759)**

The target compound (GT-06759) was prepared as a white solid (24 mg, yield: 38%) according to the method described in synthetic method II. $^1H$ NMR (400 MHz, DMSO-d6) δ 10.96 (s, 1H), 8.01 - 7.73 (m, 1H), 7.70 - 7.56 (m, 2H), 7.55 - 7.09 (m, 7H), 7.12 - 6.93 (m, 1H), 5.36 - 4.96 (m, 2H), 4.43 - 4.22 (m, 2H), 3.99 (dd, J = 34.3, 10.9 Hz, 1H), 3.64 - 3.39 (m, 3H), 3.17 - 3.02 (m, 1H), 2.99 - 2.87 (m, 1H), 2.60 (d, J = 17.6 Hz, 1H), 2.45 - 2.30 (m, 1H), 2.08 - 1.90 (m, 1H). LCMS (ESI) calcd for $C_{29}H_{26}F_2N_3O_4^+$ $[M+H]^+$: 518.19, found, 518.2.

**Example 110: Preparation of 3-(5-((1-(bis(3-fluorophenyl)methyl)azetidin-3-yl)oxy)-1-oxoisoindolin-2-yl)piperidine-2,6-dione (GT-06352)**

The target compound (GT-06352) was prepared as a white solid (21 mg, yield: 33%) according to the method described in synthetic method II. $^1H$ NMR (400 MHz, DMSO-d6) δ 10.90 (d, J = 7.9 Hz, 1H), 7.59 - 7.55 (m, 1H), 7.49 - 7.32 (m, 6H), 7.20 - 7.08 (m, 2H), 7.00 (s, 1H), 6.94 (d, J = 8.1 Hz, 1H), 5.38 - 5.16 (m, 1H), 5.04 - 4.97 (m, 1H), 4.37 - 4.14 (m, 5H), 3.97 - 3.88 (m, 1H), 2.89 - 2.78 (m, 1H), 2.54 - 2.48 (m, 2H), 2.37 - 2.22 (m, 1H), 1.99 - 1.82 (m, 1H). LCMS (ESI) calcd for $C_{29}H_{26}F_2N_3O_4^+$ $[M+H]^+$: 518.19, found, 518.2.

**Example 111: Preparation of 3-(5-((1-(bis(4-fluorophenyl)methyl)azetidin-3-yl)oxy)-1-oxoisoindolin-2-yl)piperidine-2,6-dione (GT-06519)**

The target compound (GT-06519) was prepared as a white solid (11 mg, yield: 17%) according to the method described in synthetic method II. $^1H$ NMR (400 MHz, DMSO-d6) δ 10.98 (s, 1H), 7.95 - 7.54 (m, 5H), 7.43 - 7.21 (m, 4H), 7.16 - 6.89 (m, 2H), 6.23 - 5.88 (m, 1H), 5.19 - 4.92 (m, 2H), 4.65 - 3.49 (m, 2H), 4.35 - 4.11 (m, 4H), 2.95 - 2.86 (m, 1H), 2.34 - 2.25 (m, 2H), 2.04 - 1.91 (m, 1H). LCMS (ESI) calcd for $C_{29}H_{26}F_2N_3O_4^+$ $[M+H]^+$: 518.19, found, 518.2.

**Example 112: Preparation of 3-(5-((1-(bis(4-chlorophenyl)methyl)azetidin-3-yl)oxy)-1-oxoisoindolin-2-yl)piperidine-2,6-dione (GT-06520)**

The target compound (GT-06520) was prepared as a white solid (20 mg, yield: 30%) according to the method described in synthetic method II. $^1H$ NMR (400 MHz, DMSO-d6) δ 10.89 (s, 1H), 7.80 - 7.49 (m, 5H), 7.49 - 7.17 (m, 4H), 7.00 - 6.93 (m, 2H), 6.12 - 5.79 (m, 1H), 5.00 (dd, J = 13.3, 5.0 Hz, 1H), 4.58 - 4.46 (m, 1H), 4.29 (d, J = 17.5 Hz, 1H), 4.18 (d, J = 17.4 Hz, 1H), 4.13 - 4.00 (m, 1H), 3.29 - 3.23 (m, 3H), 2.87 - 2.78 (m, 1H), 2.52 (d, J = 16.6 Hz, 1H), 2.36 - 2.24 (m, 1H), 1.92 - 1.88 (m, 1H). LCMS (ESI) calcd for $C_{29}H_{26}Cl_2N_3O_4^+$ $[M+H]^+$: 550.13, found, 550.2.

**Example 113: Preparation of 3-(5-((1-(bis(4-methoxyphenyl)methyl)azetidin-3-yl)oxy)-1-oxoisoindolin-2-yl)piperidine-2,6-dione (GT-06959)**

The target compound (GT-06959) was prepared as a white solid (10 mg, yield: 14%) according to the method described in synthetic method II. $^1H$ NMR (400 MHz, DMSO) δ 10.89 (s, 1H), 7.58 - 7.46 (m, 4H), 7.36 - 7.15 (m, 1H), 7.02 - 6.84 (m, 6H), 5.78 - 5.70 (m, 1H), 5.02 - 4.97 (m, 1H), 4.45 - 4.37 (m, 2H), 4.30 (d, J = 17.5 Hz, 1H), 4.18 (d, J = 17.9 Hz, 1H), 4.09 - 4.01 (m, 2H), 3.68 (s, 6H), 2.92 - 2.76 (m, 1H), 2.52 (d, J = 17.2 Hz, 1H), 2.37 - 2.24 (m, 1H), 2.22 - 2.11 (m, 1H), 1.97 - 1.85 (m, 1H). LCMS (ESI) calcd for $C_{31}H_{31}N_3NaO_6^+$ $[M+H]^+$: 564.21, found, 564.2.

**Example 114: Preparation of 3-(1-oxo-5-((1-(phenyl(pyridin-2-yl)methyl)azetidin-3-yl)oxy)isoindolin-2-yl)piperidine-2,6-dione (GT-06559)**

The target compound (GT-06559) was prepared as a white solid (30 mg, yield: 51%) according to the method described in synthetic method II. $^1H$ NMR (400 MHz, DMSO-d6) δ 10.90 (s, 1H), 10.21 (s, 1H), 8.60 (s, 1H), 7.82 - 7.78 (m, 1H), 7.64 - 7.54(m, 3H), 7.48 - 7.28 (m, 6H), 7.14 - 7.10 (m, 1H), 5.25 - 5.11 (m, 1H), 5.07 - 4.93 (m, 1H), 4.48 - 4.12 (m, 3H), 4.04 - 3.87 (m, 2H), 3.21 - 3.11 (m, 2H), 2.89 - 2.78 (m, 1H), 2.53 (d, J = 17.0 Hz, 1H), 2.40 - 2.21 (m, 1H), 2.00 - 1.81 (m, 1H). LCMS (ESI) calcd for $C_{28}H_{27}N_4O_4^+$ $[M+H]^+$: 483.20, found, 483.2.

**Example 115: Preparation of 3-(1-oxo-5-((1-(phenyl(pyridin-3-yl)methyl)azetidin-3-yl)oxy)isoindolin-2-yl)piperidine-2,6-dione (GT-06760)**

The target compound (GT-06760) was prepared as a white solid (16 mg, yield: 27%) according to the method described in synthetic method II. $^1$H NMR (400 MHz, DMSO-d6) δ 10.99 (s, 1H), 9.00 (s, 1H), 8.67 (t, J = 4.8 Hz, 1H), 8.44 - 8.40 (m, 1H), 7.81 - 7.74 (m, 2H), 7.72 - 7.61 (m, 2H), 7.52 - 7.31 (m, 4H), 7.24 - 7.21 (m, 1H), 5.10 (dd, J = 13.7, 8.7 Hz, 1H), 4.52 - 4.39 (m, 2H), 4.35 - 4.27 (m, 1H), 4.13 - 3.93 (m, 2H), 3.38 - 3.21 (m, 3H), 2.97 - 2.87 (m, 1H), 2.60 (d, J = 17.4 Hz, 1H), 2.45 - 2.34 (m, 1H), 2.07 - 1.89 (m, 1H). LCMS (ESI) calcd for $C_{28}H_{27}N_4O_4^+$ [M+H]$^+$: 483.20, found, 483.2.

**Example 116: Preparation of 3-(5-((1-(di(pyridin-2-yl)methyl)azetidin-3-yl)oxy)-1-oxoisoindolin-2-yl)piperidine-2,6-dione (GT-06814)**

The target compound (GT-06814) was prepared as a white solid (19 mg, yield: 32%) according to the method described in synthetic method II. $^1$H NMR (400 MHz, DMSO-d6) δ 10.99 (s, 1H), 8.64 (s, 2H), 7.98 - 7.83 (m, 2H), 7.71 - 7.65 (m, 2H), 7.60 (d, J = 7.6 Hz, 1H), 7.51 - 7.38 (m, 2H), 7.30 (brs, 1H), 7.22 - 7.18 (m, 1H), 6.02 (brs, 1H), 5.29 (brs, 1H), 5.10 (dd, J = 13.4, 5.1 Hz, 1H), 4.45 - 4.29 (m, 2H), 4.09 - 3.91 (m, 2H), 3.00 - 2.85 (m, 2H), 2.61 (d, J = 17.2 Hz, 2H), 2.43 - 2.26 (m, 2H), 2.04 - 1.93 (m, 1H). LCMS (ESI) calcd for $C_{27}H_{26}N_5O_4^+$ [M+H]$^+$: 484.20, found, 484.2.

**Biological activity assay**

**[001019] Reagents and Materials:**

| Reagents and materials | Suppliers or Source |
|---|---|
| Human T cell acute lymphoblastic leukemia cell line: CCRF-CEM | Dalian Meilun Biotech Co., Ltd. |
| Human Burkitt's lymphoma cells: Daudi | Dalian Meilun Biotech Co., Ltd. |
| Human acute lymphoblastic leukemia cell: Kasumi-1 | ATCC (American Type Culture Collection) |
| Human breast cancer cell: MDA-MB-231 | Dalian Meilun Biotech Co., Ltd. |
| Human multiple myeloma cells: MM.1S | ATCC |
| Human mantle cell lymphoma cells: JEKO-1 | ATCC |
| Human Monocyte Leukemia cells: THP-1 | ATCC |
| Human myelomonocytic leukemia cells: MV-4-11 | ATCC |
| Human multiple myeloma cell line resistant to dexamethasone: MM.1R | ATCC |
| Human thyroid carcinoma cells: CAL-62 | ATCC |
| RPMI-1640 Medium | Dalian Meilun Biotech Co., Ltd. |
| IMDM Medium | Gibco Company |
| DMEM Medium | Dalian Meilun Biotech Co., Ltd. |
| Meilun Cell Counting Kit-8 | Dalian Meilun Biotech Co., Ltd. |
| Fetal bovine serum (FBS) | Sunrise Science Products Company |
| Penicillin-Streptomycin | Beijing TransGen Biotech Co., Ltd. |
| Mycoplasma detection kit | Beijing TransGen Biotech Co., Ltd. |
| STR genotype detection | Shanghai Biowing Applied Biotechnology Co. Ltd. |

Methods:

**Cell cultures**

The tumor cells used herein were cultured in a cell culture medium listed in table A at 37°C in an incubator with 5% $CO_2$. Prior to experimentation, all cells used were correctly identified by STR profiling, and tested negative for mycoplasma through routine screenings.

Table A. Tumor cells and cell culture medium used in the present disclosure

| Cell line | Cell culture medium used |
|---|---|
| CCRF-CEM | RPMI 1640 culture medium supplemented with 10% FBS, as well as penicillin at a final concentration of 100 U/mL and streptomycin at a final concentration of 100 μg/mL |
| Daudi | |
| Kasumi-1 | |
| MM.1S | |
| JEKO-1 | |
| THP-1 | |
| MM.1R | |
| MDA-MB-231 | DMEM culture medium supplemented with 10% FBS, as well as penicillin at a final concentration of 100 U/mL and streptomycin at a final concentration of 100 μg/mL |
| CAL-62 | |
| MV-4-11 | IMDM culture medium supplemented with 10% FBS, as well as penicillin at a final concentration of 100 U/mL and streptomycin at a final concentration of 100 μg/mL |

**I. Determination of half inhibitory concentration ($IC_{50}$) of the compounds against tumor cells:**

$IC_{50}$ values of the compounds of the present disclosure (including the compounds in Tables 1 and 2, and the Examples A1-A20 and 1-116 compounds) were measured using the Meilun Cell Counting Kit-8 kit from Dalian Meilun Biotech Co., Ltd. Assay details are as follows: tumor cells were seeded in a 96-well plate at a density listed in Table B. After 24h, 100 μL of the inoculated cells were treated with 0.5μL of the compounds of the present disclosure (including the compounds in Tables 1 and 2, and the Examples A1-A20 and 1-116 compounds) at 10 different successively decreasing concentrations (starting at the highest concentration of 10μM; 5-fold serial dilutions). After the cells were treated with the compounds of the present disclosure for a period of time, the cell viability detection reagent was added to the culture medium for cell viability assessment according to the instructions provided with the CCK-8 kit. DMSO served as the negative control, and corresponding commercial inhibitors immunomodulatory agents (including lenalidomide and pomalidomide) were used as the positive control, both of which treated the cells using the same protocol as that of the compounds of the present disclosure. The growth inhibition of the compounds of the present disclosure on cells was plotted by Prism Graphpad software, and the $IC_{50}$ values of the compounds of the present disclosure were calculated therefrom.

Table B. Seeding protocol and treatment time for tumor cells

| Cells | Cells seeding protocol | The times for treating cells with the compounds of the present disclosure (hours) |
|---|---|---|
| Kasumi-1 | cells were seeded at a density of 10,000 cells per well in 100 μL of serum-containing RPMI-1640 medium per well | 96 h |
| MM.1S | | |
| THP-1 | | |
| MM.1R | | |
| CAL-62 | cells were seeded at a density of 2,000 cells per well in 100 μL of serum-containing DMEM medium per well | 96 h |
| CCRF-CEM | cells were seeded at a density of 4,000 cells per well in 100 μL of serum-containing RPMI-1640 medium per well | 96 h |
| Daudi | | |
| JEKO-1 | | |
| MDA-MB-231 | cells were seeded at a density of 4,000 cells per well in 100 μL of serum-containing DMEM medium per well | 96 h |

(continued)

| Cells | Cells seeding protocol | The times for treating cells with the compounds of the present disclosure (hours) |
|---|---|---|
| MV-4-11 | cells were seeded at a density of 10,000 cells per well in 100 μL of serum-containing IMDM medium per well | 96 h |

Results of inhibiting tumor cell proliferation were shown in Table C below.

Table C. The inhibitory activity ($IC_{50}$, nM) of the compounds of the present disclosure designed based on immunomodulatory drugs against tumor cells proliferation

| Comp. No./N ame | CCRF -CEM | MV-4-11 | Daudi | Kasum i-1 | JEKO-1 | THP-1 | MDA-MB-231 | MM.1 R | MM.1 S | CAL-62 |
|---|---|---|---|---|---|---|---|---|---|---|
| lenalid omide | F | F | F | F | F | F | F | F | F | F |
| pomali domid e | F | F | F | F | F | F | F | F | C | F |
| GT-03360 | A | B | A+++ | B | B | A+ | B | A | B | A |
| GT-03357 | B | - | B | - | - | - | - | - | - | - |
| GT-03363 | B | - | - | - | B | - | - | - | - | - |
| GT-04245 | - | - | - | - | - | C | - | - | - | - |
| GT-03462 | C | - | - | - | - | - | - | - | - | B |
| GT-06873 | - | A+ | - | - | - | - | - | - | - | - |
| GT-06874 | - | B | - | - | - | - | - | - | - | - |
| GT-06960 | - | C | - | - | - | - | - | - | - | - |
| GT-06918 | - | B | - | - | - | - | - | - | - | - |
| GT-07016 | - | C | - | - | - | - | - | - | - | - |
| GT-06924 | C | B | - | - | - | - | - | - | - | - |
| GT-03892 | A+ | - | - | - | - | B | - | - | - | A+ |
| GT-06182 | - | - | - | - | - | - | - | - | A+ | - |
| GT-06925 | - | A++ | - | - | - | - | - | - | - | - |
| GT-06226 | - | - | - | - | - | - | - | - | - | - |
| GT-06645 | - | C | - | - | - | - | - | - | - | - |
| GT-06926 | A | A+ | - | B | - | - | - | - | - | - |
| GT-06758 | A | A | - | B | - | - | - | - | - | - |
| GT-06815 | - | C | - | - | - | - | - | - | - | - |
| GT-06934 | - | B | - | - | - | - | - | - | - | - |
| GT-06204 | - | - | - | - | - | - | - | - | A+ | - |
| GT-06935 | - | B | - | - | - | - | - | - | - | - |
| GT-06928 | - | A+ | - | - | - | - | - | - | - | - |
| GT-06956 | - | C | - | - | - | - | - | - | - | - |

(continued)

| Comp. No./N ame | CCRF -CEM | MV-4-11 | Daudi | Kasum i-1 | JEKO-1 | THP-1 | MDA-MB-231 | MM.1 R | MM.1 S | CAL-62 |
|---|---|---|---|---|---|---|---|---|---|---|
| GT-06955 | - | A | - | - | - | - | - | - | - | - |
| Note: In Table C, the symbols A+++, A++, A+, A, B, C, D, E, and F are defined as follows: 0 < A+++ ≤ 1 nM; 1 nM < A++ ≤ 10 nM; 10 nM < A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E ≤ 10000 nM; F > 10000 nM. The symbol "-" indicates "not detected". | | | | | | | | | | |

The results demonstrated that the compounds of the present disclosure (including but not limited to those listed in Tables 1 and 2, and Examples A1-A20 and 1-116 compounds) exhibited significant inhibitory effects on the proliferation of various cancer cell lines, including CCRF-CEM (human T cell acute lymphoblastic leukemia cell line), MV-4-11 (human myelomonocytic leukemia cells), Daudi (human Burkitt's lymphoma cells), MM.1S (human multiple myeloma cells), MM.1R (dexamethasoneresistant human multiple myeloma cells), MDA-MB-231 (human breast cancer cells), Kasumi-1 (human acute lymphoblastic leukemia cells), CAL-62 (human thyroid carcinoma cells), JEKO-1 (human mantle cell lymphoma cells), and THP-1 (human monocytic leukemia cells), as shown in Table C. These compounds of the present disclosure demonstrated superior inhibitory potency compared to their corresponding positive control drugs.

**II. Determination of CRBN-binding affinity of compounds:**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING kits, the compounds to be tested of the present disclosure and lenalidomide were serially diluted using diluent #9 (1X) solution to obtain a final concentration of 2 μM for both the tested compounds and lenalidomide solution.
**2.** 2.5 μL of the above 2 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Re reagent and GST Eu antibody working solution were added to each of the aforementioned wells. The final concentration of the tested compounds and lenalidomide in each well is 0.5 μM.
**3.** The blank control wells were sequentially added with 2.5 μL of diluent #9 (1X) solution, 2.5 μL of PROTAC binding buffer, and 5 μL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$$R_{Std0} = OD\ 665\ nm_{Std0} / OD\ 620\ nm_{Std0} - OD\ 665\ nm_{control} / OD\ 620\ nm_{control}$$

$$\text{inhibition rate } (\%) = (1 - R_{compound}/R_{Std0}) * 100$$

Note: OD 665 nm $_{compound}$ is the absorbance value of each well of the compounds to be tested of the present disclosure at an emission wavelength of 665 nm.
OD 620 nm $_{compound}$ is the absorbance value of each well of the compounds to be tested of the present disclosure at an emission wavelength of 620 nm.
OD 665 nm $_{control}$ is the absorbance value of the blank control well at an emission wavelength of 665 nm.
OD 620 nm $_{control}$ is the absorbance value of the blank control well at an emission wavelength of 620 nm.
OD 665 nm $_{Std0}$ is the absorbance value of the solvent control well at an emission wavelength of 665 nm.
OD 620 nm $_{Std0}$ is the absorbance value of the solvent control well at an emission wavelength of 620 nm.

The test results were shown in Table D below.

Table D. HTRF inhibitory activity ($IC_{50}$, μM) of the compounds of the present disclosure (including but not limited to the compounds in Tables 1 and 2, and the Examples A1-A20 and 1-116 compounds)

| Comp. No. | HTRF $IC_{50}$ (μM) | Comp. No. | HTRF $IC_{50}$ (μM) | Comp. No. | HTRF $IC_{50}$ (μM) | Comp. No. | **HTRF** $IC_{50}$ (μM) |
|---|---|---|---|---|---|---|---|
| lenalidomide | d | GT-06925 | b | GT-06872 | c | GT-06561 | a |
| GT-03464 | a | GT-06225 | a | GT-06815 | c | GT-06185 | a |
| GT-06515 | b | GT-06226 | b | GT-06929 | b | GT-06819 | a |
| GT-06757 | c | GT-06350 | a | GT-06933 | c | GT-06195 | a |
| GT-06812 | c | GT-06808 | b | GT-06816 | a | GT-06928 | b |
| GT-04245 | a | GT-06517 | a | GT-06192 | b | GT-06956 | a |
| GT-03462 | b | GT-03457 | a | GT-07018 | a | GT-06955 | a |
| GT-06641 | c | GT-06301 | a | GT-07025 | a | GT-06759 | a |
| GT-06813 | c | GT-06351 | a | GT-07019 | a | GT-06352 | b |
| GT-06873 | c | GT-06513 | a | GT-07020 | b | GT-06519 | b |
| GT-06874 | c | GT-06516 | c | GT-03363 | b | GT-06520 | c |
| GT-06137 | b | GT-06514 | b | GT-06817 | b | GT-06959 | a |
| GT-06139 | b | GT-06138 | b | GT-06193 | c | GT-06559 | a |
| GT-06140 | b | GT-06196 | b | GT-06934 | a | GT-06760 | a |
| GT-06642 | c | GT-06184 | b | GT-06953 | a | GT-06814 | a |
| GT-07022 | b | GT-06645 | a | GT-06183 | a | GT-03613 | a |
| GT-07015 | b | GT-06926 | c | GT-06818 | a | GT-03616 | c |
| GT-06918 | b | GT-06920 | a | GT-06204 | a | GT-06995 | c |
| GT-07016 | c | GT-06958 | a | GT-06935 | a | GT-06992 | b |
| GT-07017 | c | GT-06922 | a | GT-06954 | a | GT-06993 | c |
| GT-03360 | a | GT-06758 | a | GT-06230 | b | GT-06997 | b |
| GT-04195 | c | GT-06349 | b | GT-06809 | b | GT-06998 | c |
| GT-06643 | c | GT-06503 | b | GT-06353 | b | GT-06996 | c |
| GT-06914 | c | GT-06504 | b | GT-06562 | a | GT-06999 | b |
| GT-03892 | a | GT-06923 | a | GT-03459 | b | GT-03612 | c |
| GT-06181 | a | GT-06518 | a | GT-06302 | b | GT-07000 | a |
| GT-06182 | a | GT-06807 | a | GT-06354 | a | GT-07005 | c |
| GT-06644 | a | GT-06811 | a | GT-06560 | b | GT-07004 | c |
| GT-07023 | a | GT-03369 | b | GT-06563 | b | | |
| GT-07024 | a | GT-06952 | b | | | | |
| In Table D, the symbols a, b, and c are defined as follows: 0<a≤0.5μM, 0.5μM <b≤1μM, 1μM <c<1.9μM, 1.9μM ≤ d. | | | | | | | |

The results show that the compounds of the present disclosure (including the compounds listed in Tables 1 and 2, and the Examples A1-A20 and 1-116 compounds) exhibit strong binding affinity to CRBN.

**III. Western-blot assay**

The effect of the compounds of the present disclosure on the expression of target proteins in cells was detected using conventional Western blot analysis.

Human peripheral blood mononuclear cells (hPBMCs) at a density of $1\times10^6$ cells/mL were thawed according to the PBMC Recovery Protocol (Milecell Biotechnologies Inc, Shanghai). The cells were then treated with the compounds of the present disclosure (including the compounds listed in Tables 1 and 2, and the Examples A1-A20 and 1-116 compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing phosphatase inhibitors on ice for 30-60 min, followed by centrifugation. The supernatant was aspirated as the extracted total cell protein. The protein concentration of the supernatant was determined using the conventional BCA (Bicinchoninic Acid) assay. The supernatant was then mixed with 4X SDS sample loading buffer, denatured at 95°C for 5 minutes, and then subjected to SDS-PAGE electrophoresis on a polyacrylamide gel. Proteins were then transferred to a nitrocellulose (NC) membrane, blocked at room temperature for 1-2 h, and processed for antibody incubation and detection according to the manufacturer's instructions (Cell Signaling Technology).

Half-Maximal Degradation Concentration value (the drug concentration required for degrading proteins by 50%, abbreviated as $DC_{50}$) reads method: comparing the grayscale values of Western blot bands from drug-treated samples with those from blank DMSO-treated controls, and identifying the drug concentration range at which the grayscale value is half that of the DMSO-treated control bands.

$DC_{50}$ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in hPBMC cells after treatment with protein degrader compounds.

Calculation method for protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

Target protein degradation rate (%) = 100% - target protein remaining (%).

The effects of the compounds of the present disclosure (including the compounds listed in Tables 1 and 2, and the Examples A1-A20 and 1-116 compounds) on substrate proteins expression in hPBMC cells were shown in Table E below.

Table E: Degradation results (substrate protein remaining %) of the compounds of the present disclosure (including the compounds listed in Tables 1 and 2, and the Examples A1-A20 and 1-116 compounds) on substrate proteins

| Comp. No. | **Wee1** | **IKZF1** | **IKZF2** | **IKZF3** | **GSPT1** | CK1α |
|---|---|---|---|---|---|---|
| GT-03464 | A | A | | | A | A |
| GT-06515 | A | A | | A | A | A |
| GT-06812 | A | A | | | A | |
| GT-04245 | | | A | | A | |
| GT-03462 | A | | | | A | |
| GT-06641 | | | A | | | |
| GT-06813 | A | | A | | A | |
| GT-06873 | A | A | A | A | A | A |
| GT-06874 | A | | A | A | A | A |
| GT-06960 | A | | | | A | A |
| GT-06137 | A | A | | | A | |
| GT-06140 | A | | | | | |
| GT-06642 | | | A | | A | |
| GT-07022 | | | | A | | A |
| GT-07015 | | | A | A | | |

(continued)

| Comp. No. | **Wee1** | **IKZF1** | **IKZF2** | **IKZF3** | **GSPT1** | CK1α |
|---|---|---|---|---|---|---|
| GT-06918 | A | A | A | A | | |
| GT-03360 | | A | A | A | A | A |
| GT-03357 | | | | A | A | |
| GT-06643 | A | | A | | | |
| GT-06914 | A | | A | | | |
| GT-06924 | | A | | | A | A |
| GT-03892 | A | A | A | A | A | |
| GT-06181 | A | | | A | A | |
| GT-06182 | A | A | | A | A | A |
| GT-06644 | | | | | A | |
| GT-07023 | A | | | | | |
| GT-07024 | A | | A | | | |
| GT-06925 | A | A | A | A | A | A |
| GT-06225 | | A | | | A | |
| GT-06226 | A | A | | | | |
| GT-06350 | | A | A | A | A | A |
| GT-06808 | A | A | A | | | A |
| GT-03457 | | A | A | A | A | A |
| GT-06301 | A | | A | | A | |
| GT-06351 | | A | A | A | A | A |
| GT-06513 | | | | | A | |
| GT-06516 | A | | A | A | A | A |
| GT-06514 | A | A | A | A | A | |
| GT-06138 | A | | | | A | |
| GT-06196 | | | A | | A | A |
| GT-06184 | | | | A | A | |
| GT-06645 | | | | | A | |
| GT-06926 | A | A | A | A | A | A |
| GT-06920 | A | A | | | | |
| GT-06958 | A | | | A | A | A |
| GT-06922 | A | A | | A | A | A |
| GT-06758 | | A | A | | A | |
| GT-06349 | | A | A | A | A | A |
| GT-06503 | A | A | | | A | A |
| GT-06518 | | | A | | A | A |
| GT-06811 | A | A | A | A | A | |
| GT-03369 | A | A | | A | A | |
| GT-06952 | | A | | A | A | A |
| GT-06872 | A | | A | A | A | A |

(continued)

| Comp. No. | **Wee1** | **IKZF1** | **IKZF2** | **IKZF3** | **GSPT1** | CK1α |
|---|---|---|---|---|---|---|
| GT-06815 | A | A | A | A | A | A |
| GT-06929 | A | | A | A | | |
| GT-06933 | A | A | A | A | | |
| GT-06816 | A | | A | | A | A |
| GT-06192 | A | | A | | | |
| GT-07025 | | | A | | | A |
| GT-07019 | A | | | | | |
| GT-07020 | A | | | | A | |
| GT-03363 | A | A | | A | A | A |
| GT-06817 | A | | A | A | A | A |
| GT-06193 | A | | A | | A | |
| GT-06934 | A | | A | | | A |
| GT-06953 | A | | | | | A |
| GT-06183 | A | | | | | |
| GT-06818 | A | | | | A | A |
| GT-06204 | | | A | A | A | A |
| GT-06935 | A | | | | A | A |
| GT-06954 | A | A | A | | A | |
| GT-06227 | A | | | | A | A |
| GT-06230 | A | | | | A | A |
| GT-06809 | A | A | A | A | A | A |
| GT-06353 | A | A | | A | A | A |
| GT-06562 | A | A | | | A | |
| GT-03459 | A | A | A | A | A | A |
| GT-06228 | A | | | | A | A |
| GT-06302 | A | A | A | A | A | A |
| GT-06354 | A | A | | A | A | A |
| GT-06560 | A | | | A | | |
| GT-06563 | A | | | | | |
| GT-06561 | A | | A | | | |
| GT-06185 | | | A | A | A | |
| GT-06819 | A | | A | A | A | A |
| GT-06195 | A | A | | A | A | A |
| GT-06928 | A | | A | | | |
| GT-06956 | A | A | A | A | | A |
| GT-06955 | | A | A | | | A |
| GT-06759 | | | A | | A | |
| GT-06352 | | A | | | A | A |
| GT-06519 | | | | | | A |

(continued)

| Comp. No. | **Wee1** | **IKZF1** | **IKZF2** | **IKZF3** | **GSPT1** | CK1α |
|---|---|---|---|---|---|---|
| GT-06520 | | | | | A | |
| GT-06959 | A | | | A | A | A |
| GT-06559 | A | | A | | A | A |
| GT-06760 | A | A | A | | | |
| GT-06814 | A | | A | A | A | A |
| GT-07061 | | | | | | A |
| GT-07062 | | | | | | A |
| GT-07063 | | | | | | A |
| GT-07064 | | | | | | A |
| GT-07065 | | | | | | A |
| GT-03613 | A | A | A | | A | A |
| GT-04309 | | | | | A | |
| GT-03616 | | A | A | | A | A |
| GT-06995 | | | | | | A |
| GT-06994 | | A | A | | | |
| GT-06997 | | | A | | A | |
| GT-03614 | A | | | | | |
| GT-03612 | A | A | A | A | A | A |
| GT-07003 | A | | | | | |
| GT-07005 | | | A | | | |
| In Table E, A represents 0% ≤ substrate protein remaining % ≤ 80%. | | | | | | |

As demonstrated in Table E, the compounds of the present disclosure (including the compounds in Tables 1 and 2, and the Examples A1-A20 and 1-116 compounds) effectively degraded one or more substrate proteins (e.g., Wee1, IKZF1, IKZF2, IKZF3, GSPT1, CK1α, etc.), showing significantly superior activity compared to the marketed drug lenalidomide. Lenalidomide failed to effectively degrade Wee1, IKZF2, GSPT1, and CK1α proteins in hPBMC cells at concentrations ≤1000 nM (as shown in Figure 1). Western blot results demonstrated that the compounds of the present disclosure exhibited significantly enhanced degradation efficiency against substrate proteins and a markedly expanded range of degradable substrates proteins compared to lenalidomide. In summary, the structural diversity of the compounds of the present disclosure enables effective degradation of multiple substrate proteins, making them potentially useful for treating diseases associated with these substrate proteins.

### IV. TNF-α Activity Inhibition Assay

PBMCs were cultured at 37°C with 5% $CO_2$ and seeded in 96-well plates at 1×10^7 cells/well. The compounds to be tested (including those listed in Tables 1 and 2, and Examples A1-A20 and 1-116) were dissolved in DMSO and diluted to appropriate concentrations, ensuring the final DMSO concentration in cell culture did not exceed 0.5%. After 1-hour incubation in medium with or without the compounds, the cells were stimulated with LPS (1 ng/ml) for 18-20 hours. Culture supernatants were then collected, diluted with serum-free medium, and analyzed for TNF-α levels using an ELISA kit. $IC_{50}$ values were calculated using GraphPad Prism 7.0.

TNF-α downregulation is a key mechanism of immunomodulatory agents' antitumor effects. The compounds of the present disclosure demonstrated dose-dependent inhibition of TNF-α levels.

### V. Pharmacokinetic Study of the compounds of the present disclosure

The pharmacokinetic properties of the compounds of the present disclosure were evaluated using conventional

pharmacokinetic experimental methods.

The test compounds of the present disclosure (including the compounds in Tables 1 and 2, and the Examples A1-A20 and 1-116 compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). In the present disclosure, mice were used as the experimental animals.

The experimental animals were divided into two groups: a control group (for blank plasma collection) and an oral administration group (dosed at 10 mg/kg or 30 mg/kg). Blood samples were collected at predetermined time points after administration, including: 0.25h, 0.5h, 1h, 2h, 4h, 8h, and 24h post-dose. Blank plasma was collected from the control group.

Prior to analysis, plasma samples were processed as follows: To 10 μL of plasma sample were added 10 μL of 50% acetonitrile and 200 μL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with noncompartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

## VI. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

### VI.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

### VI.2 Methods

#### VI.2.1 Cell Thawing and Plating

A complete medium was prepared and pre-warmed to 37°C. Cryovials were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of $1 \times 10^5$ cells/well

(75 μL/well) and incubated for 2 h at 37°C with 5% $CO_2$ under high humidity conditions.

VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) 2) The test compounds were further diluted in culture medium to five times the final working concentration intended for use.
4) The diluted compounds (25 μL/well) were added to designated wells according to the plate layout, achieving final concentrations starting from 1 μM with 5-fold serial dilution (9 concentration points in total).
5) After 1 h incubation, LPS or PHA (25 μL/well) was added to the cell wells to reach final concentrations of: 1 μg/mL LPS (for TNF-α, IL-10, IL-6, GM-CSF and IL-12p40), 5 μg/mL LPS (IL-1β), 50 μg/mL LPS (IFN-γ), or 1 μg/mL PHA (IL-2).
6) The plates were incubated in a incubator for 24 h at 37°C with 5% $CO_2$ under high humidity.

Cell plate layout:

<table>
<tr><td></td><td>1</td><td>2</td><td>3</td><td>4</td><td>5</td><td>6</td><td>7</td><td>8</td><td>9</td><td>10</td><td>11</td><td>12</td></tr>
<tr><td>A</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td></tr>
<tr><td>B</td><td>$H_2O$</td><td colspan="9" rowspan="2">Cmpd 1# (1 μM Starting, 5-fold dilution, 9 points)</td><td rowspan="4">LPS/PHA</td><td>$H_2O$</td></tr>
<tr><td>C</td><td>$H_2O$</td><td>$H_2O$</td></tr>
<tr><td>D</td><td>$H_2O$</td><td colspan="9" rowspan="2">Cmpd 2# (1 μM Starting, 5-fold dilution, 9 points)</td><td>$H_2O$</td></tr>
<tr><td>E</td><td>$H_2O$</td><td>$H_2O$</td></tr>
<tr><td>F</td><td>$H_2O$</td><td colspan="9" rowspan="2">Cmpd 3# (1 μM Starting, 5-fold dilution, 9 points)</td><td rowspan="2">Blank</td><td>$H_2O$</td></tr>
<tr><td>G</td><td>$H_2O$</td><td>$H_2O$</td></tr>
<tr><td>H</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td></tr>
</table>

VI.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions.

The concentrations of TNF- α , IL-10, IL-6, IL-1 β , IFN- γ , GM-CSF, IL-2, and IL-12p40 in the samples were calculated based on the standard curve. Inhibition rate% = [(Ac-As)/(Ac-Ab)] × 100%
As: OA of the samples (cells + LPS/PHA + test compound)
Ac: OA of positive cell control (cells + LPS/PHA + DMSO)
Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results demonstrated that the compounds of the present disclosure can modulate the production levels of cytokines associated with autoimmune diseases and may be used for the treatment of autoimmune diseases.

**VII. Efficacy experiment of the compounds of the present disclosure on Psoriasis-like skin lesions induced by Imiquimod in Mice**

The compounds of the present disclosure (test compounds, including the compounds in Tables 1 and 2, and the Examples A1-A20 and 1-116 compounds) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a solvent control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-$\alpha$ were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean $\pm$ SD. Statistical analysis between two groups was performed using SPSS, with $p < 0.05$ considered statistically significant.

The experimental results demonstrated that the compounds of the present disclosure improve psoriasis-like skin lesions on the back of mice induced by imiquimod and may be used for the treatment of psoriasis.

**VIII. Efficacy Experiment of the compounds of the present disclosure on the CIA Model in Rats Induced by Type II Collagen**

The compounds of the present disclosure (test compounds, including the compounds in Tables 1 and 2, and the Examples A1-A20 and 1-116 compounds) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Between 3 and 7 days after the second booster immunization, when the AI score of the affected feet reached 1 to 2, a solvent control, a positive control drug, or the test compounds were administered orally via gavage (or subcutaneously) to the animals in each group, according to the above table, once daily for 21 consecutive days.

Starting 3 days after the second booster immunization, the volume of both hind paws (or toes) was measured twice a week and recorded.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-$\alpha$, IL-1$\beta$, and IL-6 in serum were measured by ELISA.
After the experiment, the animals were euthanized by $CO_2$ inhalation. The spleen and thymus of the animals were collected and weighed, and the organ coefficients were calculated.
Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.
Experimental data were presented as Mean $\pm$ SEM. Data between two groups were analyzed using Excel-T test, with $p<0.05$ considered statistically significant. Scoring data were analyzed using the non-parametric Mann-Whitney U test in SPSS, with $p<0.05$ considered statistically significant.
The experimental results demonstrated that the compounds of the present disclosure can regulate the serum levels of inflammatory cytokines in rats with collagen-induced arthritis (CIA) model that was induced using bovine type II collagen, and significantly improve the swelling of rat limbs, and thus may be used for the treatment of arthritis.
The above descriptions represent only preferred embodiments of the present invention, but the scope of protection is not limited thereto. Any person skilled in the art, within the technical scope disclosed by the invention, may make equivalent replacements or modifications based on the technical solutions and inventive concepts of the present invention, and such changes shall fall within the protection scope of the invention.

## Claims

**1.** A compound of Formula (I)

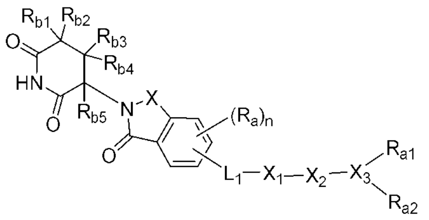

Formula I

or salts, enantiomers, stereoisomer, polymorphs or solvates thereof,

wherein X is selected from the group consisting of CO or $CH_2$;
$L_1$ is selected from the group consisting of S or O;
$X_1$ is selected from the group consisting of unsubstituted or substituted linear or branched alkylene, or $X_1$ is absent;
$X_2$ represents a structure of Formula II:

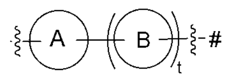

Formula II

wherein ring A and ring B are each independently selected from the group consisting of unsubstituted or substituted arylene, unsubstituted or substituted heteroarylene, unsubstituted or substituted heterocyclylene, or unsubstituted or substituted cycloalkylene;
t represents an integer of 0, 1 or 2; and
symbol # indicates the point of attachment to $X_3$;

$X_3$ is selected from the group consisting of $CR_1$, N, or CO, wherein $R_1$ is selected from the group consisting of hydrogen, deuterium, halogen, nitro, cyano, hydroxy, mercapto, trifluoromethoxy, unsubstituted or substituted amino, unsubstituted or substituted silyl, unsubstituted or substituted boryl, unsubstituted or substituted linear or branched alkyl, or unsubstituted or substituted cycloalkyl;
$R_{a1}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl;
$R_{a2}$ is selected from the group consisting of hydrogen, deuterium, unsubstituted or substituted aryl, unsubstituted or substituted heteroaryl, unsubstituted or substituted cycloalkyl, or unsubstituted or substituted heterocyclyl, or $R_{a2}$ is absent; and when $X_3$ represents CO, $R_{a2}$ is absent and $R_{a1}$ is neither hydrogen nor deuterium, and when $R_{a2}$ represents hydrogen or deuterium, $X_3$ represents $CR_1$ or N, and $R_{a1}$ is neither hydrogen nor deuterium;
$R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl;
$R_{b1}$, $R_{b2}$, $R_{b3}$, $R_{b4}$ and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl; and
n is an integer of 0, 1, 2 or 3, and when n is an integer of 2 or 3, each $R_a$ is independently identical to or different from each other.

**2.** The compound or salts, enantiomers, stereoisomers, polymorphs or solvates thereof as claimed in claim 1, wherein $X_1$ represents unsubstituted or substituted linear or branched $C_1$-$C_5$ alkylene, or $X_1$ is absent;

and/or, $X_2$ represents the structure of Formula II:

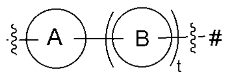

Formula II

wherein ring A is selected from the group consisting of unsubstituted or substituted $C_5$-$C_{30}$ arylene, unsubstituted or substituted 5- to 30-membered heteroarylene, unsubstituted or substituted 4- to 30-membered heterocyclylene, or unsubstituted or substituted $C_3$-$C_{30}$ cycloalkylene; preferably, ring A is selected from the group consisting of unsubstituted or substituted $C_5$-$C_{20}$ arylene, unsubstituted or substituted 5- to 20-membered heteroarylene, unsubstituted or substituted 4- to 20-membered heterocyclylene, or unsubstituted or substituted $C_3$-$C_{20}$ cycloalkylene;

ring B is selected from the group consisting of unsubstituted or substituted $C_5$-$C_{30}$ arylene, unsubstituted or substituted 5- to 30-membered heteroarylene, unsubstituted or substituted 4- to 30-membered heterocyclylene, or unsubstituted or substituted $C_3$-$C_{30}$ cycloalkylene; preferably, ring B is selected from the group consisting of unsubstituted or substituted $C_5$-$C_{20}$ arylene, unsubstituted or substituted 5- to 20-membered heteroarylene, unsubstituted or substituted 4- to 20-membered heterocyclylene, or unsubstituted or substituted $C_3$-$C_{20}$ cycloalkylene;

t represents an integer of 0, 1 or 2; preferably, t represents an integer of 0 or 1; and

symbol # indicates the point of attachment to $X_3$;

and/or, $R_1$ is selected from the group consisting of hydrogen, deuterium, F, Cl, Br, I, nitro, cyano, hydroxy, mercapto, trifluoromethoxy, unsubstituted or substituted amino, unsubstituted or substituted silyl, unsubstituted or substituted boryl, unsubstituted or substituted linear or branched $C_1$-$C_{10}$ alkyl, or unsubstituted or substituted $C_3$-$C_{15}$ cycloalkyl;

and/or, $R_{a1}$ is selected from the group consisting of hydrogen, deuterium, unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl; preferably, $R_{a1}$ is selected from the group consisting of hydrogen, deuterium, unsubstituted or substituted $C_5$-$C_{20}$ aryl, unsubstituted or substituted 5- to 20-membered heteroaryl, unsubstituted or substituted $C_3$-$C_{20}$ cycloalkyl, or unsubstituted or substituted 4- to 20-membered heterocyclyl;

and/or, $R_{a2}$ is selected from the group consisting of hydrogen, deuterium, unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl, or $R_{a2}$ is absent; preferably, $R_{a2}$ is selected from the group consisting of hydrogen, deuterium, unsubstituted or substituted $C_5$-$C_{20}$ aryl, unsubstituted or substituted 5- to 20-membered heteroaryl, unsubstituted or substituted $C_3$-$C_{20}$ cycloalkyl, or unsubstituted or substituted 4- to 20-membered heterocyclyl, or $R_{a2}$ is absent;

and/or, $R_a$ is selected from the group consisting of deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl; preferably, $R_a$ is selected from the group consisting of deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl;

and/or, $R_{b1}$, $R_{b2}$, $R_{b3}$, $R_{b4}$ and $R_{b5}$ are each independently selected from the group consisting of hydrogen, deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, or unsubstituted or substituted linear or branched $C_1$-$C_5$ alkyl; preferably, $R_{b1}$, $R_{b2}$, $R_{b3}$, $R_{b4}$ and $R_{b5}$ are each independently selected from the group consisting of hydrogen, deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, or unsubstituted or substituted linear or branched $C_1$-$C_5$ alkyl.

**3.** The compound or salts, enantiomers, stereoisomers, polymorphs or solvates thereof as claimed in claim 1 or 2, wherein the structure of said compound is shown in Formula I-1 or Formula I-2:

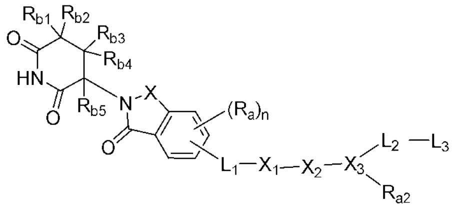

Formula I-1

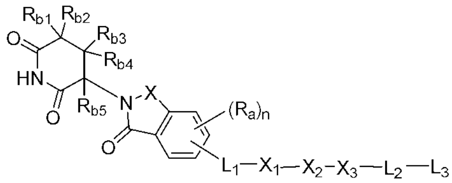

Formula I-2

in Formula I-1 or Formula I-2, X, $L_1$, $X_1$, $X_2$, $R_a$, $R_{b1}$, $R_{b2}$, $R_{b3}$, $R_{b4}$, $R_{b5}$, and n are as defined in claim 1 or 2;
$L_2$ is selected from the group consisting of unsubstituted or substituted $C_5$-$C_{30}$ arylene, unsubstituted or substituted 5- to 30-membered heteroarylene, unsubstituted or substituted $C_3$-$C_{30}$ cycloalkylene, or unsubstituted or substituted 4- to 30-membered heterocyclylene, or $L_2$ is absent;
$L_3$ is selected from the group consisting of unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl;
$R_{a2}$ is selected from the group consisting of unsubstituted or substituted $C_5$-$C_{30}$ aryl, unsubstituted or substituted 5- to 30-membered heteroaryl, unsubstituted or substituted $C_3$-$C_{30}$ cycloalkyl, or unsubstituted or substituted 4- to 30-membered heterocyclyl;
wherein in Formula I-1, $X_3$ is selected from the group consisting of $CR_1$ or N, and in Formula I-2, $X_3$ is selected from the group consisting of $CR_1$, N or CO, wherein $R_1$ is as defined in claim 1 or 2.

**4.** The compound or salts, enantiomers, stereoisomers, polymorphs or solvates thereof as claimed in any one of claims 1-3, wherein $X_1$ is selected from the group consisting of methylene, ethylene, propylene or isopropylene, or $X_1$ is absent.

**5.** The compound or salts, enantiomers, stereoisomers, polymorphs or solvates thereof as claimed in any one of claims 1-3, wherein $X_2$ represents the structure of Formula II:

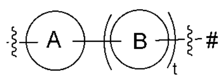

Formula II

wherein ring A is selected from the group consisting of unsubstituted or substituted $C_5$-$C_{15}$ arylene, unsubstituted or substituted 5- to 15-membered heteroarylene, unsubstituted or substituted 4- to 15-membered heterocyclylene, or unsubstituted or substituted $C_3$-$C_{15}$ cycloalkylene, wherein the substituent of the substituted ring A is optionally one or more substituents selected from the group consisting of halogen, mercapto, hydroxy, amino, cyano, $C_1$-$C_6$ alkyl, $C_1$-$C_6$ alkoxy, halogenated $C_1$-$C_6$ alkyl or any combination thereof;
ring B is selected from the group consisting of unsubstituted or substituted $C_5$-$C_{15}$ arylene, unsubstituted or substituted 5- to 15-membered heteroarylene, unsubstituted or substituted 4- to 15-membered heterocyclylene, or unsubstituted or substituted $C_3$-$C_{15}$ cycloalkylene; wherein the substituent of the substituted ring B is optionally one or more substituents selected from the group consisting of halogen, mercapto, hydroxy, amino, cyano, $C_1$-$C_6$ alkyl, $C_1$-$C_6$ alkoxy, halogenated $C_1$-$C_6$ alkyl or any combination thereof;
t represents an integer of 0, 1 or 2; preferably, t represents an integer of 0 or 1; and
symbol # indicates the point of attachment to $X_3$.

6. The compound or salts, enantiomers, stereoisomers, polymorphs or solvates thereof as claimed in any one of claims 1-3, wherein the ring A and ring B are each independently selected from the group consisting of the following groups:

azetidinylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, azacyclooctylene, diazacycloheptanylene, diazacyclooctylene, bridged heterocyclylene (e.g., 5- to 15-membered bridged heterocyclylene, such as azabicyclohexylene, azabicycloheptanylene, or azabicyclooctylene), or spiro-heterocyclylene (e.g., 5- to 15-membered spiro-heterocyclylene, such as 3-azaspiro[5.5]undecanylene or 7-azaspiro[3.5]nonanylene); or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene; or

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_{5-15}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), or bridged cycloalkylene (e.g., $C_{5-15}$ bridged cycloalkylene, such as adamantanylene, noradamantanylene, bornylene, bicyclo[2.2.1]heptylene, 2-oxobicyclo[2.2.1]heptylene, or bicyclo[2.2.1]heptentylene); or

phenylene or naphthylene;

wherein the ring A and ring B are each independently optionally substituted by one or more substituents selected from the group consisting of deuterium, halogen, nitro, mercapto, hydroxy, cyano, $C_1$-$C_6$ alkyl, $C_1$-$C_6$ alkoxy, halogenated $C_1$-$C_6$ alkoxy, halogenated $C_1$-$C_6$ alkyl, unsubstituted or substituted amino, unsubstituted or substituted silyl, unsubstituted or substituted boryl or any combination thereof.

7. The compound or salts, enantiomers, stereoisomers, polymorphs or solvates thereof as claimed in claim 3 or 5, wherein $X_2$ is selected from the group consisting of :

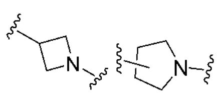 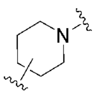 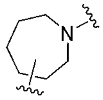 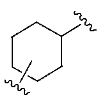 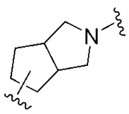 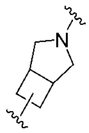 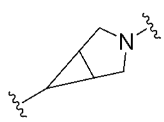 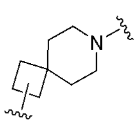

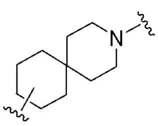 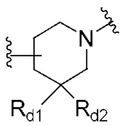 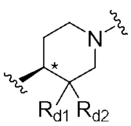 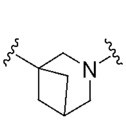 or 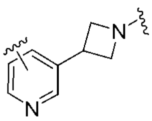 ;

wherein $R_{d1}$ and $R_{d2}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, and unsubstituted or substituted linear or branched $C_1$-$C_5$ alkyl; and $R_{d1}$ and $R_{d2}$ are not hydrogen at the same time.

8. The compound or salts, enantiomers, stereoisomers, polymorphs or solvates thereof as claimed in claim 1, wherein when $R_{a2}$ is present, $X_3$ is selected from the group consisting of CH or N;
when $R_{a2}$ is absent, $X_3$ represents CO.

9. The compound or salts, enantiomers, stereoisomers, polymorphs or solvates thereof as claimed in claim 3, wherein

$L_2$ is selected from the group consisting of unsubstituted or substituted 5- to 20-membered heteroarylene (e.g., unsubstituted or substituted 5- to 15-membered heteroarylene); preferably, the heteroarylene is selected from the

group consisting of pyridylene, pyrrolylene, pyrimidinylene, pyridazinylene, furanylene, thienylene, imidazolylene, pyrazolylene, oxazolylene, isoxazolylene, thiazolylene, isothiazolylene, triazolylene, oxadiazolylene, thiadiazolylene, tetrazolylene, pyranylene, thiopyranylene, pyrazinylene, pyridazinylene, thiazinylene, triazinylene, tetrazinylene, quinolinylene, isoquinolinylene, quinolinylene, quinazolinylene, quinoxalinylene, cinnolinylene, naphthyridinylene, acridinylene, xanthenylene, phenanthridinylene, diazanaphthylene, triazaindenylene, indolylene, indolinylene, indolizinylene, phthalazinylene, pyrazolopyridylene, pyrazolopyrimidinylene, pyridopyrimidinylene, pyridopyrazinylene, pyrazinopyrazinylene, benzothiazolylene, benzoxazolylene, benzimidazolylene, benzothienylene, benzofuranylene, isobenzofuranylene, dibenzothienylene, dibenzofuranylene, indazolylene, carbazolylene, benzocarbazolylene, dibenzocarbazolylene, indolocarbazolylene, indenocarbazolylene, phenazinylene, imidazopyridinylene, phenazinylene, phenanthridinylene, phenanthrolinylene, phenothiazinylene, imidazopyridinylene, imidazophenanthridinylene, pyrrolopyridinylene, pyrrolothiazolylene, imidazothiazolylene, benzimidazoquinazolinylene, benzimidazophenanthridinylene, pyrenylene, dinaphthofuranylene, naphthobenzofuranylene, dinaphthothienylene, or naphthobenzothienylene;

and/or, $L_2$ is selected from the group consisting of unsubstituted or substituted $C_3$-$C_{20}$ cycloalkylene (e.g., unsubstituted or substituted $C_3$-$C_{15}$ cycloalkylene); preferably, $L_2$ is selected from the group consisting of cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{15}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), or bridged cycloalkylene (e.g., $C_5$-$C_{15}$ bridged cycloalkylene, adamantanylene, noradamantanylene, bornylene, bicyclo[2.2.1]heptylene, 2-oxobicyclo[2.2.1]heptylene, or bicyclo[2.2.1]heptentylene);

and/or, $L_2$ is selected from the group consisting of unsubstituted or substituted $C_5$-$C_{20}$ arylene (e.g., unsubstituted or substituted $C_5$-$C_{15}$ arylene); preferably, $L_2$ is selected from the group consisting of phenylene, biphenylene, terphenylene, naphthylene, anthrylene, phenanthrylene, fluorenylene, 9,9'-dimethylfluorenylene;

and/or, $L_2$ is selected from the group consisting of unsubstituted or substituted 4- to 20-membered heterocyclylene (e.g., unsubstituted or substituted 4- to 15-membered heterocyclylene); preferably, $L_2$ is selected from the group consisting of azetidinylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, azacyclooctylene, diazacycloheptanylene, diazacyclooctylene, bridged heterocyclylene (e.g., 5- to 15-membered bridged heterocyclylene, such as azabicyclohexylene, azabicycloheptanylene, diazabicycloheptanylene, azabicyclooctylene), or spiro-heterocyclylene (e.g., 5- to 15-membered spiro-heterocyclylene, such as 3-azaspiro[5.5]undecanylene or 7-azaspiro[3.5]nonanylene);

wherein the substituent of the substituted $L_2$ is optionally one or more substituents selected from the group consisting of deuterium, halogen, nitro, mercapto, hydroxy, cyano, halogenated $C_1$-$C_6$ alkyl linear or branched $C_1$-$C_6$ alkyl, halogenated $C_1$-$C_6$ alkoxy, $C_1$-$C_6$ alkoxy, halogenated $C_1$-$C_6$ alkyl, unsubstituted or substituted amino, unsubstituted or substituted silyl, unsubstituted or substituted boryl or any combination thereof;

or $L_2$ is absent.

**10.** The compound or salts, enantiomers, stereoisomers, polymorphs or solvates thereof as claimed in claim 3 or 9, wherein

$L_2$ is selected from the group consisting of:

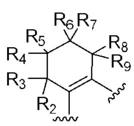, 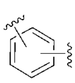, 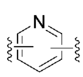, 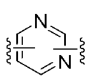, 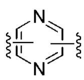, 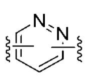, 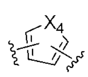, 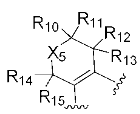, 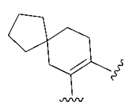, 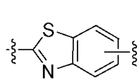,

or

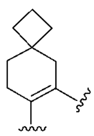;

wherein $X_4$ is selected from the group consisting of O, S, or NH;

$X_5$ is selected from the group consisting of O, S, or $NR_{16}$, wherein $R_{16}$ represents hydrogen or $C_1$-$C_3$ alkyl; $R_2$, $R_3$, $R_4$, $R_5$, $R_6$, $R_7$, $R_8$, $R_9$, $R_{10}$, $R_{11}$, $R_{12}$, $R_{13}$, $R_{14}$ and $R_{15}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, linear or branched $C_1$-$C_5$ alkyl or halogenated $C_1$-$C_5$ alkyl, wherein preferably, the halogenated $C_1$-$C_6$ alkoxy is selected from the group consisting of trifluoromethoxy; or
$L_2$ is absent.

**11.** The compound or salts, enantiomers, stereoisomers, polymorphs or solvates thereof as claimed in claim 3, wherein

$L_3$ and $R_{a2}$ are each independently selected from the group consisting of unsubstituted or substituted $C_5$-$C_{20}$ aryl (e.g., unsubstituted or substituted $C_5$-$C_{15}$ aryl); preferably, $L_3$ and $R_{a2}$ are each independently selected from the group consisting of biphenyl, terphenyl, naphthyl, anthryl, phenanthryl, fluorenyl, and 9,9'-dimethylfluorenyl;
and/or, $L_3$ and $R_{a2}$ are each independently selected from the group consisting of unsubstituted or substituted 5- to 25-membered heteroaryl (preferably, unsubstituted or substituted 5- to 20-membered heteroaryl, or unsubstituted or substituted 5- to 15-membered heteroaryl); more preferably, $L_3$ and $R_{a2}$ are each independently selected from the group consisting of pyridyl, pyrrolyl, pyrimidinyl, pyridazinyl, furanyl, thienyl, imidazolyl, pyrazolyl, oxazolyl, isoxazolyl, thiazolyl, isothiazolyl, triazolyl, oxadiazolyl, thiadiazolyl, tetrazolyl, pyranyl, thiopyranyl, pyrazinyl, oxazinyl, thiazinyl, triazinyl, tetrazinyl, quinolinyl, isoquinolinyl, quinazolinyl, quinoxalinyl, cinnolinyl, naphthyridinyl, acridinyl, xanthenyl, phenanthridinyl, diazanaphthyl, triazaindenyl, indolyl, indolinyl, indolizinyl, phthalazinyl, pyridopyrimidinyl, pyridopyrazinyl, pyrazinopyrazinyl, benzothiazolyl, benzoxazolyl, benzimidazolyl, benzothienyl, benzofuranyl, dibenzothienyl, dibenzofuranyl, carbazolyl, benzocarbazolyl, dibenzocarbazolyl, indolocarbazolyl, indenocarbazolyl, phenazinyl, imidazopyridinyl, phenanthrolinyl, phenothiazinyl, imidazopyridinyl, imidazophenanthridinyl, benzimidazoquinazolinyl, benzimidazophenanthridinyl, spiro[fluorene-9,9'-xanthene], pyrenyl, dinaphthofuranyl, naphthobenzofuranyl, dinaphthothienyl, or naphthobenzothienyl;
and/or, $L_3$ and $R_{a2}$ are each independently selected from the group consisting of unsubstituted or substituted 4- to 20-membered heterocyclyl (e.g., unsubstituted or substituted 4- to 15-membered heterocyclyl); preferably, $L_3$ and $R_{a2}$ are each independently selected from the group consisting of azetidinyl, oxetanyl, pyrrolidinyl, imidazolidinyl, pyrazolidyl, tetrahydrofuranyl, tetrahydropyranyl, dihydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, dioxacyclohexenyl, azacycloheptanyl, azacyclooctyl, diazacycloheptanyl, diazacyclooctyl, bridged heterocyclyl (e.g., 5- to 15-membered bridged heterocyclyl, such as azabicyclohexyl, azabicycloheptanyl, or azabicyclooctyl), or spiro-heterocyclyl (e.g., 5- to 15-membered spiro-heterocyclyl, such as 3-azaspiro[5.5]undecanyl or 7-azaspiro[3.5]nonanyl);
and/or, $L_3$ and $R_{a2}$ are each independently selected from the group consisting of unsubstituted or substituted $C_3$-$C_{20}$ cycloalkyl (e.g., unsubstituted or substituted $C_3$-$C_{15}$ cycloalkyl); preferably, $L_3$ and $R_{a2}$ are each independently selected from the group consisting of cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{15}$ spiro-cycloalkyl, such as spiro[3.3]heptyl, spiro[2.5]octyl, spiro[3.5]nonyl, spiro[4.4]nonyl, spiro[4.5]decyl, or spiro[5.5]undecyl), or bridged cycloalkyl (e.g., $C_5$-$C_{15}$ bridged cycloalkyl, such as adamantanyl, noradamantanyl, bornyl, bicyclo[2.2.1]heptyl, 2-oxobicyclo[2.2.1]heptyl or bicyclo[2.2.1]heptentyl);
wherein the substituents of the substituted $L_3$ and $R_{a2}$ are each independently optionally one or more substituents selected from the group consisting of deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, linear or branched $C_1$-$C_5$ alkyl or halogenated $C_1$-$C_5$ alkyl.

**12.** The compound or salts, enantiomers, stereoisomers, polymorphs or solvates thereof as claimed in claim 3 or 11, wherein $L_3$ and $R_{a2}$ are each independently selected from the group consisting of :

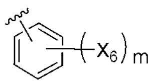 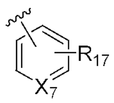 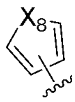 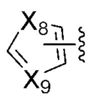 or 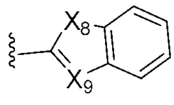 ;

wherein $X_6$ is selected from the group consisting of deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, linear or branched $C_1$-$C_6$ alkyl or halogenated $C_1$-$C_6$ alkyl;
m is an integer of 0, 1, 2, 3, 4 or 5, and when m is an integer of 2, 3, 4 or 5, each $X_6$ is independently identical to or

different from each other;

$X_7$ and $X_9$ are each independently selected from the group consisting of CH or N;

each $X_8$ is independently selected from the group consisting of O, S or NH;

$R_{17}$ is selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, linear or branched $C_1$-$C_5$ alkyl or halogenated $C_1$-$C_5$ alkyl, wherein the halogenated $C_1$-$C_6$ alkoxy is preferably selected from the group consisting of trifluoromethoxy.

**13.** The compound or salts, enantiomers, stereoisomers, polymorphs or solvates thereof as claimed in claim 1, wherein the chemical structure of said compound is selected from the group consisting of any one of the following compounds:

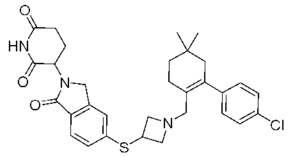
1

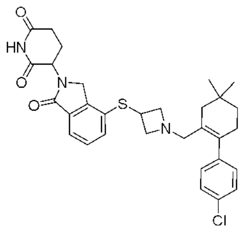
2

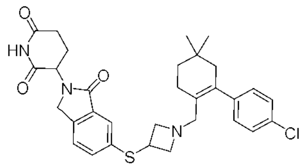
3

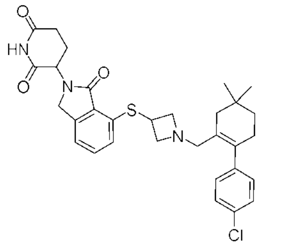
4

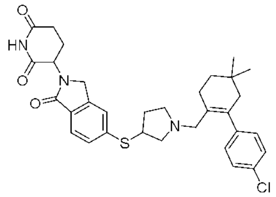
5

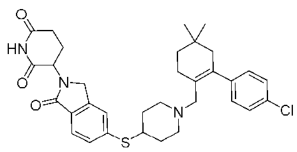
6

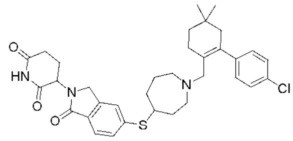
7

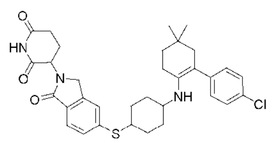
8

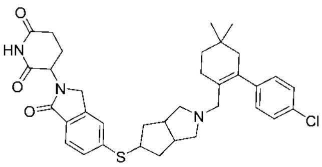
9

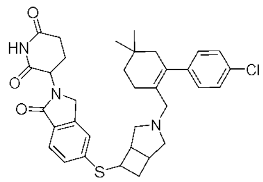
10

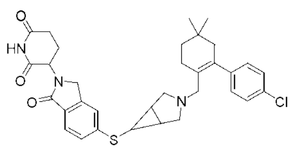
11

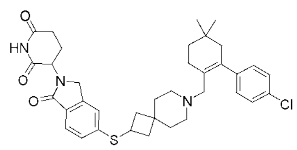
12

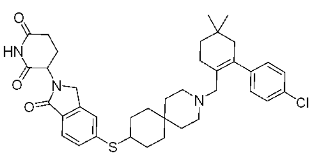
13

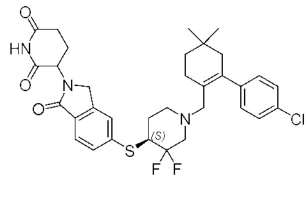
14

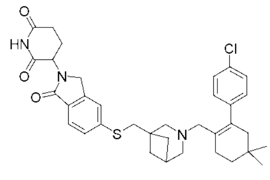
15

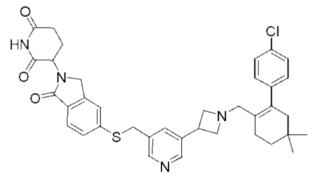
16

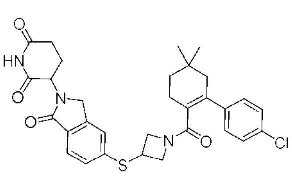
17

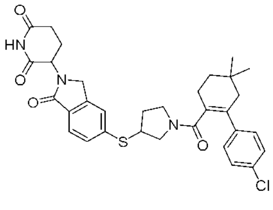
18

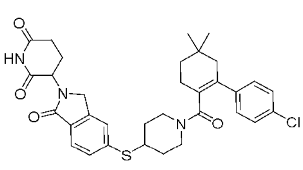
19

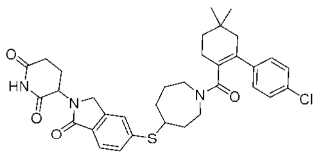
20

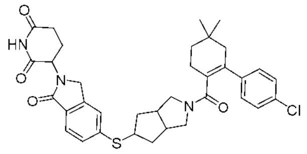

21

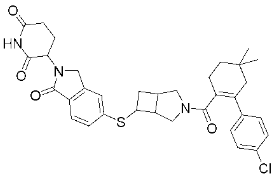

22

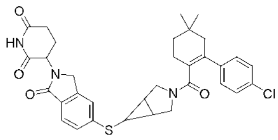

23

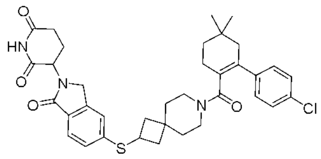

24

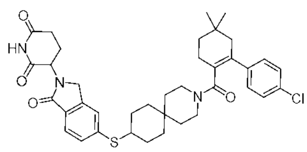

25

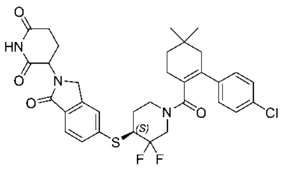

26

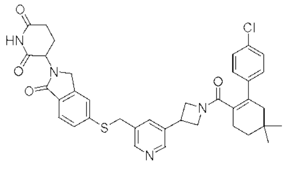

27

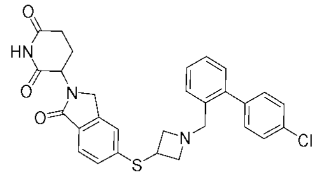

28

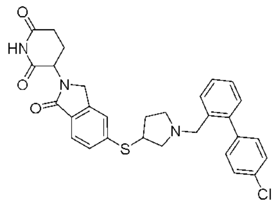

29

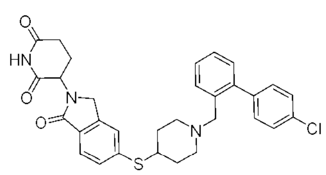

30

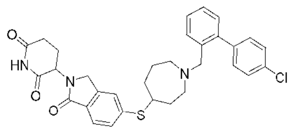

31

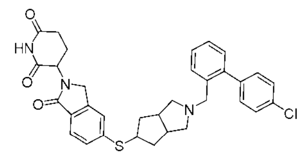

32

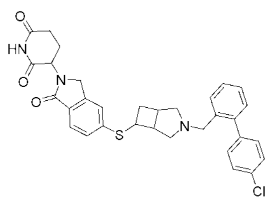

33

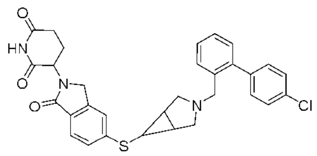

34

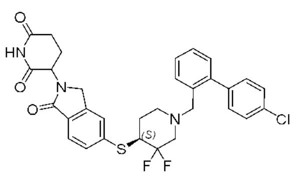

35

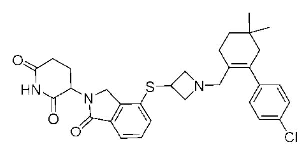

36

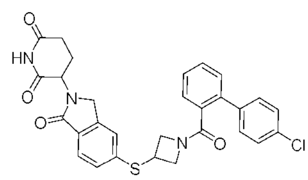

37

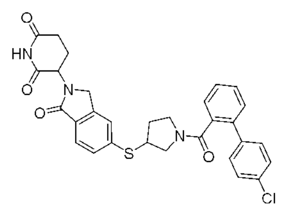

38

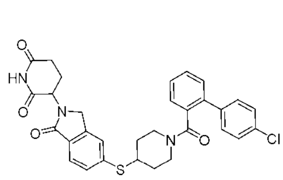

39

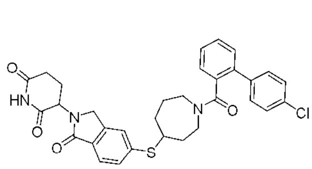

40

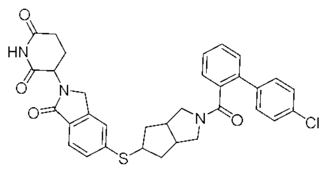

41

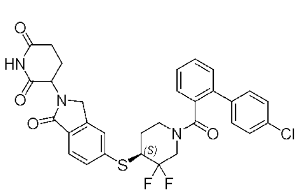

42

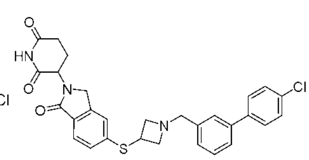

43

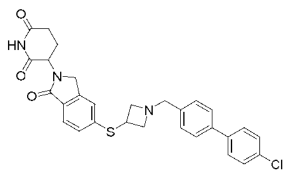

44

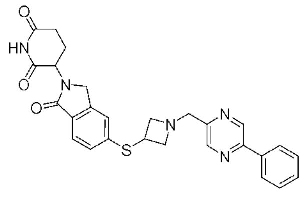
45

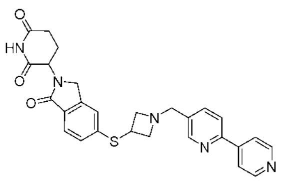
46

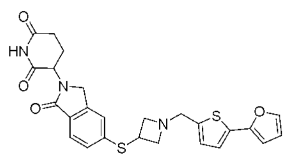
47

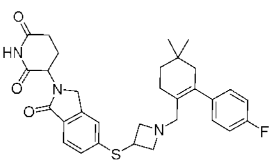
48

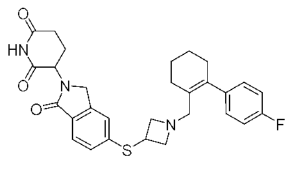
49

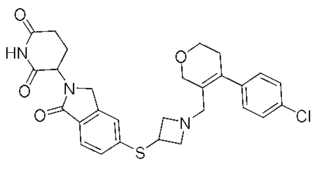
50

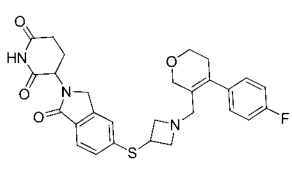
51

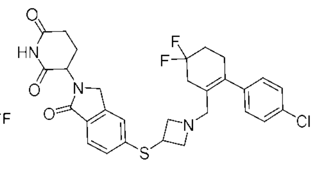
52

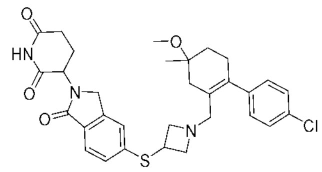
53

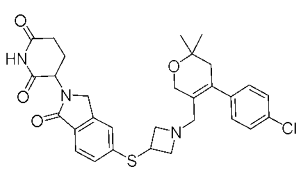
54

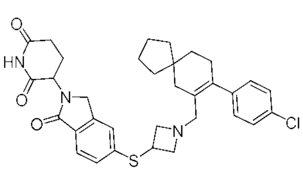
55

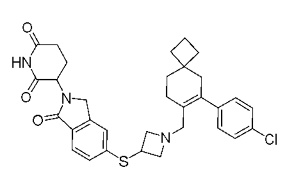
56

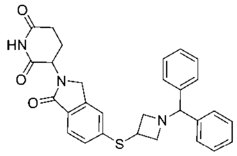
57

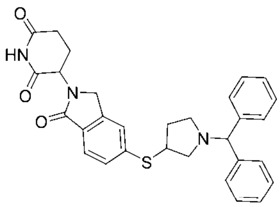
58

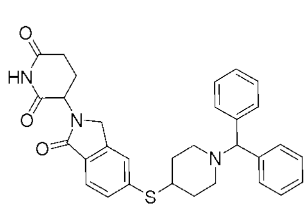
59

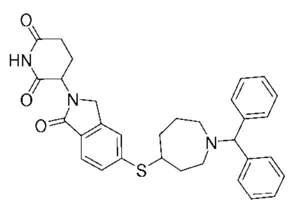
60

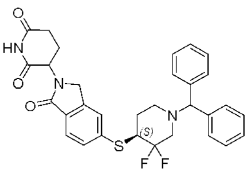
61

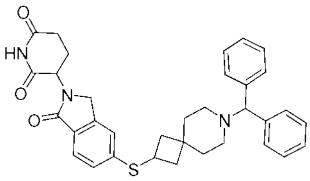
62

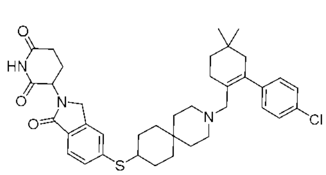
63

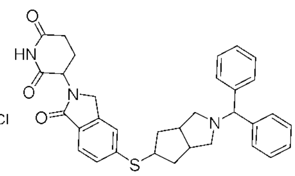
64

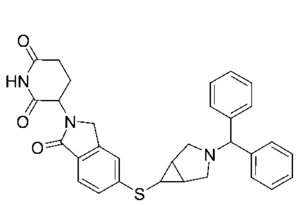
65

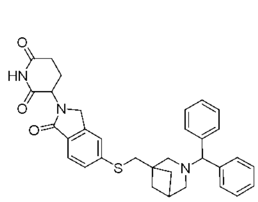
66

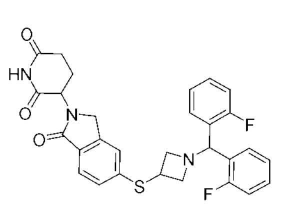
67

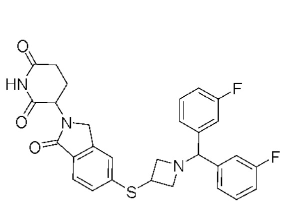
68

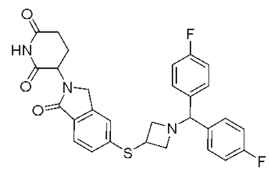
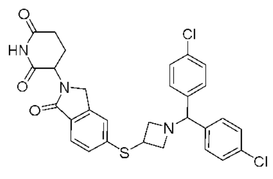
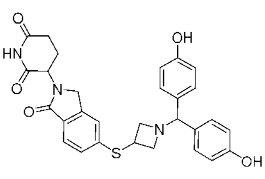
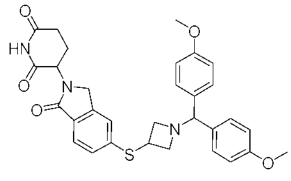

69 70 71 72

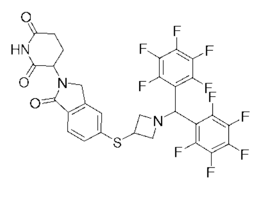
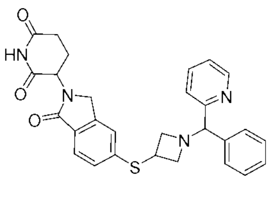
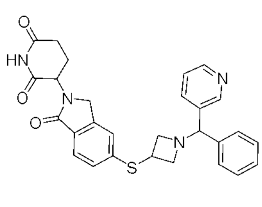
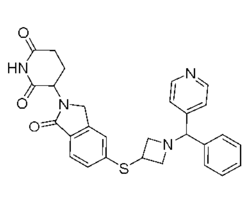

73 74 75 76

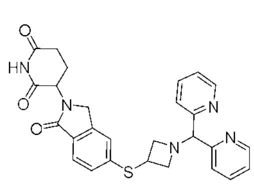
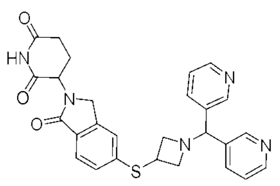
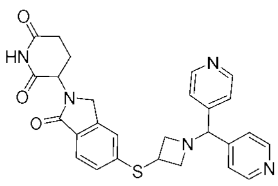
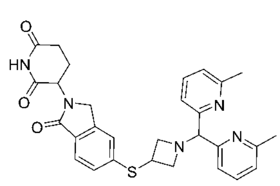

77 78 79 80

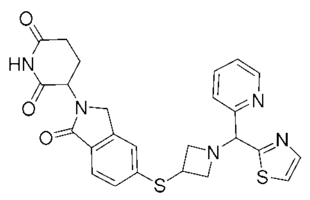
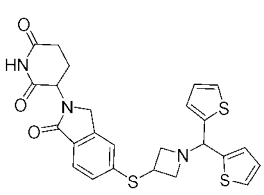
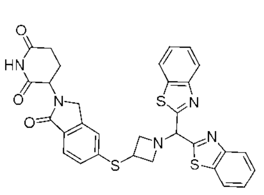
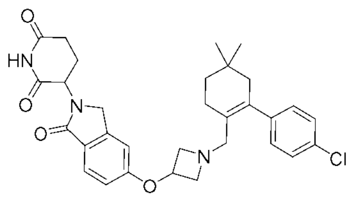

81 82 83 84

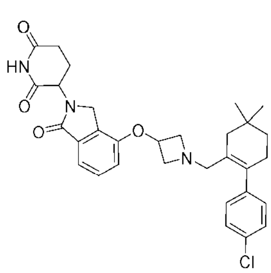
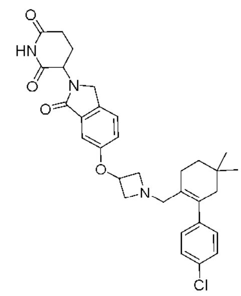
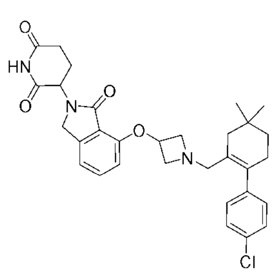
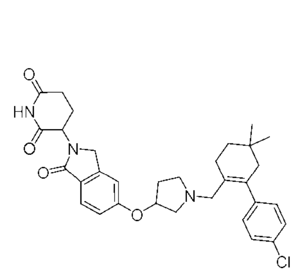

85 86 87 88

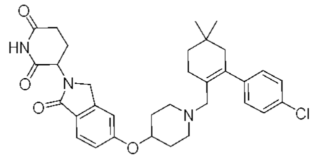
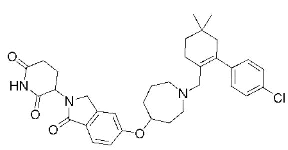
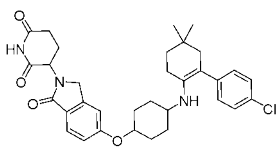
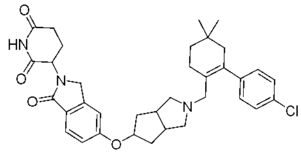

89 90 91 92

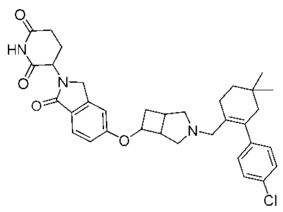 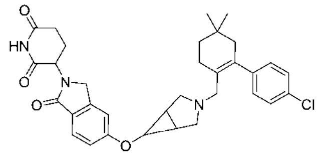 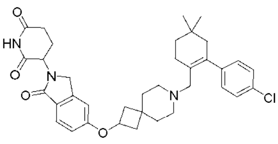 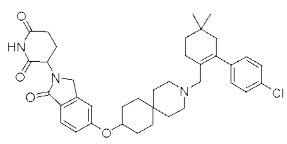

93 94 95 96

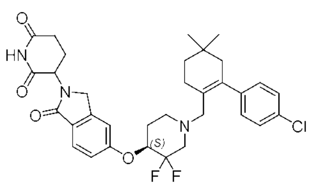 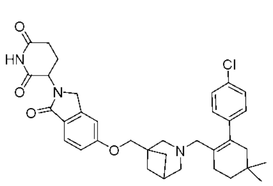 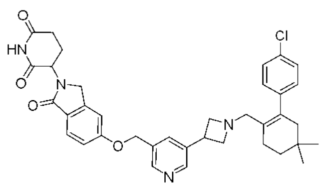 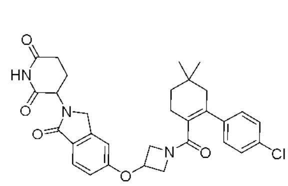

97 98 99 100

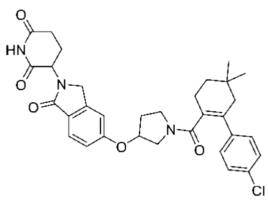 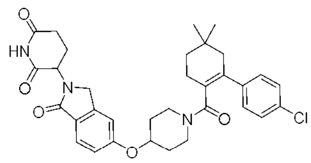 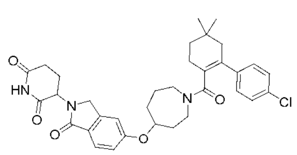 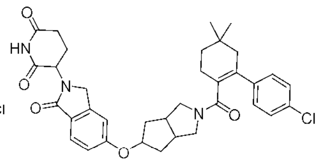

101 102 103 104

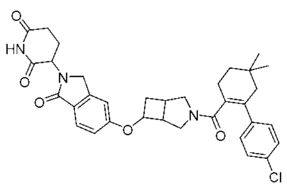 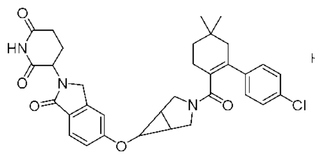 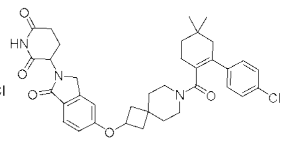 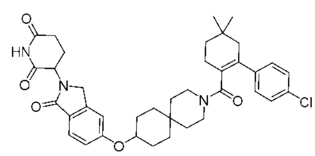

105 106 107 108

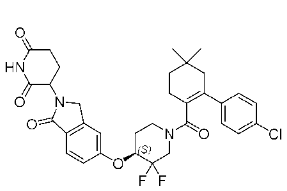 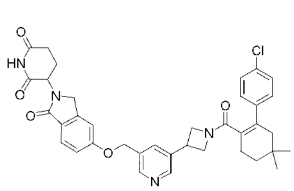 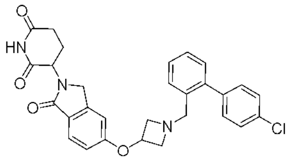 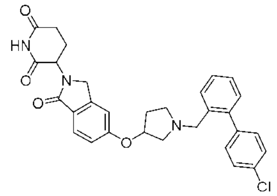

109 110 111 112

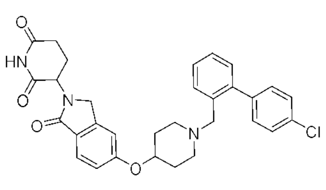 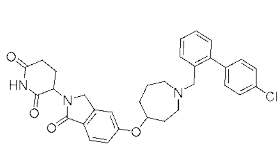 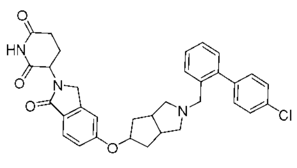 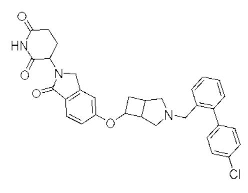

113 114 115 116

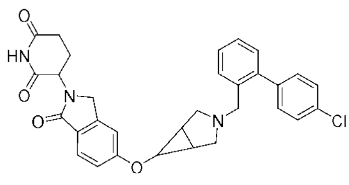
117

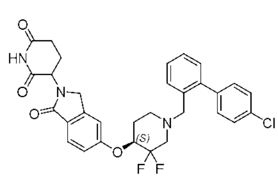
118

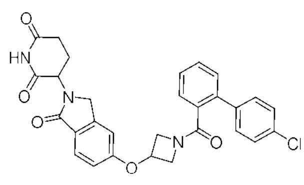
119

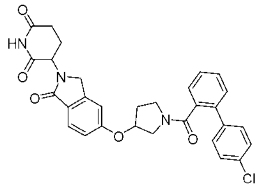
120

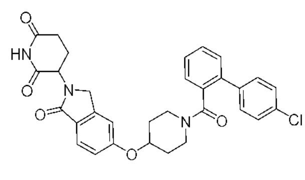
121

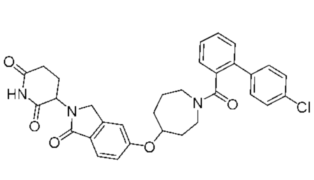
122

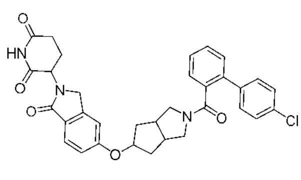
123

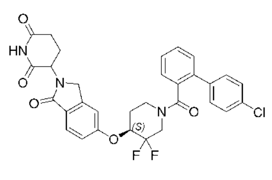
124

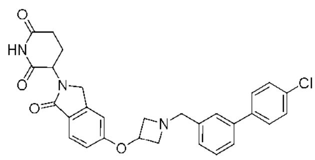
125

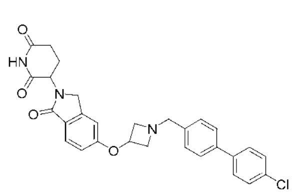
126

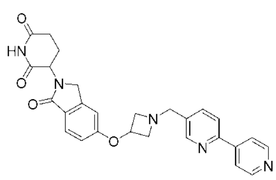
127

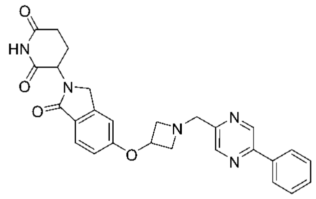
128

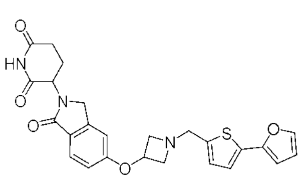
129

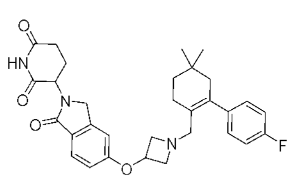
130

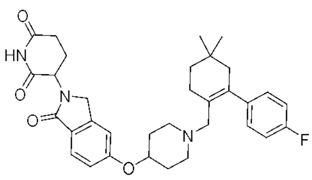
131

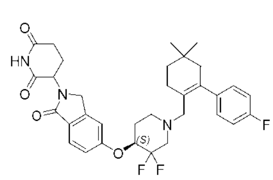
132

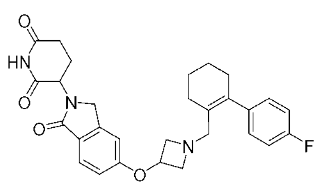
133

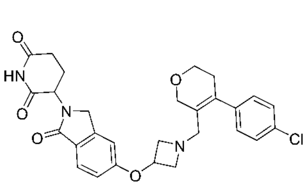
134

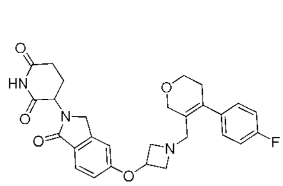
135

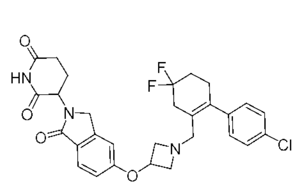
136

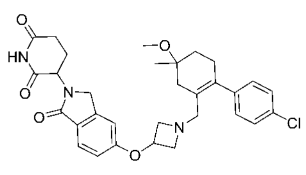
137

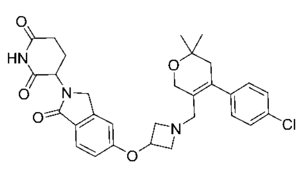
138

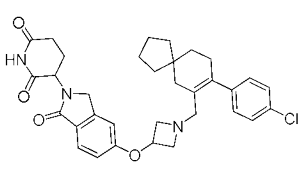
139

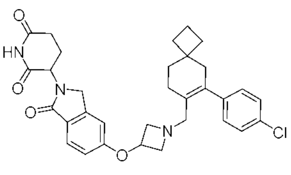
140

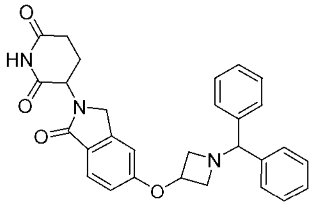
141
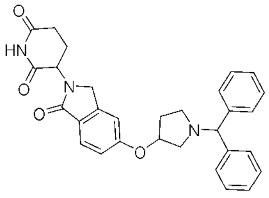
142
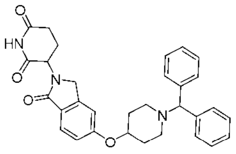
143
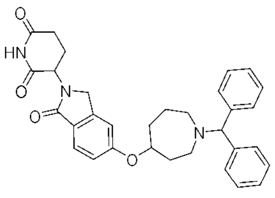
144

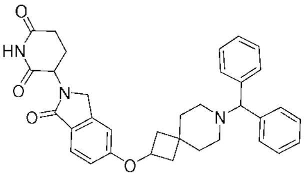
145
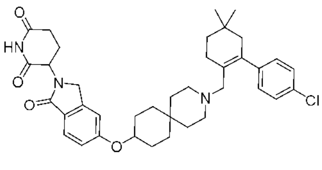
146
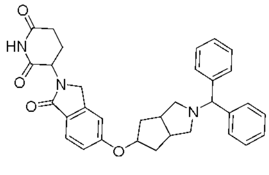
147
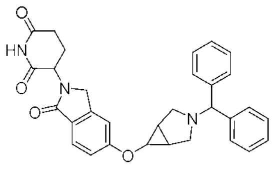
148

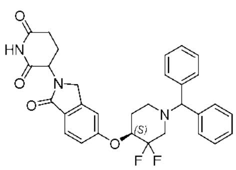
149
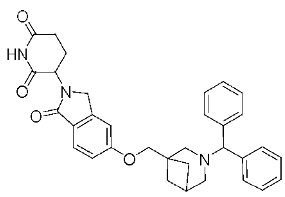
150
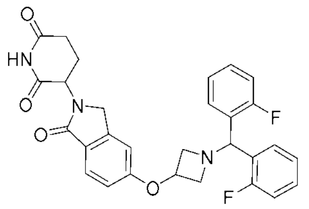
151
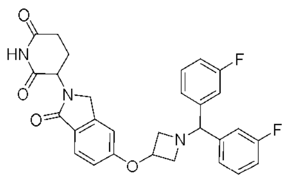
152

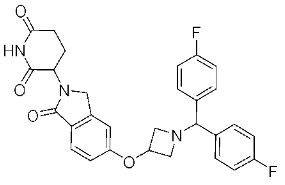
153
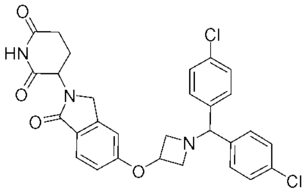
154
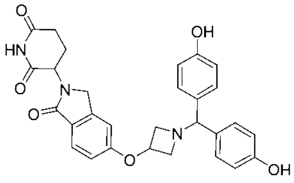
155
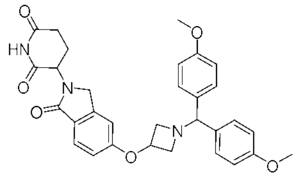
156

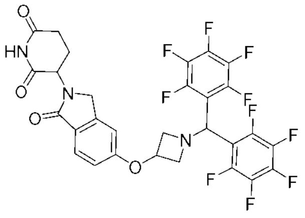
157
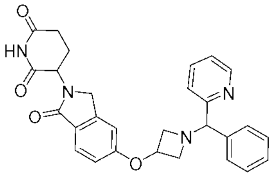
158
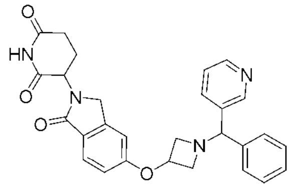
159
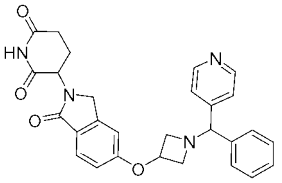
160

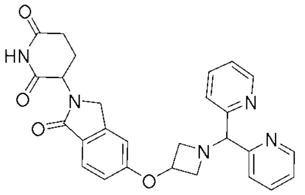
161
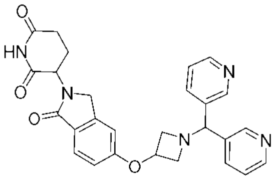
162
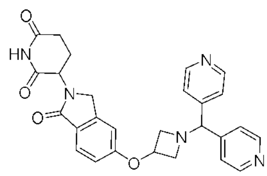
163
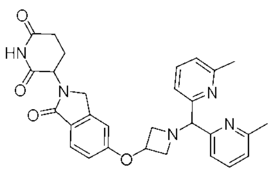
164

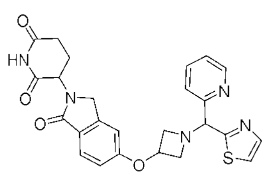 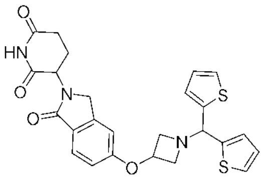 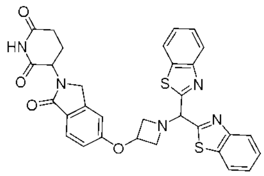 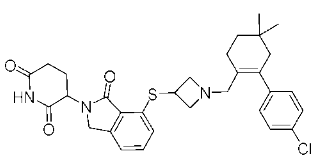

165 166 167 168

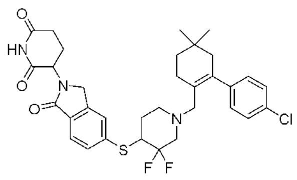 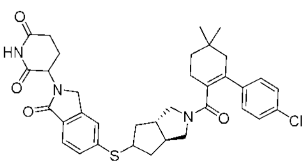 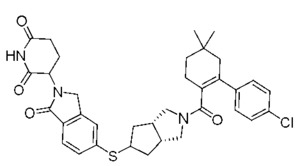 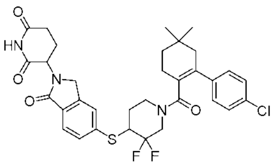

169 170 171 172

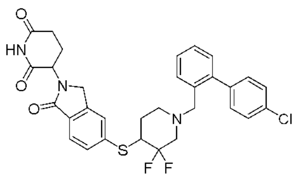 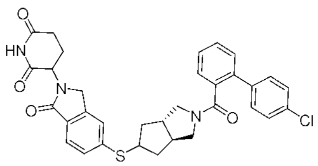 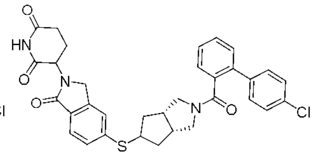 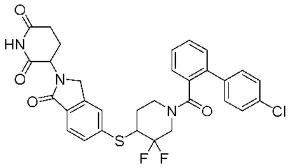

173 174 175 176

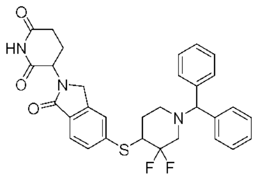 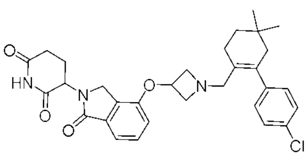 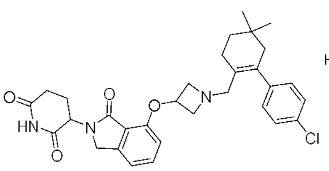 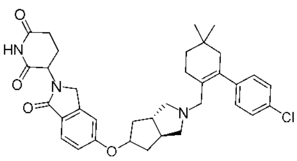

177 178 179 180

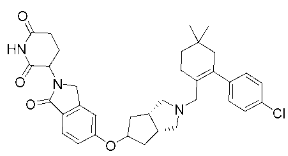 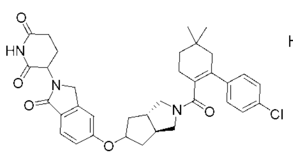 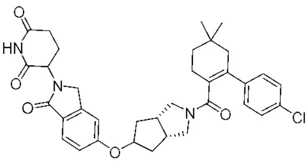 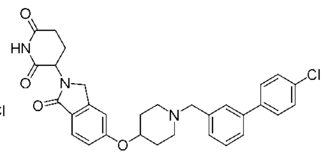

181 182 183 and 184.

**14.** The compound of Formula I or salts, enantiomers, stereoisomers, polymorphs or solvates thereof as claimed in any one of claims 1-13, which is hydrohalides (including hydrochlorides and hydrobromates), sulfates, citrates, maleates, mesylates, citrates, lactates, L-tartrates, fumarates, L-malates, phosphates, dihydrophosphates, pyrophosphates, metaphosphates, oxalates, malonates, benzoates, mandelates, succinates, trifluoroacetates, hippurates, glycolates, or p-toluenesulfonates of the compound of Formula I.

**15.** A compound of Formula A or salts, enantiomers, stereoisomers, polymorphs or solvates thereof,

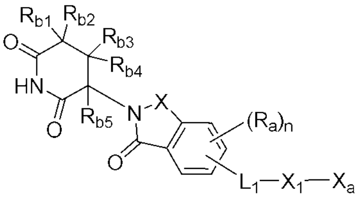

Formula A

wherein X is selected from the group consisting of CO or $CH_2$;
$L_1$ is selected from the group consisting of S or O;
$X_1$ is selected from the group consisting of unsubstituted or substituted linear or branched alkylene, or $X_1$ is absent;
$X_a$ represents the structure of Formula A1:

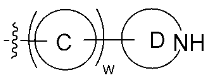

Formula A1

wherein ring C is selected from the group consisting of unsubstituted or substituted arylene, unsubstituted or substituted heteroarylene, unsubstituted or substituted heterocyclylene, or unsubstituted or substituted cycloalkylene, and w represents an integer of 0 or 1; and
ring D represents unsubstituted or substituted nitrogen-containing heterocyclyl;

$R_a$ is selected from the group consisting of deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl, unsubstituted or substituted cycloalkyl, unsubstituted or substituted heterocyclyl, unsubstituted or substituted aryl, or unsubstituted or substituted heteroaryl;
$R_{b1}$, $R_{b2}$, $R_{b3}$, $R_{b4}$ and $R_{b5}$ are each independently selected from the group consisting of hydrogen, deuterium, halogen, nitro, cyano, hydroxy, mercapto, alkoxy, halogenated alkoxy, unsubstituted or substituted amino, unsubstituted or substituted silyl, unsubstituted or substituted boryl, unsubstituted or substituted linear or branched alkyl; and
n is an integer of 0, 1, 2 or 3, and when n is an integer of 2 or 3, each $R_a$ is independently identical to or different from each other.

**16.** The compound of Formula A or salts, enantiomers, stereoisomers, polymorphs or solvates thereof as claimed in claim 15, wherein $X_1$ represents unsubstituted or substituted linear or branched $C_1$-$C_5$ alkylene, or $X_1$ is absent;

and/or, $X_a$ represents the structure of Formula A1:

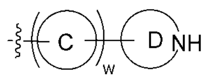

Formula A1

wherein ring C is selected from the group consisting of unsubstituted or substituted $C_5$-$C_{30}$ arylene, unsubstituted or substituted 5- to 30-membered heteroarylene, unsubstituted or substituted 4- to 30-membered heterocyclylene, or unsubstituted or substituted $C_3$-$C_{30}$ cycloalkylene; preferably, ring C is selected from the group consisting of unsubstituted or substituted $C_5$-$C_{20}$ arylene, unsubstituted or substituted 5- to 20-membered heteroarylene, unsubstituted or substituted 4- to 20-membered heterocyclylene, or unsubstituted or substituted $C_3$-$C_{20}$ cycloalkylene;
w represents an integer of 0 or 1; and
ring D represents unsubstituted or substituted 4- to 30-membered nitrogen-containing heterocyclyl; preferably, ring D represents unsubstituted or substituted 4- to 20-membered nitrogen-containing heterocyclyl;

and/or, $R_a$ is selected from the group consisting of deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl; preferably, $R_a$ is selected from the group consisting of deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, unsubstituted or substituted linear or branched $C_1$-$C_5$ alkyl, unsubstituted or substituted $C_3$-$C_{15}$ cycloalkyl, unsubstituted or substituted 4- to 15-membered heterocyclyl, unsubstituted or substituted $C_5$-$C_{15}$ aryl, or unsubstituted or substituted 5- to 15-membered heteroaryl;

and/or, $R_{b1}$, $R_{b2}$, $R_{b3}$, $R_{b4}$ and $R_{b5}$ are each independently selected from the group consisting of hydrogen, deuterium, F, Cl, Br, I, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, or unsubstituted or substituted linear or branched $C_1$-$C_5$ alkyl; preferably, $R_{b1}$, $R_{b2}$, $R_{b3}$, $R_{b4}$ and $R_{b5}$ are each independently selected from the group consisting of hydrogen, deuterium, F, Cl, Br, I, nitro, cyano, hydroxy, mercapto, $C_1$-$C_6$ alkoxy, trifluoromethoxy, unsubstituted or substituted amino, unsubstituted or substituted silyl, unsubstituted or substituted boryl, or unsubstituted or substituted linear or branched $C_1$-$C_5$ alkyl.

**17.** The compound of Formula A or salts, enantiomers, stereoisomers, polymorphs or solvates thereof as claimed in claim 15, wherein $X_1$ represents methylene, ethylene, propylene or isopropylene, or $X_1$ is absent.

**18.** The compound of Formula A or salts, enantiomers, stereoisomers, polymorphs or solvates thereof as claimed in claim 15, wherein $X_a$ represents the structure of Formula A1:

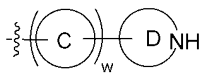

Formula A1

wherein the ring C is selected from the group consisting of unsubstituted or substituted $C_5$-$C_{15}$ arylene, unsubstituted or substituted 5- to 15-membered heteroarylene, unsubstituted or substituted 4- to 15-membered heterocyclylene, or unsubstituted or substituted $C_3$-$C_{15}$ cycloalkylene, wherein the substituent of the substituted ring C is optionally one or more substituents selected from the group consisting of halogen, mercapto, hydroxy, amino, cyano, $C_1$-$C_6$ alkyl, $C_1$-$C_6$ alkoxy, halogenated $C_1$-$C_6$ alkyl or any combination thereof;
w represents an integer of 0 or 1; and
the ring D represents unsubstituted or substituted 4- to 15-membered nitrogen-containing heterocyclyl.

**19.** The compound of Formula A or salts, enantiomers, stereoisomers, polymorphs or solvates thereof as claimed in claim 15, wherein

(1) the ring C is selected from the group consisting of the following groups:

azetidinylene, oxetanylene, pyrrolidinylene, imidazolidinylene, pyrazolidylene, tetrahydrofuranylene, tetrahydropyranylene, dihydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, dioxacyclohexenylene, azacycloheptanylene, azacyclooctylene, diazacycloheptanylene, diazacyclooctylene, bridged heterocyclylene (e.g., 5- to 15-membered bridged heterocyclylene, such as azabicyclohexylene, azabicycloheptanylene or azabicyclooctylene), or spiro-heterocyclylene (e.g., 5- to 15-membered spiro-heterocyclylene, such as 3-azaspiro[5.5]undecanylene or 7-azaspiro[3.5]nonanylene); or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene; or

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{15}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), or bridged cycloalkylene (e.g., $C_5$-$C_{15}$ bridged cycloalkylene, adamantanylene, noradamantanylene, bornylene, bicyclo[2.2.1]heptylene, 2-oxobicyclo[2.2.1]heptylene, or bicyclo[2.2.1]heptentylene); or
phenylene or naphthylene;

wherein the ring C is optionally substituted by one or more substituents selected from the group consisting of deuterium, halogen, nitro, mercapto, hydroxy, cyano, $C_1$-$C_6$ alkyl, $C_1$-$C_6$ alkoxy, halogenated $C_1$-$C_6$ alkoxy, halogenated $C_1$-$C_6$ alkyl, unsubstituted or substituted amino, unsubstituted or substituted silyl, unsubstituted or substituted boryl or any combination thereof;
and/or, (2) the ring D is selected from the group consisting of the following groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptanyl, azacyclooctyl, diazacycloheptanyl, diazacyclooctyl, nitrogen-containing bridged heterocyclyl (e.g., 5- to 15-membered bridged heterocyclyl, such as azabicyclohexyl, azabicycloheptanyl or azabicyclooctyl), or nitrogen-containing spiro-heterocyclyl (e.g., 5- to 15-membered spiro-heterocyclyl, such as 3-azaspiro[5.5] undecanyl or 7-azaspiro[3.5]nonanyl);
wherein the ring D is optionally substituted by one or more substituents selected from the group consisting of deuterium, halogen, nitro, mercapto, hydroxy, cyano, oxo, $C_1$-$C_6$ alkyl, $C_1$-$C_6$ alkoxy, halogenated $C_1$-$C_6$ alkoxy, halogenated $C_1$-$C_6$ alkyl, unsubstituted or substituted amino, unsubstituted or substituted silyl, unsubstituted or substituted boryl or any combination thereof.

**20.** The compound of Formula A or salts, enantiomers, stereoisomers, polymorphs or solvates thereof as claimed in claim 15, wherein $X_a$ is selected from the group consisting of:

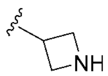 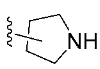 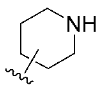 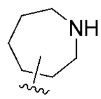 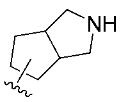 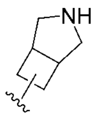 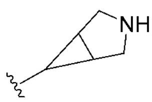 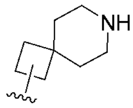 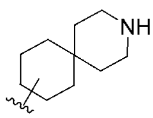

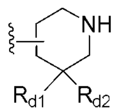 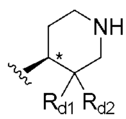 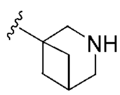 or 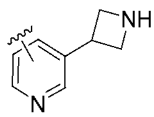 ;

wherein $R_{d1}$ and $R_{d2}$ are each independently selected from the group consisting of hydrogen, deuterium, halogen, nitro, cyano, hydroxy, mercapto, $C_1$-$C_6$ alkoxy, halogenated $C_1$-$C_6$ alkoxy, unsubstituted or substituted amino, unsubstituted or substituted silyl, unsubstituted or substituted boryl, unsubstituted or substituted linear or branched $C_1$-$C_5$ alkyl; and $R_{d1}$ and $R_{d2}$ are not hydrogen at the same time.

**21.** The compound of Formula A or salts, enantiomers, stereoisomers, polymorphs or solvates thereof as claimed in claim 15, which is selected from the group consisting of:

3-(5-(azetidin-3-ylthio)-1-oxoisoindolin-2-yl)piperidine-2,6-dione;
3-(1-oxo-5-(pyrrolidin-3-ylthio)isoindolin-2-yl)piperidine-2,6-dione;
3-(1-oxo-5-(piperidin-4-ylthio)isoindolin-2-yl)piperidine-2,6-dione;
3-(5-(azepan-4-ylthio)-1-oxoisoindolin-2-yl)piperidine-2,6-dione;
3-(4-(azetidin-3-ylthio)-1-oxoisoindolin-2-yl)piperidine-2,6-dione;
3-(6-(azetidin-3-ylthio)-1-oxoisoindolin-2-yl)piperidine-2,6-dione;
3-(7-(azetidin-3-ylthio)-1-oxoisoindolin-2-yl)piperidine-2,6-dione;
3-(5-((7-azaspiro[3.5]nonan-2-yl)thio)-1-oxoisoindolin-2-yl)piperidine-2,6-dione;
3-(5-((3-azaspiro[5.5]undecan-9-yl)thio)-1-oxoisoindolin-2-yl)piperidine-2,6-dione;
3-(5-((octahydrocyclopenta[c]pyrrol-5-yl)thio)-1-oxoisoindolin-2-yl)piperidine-2,6-dione;
3-(5-((3,3-difluoropiperidin-4-yl)thio)-1-oxoisoindolin-2-yl)piperidine-2,6-dione;
3-(5-((3-azabicyclo[3.2.0]heptan-6-yl)thio)-1-oxoisoindolin-2-yl)piperidine-2,6-dione;
3-(5-((3-azabicyclo[3.1.0]hexan-6-yl)thio)-1-oxoisoindolin-2-yl)piperidine-2,6-dione;

3-(5-(((3-azabicyclo[3.1.1]heptan-1-yl)ymethyl)thio)-1-oxoisoindolin-2-yl)piperidine-2,6-dione;
3-(5-(((5-(azetidin-3-yl)pyridin-3-yl)methyl)thio)-1-oxoisoindolin-2-yl)piperidine-2,6-dione;
3-(5-(azetidin-3-yloxy)-1-oxoisoindolin-2-yl)piperidine-2,6-dione;
3-(1-oxo-5-(pyrrolidin-3-yloxy)isoindolin-2-yl)piperidine-2,6-dione;
3-(1-oxo-5-(piperidin-4-yloxy)isoindolin-2-yl)piperidine-2,6-dione;
3-(5-(azepan-4-yloxy)-1-oxoisoindolin-2-yl)piperidine-2,6-dione;
3-(4-(azetidin-3-yloxy)-1-oxoisoindolin-2-yl)piperidine-2,6-dione;
3-(6-(azetidin-3-yloxy)-1-oxoisoindolin-2-yl)piperidine-2,6-dione;
3-(7-(azetidin-3-yloxy)-1-oxoisoindolin-2-yl)piperidine-2,6-dione;
3-(5-((7-azaspiro[3.5]nonan-2-yl)oxy)-1-oxoisoindolin-2-yl)piperidine-2,6-dione;
3-(5-((octahydrocyclopenta[c]pyrrol-5-yl)oxy)-1-oxoisoindolin-2-yl)piperidine-2,6-dione;
3-(5-((3-azaspiro[5.5]undecan-9-yl)oxy)-1-oxoisoindolin-2-yl)piperidine-2,6-dione;
3-(5-((3,3-difluoropiperidin-4-yl)oxy)-1-oxoisoindolin-2-yl)piperidine-2,6-dione;
3-(5-((3-azabicyclo[3.2.0]heptan-6-yl)oxy)-1-oxoisoindolin-2-yl)piperidine-2,6-dione;
3-(5-((3-azabicyclo[3.1.0]hexan-6-yl)oxy)-1-oxoisoindolin-2-yl)piperidine-2,6-dione;
3-(5-((3-azabicyclo[3.1.1]heptan-1-yl)methoxy)-1-oxoisoindolin-2-yl)piperidine-2,6-dione; and
3-(5-((5-(azetidin-3-yl)pyridin-3-yl)methoxy)-1-oxoisoindolin-2-yl)piperidine-2,6-dione.

**22.** The compound of Formula A or salts, enantiomers, stereoisomers, polymorphs or solvates thereof as claimed in any one of claims 15-21, which is hydrohalides (including hydrochlorides and hydrobromates), sulfates, citrates, maleates, mesylates, citrates, lactates, L-tartrates, fumarates, L-malates, phosphates, dihydrophosphates, pyrophosphates, metaphosphates, oxalates, malonates, benzoates, mandelates, succinates, trifluoroacetates, hippurates, glycolates, or p-toluenesulfonates of the compound of Formula A.

**23.** A pharmaceutical composition comprising:

the compound or salts, enantiomers, stereoisomers, polymorphs or solvates thereof as claimed in any one of claims 1-14, and at least one pharmaceutically acceptable carrier or excipient; or
the compound of Formula A or salts, enantiomers, stereoisomers, polymorphs or solvates thereof as claimed in any one of claims 15-22, and at least one pharmaceutically acceptable carrier or excipient;
and optionally comprising at least one additional therapeutic agent, e.g., an anticancer agent.

**24.** A medicine kit or reagent kit comprising:

the compound or a pharmaceutically acceptable salt thereof as claimed in any one of claims 1-14; or
the compound of Formula A or a pharmaceutically acceptable salt thereof as claimed in any one of claims 15-22; or
the pharmaceutical composition as claimed in claim 23.

**25.** The compound or salts, enantiomers, stereoisomers, polymorphs or solvates thereof as claimed in any one of claims 1-14, for use as a medicament.

**26.** The compound of Formula A or salts, enantiomers, stereoisomers, polymorphs or solvates thereof as claimed in any one of claims 15-22, for use as a medicament.

**27.** The compound as claimed in claim 25, or the compound of Formula A as claimed in claim 26, or salts, enantiomers, stereoisomers, polymorphs or solvates thereof, for use in the treatment of a disease or disorder associated with cereblon protein.

**28.** Use of the compound as claimed in any one of claims 1-14, or the compound of Formula A as claimed in any one of claims 15-22, or salts, enantiomers, stereoisomers, polymorphs or solvates thereof, or the pharmaceutical composition as claimed in claim 23 for the manufacture of a medicament for the treatment of a disease or disorder associated with cereblon protein.

**29.** A method for treating or preventing a disease or disorder associated with cereblon protein, comprising administering to a subject a therapeutically effective amount of the compound as claimed in any one of claims 1-14, or the compound of Formula A as claimed in any one of claims 15-22, or salts, enantiomers, stereoisomers, polymorphs or solvates thereof, or the pharmaceutical composition as claimed in claim 23.

**30.** The compound, use, or method as claimed in any one of claims 27-29, wherein the disease or disorder associated with cereblon protein is selected from the group consisting of tumor, infectious disease, inflammatory disease, immune system diseases, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**31.** The compound, use, or method as claimed in claim 30, wherein the disease or disorder associated with cereblon protein is selected from the group consisting of: myeloma (e.g., multiple myeloma, plasma cell myeloma, smoldering myeloma, smoldering multiple myeloma); myelofibrosis; bone marrow disease; myelodysplastic syndrome (MDS); previously treated myelodysplastic syndrome; transplantation-related cancer; neutropenia; leukemia (e.g., acute myeloid leukemia, chronic myelogenous leukemia, B-cell chronic lymphocytic leukemia, leukemia-associated anemia, acute myeloid leukemia (AML), monocytic leukemia, acute lymphoblastic leukemia (ALL), acute B-cell lymphoblastic leukemia, acute T-cell lymphoblastic leukemia, chronic lymphocytic leukemia, lymphoma cell leukemia, T-cell lymphoblastic leukemia, myelomonocytic leukemia); lymphoma (e.g., diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma); thyroid cancer; melanoma; lung cancer (e.g., lung adenocarcinoma, lung squamous cell carcinoma, non-small cell lung cancer, and small cell lung cancer); inflammatory myofibroblastoma; colorectal cancer; intestinal cancer; brain glioma; astroblastoma; neuroglioma; peripheral neuroepithelioma; ovarian cancer; bronchial cancer; prostate cancer; breast cancer (e.g., triple negative breast cancer, sporadic breast cancer, and patients with Cowden syndrome); pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma (e.g., rhabdomyosarcoma, various lipogenic tumors, Ewing's sarcoma/primitive neuroectodermal tumors (Ewing/PNETs), and leiomyosarcoma); urothelial carcinoma; basal cell carcinoma; oral squamous cell carcinoma; cholangiocarcinoma; bone cancer; cervical cancer; skin cancer; bone tumors; brain tumors; lung or mesothelium tumor; colonic tumors; ureteral tumors; Richter syndrome (RS); sepsis syndrome; autoimmune diseases (e.g., rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, atopic dermatitis, vitiligo, experimental autoimmune encephalomyelitis, and Arthus reaction); keratoconjunctivitis; inflammatory diseases (e.g., Crohn's disease and ulcerative colitis, pneumonia, osteoarthritis, synovitis, systemic inflammatory response syndrome, airway inflammation, bronchitis, rheumatoid spondylitis, gouty arthritis, eczema, psoriasis, dermatitis, uveitis, conjunctivitis, acute respiratory distress syndrome, bacteremia, endotoxemia, aphthous ulcer, gingivitis, pancreatitis, regional ileitis, atrophic gastritis, peritonitis, pepticulcer, or irritable bowel syndrome); cerebral malaria; infectious diseases (e.g., viral pneumonia, Acquired immunodeficiency syndrome (AIDS), COVID-19 novel coronavirus infection, gram-negative bacteria infection, gram-positive bacteria infection, tuberculosis); septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**32.** Use of the compound of Formula A or salts, enantiomers, stereoisomers, polymorphs or solvates thereof as claimed in any one of claims 15-22 for the manufacture of a Cereblon protein (CRBN)-binding agent.

**33.** Use of the compound of Formula A or salts, enantiomers, polymorphs or solvates thereof as claimed in any one of claims 15-22 for the preparation of the compound or salts, enantiomers, polymorphs or solvates thereof as claimed in claim 1.

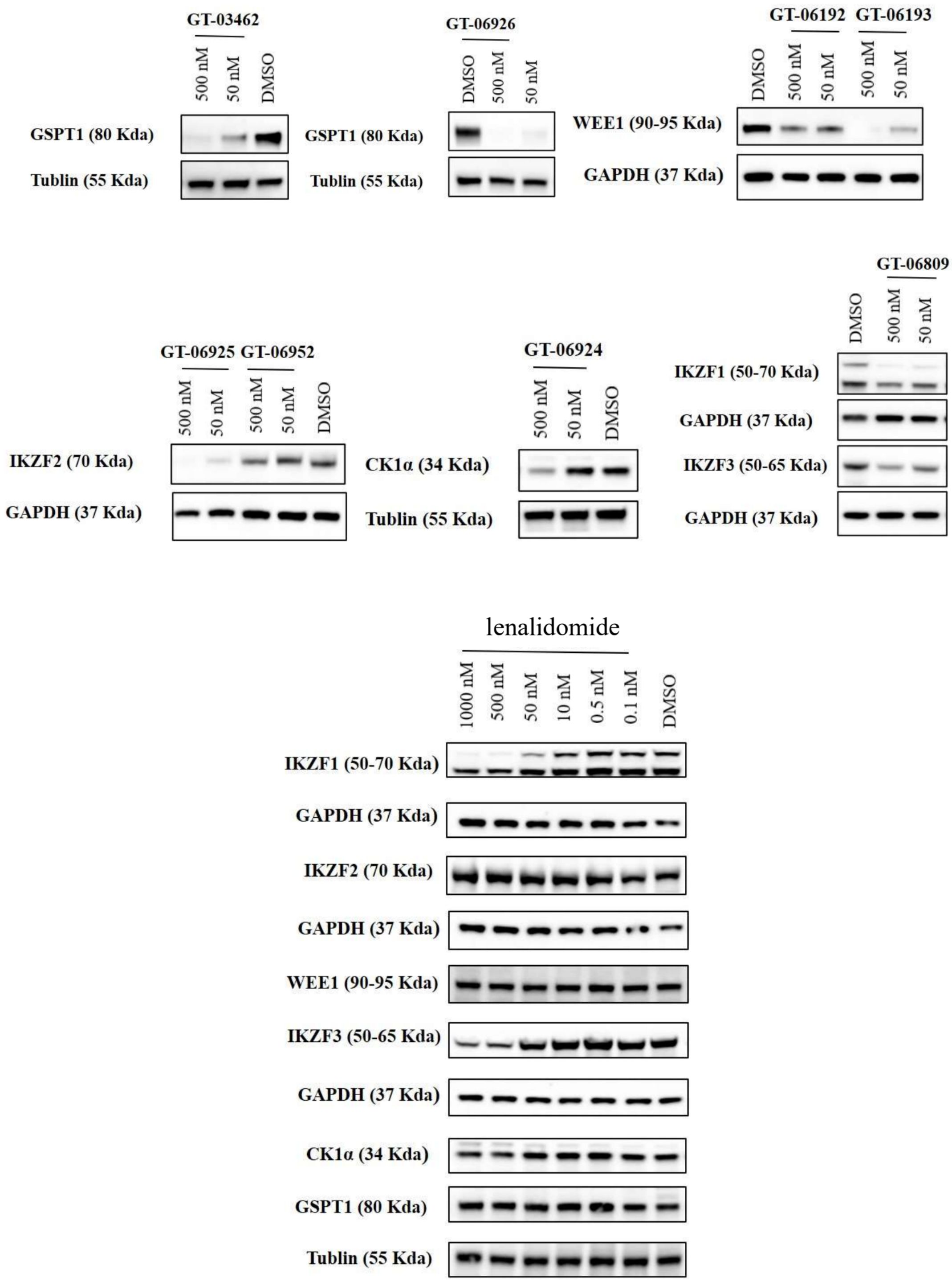
GT-03462
500 nM
50 nM
DMSO
GSPT1 (80 Kda)
Tublin (55 Kda)
GT-06926
DMSO
500 nM
50 nM
GSPT1 (80 Kda)
Tublin (55 Kda)
GT-06192
GT-06193
DMSO
500 nM
50 nM
500 nM
50 nM
WEE1 (90-95 Kda)
GAPDH (37 Kda)
GT-06925
GT-06952
500 nM
50 nM
500 nM
50 nM
DMSO
IKZF2 (70 Kda)
GAPDH (37 Kda)
GT-06924
500 nM
50 nM
DMSO
CK1α (34 Kda)
Tublin (55 Kda)
GT-06809
DMSO
500 nM
50 nM
IKZF1 (50-70 Kda)
GAPDH (37 Kda)
IKZF3 (50-65 Kda)
GAPDH (37 Kda)
lenalidomide
1000 nM
500 nM
50 nM
10 nM
0.5 nM
0.1 nM
DMSO
IKZF1 (50-70 Kda)
GAPDH (37 Kda)
IKZF2 (70 Kda)
GAPDH (37 Kda)
WEE1 (90-95 Kda)
IKZF3 (50-65 Kda)
GAPDH (37 Kda)
CK1α (34 Kda)
GSPT1 (80 Kda)
Tublin (55 Kda)

Figure 1

**INTERNATIONAL SEARCH REPORT**

International application No.
**PCT/CN2023/141787**

**A. CLASSIFICATION OF SUBJECT MATTER**

C07D401/14(2006.01)i; C07D409/14(2006.01)i; C07D417/14(2006.01)i; C07D413/14(2006.01)i; A61K31/40(2006.01)i; A61K 31/4545(2006.01)i; A61K31/5377(2006.01)i; A61K 31/454(2006.01)i; A61P29/00(2006.01)i; A61P35/00(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT; DWPI; SIPOABS; CNKI; REGISTRY(STN); CAPLUS(STN); MARPAT(STN); 标新; GLUETACS; 泛素连接酶; 结合蛋白; 戊二酰亚胺; 异吲哚啉酮; cereblon; CRBN; Glutarimide; Isoindolinone; 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 112745298 A (SHANGHAI INSTITUTE OF MATERIA MEDICA, CHINESE ACADEMY OF SCIENCES) 04 May 2021 (2021-05-04)<br>claim 1, and description, paragraphs [0309]-[0324], and compounds 1-161, etc. | 1-33 |
| X | CN 115397821 A (ARVINAS OPERATIONS, INC.) 25 November 2022 (2022-11-25)<br>claim 1, and description, paragraphs [0995]-[1034], and embodiments 1, 36-37, 44-47 and 154, etc. | 1-33 |
| X | WO 2022087125 A1 (ARVINAS OPERATIONS, INC.) 28 April 2022 (2022-04-28)<br>claim 1, and description, paragraphs [00137]- [00179], Scheme 43, and embodiments 93 and 162, etc. | 1-33 |
| X | CN 113874016 A (FOGHORN THERAPEUTICS INC.) 31 December 2021 (2021-12-31)<br>description, paragraphs [0930]-[0947], and embodiments 47 and 50-52, etc. | 1-12, 14-20, 22-23, 25-33 |
| X | WO 2020160198 A1 (FOGHORN THERAPEUTICS INC.) 06 August 2020 (2020-08-06)<br>claims 306-326, and description, page 226, paragraph 5, and compounds D47-D50, etc. | 1-12, 14-20, 22-33 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 March 2024** | **07 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/141787**

**C. DOCUMENTS CONSIDERED TO BE RELEVANT**

| Category* | Citation of document, with indication, where appropriate, of the relevant passages | Relevant to claim No. |
|---|---|---|
| X | CN 114828959 A (NOVARTIS AG) 29 July 2022 (2022-07-29) claims 74-101, and compounds I50a to I50ew, etc. | 1-12, 14-20, 22-23, 25-33 |
| X | CN 110291087 A (ARVINAS OPERATIONS, INC.) 27 September 2019 (2019-09-27) claims 24-31, synthesis solutions 3-39, and compounds 426 and 436, etc. | 1-12, 14-20, 22-23, 25-33 |
| X | CN 114867727 A (ARVINAS OPERATIONS, INC.) 05 August 2022 (2022-08-05) description, paragraphs [1070]-[1072], and compounds 54, 58 and 386-387, etc. | 1-12, 14-20, 22-23, 25-33 |
| X | CN 110741004 A (ARVINAS OPERATIONS INC. et al.) 31 January 2020 (2020-01-31) description, paragraphs [1494]-[1533], and compounds 184 and 186, etc. | 1-12, 14, 23, 25-31 |

Form PCT/ISA/210 (second sheet) (July 2022)

**INTERNATIONAL SEARCH REPORT**

International application No.
**PCT/CN2023/141787**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29-31**
because they relate to subject matter not required to be searched by this Authority, namely:

The subject matter of claims 29-31 relates to a method for treating diseases of the human or animal body, which falls within the cases set out in PCT Rule 39.1(iv). The present search report is provided on the basis of the use of the compound or a pharmaceutically acceptable salt or pharmaceutical composition thereof in the preparation of a drug for corresponding diseases.

2. ☑ Claims Nos.: **1-12, 15-20**
because they relate to parts of the international application that do not comply with the prescribed requirements to such an extent that no meaningful international search can be carried out, specifically:

The range of the structures defined in claims 1-12 and 15-20 is very broad, and the related compound covers a large number of known compounds in the prior art. Claims 14 and 22-33 substantially refer to any one of said claims. It is difficult for the examiner to carry out an exhaustive search of the prior art with regard to the current scope of the claims. In the present report, a search is performed on the basis of the reasonably summarized scope of most compounds in the embodiments, i.e., in formula I of claim 1, X is CO or CH2, L1 is S or O, X1 is alkylene or does not exist, X2 is a nitrogen-containing heterocyclic ring, X3 is C, N or CO, and Ra1 is -ring-ring; and in formula A of claim 15, X is CO or CH2, L1 is S or O, X1 is alkylene or does not exist, and Xa is a nitrogen-containing heterocyclic ring.

3. ☐ Claims Nos.:
because they are dependent claims and are not drafted in accordance with the second and third sentences of Rule 6.4(a).

## INTERNATIONAL SEARCH REPORT
**Information on patent family members**

International application No. **PCT/CN2023/141787**

| Patent document cited in search report | Publication date (day/month/year) | Patent family member(s) | Publication date (day/month/year) |
|---|---|---|---|
| CN 112745298 A | 04 May 2021 | WO 2021083328 A1 | 06 May 2021 |
| CN 115397821 A | 25 November 2022 | CO 2022006490 A2 | 31 May 2022 |
| | | CA 3154386 A1 | 22 April 2021 |
| | | KR 20220102156 A | 19 July 2022 |
| | | BR 112022007380 A2 | 05 July 2022 |
| | | WO 2021077010 A1 | 22 April 2021 |
| | | IL 292200 A | 01 June 2022 |
| | | AU 2020368542 A1 | 05 May 2022 |
| | | AU 2020368542 B2 | 29 February 2024 |
| | | MX 2022004526 A | 21 July 2022 |
| | | US 2022323457 A1 | 13 October 2022 |
| | | EP 4045496 A1 | 24 August 2022 |
| | | JP 2022552006 A | 14 December 2022 |
| WO 2022087125 A1 | 28 April 2022 | CA 3199074 A1 | 28 April 2022 |
| | | JP 2023547084 A | 09 November 2023 |
| | | IL 302137 A | 01 June 2023 |
| | | AU 2021365850 A1 | 08 June 2023 |
| | | US 2023084249 A1 | 16 March 2023 |
| | | KR 20230111615 A | 25 July 2023 |
| | | EP 4232445 A1 | 30 August 2023 |
| CN 113874016 A | 31 December 2021 | JP 2022523073 A | 21 April 2022 |
| | | US 2023077730 A1 | 16 March 2023 |
| | | WO 2020160192 A1 | 06 August 2020 |
| | | IL 285178 A | 30 September 2021 |
| | | EP 3917526 A1 | 08 December 2021 |
| | | EP 3917526 A4 | 02 November 2022 |
| | | AU 2020214802 A1 | 12 August 2021 |
| WO 2020160198 A1 | 06 August 2020 | US 2024002382 A1 | 04 January 2024 |
| | | US 2024067642 A1 | 29 February 2024 |
| | | US 2023142883 A1 | 11 May 2023 |
| | | US 2023331722 A1 | 19 October 2023 |
| | | EP 3917517 A1 | 08 December 2021 |
| | | EP 3917517 A4 | 25 January 2023 |
| | | US 2023416246 A1 | 28 December 2023 |
| CN 114828959 A | 29 July 2022 | UY 38986 A | 30 July 2021 |
| | | IL 293530 A | 01 August 2022 |
| | | BR 112022011796 A2 | 30 August 2022 |
| | | AU 2020410514 A1 | 30 June 2022 |
| | | CR 20220278 A | 01 July 2022 |
| | | ECSP 22055267 A | 31 August 2022 |
| | | US 2022402904 A1 | 22 December 2022 |
| | | US 11566022 B2 | 31 January 2023 |
| | | KR 20220114065 A | 17 August 2022 |
| | | MX 2022007351 A | 19 July 2022 |
| | | AR 120773 A1 | 16 March 2022 |
| | | DOP 2022000121 A | 15 August 2022 |
| | | TW 202130631 A | 16 August 2021 |
| | | WO 2021124172 A1 | 24 June 2021 |
| | | JP 2023506642 A | 17 February 2023 |

Form PCT/ISA/210 (patent family annex) (July 2022)

**INTERNATIONAL SEARCH REPORT**
**Information on patent family members**

International application No.
**PCT/CN2023/141787**

| Patent document cited in search report | Publication date (day/month/year) | Patent family member(s) | Publication date (day/month/year) |
|---|---|---|---|
| | | EP 4076650 A1 | 26 October 2022 |
| | | EP 4076650 B1 | 28 February 2024 |
| | | PE 20230159 A1 | 01 February 2023 |
| | | CA 3164832 A1 | 24 June 2021 |
| | | CO 2022008243 A2 | 08 July 2022 |
| | | JOP 20220152 A1 | 30 January 2023 |
| CN 110291087 A | 27 September 2019 | IL 266842 A | 31 July 2019 |
| | | IL 266842 B | 01 September 2022 |
| | | EP 3689868 A1 | 05 August 2020 |
| | | EP 3689868 B1 | 27 September 2023 |
| | | RS 64976 B1 | 31 January 2024 |
| | | WO 2018102725 A1 | 07 June 2018 |
| | | EP 3548483 A1 | 09 October 2019 |
| | | EP 3548483 A4 | 10 June 2020 |
| | | CO 2019007091 A2 | 09 October 2019 |
| | | KR 20190082989 A | 10 July 2019 |
| | | BR 112019011200 A2 | 08 October 2019 |
| | | RU 2019120120 A | 28 December 2020 |
| | | RU 2019120120 A3 | 24 March 2021 |
| | | US 2024076281 A1 | 07 March 2024 |
| | | AU 2020201793 A1 | 26 March 2020 |
| | | AU 2020201793 B2 | 27 August 2020 |
| | | SI 3689868 T1 | 29 February 2024 |
| | | FI 3689868 T3 | 18 December 2023 |
| | | CA 3042968 A1 | 07 June 2018 |
| | | CA 3042968 C | 17 October 2023 |
| | | JP 2023021211 A | 10 February 2023 |
| | | US 2021139458 A1 | 13 May 2021 |
| | | US 11104666 B2 | 31 August 2021 |
| | | MX 2022010583 A | 13 December 2022 |
| | | IL 297717 A | 01 December 2022 |
| | | JP 2020504089 A | 06 February 2020 |
| | | JP 6957620 B2 | 02 November 2021 |
| | | LT 3689868 T | 27 December 2023 |
| | | AU 2017366693 A1 | 18 April 2019 |
| | | AU 2017366693 A9 | 02 May 2019 |
| | | AU 2017366693 B2 | 01 April 2021 |
| | | AU 2021204549 A1 | 05 August 2021 |
| | | AU 2021204549 B2 | 06 July 2023 |
| | | IL 272527 A | 31 March 2020 |
| | | IL 272527 B | 29 July 2021 |
| | | US 2022274955 A1 | 01 September 2022 |
| | | US 11597720 B2 | 07 March 2023 |
| | | IL 284424 A | 29 July 2021 |
| | | IL 284424 B | 01 December 2022 |
| | | IL 284424 B2 | 01 April 2023 |
| | | JP 2020100659 A | 02 July 2020 |
| | | JP 6972211 B2 | 24 November 2021 |
| | | AU 2021257895 A1 | 18 November 2021 |
| | | US 2018155322 A1 | 07 June 2018 |

Form PCT/ISA/210 (patent family annex) (July 2022)

**INTERNATIONAL SEARCH REPORT**
**Information on patent family members**

International application No.
**PCT/CN2023/141787**

| Patent document cited in search report | Publication date (day/month/year) | Patent family member(s) | Publication date (day/month/year) |
|---|---|---|---|
| | | US 10647698 B2 | 12 May 2020 |
| | | US 10899742 B1 | 26 January 2021 |
| | | KR 20200020988 A | 26 February 2020 |
| | | KR 102173464 B1 | 04 November 2020 |
| | | KR 102173464 B9 | 23 May 2022 |
| | | MX 2019006402 A | 11 September 2019 |
| | | RU 2020106142 A | 13 April 2020 |
| | | RU 2020106142 A3 | 07 August 2020 |
| | | RU 2750484 C2 | 28 June 2021 |
| | | US 2022388984 A1 | 08 December 2022 |
| | | DK 3689868 T3 | 02 January 2024 |
| | | PT 3689868 T | 02 January 2024 |
| | | JP 2021169479 A | 28 October 2021 |
| | | JP 7160497 B2 | 25 October 2022 |
| | | JP 2022023103 A | 07 February 2022 |
| CN 114867727 A | 05 August 2022 | US 2022315575 A1 | 06 October 2022 |
| | | US 11912699 B2 | 27 February 2024 |
| | | WO 2021011913 A1 | 21 January 2021 |
| | | EP 3999182 A1 | 25 May 2022 |
| | | JP 2022540935 A | 20 September 2022 |
| CN 110741004 A | 31 January 2020 | JP 2023065395 A | 12 May 2023 |
| | | RU 2019121565 A3 | 14 January 2021 |
| | | BR 112019012878 A2 | 26 November 2019 |
| | | KR 20190102019 A | 02 September 2019 |
| | | KR 102564201 B1 | 07 August 2023 |
| | | AU 2021200099 A1 | 18 March 2021 |
| | | AU 2021200099 B2 | 19 January 2023 |
| | | JP 2020504741 A | 13 February 2020 |
| | | WO 2018119448 A1 | 28 June 2018 |
| | | CO 2019007894 A2 | 09 October 2019 |
| | | MX 2019007649 A | 10 September 2019 |
| | | IL 267410 A | 29 August 2019 |
| | | US 2018179183 A1 | 28 June 2018 |
| | | US 10723717 B2 | 28 July 2020 |
| | | AU 2017382436 A1 | 04 July 2019 |
| | | AU 2017382436 B2 | 28 January 2021 |
| | | AU 2017382436 C1 | 27 May 2021 |
| | | IL 294423 A | 01 August 2022 |
| | | IL 294423 B1 | 01 September 2023 |
| | | IL 294423 B2 | 01 January 2024 |
| | | IL 304704 A | 01 September 2023 |
| | | KR 20230119252 A | 16 August 2023 |
| | | CA 3047784 A1 | 28 June 2018 |
| | | EP 3558994 A1 | 30 October 2019 |
| | | EP 3558994 A4 | 12 May 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Pub. Co, 1991